(19) Europäisches Patentamt
European Patent Office
Office européen des brevets

(11) **EP 2 392 662 A2**

(12) **EUROPEAN PATENT APPLICATION**

(43) Date of publication:
**07.12.2011 Bulletin 2011/49**

(21) Application number: **11176674.7**

(22) Date of filing: **23.04.2008**

(51) Int Cl.:
***C12N 15/82*** *(2006.01)* ***A01N 43/56*** *(2006.01)*
***A01N 47/02*** *(2006.01)* ***A01N 47/24*** *(2006.01)*

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **23.04.2007 US 913349 P**

(62) Document number(s) of the earlier application(s) in accordance with Art. 76 EPC:
**08749670.9 / 2 076 602**

(71) Applicant: **BASF SE**
**67056 Ludwigshafen (DE)**

(72) Inventors:
- **Voeste, Dirk**
  **67117 Limburgerhof (DE)**
- **Haden, Egon**
  **67346 Speyer (DE)**
- **McKersie, Bryan**
  **Raleigh, NC 27617 (US)**
- **Wang, Xi-Qing**
  **Chapel Hill, NC 27516 (US)**
- **Hudelson, Timothy**
  **Durham, NC 27712 (US)**

(74) Representative: **Fitzner, Uwe et al**
**Hauser Ring 10**
**40878 Ratingen (DE)**

Remarks:
- The complete document including Reference Tables and the Sequence Listing can be downloaded from the EPO website
- This application was filed on 05-08-2011 as a divisional application to the application mentioned under INID code 62.

(54) **Plant produtivity enhancement by combining chemical agents with transgenic modifications**

(57) The present invention relates to the enhancement of plant productivity by combining chemical agents with transgenic modifications.

Fig. 1

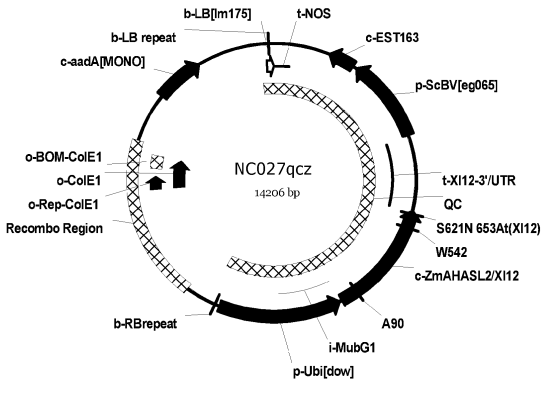


EP 2 392 662 A2

## Description

The present invention relates to the enhancement of plant productivity by combining chemical agents with transgenic modifications.

Chemical amendments and transgenic modifications can both increase plant performance. Chemical agents can be applied e.g. as a seed coating or as a spray on a growing plant. When applied as a coating, chemical amendments may improve seed resistance to biotic and abiotic stresses and stimulate and/or improve germination as well as early germination. Seedlings are thus better able to establish in the greenhouse or the field. Spray applications on growing plants may affect growth by e.g. enhancing plant metabolism, inducing plant vigor, modifying plant canopy and stem architecture, or by improving fruit set.

Many products that enhance plant performance or improve grain quality and quantity are on the market. For instance, fipronil is a broad-spectrum insecticide from the insecticide chemistry class of phenyl pyrazoles. It is highly effective against all major insect pests in crop and non-crop markets. The most important applications are soil and seed treatment in crops and termite control in non-crops. Fipronil has been reported to enhance overall root system and root hair development, increase tiller number and productivity, increase photosynthetic capacity (plant greenness), increase leaf area and plant height, stimulate early flowering and grain maturation leading to significant increases in yield (Fipronil-Worldwide technical Bulletin, BASF). Transgenic plants, such as Round-up ready crops, have exhibited enhanced productivity, primarily due to elimination of competition for water and nutrients with weeds. Round-up ready crops are not susceptible to the herbicide when applied at field rates (24-32 oz/A). Most weeds are susceptible to the herbicide, allowing more of the available nutrients to be sequestered by the crop plants and, therefore, increase yield.

Combinations of chemical, physical, biological, and transgenic approaches are used in integrated pest management (IPM). For example, a transgenic plant with increased resistance is the so called Bt corn (Bt = *Bacillus thuringiensis*), which has been modified to increase levels of certain crystal proteins. Bt is effective at controlling Lepidoptera larvae, most notably the corn borer (http://www.extension.umn.edu/distribution/cropsystems/DC7055.html#ch2). The protein is selective, generally not harming insects of other orders, such as the *Trichogramma minutum* wasp, which is also used to control corn borer (http://ipmworld.umn.edu/chapters/chippen.htm). Crops carrying the Bt gene, therefore, have been considered compatible with biological control programs.

A combination of a transgenic event having activity against corn rootworm and treatment of the seed with certain pesticides provides unexpectedly synergistic advantages to seeds having such treatment, showing unexpectedly superior efficacy in the protection against damage by corn rootworm is disclosed in US 6,593,273.

According to the teaching of the WO1999035913 treating transgenic useful plants which carry one or more genes expressing a pesticidal active ingredient, or which are tolerant against herbicides or which are resistant against the attack of fungi, with a nitroimino-or nitroguanidino-compound for controlling pests, has a synergistic effect on the pests to be controlled.

A method for increasing the vigor and the yield of an agronomic plant is known from US 20030060371, US 20040023081 or US 20030114308. The method includes treatment of a plant with herbicides or other pesticides, whereby the plant is a transgenic plant having a transgenic event that confers resistance to the herbicide or pesticide which is employed.

The advantages of applying chemicals and/or chemical compositions comprising at least one active ingredient on the transgenic plants themselves to improve performance, however, are unknown. There is no known product system, which combines agrochemical application with transgenic modifications to enhance plant productivity and grain quality.

Object of the present invention is a new process for increasing plant health and/or controlling pests.

A further object is a method which integrates chemicals and transgenic material to improve plant performance.

The object is achieved according to the invention by providing the present method. Summarized, the present invention relates to a method for increasing plant health and/or controlling pests in plants with at least one transgenic modification related to yield increase as compared to a corresponding wild-type plant comprising treating the location where the plant with at least one transgenic modification is growing or is expected to grow and/or the transgenic plant with at least one transgenic modification or propagation material of the plant with at least one transgenic modification with an effective amount of a chemical composition comprising at least one active ingredient.

In one embodiment of the invention the method of the invention comprises

a) treating the location where the plant with at least one transgenic modification is growing or is expected to grow and/or treating the plant with at least one transgenic modification or propagation material of the plant with at least one transgenic modification with an effective amount of a chemical composition comprising at least one active ingredient and

b) cultivating a transgenic plant with increased plant health and increased productivity as compared to a corresponding non-treated plant and growing said plant under conditions which permit the development of the plant.

In one embodiment the invention is related to a method for increasing plant productivity which comprises an increase in plant health and/or pest control.

Surprisingly, it has now been found that treating that plant with at least one transgenic modification related to yield increase with an effective amount of a chemical composition comprising at least one active ingredient results in a synergistic effect.

Synergistic effect in the present context means that

a) the use of a pesticide in combination with a transgenic modification exceeds the additive effect, to be expected on the pests to be controlled and thus extends the range of action of the active ingredient and of the active principle expressed by the transgenic plant and/or

b) results in an increase in plant health and increased yield.

The term "synergistic", however, is to be understood in this connection as synergistic pesticidal activity and/or synergistic plant health effects.

Synergistic pesticidal activity means extension of the pesticidal spectrum of action to other pests, for example to resistant strains; and/orreduction in the application rate of the pesticides, and/or sufficient control of the pests with the aid of the pesticides even at an application rate of the pesticides alone and the transgenic plant alone are entirely ineffective.

In one embodiment of the invention at least one transgenic modification of said plant does not confer resistance to the active ingredient which is employed.

According to the present invention, "increasing the plant productivity" means that certain plant traits are increased or improved by a measurable or noticeable amount over the same factor of the control, e.g. the plant produced under the same conditions, but without the application of the present invention, such as selected from the group consisting of: delay of senescence, root growth, longer panicles, increased or improved plant stand, the plant weight, plant height, emergence, improved visual appearance, improved protein content, more developed root system, tillering increase, increase in plant height, bigger leaf blade, less dead basal leaves, stronger tillers, greener leaf color, pigment content, photosynthetic activity, less fertilizers needed, less seeds needed, more productive tillers, earlier flowering, early grain maturity, less plant verse (lodging), increased shoot growth, early and improved germination, improved vitality of the plant, improved quality of the plant, improved quality of the fruits or vegetables (or other products produced by the plant), improved self defence mechanism of the plant such as induced tolerance against fungi, bacteria, viruses and/or insects.

Synergistic pest control means as stated above that the use of a pesticide in combination with a transgenic modification exceeds the additive effect, to be expected on the pests to be controlled and thus extends the range of action of the pesticides and of the activity caused by the transgenic modification expressed by the transgenic plant and may be accompanied by effects such as improved self defence mechanism of the plant such as induced tolerance against fungi, bacteria, viruses and/or insects, but is not limited to improved self defence mechanism of the plant such as induced tolerance against fungi, bacteria, viruses and/or insects.

Pests mean not only insects, nematodes or arachnids but also phytophatogenic fungi.

In one embodiment of the invention, increased plant productivity means plants with improvements in fresh weight (FW), dry weight, and/or plant volume.

In one embodiment of the invention, increased plant productivity means e.g. plants with increased internode length, representing better plant growth and/or green vs. yellow plant material, representing greater photosynthetic capacity than the control.

In one embodiment of the invention, increased plant productivity means plants with increased greenness, internode length, leaf angle, implying that leaves were laying flatter and thus received more photosynthetic radiation, and/or increased plant area, representing more leaf surface area for photosynthesis than the control.

In one embodiment of the invention, increased plant productivity means plants with increased the yield and/or improved vigor.

In one embodiment of the invention, increased plant productivity means plants with an increase in any of the aforementioned traits or any combination of two or more of the aforementioned traits.

In one embodiment of the invention, an increased yield means an increase in a trait selected from the group consisting of biomass production, grain yield, starch content, oil content or protein content.

In one embodiment of the invention, an increased yield is obtained based upon an increase in plant productivity, e.g. increased plant health and/or pest control.

According to the present invention, "increased plant health" means that certain plant characteristics are increased or improved as compared to the wild-type plant such as selected from the group consisting of: delay of senescence, root growth, longer panicles, plant weight, plant height, emergence, improved visual appearance, protein content, oil content, starch content, more developed root system (improved root growth), reduced ethylene (reduced production

and/or inhibition of reception), tillering increase, increase in plant height, bigger leaf blade, less dead basal leaves, stronger tillers, greener leaf color, pigment content, photosynthetic activity, less input needed (such as fertilizers or water), less seeds needed, enhanced plant vigor, more productive tillers, earlier flowering, early grain maturity, less plant verse (lodging), increased shoot growth, increased plant stand and early and better germination, improved vitality of the plant, improved quality of the plant, improved quality of the fruits or vegetables (or other products produced by the plant), improved self-defense mechanism of the plant such as induced and improved tolerance against stress factors such as heat stress, cold stress, drought stress, UV stress and/or salt stress. Advantageous properties, obtained especially from treated seeds, are selected from the group cocsisting of improved germination and field establishment, better vigor, more homogen field establishment. Advantageous properties, obtained especially from foliar and/or in-furrow application are e.g. improved plant growth and plant development, better growth, more tillers, greener leaves, larger leaves, more biomass, better roots, improved abiotic stress tolerance of the plants, more grain yield, more biomass harvested, improved quality of the harvest (content of fatty acids, metabolites, oil etc.), more marketable products (e.g. improved size), improved process (e.g. longer shelf-life, better extraction of compounds), improved quality of seeds (for being seeded in the following seasons for seed production); or any other advantages familiar to a person skilled in the art.

In one embodiment of the invention, increased plant health means plants with an increase in any of the aforementioned traits or any combination of two or more of the aforementioned traits.

In one embodiment the invention provides a method for controlling pests and/or increasing plant health in plants with at least one transgenic modification related to yield increase comprising the application of a composition comprising at least one pesticide to the pests, or to the plant with at least one transgenic modification or propagation material of the plant with at least one transgenic modification.

According to the present invention, "pest control" or "controlling pests" means in one embodiment efficiently combating one or more pests selected from the group consisting of: insects from the order of the lepidopterans (*Lepidoptera*), e.g. *Agrotis ypsilon, Agrotis segetum, Alabama argillacea, Anticarsia gemmatalis, Argyresthia conjugella, Autographa gamma, Bupalus piniarius, Cacoecia murinana, Capua reticulana, Cheimatobia brumata, Choristoneura fumiferana, Choristoneura occidentalis, Cirphis unipuncta, Cydia pomonella, Dendrolimus pini, Diaphania nitidalis, Diatraea grandiosella, Earias insulana, Elasmopalpus lignosellus, Eupoecilia ambiguella, Evetria bouliana, Feltia subterranea, Galleria mellonella, Grapholitha funebrana, Grapholitha molesta, Heliothis armigera, Heliothis virescens, Heliothis zea, Hellula undalis, Hibernia defoliaria, Hyphantria cunea, Hyponomeuta malinellus, Keiferia lycopersicella, Lambdina fiscellaria, Laphygma exigua, Leucoptera coffeella, Leucoptera scitella, Lithocolletis blancardella, Lobesia botrana, Loxostege sticticalis, Lymantria dispar, Lymantria monacha, Lyonetia clerkella, Malacosoma neustria, Mamestra brassicae, Orgyia pseudotsugata, Ostrinia nubilalis, Panolis flammea, Pectinophora gossypiella, Peridroma saucia, Phalera bucephala, Phthorimaea operculella, Phyllocnistis citrella, Pieris brassicae, Plathypena scabra, Plutella xylostella, Pseudoplusia includens, Rhyacionia frustrana, Scrobipalpula absoluta, Sitotroga cerealella, Sparganothis pilleriana, Spodoptera frugiperda, Spodoptera littoralis, Spodoptera litura, Thaumatopoea pityocampa, Tortrix viridana, Trichoplusia ni* and *Zeiraphera canadensis,*

beetles (*Coleoptera*), e.g. *Agrilus sinuatus, Agriotes lineatus, Agriotes obscurus, Amphimallus solstitialis, Anisandrus dispar, Anthonomus grandis, Anthonomus pomorum, Aphthona euphoridae, Athous haemorrhoidalis, Atomaria linearis, Blastophagus piniperda, Blitophaga undata, Bruchus rufimanus, Bruchus pisorum, Bruchus lentis, Byctiscus betulae, Cassida nebulosa, Cerotoma trifurcata, Cetonia aurata, Ceuthorrhynchus assimilis, Ceuthorrhynchus napi, Chaetocnema tibialis, Conoderus vespertinus, Crioceris asparagi, Ctenicera ssp., Diabrotica longicornis, Diabrotica semipunctata, Diabrotica 12-punctata Diabrotica speciosa, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Oulema oryzae, Otiorrhynchus sulcatus, Otiorrhynchus ovatus, Phaedon cochleariae, Phyllobius pyri, Phyllotreta chrysocephala, Phyllophaga sp., Phyllopertha horticola, Phyllotreta nemorum, Phyllotreta striolata, Popillia japonica, Sitona lineatus* and *Sitophilus granaria,*

flies, mosquitoes (Diptera), e.g. *Aedes aegypti, Aedes albopictus, Aedes vexans, Anastrepha ludens, Anopheles maculipennis, Anopheles crucians, Anopheles albimanus, Anopheles gam-biae, Anopheles freeborni, Anopheles leucosphyrus, Anopheles minimus, Anopheles quadri-maculatus, Calliphora vicina, Ceratitis capitata, Chrysomya bezziana, Chrysomya hominivorax, Chrysomya macellaria, Chrysops discalis, Chrysops silacea, Chrysops atlanticus, Cochliomyia hominivorax, Contarinia sorghicola Cordylobia anthropophaga, Culicoides furens, Culex pipiens, Culex nigripalpus, Culex quinquefasciatus, Culex tarsalis, Culiseta inornata, Culiseta melanura, Dacus cucurbitae, Dacus oleae, Dasineura brassicae, Delia antique, Delia coarc-tata, Delia platura, Delia radicum, Dermatobia hominis, Fannia canicularis, Geomyza Tripunc-tata, Gasterophilus intestinalis, Glossina morsitans, Glossina palpalis, Glossina fuscipes, Glossina tachinoides, Haematobia irritans, Haplodiplosis equestris, Hippelates spp., Hylemyia platura, Hypoderma lineata, Leptoconops torrens, Liriomyza sativae, Liriomyza trifolii, Lucilia caprina, Lucilia cuprina, Lucilia sericata, Lycoria pectoralis, Mansonia titillanus, Mayetiola destructor, Musca autumnalis, Musca domestica, Muscina stabulans, Oestrus ovis, Opomyza florum, Oscinella frit, Pegomya hysocyami, Phorbia antiqua, Phorbia brassicae, Phorbia coarc-tata,*

*Phlebotomus argentipes, Psorophora columbiae, Psila* rosae, *Psorophora discolor, Prosimulium mixtum, Rhagoletis cerasi, Rhagoletis pomonella, Sarcophaga haemorrhoidalis, Sarcophaga spp., Simulium vittatum, Stomoxys calcitrans, Tabanus bovinus, Tabanus atratus, Tabanus lineola,* and *Tabanus similis, Tipula oleracea,* and *Tipula paludosa*

thrips (*Thysanoptera*), e.g. *Dichromothrips corbetti, Dichromothrips ssp., Frankliniella fusca, Frankliniella occidentalis, Frankliniella tritici, Scirtothrips citri, Thrips oryzae, Thrips palmi* and *Thrips tabaci,*

termites (Isoptera), e.g. *Calotermes flavicollis, Leucotermes flavipes, Heterotermes aureus, Reticulitermes flavipes, Reticulitermes virginicus, Reticulitermes lucifugus, Reticulitermes santonensis, Reticulitermes grassei, Termes natalensis,* and *Coptotermes formosanus,* cockroaches (Blattaria - Blattodea), e.g. *Blattella germanica, Blattella asahinae, Periplaneta americana, Periplaneta japonica, Periplaneta brunnea, Periplaneta fuligginosa, Periplaneta australasiae,* and *Blatta orientalis,*

bugs, aphids, leafhoppers, whiteflies, scale insects, cicadas (*Hemiptera*), e.g. *Acrosternum hilare, Blissus leucopterus, Cyrtopeltis notatus, Dysdercus cingulatus, Dysdercus intermedius, Eurygaster integriceps, Euschistus impictiventris, Leptoglossus phyllopus, Lygus lineolaris, Lygus pratensis, Nezara viridula, Piesma quadrata, Solubea insularis , Thyanta perditor, Acyrthosiphon onobrychis, Adelges laricis, Aphidula nasturtii, Aphis fabae, Aphis forbesi, Aphis pomi, Aphis gossypii, Aphis grossulariae, Aphis schneideri, Aphis spiraecola, Aphis sambuci, Acyrthosiphon pisum, Aulacorthum solani, Bemisia argentifolii, Brachycaudus cardui, Brachycaudus helichrysi, Brachycaudus persicae, Brachycaudus prunicola, Brevicoryne brassicae, Capitophorus horni, Cerosipha gossypii, Chaetosiphon fragaefolii, Cryptomyzus ribis, Dreyfusia nordmannianae, Dreyfusia piceae, Dysaphis radicola, Dysaulacorthum pseudosolani, Dysaphis plantaginea, Dysaphis pyri, Empoasca fabae, Hyalopterus pruni, Hyperomyzus lactucae, Macrosiphum avenae, Macrosiphum euphorbiae, Macrosiphon rosae*, *Megoura viciae, Melanaphis pyrarius, Metopolophium dirhodum, Myzus persicae, Myzus ascalonicus, Myzus cerasi, Myzus varians, Nasonovia ribis-nigri, Nilaparvata lugens, Pemphigus bursarius, Perkinsiella sacchari-cida, Phorodon humuli, Psylla mali, Psylla piri, Rhopalomyzus ascalonicus, Rhopalosiphum maidis, Rhopalosiphum padi, Rhopalosiphum insertum, Sappaphis mala, Sappaphis mali, Schizaphis graminum, Schizoneura lanuginosa, Sitobion avenae, Trialeurodes vaporariorum, Toxoptera aurantiiand, Viteus vitifolii, Cimex lectularius, Cimex hemipterus, Reduvius senilis, Triatoma* spp., and *Arilus critatus.*

ants, bees, wasps, sawflies (Hymenoptera), e.g. *Athalia rosae, Atta cephalotes, Atta capiguara, Atta cephalotes, Atta laevigata, Atta robusta, Atta sexdens, Atta texana, Cremato-gaster spp., Hoplocampa minuta, Hoplocampa testudinea, Lasius niger, Monomorium pharaonis, Solenopsis geminata, Solenopsis invicta, Solenopsis richteri, Solenopsis xyloni, Pogonomyrmex barbatus, Pogonomyrmex californicus, Pheidole megacephala, Dasymutilla occidentalis, Bombus spp., Vespula squamosa*, *Paravespula vulgaris, Paravespula pennsyl-vanica, Paravespula germanica, Dolichovespula maculata, Vespa crabro, Polistes rubiginosa, Camponotus floridanus,* and *Linepithema humile,*

crickets, grasshoppers, locusts (Orthoptera), e.g. *Acheta domestica, Gryllotalpa gryllotalpa, Locusta migratoria, Melanoplus bivittatus, Melanoplus femurrubrum, Melanoplus mexicanus, Melanoplus sanguinipes, Melanoplus spretus, Nomadacris septemfasciata, Schistocerca americana, Schistocerca gregaria, Dociostaurus maroccanus, Tachycines asynamorus, Oedaleus senegalensis, Zonozerus variegatus, Hieroglyphus daganensis, Kraussaria angulifera, Calliptamus italicus, Chortoicetes terminifera,* and *Locustana pardalina,*

Arachnoidea, such as arachnids (Acarina), e.g. of the families Argasidae, Ixodidae and Sar-coptidae, such as *Amblyomma americanum, Amblyomma variegatum, Ambryomma macula-tum, Argas persicus, Boophilus annulatus, Boophilus decoloratus, Boophilus microplus, Dermacentor silvarum, Dermacentor andersoni, Dermacentor variabilis, Hyalomma truncatum, Ixodes ricinus, Ixodes rubicundus, Ixodes scapularis, Ixodes holocyclus, Ixodes pacificus, Ornithodorus moubata, Ornithodorus hermsi, Ornithodorus turicata, Ornithonyssus bacoti, Otobius megnini, Dermanyssus gallinae, Psoroptes ovis, Rhipicephalus sanguineus, Rhipicephalus appendiculatus, Rhipicephalus evertsi, Sarcoptes scabiei,* and Eriophyidae spp. such as *Acu-lus schlechtendali, Phyllocoptrata oleivora* and *Eriophyes sheldoni;* Tarsonemidae spp. such as *Phytonemus pallidus and Polyphagotarsonemus latus*; Tenuipalpidae spp. such as *Brevi-palpus phoenicis;* Tetranychidae spp. such as *Tetranychus cinnabarinus, Tetranychus kan-zawai, Tetranychus pacificus, Tetranychus telarius* and *Tetranychus* urticae, Panonychus ulmi, Panonychus citri, and *Oligonychus pratensis;* Araneida, e.g. *Latrodectus mactans,* and *Loxosceles reclusa,*

fleas (Siphonaptera), e.g. *Ctenocephalides felis, Ctenocephalides canis, Xenopsylla cheopis, Pulex irritans, Tunga penetrans,* and *Nosopsyllus fasciatus,*

silverfish, firebrat (Thysanura), e.g. *Lepisma saccharina* and *Thermobia domestica,*

centipedes (Chilopoda), e.g. *Scutigera coleoptrata,*

millipedes (Diplopoda), e.g. *Narceus spp.,*

Earwigs (Dermaptera), e.g. *forficula auricularia,*

lice (Phthiraptera), e.g. Pediculus humanus capitis, Pediculus humanus corporis, Pthirus pubis, Haematopinus eurysternus, Haematopinus suis, Linognathus vituli, Bovicola bovis, Menopon gallinae, Menacanthus stramineus and Solenopotes capillatus.

According to the present invention, "pest control" or "controlling pests" means in one embodiment efficiently combating the pest selected from the group consisting of: phytopathogenic fungi, including soil-borne fungi, which derive

especially from the classes of the Plasmodiophoromycetes, Peronosporomycetes (syn. Oomycetes), Chytridiomycetes, Zygomycetes, Ascomycetes, Basidiomycetes and Deuteromycetes (syn. Fungi imperfecti).

According to the present invention, "pest control" or "controlling pests" means in one embodiment efficiently controlling the plant diseases selected from the group consisting of: *Albugo* spp. (white rust) on ornamentals, vegetables (e. g. *A. candida*) and sunflowers (e. g. *A. tragopogonis*); *Alternaria* spp. (Alternaria leaf spot) on vegetables, rape (*A. brassicola* or *brassicae*), sugar beets (*A. tenuis*), fruits, rice, soybeans, potatoes (e. g. A. *solani* or *A. alternata*), tomatoes (e. g. *A. solani* or *A. alternata*) and wheat; *Aphanomyces* spp. on sugar beets and vegetables; *Ascochyta* spp. on cereals and vegetables, e. g. *A. tritici* (anthracnose) on wheat and *A. hordei* on barley; *Bipolaris* and *Drechslera* spp. (teleomorph: *Cochliobolus* spp.) on corn (e. g. *D. maydis*), cereals (e. g. *B. sorokiniana*: spot blotch), rice (e. g. *B. oryzae*) and turfs; *Blumeria* (formerly *Erysiphe*) *graminis* (powdery mildew) on cereals (e. g. on wheat or barley); *Botrytis cinerea* (teleomorph: *Botryotinia fuckeliana:* grey mold) on fruits and berries (e. g. strawberries), vegetables (e. g. lettuce, carrots, celery and cabbages), rape, flowers, vines, forestry plants and wheat; *Bremia lactucae* (downy mildew) on lettuce; *Ceratocystis* (syn. *Ophiostoma*) spp. (rot or wilt) on broad-leaved trees and evergreens, e. g. *C. ulmi* (Dutch elm disease) on elms; *Cercospora* spp. (Cercospora leaf spots) on corn, rice, sugar beets (e. g. *C. beticola*), sugar cane, vegetables, coffee, soybeans (e. g. *C. sojina* or *C. kikuchii*) and rice; *Cladosporium* spp. on tomatoes (e. g. *C. fulvum*: leaf mold) and cereals, e. g. *C. herbarum* (black ear) on wheat; *Claviceps purpurea* (ergot) on cereals; *Cochliobolus* (anamorph: *Helminthosporium* of *Bipolaris*) spp. (leaf spots) on corn (*C. carbonum*), cereals (e. g. *C. sativus*, anamorph: *B. sorokiniana*) and rice (e. g. *C. miyabeanus*, anamorph: *H. oryzae*); *Colleto-trichum* (teleomorph: *Glomerella*) spp. (anthracnose) on cotton (e. g. *C. gossypii*), corn (e. g. *C. graminicola*), soft fruits, potatoes (e. g. *C. coccodes*: black dot), beans (e. g. *C. lindemu-thianum*) and soybeans (e. g. *C. truncatum* or *C. gloeosporioides*); *Corticium* spp., e. g. C. sa-*sakii* (sheath blight) on rice; *Corynespora cassiicola* (leaf spots) on soybeans and ornamentals; *Cycloconium* spp., e. g. *C. oleaginum* on olive trees; *Cylindrocarpon* spp. (e. g. fruit tree canker or young vine decline, teleomorph: *Nectria* or *Neonectria* spp.) on fruit trees, vines (e. g. *C. liriodendri*, teleomorph: *Neonectria liriodendri*: Black Foot Disease) and ornamentals; *Demato-phora* (teleomorph: *Rosellinia*) necatrix (root and stem rot) on soybeans; *Diaporthe* spp., e. g. *D. phaseolorum* (damping off) on soybeans; *Drechslera* (syn. *Helminthosporium*, teleomorph: *Pyrenophora*) spp. on corn, cereals, such as barley (e. g. *D. teres*, net blotch) and wheat (e. g. *D. tritici-repentis*: tan spot), rice and turf; Esca (dieback, apoplexy) on vines, caused by *Formitiporia* (syn. *Phellinus*) *punctata*, *F. mediterranea*, *Phaeomoniella chlamydospora* (earlier *Phaeoacremonium chlamydosporum*), *Phaeoacremonium aleophilum* and/or *Botryosphaeria obtusa; Elsinoe* spp. on pome fruits (*E. pyri*), soft fruits (*E. veneta*: anthracnose) and vines (*E. ampelina*: anthracnose); *Entyloma oryzae* (leaf smut) on rice; *Epicoccum* spp. (black mold) on wheat; *Erysiphe* spp. (powdery mildew) on sugar beets (*E. betae*), vegetables (e. g. *E. pisi*), such as cucurbits (e. g. *E. cichoracearum*), cabbages, rape (e. g. *E. cruciferarum*); *Eutypa lata* (Eutypa canker or dieback, anamorph: *Cytosporina lata*, syn. *Libertella blepharis*) on fruit trees, vines and ornamental woods; *Exserohilum* (syn. *Helminthosporium*) spp. on corn (e. g. *E. turcicum*); *Fusarium* (teleomorph: *Gibberella*) spp. (wilt, root or stem rot) on various plants, such as *F. graminearum* or *F. culmorum* (root rot, scab or head blight) on cereals (e. g. wheat or barley), *F. oxysporum* on tomatoes, *F. solani* on soybeans and *F. verticillioides* on corn; *Gaeumannomyces graminis* (take-all) on cereals (e. g. wheat or barley) and corn; *Gibberella* spp. on cereals (e. g. *G. zeae*) and rice (e. g. *G. fujikuroi*: Bakanae disease); *Glomerella cingu-lata* on vines, pome fruits and other plants and *G. gossypii* on cotton; Grainstaining complex on rice; *Guignardia bidwellii* (black rot) on vines; *Gymnosporangium* spp. on rosaceous plants and junipers, e. g. *G. sabinae* (rust) on pears; *Helminthosporium* spp. (syn. *Drechslera*, teleomorph: *Cochliobolus*) on corn, cereals and rice; *Hemileia* spp., e. g. *H. vastatrix* (coffee leaf rust) on coffee; *Isariopsis clavispora* (syn. *Cladosporium vitis*) on vines; *Macrophomina phase-olina* (syn. *phaseoli*) (root and stem rot) on soybeans and cotton; *Microdochium* (syn. Fusa*rium*) *nivale* (pink snow mold) on cereals (e. g. wheat or barley); *Microsphaera diffusa* (powdery mildew) on soybeans; *Monilinia* spp., e. g. *M. laxa, M. fructicola* and *M. fructigena* (bloom and twig blight, brown rot) on stone fruits and other rosaceous plants; *Mycosphaerella* spp. on cereals, bananas, soft fruits and ground nuts, such as e. g. *M. graminicola* (anamorph: Septo*ria tritici*, Septoria blotch) on wheat or *M. fijiensis* (black Sigatoka disease) on bananas; Per-onospora spp. (downy mildew) on cabbage (e. g. *P. brassicae*), rape (e. g. *P. parasitica*), onions (e. g. *P. destructor*), tobacco (*P. tabacina*) and soybeans (e. g. *P. manshurica*); *Phakop*-sora *pachyrhizi* and *P. meibomiae* (soybean rust) on soybeans; *Phialophora* spp. e. g. on vines (e. g. *P. tracheiphila* and *P. tetraspora*) and soybeans (e. g. *P. gregata*: stem rot); *Phoma lin*-gam (root and stem rot) on rape and cabbage and *P. betae* (root rot, leaf spot and damping-off) on sugar beets; *Phomopsis* spp. on sunflowers, vines (e. g. *P. viticola*: can and leaf spot) and soybeans (e. g. stem rot: *P. phaseoli*, teleomorph: *Diaporthe phaseolorum*); *Physoderma maydis* (brown spots) on corn; *Phytophthora* spp. (wilt, root, leaf, fruit and stem root) on various plants, such as paprika and cucurbits (e. g. *P. capsici*), soybeans (e. g. *P. megasperma,* syn. *P. sojae*), potatoes and tomatoes (e. g. *P. infestans*: late blight) and broad-leaved trees (e. g. *P. ramorum*: sudden oak death); *Plasmodiophora brassicae* (club root) on cabbage, rape, radish and other plants; *Plasmopara* spp., e. g. *P. viticola* (grapevine downy mildew) on vines and *P. halstedii* on sunflowers; *Podosphaera* spp. (powdery mildew) on rosaceous plants, hop, pome and soft fruits, e. g. *P. leucotricha* on apples; *Polymyxa* spp., e. g. on cereals, such as barley and wheat (*P. graminis*) and sugar beets (*P. betae*) and thereby transmitted viral diseases; *Pseudocercosporella herpotrichoides* (eyespot, teleomorph: *Tapesia*

*yallun-dae*) on cereals, e. g. wheat or barley; *Pseudoperonospora* (downy mildew) on various plants, e. g. *P. cubensis* on cucurbits or *P. humili* on hop; *Pseudopezicula tracheiphila* (red fire disease or ‚rotbrenner', anamorph: *Phialophora*) on vines; *Puccinia* spp. (rusts) on various plants, e. g. *P. triticina* (brown or leaf rust), *P. striiformis* (stripe or yellow rust), *P. hordei* (dwarf rust), *P. graminis* (stem or black rust) or *P. recondita* (brown or leaf rust) on cereals, such as e. g. wheat, barley or rye, and asparagus (e. g. *P. asparagi*); *Pyrenophora* (anamorph: *Drechslera*) *tritici-repentis* (tan spot) on wheat or *P. teres* (net blotch) on barley; *Pyricularia* spp., e. g. *P. oryzae* (teleomorph: *Magnaporthe grisea*, rice blast) on rice and *P. grisea* on turf and cereals; *Pythium* spp. (damping-off) on turf, rice, corn, wheat, cotton, rape, sunflowers, soybeans, sugar beets, vegetables and various other plants (e. g. *P. ultimum* or *P. aphanidermatum*); *Ramularia* spp., e. g. *R. collo-cygni* (Ramularia leaf spots, Physiological leaf spots) on barley and *R. beticola* on sugar beets; *Rhizoctonia* spp. on cotton, rice, potatoes, turf, corn, rape, potatoes, sugar beets, vegetables and various other plants, e. g. *R. solani* (root and stem rot) on soybeans, *R. solani* (sheath blight) on rice or *R. cerealis* (Rhizoctonia spring blight) on wheat or barley; *Rhizopus stolonifer* (black mold, soft rot) on strawberries, carrots, cabbage, vines and tomatoes; *Rhynchosporium secalis* (scald) on barley, rye and triticale; *Sarocladium oryzae* and *S. attenuatum* (sheath rot) on rice; *Sclerotinia* spp. (stem rot or white mold) on vegetables and field crops, such as rape, sunflowers (e. g. *S. sclerotiorum*) and soybeans (e. g. *S. rolfsii* or *S. sclerotiorum*); *Septoria* spp. on various plants, e. g. *S. glycines* (brown spot) on soybeans, *S. tritici* (Septoria blotch) on wheat and *S.* (syn. *Stagonospora*) nodorum (Stagonospora blotch) on cereals; *Uncinula* (syn. *Erysiphe*) *necator* (powdery mildew, anamorph: *Oidium tuckeri*) on vines; *Setospaeria* spp. (leaf blight) on corn (e. g. *S. turcicum*, syn. *Helminthosporium turcicum*) and turf; *Sphacelotheca* spp. (smut) on corn, (e. g. *S. reiliana:* head smut), sorghum und sugar cane; *Sphaerotheca fuliginea* (powdery mildew) on cucurbits; *Spongospora subterranea* (powdery scab) on potatoes and thereby transmitted viral diseases; *Stagonospora* spp. on cereals, e. g. *S. nodorum* (Stagonospora blotch, teleomorph: *Lep-tosphaeria* [syn. *Phaeosphaeria*] *nodorum*) on wheat; *Synchytrium endobioticum* on potatoes (potato wart disease); *Taphrina* spp., e. g. *T. deformans* (leaf curl disease) on peaches and *T. pruni* (plum pocket) on plums; *Thielaviopsis* spp. (black root rot) on tobacco, pome fruits, vegetables, soybeans and cotton, e. g. *T. basicola* (syn. *Chalara elegans*); *Tilletia* spp. (common bunt or stinking smut) on cereals, such as e. g. *T. tritici* (syn. *T. caries*, wheat bunt) and *T. con-troversa* (dwarf bunt) on wheat; *Typhula incarnata* (grey snow mold) on barley or wheat; *Urocystis* spp., e. g. *U. occulta* (stem smut) on rye; *Uromyces* spp. (rust) on vegetables, such as beans (e. g. *U. appendiculatus,* syn. *U. phaseoli*) and sugar beets (e. g. *U. betae*); *Ustilago* spp. (loose smut) on cereals (e. g. *U. nuda* and *U. avaenae*), corn (e. g. *U. maydis*: corn smut) and sugar cane; *Venturia* spp. (scab) on apples (e. g. *V. inaequalis*) and pears; and *Verticillium* spp. (wilt) on various plants, such as fruits and ornamentals, vines, soft fruits, vegetables and field crops, e. g. *V. dahliae* on strawberries, rape, potatoes and tomatoes.

Further harmful fungi are selected from the group consisting of : Ascomycetes such as *Ophiostoma* spp., *Ceratocystis* spp., *Aureobasidium pullulans*, *Sclerophoma* spp., *Chaetomium* spp., *Humicola* spp., *Petriella* spp., *Trichurus* spp.; Basidiomycetes such as *Coniophora* spp., *Corio-lus* spp., *Gloeophyllum* spp., *Lentinus* spp., *Pleurotus* spp., *Poria* spp., *Serpula* spp. and *Ty-romyces* spp., Deuteromycetes such as *Aspergillus* spp., *Cladosporium* spp., *Penicillium* spp., *Trichorma* spp., *Alternaria* spp., *Paecilomyces* spp. and Zygomycetes such as *Mucorspp.*, and in addition in the protection of stored products the following yeast fungi are worthy of note: *Candida* spp. and *Saccharomyces cerevisae.*

According to the present invention, "pesticide" means a composition comprising in free form or in agrochemically useful salt form as active ingredient and at least one auxiliary.

In one embodiment the invention relates to compositions and to the use of these for controlling harmful plants.

According to the invention, the term "transgenic plant" means a plant with transgenic modification.

A "plant with transgenic modification" means a plant whose genetic material has been altered using techniques in genetics generally known as recombinant DNA technology.

The plant has at least one transgenic modification as compared with the wild-type plant, but it may have further transgenic modifications, so in total 2, 3, 4, 5, 6, 7, 8, 9 or even more.

The term "wild-type" means a plant without the aforementioned modification.

The terms "wild type", "control" or "reference" are exchangeable and can be a cell or a part of a plant such as an organelle or tissue, or a plant, which was not modified or treated according to the herein described process according to the invention. Accordingly, the plant used as wild type, control or reference corresponds to the plant as much as possible and is in any other property but in the result of the process of the invention as identical to the subject matter of the invention as possible. Thus, the wild type, control or reference is treated identically or as identical as possible, saying that only conditions or properties might be different which do not influence the quality of the tested property.

Preferably, any comparison is carried out under analogous conditions. The term "analogous conditions" means that all conditions such as, for example, culture or growing conditions, assay conditions (such as buffer composition, temperature, substrates, pathogen strain, concentrations and the like) are kept identical between the experiments to be compared.

The "reference", "control", or "wild type" is preferably a subject, e.g. an organelle, a cell, a tissue, in particular a plant, which was not modified or treated according to the herein described process and is in any other property as

similar to the subject matter of the invention as possible, preferably 95%, more preferred are 98%, even more preferred are 99,00%, in particular 99,10%, 99,30%, 99,50%, 99,70%, 99,90%, 99,99%, 99, 999% or more.

In one embodiment the transgenic plant used according to the invention with at least one transgenic modification as compared to a corresponding wild-type plant is a plant selected from the group according to table A:

Table A

| | |
|---|---|
| A1 | a) Plant with one or more increased or generated activities in a plant cell conferring an altered trait as compared with the wild type,<br>b) Plant with one or more increased or generated activities in a plant cell conferring an altered trait as compared with the wild type, whereby said trait is not resistance against the active ingredient of the invention. |
| A2 | a) Plant with one or more reduced, repressed or deleted activities in a plant cell conferring an altered trait as compared with the wild type,<br>b) Plant with one or more reduced, repressed or deleted activities in a plant cell conferring an altered trait as compared with the wild type, whereby said trait is not resistance against the active ingredient of the invention. |

The term "activity" of a compound refers to the function of a compound in a biological system such as a cell, an organ or an organism. For example, the term "activity" of a compound refers to the enzymatic function, regulatory function or its function as binding partner, transporter, regulator, or carrier, etc. of a compound.

The term "plant" includes according to the invention a plant cell, organelle, a plant tissue or a part thereof, such as seed, root, tuber, fruit, leave, flower, plant propagation material etc.

The term "plant propagation material" as used herein includes all the generative parts of the plant such as seeds and vegetative plant material such as cuttings and tubers (e.g. potatoes), which can be used for the multiplication of the plant. This includes seeds, roots, fruits, tubers, bulbs, rhizomes, shoots, sprouts and other parts of theplants. Seedlings and young plants, which are to be transplanted after germination or after emergence from soil, may also be included. These plant propagation materials may be treated prophylactically with a plant protection compound either at or before planting or transplanting.

For the purposes of the invention, as a rule the plural is intended to encompass the singular and vice versa.

Unless otherwise specified, the terms "polynucleotides", "nucleic acid" and "nucleic acid molecule" are interchangeably in the present context. Unless otherwise specified, the terms "peptide", "polypeptide" and "protein" are interchangeably in the present context. The term "sequence" may relate to polynucleotides, nucleic acids, nucleic acid molecules, peptides, polypeptides and proteins, depending on the context in which the term "sequence" is used. The terms "gene(s)", "polynucleotide", "nucleic acid sequence", "nucleotide sequence", or "nucleic acid molecule(s)" as used herein refer to a polymeric form of nucleotides of any length, either ribonucleotides or deoxyribonucleotides. The terms refer only to the primary structure of the molecule.

Thus, the terms "gene(s)", "polynucleotide", "nucleic acid sequence", "nucleotide sequence", or "nucleic acid molecule(s)" as used herein include double- and single-stranded DNA and/or RNA. They also include known types of modifications, e.g., methylation, "caps", substitutions of one or more of the naturally occurring nucleotides with an analog. Preferably, the DNA or RNA sequence comprises a coding sequence encoding the herein defined polypeptide.

A "coding sequence" is a nucleotide sequence, which is transcribed into a RNA, e.g. a regulatory RNA, such as a miRNA, a ta-siRNA, cosuppression molecule, a RNAi, a ribozyme, etc. or into a mRNA which is translated into a polypeptide when placed under the control of appropriate regulatory sequences. The boundaries of the coding sequence are determined by a translation start codon at the 5'-terminus and a translation stop codon at the 3'-terminus. A coding sequence can include, but is not limited to mRNA, cDNA, recombinant nucleotide sequences or genomic DNA, while introns may be present as well under certain circumstances.

As used in the present context a nucleic acid molecule may also encompass the untranslated sequence located at the 3' and at the 5' end of the coding gene region, for example at least 500, preferably 200, especially preferably 100, nucleotides of the sequence upstream of the 5' end of the coding region and at least 100, preferably 50, especially preferably 20, nucleotides of the sequence downstream of the 3' end of the coding gene region. Using antisense, RNAi, snRNA, dsRNA, siRNA, miRNA, ta-siRNA, cosuppression, ribozyme etc. technology, the coding regions as well as the 5'- and/or 3'-regions can advantageously be used.

However, it is often advantageous only to choose the coding region for cloning and expression purposes.

In one embodiment of the present invention, "transgenic modification" means an increased or generated activity of a polypeptide which is the expression product of a coding region of a gene.

"Polypeptide" refers to a polymer of amino acid (amino acid sequence) and does not refer to a specific length of the molecule. Thus peptides and oligopeptides are included within the definition of polypeptide. This term does also refer to or include post-translational modifications of the polypeptide, for example, glycosylations, acetylations, phosphorylations and the like. Included within the definition are, for example, polypeptides containing one or more analogs of an amino acid (including, for example, unnatural amino acids, etc.), polypeptides with substituted linkages, as well as other modifications known in the art, both naturally occurring and non-naturally occurring.

Plants and plant cells according to table A1 are disclosed in W02004018687, W02004092398, W02006032708 which are incorporated by reference.

Plants and plant cells according to table A2 are disclosed in W02004092349, W02006032707 which are incorporated by reference.

The methods for generating such transgenic plants are widely known to those skilled in the art and described, for example, in the publications mentioned above, which are incorporated by reference.

In one embodiment transgenic plants with at least one transgenic modification as compared to a corresponding wild-type plant used in the process of the invention are plants as disclosed or generated from plant cells as disclosed in the publications as depicted in table B, column 2. The publications as depicted in table B, column 2 are herewith incorporated by reference.

In one embodiment transgenic plants with at least one transgenic modification as compared to a corresponding wild-type plant used in the process of the invention are plants with one or more increased or generated activities selected from the group consisting of the activities as depicted in table B, column 5.

In one embodiment transgenic plants with at least one transgenic modification as compared to a corresponding wild-type plant used in the process of the invention are plants with one or more increased or generated activities selected from the group consisting of the activities as depicted in SEQ ID NO: 1 to 270 or homologs thereof.

In one embodiment, the method for producing a transgenic plant with at least one transgenic modification as compared to a corresponding wild-type plant comprises one or more of the following steps

a) stabilizing a protein conferring the increased expression of a protein encoded by the nucleic acid molecule as depicted in table B, column 1 or 3 or of the polypeptid as depicted in SEQ ID NO: 1 to 270;

b) stabilizing a mRNA conferring the increased expression of a protein encoded by the nucleic acid molecule as depicted in table B, column 1 or 3 or its homologs or of a mRNA encoding the polypeptide as depicted in SEQ ID NO: 1 to 270;

c) increasing the specific activity of a protein conferring the increased expression of a protein encoded by the nucleic acid molecule as depicted in table B, column 1 or 3 or of the polypeptide o as depicted in SEQ ID NO: 1 to 270 or decreasing the inhibitory regulation of the polypeptide as depicted in SEQ ID NO: 1 to 270;

d) generating or increasing the expression of an endogenous or artificial transcription factor mediating the expression of a protein conferring the increased expression of a protein encoded by the nucleic acid molecule as depicted in table B, column 1 or 3 or of the polypeptide as depicted in SEQ ID NO: 1 to 270;

e) stimulating activity of a protein conferring the increased expression of a protein encoded by the nucleic acid molecule as depicted in table B, column 1 or 3 or a polypeptide as depicted in SEQ ID NO: 1 to 270;

f) expressing a transgenic gene encoding a protein conferring the increased expression of a polypeptide encoded by the nucleic acid molecule as depicted in table B, column 1 or 3 or a polypeptide as depicted in SEQ ID NO: 1 to 270; and/or

g) increasing the copy number of a gene conferring the increased expression of a nucleic acid molecule encoding a polypeptide encoded by the nucleic acid molecule as depicted in table B, column 1 or 3 or the polypeptide as depicted in SEQ ID NO: 1 to 270, and/or;

h) increasing the expression of the endogenous gene encoding the polypeptide as depicted in SEQ ID NO: 1 to 270 or its homologs by adding positive expression or removing negative expression elements, e.g. homologous recombination can be used to either introduce positive regulatory elements like for plants the 35S enhancer into the promoter or to remove repressor elements form regulatory regions. Further gene conversion methods can be used to disrupt repressor elements or to enhance to acitivty of positive elements- positive elements can be randomly introduced in plants by T-DNA or transposon mutagenesis and lines can be identified in which the positive elements have be integrated near to a gene of the invention, the expression of which is thereby enhanced;
and/or

i) modulating growth conditions of the plant in such a manner, that the expression or activity of the gene encoding

the protein as depicted in SEQ ID NO: 1 to 270 or the protein itself is enhanced;

j) selecting of organisms with expecially high activity of the proteins of the invention from natural or from mutagenized resources and breeding them into the target organisms, eg the elite crops.

In one embodiment transgenic plants with at least one transgenic modification as compared to a corresponding wild-type plant used in the process of the invention are plants with one or more increased or generated activities selected from the group consisting of the polypeptides encoded by:

a) a nucleic acid molecule encoding the polypeptide as depicted in any of the SEQ ID NO: 1 to 270 or the homologs as depicted in SEQ ID NO: 271 to 273;

b) a nucleic acid molecule as depicted in table B, column 1 or 3;

c) a nucleic acid molecule, which, as a result of the degeneracy of the genetic code, can be derived from a polypeptide sequence as depicted in table B, column 1 or 3 and/or in SEQ ID NO: 1 to 270;

d) a nucleic acid molecule having at least 30 % identity with the nucleic acid molecule sequence of a polynucleotide comprising the nucleic acid molecule according to a) to c);

e) a nucleic acid molecule encoding a polypeptide having at least 30 % identity with the amino acid sequence of the polypeptide encoded by the nucleic acid molecule of (a) to (c) and having the activity represented by a nucleic acid molecule of (a) to (c) ;

f) nucleic acid molecule which hybridizes with a nucleic acid molecule of (a) to (c) under stringent hybridization conditions;

g) a nucleic acid molecule encoding a polypeptide which can be isolated with the aid of monoclonal or polyclonal antibodies made against a polypeptide encoded by one of the nucleic acid molecules of (a) to (e) and having the activity represented by the nucleic acid molecule comprising a polynucleotide of (a) to (c);

h) a nucleic acid molecule encoding a polypeptide comprising the consensus sequence or one or more polypeptide motifs resulting from 2 ore more of the polypeptides as depicted in table B, column 1 or 3 and/or in SEQ ID NO: 1 to 270 and/or a consensus sequence as depicted in SEQ ID NO: 274 to 277;

h) a nucleic acid molecule encoding a polypeptide having the activity represented by a protein as depicted in table B, column 1 or 3 or 5 and/or SEQ ID NO: 1 to 270;

i) nucleic acid molecule which comprises a polynucleotide, which is obtained by amplifying a cDNA library or a genomic library using the primers for the polynucleotides as depicted in table B, column 1 or 3 and/or SEQ ID NO: 1 to 270 which do not start at their 5'-end with the nucleotides ATA;
and

j) a nucleic acid molecule which is obtainable by screening a suitable nucleic acid library under stringent hybridization conditions with a probe comprising a complementary sequence of a nucleic acid molecule of (a) or (b) or with a fragment thereof, having at least 15 nt, preferably 20 nt, 30 nt, 50 nt, 100 nt, 200 nt or 500 nt of a nucleic acid molecule complementary to a nucleic acid molecule sequence characterized in (a) to (e) and encoding a polypeptide having the activity represented by a protein comprising a polypeptide as depicted in table B, column 1 or 3 and/or SEQ ID NO: 1 to 270.

In one embodiment transgenic plants with at least one transgenic modification as compared to a corresponding wild-type plant used in the process of the invention are plants with one or more increased or generated activities selected from the group consisting of the yield increasing proteins which confers an increase in yield, plant health and/or plant productivity as compared to a corresponding non-transformed wild-type plant.

In one embodiment transgenic plants with at least one transgenic modification as compared to a corresponding wild-type plant used in the process of the invention are plants with one or more increased or generated activities selected from the group consisting of the SRP (stress related proteins) which confers an increase in tolerance and/or resistance to environmental stress as compared to a corresponding non-transformed wild-type plant.

In one embodiment transgenic plants with at least one transgenic modification as compared to a corresponding wild-type plant used in the process of the invention are plantswith one or more increased or generated activities selected from the group consisting of the SRP (stress related proteins) which confers an increase in drought resistance as compared to a corresponding non-transformed wild-type plant.

In one embodiment transgenic plants with at least one transgenic modification as compared to a corresponding wild-type plant used in the process of the invention are plants with one or more increased or generated activities selected from the group consisting of the yield increasing proteins as shown in table B and/or SEQ ID NO: 1 to 270.

In one embodiment transgenic plants with at least one transgenic modification as compared to a corresponding wild-type plant used in the process of the invention are plants with one or more increased or generated activities selected from the group consisting of the yield increasing proteins as shown in table B and/or SEQ ID NO: 1 to 270.

As used herein, the term "environmental stress" refers to any sub-optimal growing condition and includes, but is not limited to, sub-optimal conditions associated with salinity, drought, temperature, metal, chemical, pathogenic and oxidative stresses, or combinations thereof. In preferred embodiments, environmental stress is drought and low water content. Wherein drought stress means any environmental stress which leads to a lack of water content in plants, lack of water uptake potential or reduction of water supply to the plants.

In one embodiment of the invention the term "increased tolerance and/or resistance to environmental stress" relates to an increased resistance to water stress, which is produced as a secondary stress by chilling, freezing, heat, and salt, as a tertiary stress by radiation, and, of course, as a primary stress during drought.

In a preferred embodiment of the invention the term "increased tolerance and/or resistance to environmental stress" relates to an increased drought resistance.

In one embodiment transgenic plants with at least one transgenic modification as compared to a corresponding wild-type plant used in the process of the invention are plants with an increased or generated activities of a polypeptide with an activity as indicated in table B, column 5, line No 1, 5, 7,10, 11, 12, 16, 19, 20, 21, 23, 25, 27, 28, 30, 31, 33, 37, 39, 40, 42, 43, 91, 105, 107, 112, 150, 159, 164, 165, 166, 167, 168, 169, 170, 171, 172, 173, 174, 175, 176, 177, 178,182, 186, 187, 188, 189, 190, 194, 196, 203, 204, 205, 206, 207, 209, 210, 211, 213, and/or SEQ ID NO: 1, 2, 3, 4, 5, 6, 7, 8, 9, 10, 11 ,12 ,13 ,14 ,15 ,16 , 17,18, 19, 20, 21,22, 23, 24, 25, 26, 27, 28, 29, 30, 31, 32, 46, 53, 266 respectively plants with an increased or generated activities of a polypeptide according to the sequence number as depicted in table B, column 3, line No 1, 5, 7,10, 11, 12, 16, 19, 20, 21, 23, 25, 27, 28, 30, 31, 33, 37, 39, 40, 42, 43, 91, 105, 107, 112, 150, 159, 164, 165, 166, 167, 168, 169, 170, 171, 172, 173, 174, 175, 176, 177, 178,182, 186, 187, 188, 189, 190, 194, 196, 203, 204, 205, 206, 207, 209, 210, 211, 213, and/or SEQ ID NO: 1, 2, 3, 4, 5, 6, 7, 8, 9, 10, 11, 12, 13 ,14 ,15 ,16 , 17,18, 19, 20, 21,22, 23, 24, 25, 26, 27, 28, 29, 30, 31, 32, 46, 53, 266 respectively plants with an increased or generated expression of the gene as depicted in table B, column 1, line No 1, 5, 7,10, 11, 12, 16, 19, 20, 21, 23, 25, 27, 28, 30, 31, 33, 37, 39, 40, 42, 43, 91, 105, 107, 112, 150, 159, 164, 165, 166, 167, 168, 169, 170, 171, 172, 173, 174, 175, 176, 177, 178,182, 186, 187, 188, 189, 190, 194, 196, 203, 204, 205, 206, 207, 209, 210, 211, 213 and/or SEQ ID NO: 1, 2, 3, 4, 5, 6, 7, 8, 9, 10, 11, 12, 13 ,14 ,15 ,16 , 17,18, 19, 20, 21,22, 23, 24, 25, 26, 27,28,29,30,31,32,46,53,266.

In one embodiment transgenic plants with at least one transgenic modification as compared to a corresponding wild-type plant used in the process of the invention are plants with an increased or generated activitiy of a polypeptide with an activity of a transcription factor, preferably of the subfamily of an "AP2 DOMAIN CONTAINING DEHYDRATION RESPONSIVE ELEMENT BINDING PROTEIN 1", preferably as depicted in SEQ ID NO: 1 and in table B, line 23, encoded by the gene EST163t.

In one embodiment the present invention relates to a method for increasing the plant health and plant productivity which comprises

a) treating the locus where the plant with at least one transgenic modification is growing or is expected to grow, and/or the seeds and/or the plant with at least one transgenic modification from which the transgenic plant grows with an effective amount of a chemical composition comprising at least one active ingredient and

b) cultivating and growing said seed and/or plant under conditions which permit the development of the plant,

and whereby the detection of increased plant health and/or increased plant productivity comprises at least one of the following steps:

i) growing transgenic seedlings and/or plants in a greenhouse under optimal, well water conditions, preferably administrating supplemental nutrients and light,

ii) collecting phenotyic data in an imaging procedure,

iii) harvesting the plants for determining fresh weight, dry weight and/or plant volume and standardize and correlate the data from the imaging procedure with the physiological features,

iv) measuring with the imaging system, preferably scanalyzer, the plant volume, internode length, greenness, yellowness, leaf angle, area of the leaves, number of leaves and/or stem length of the plants

v) comparing the data of chemically treated plants with non treated plants

In one embodiment transgenic plants with at least one transgenic modification as compared to a corresponding wild-type plant used in the process of the invention are plants with one or more increased or generated activities selected from the group consisting of homologs of the above mentioned polypeptides and/or homologs of the nucleic acid molecules encoding said polypeptides.

These homologs which are advantageously for the process according to the invention can be isolated based on their homology to the nucleic acid molecules disclosed herein using the sequences or part thereof as hybridization probe and following standard hybridization techniques under stringent hybridization conditions. In this context, it is possible to use, for example, isolated nucleic acid molecules of at least 15, 20, 25, 30, 35, 40, 50, 60 or more nucleotides, preferably of at least 15, 20 or 25 nucleotides in length which hybridize under stringent conditions with the above-described nucleic acid molecules, in particular with those which encompass a nucleotide sequence of the nucleic acid molecule used in the process of the invention or encoding a protein used in the invention or of the nucleic acid molecule of the invention. Nucleic acid molecules with 30, 50, 100, 250 or more nucleotides may also be used.

The term "homology" means that the respective nucleic acid molecules or encoded proteins are functionally and/or structurally equivalent. The nucleic acid molecules that are homologous to the nucleic acid molecules described above and that are derivatives of said nucleic acid molecules are, for example, variations of said nucleic acid molecules which represent modifications having the same biological function, in particular encoding proteins with the same or substantially the same biological function. They may be naturally occurring variations, such as sequences from other plant varieties or species, or mutations. These mutations may occur naturally or may be obtained by mutagenesis techniques. The allelic variations may be naturally occurring allelic variants as well as synthetically produced or genetically engineered variants. Structurally equivalents can, for example, be identified by testing the binding of said polypeptide to antibodies or computer based predictions. Structurally equivalent to have the similar immunological characteristic, e.g. comprise similar epitopes.

By "hybridizing" it is meant that such nucleic acid molecules hybridize under conventional hybridization conditions, preferably under stringent conditions such as described by, e.g., Sam-brook (Molecular Cloning; A Laboratory Manual, 2nd Edition, Cold Spring Harbor Laboratory Press, Cold Spring Harbor, NY (1989)) or in Current Protocols in Molecular Biology, John Wiley & Sons, N. Y. (1989), 6.3.1-6.3.6.

Homolog polypeptides are polypeptides which comprises an amino acid sequence at least about 50% identical to an amino acid sequence shown in SEQ ID NO: 1 to 270. Preferably, the protein encoded by the nucleic acid molecule is at least about 60% identical to the sequence shown in SEQ ID NO: 1 to 270, more preferably at least about 70% identical to one of the sequences shown in SEQ ID NO: 1 to 270, even more preferably at least about 80%, 90%, 95% homologous to the sequence shown in SEQ ID NO: 1 to 270, and most preferably at least about 96%, 97%, 98%, or 99% identical to the sequence shown in SEQ ID NO: 1 to 270.

To determine the percentage homology (= identity, herein used interchangeably) of two amino acid sequences or of two nucleic acid molecules, the sequences are written one underneath the other for an optimal comparison (for example gaps may be inserted into the sequence of a protein or of a nucleic acid in order to generate an optimal alignment with the other protein or the other nucleic acid).

The amino acid residues or nucleic acid molecules at the corresponding amino acid positions or nucleotide positions are then compared. If a position in one sequence is occupied by the same amino acid residue or the same nucleic acid molecule as the corresponding position in the other sequence, the molecules are homologous at this position (i.e. amino acid or nucleic acid "homology" as used in the present context corresponds to amino acid or nucleic acid "identity". The percentage homology between the two sequences is a function of the number of identical positions shared by the sequences (i.e. % homology = number of identical positions/total number of positions x 100). The terms "homology" and "identity" are thus to be considered as synonyms.

For the determination of the percentage homology (= identity) of two or more amino acids or of two or more nucleotide sequences several computer software programs have been developed. The homology of two or more sequences can be calculated with for example the software fasta, which presently has been used in the version fasta 3 (W. R. Pearson and D. J. Lipman (1988), Improved Tools for Biological Sequence Comparison. PNAS 85:2444- 2448; W. R. Pearson (1990) Rapid and Sensitive Sequence Comparison with FASTP and FASTA, Methods in Enzymology 183:63 - 98; W. R. Pearson and D. J. Lipman (1988) Improved Tools for Biological Sequence Comparison.PNAS 85: 2444- 2448; W. R. Pearson (1990); Rapid and Sensitive Sequence Comparison with FASTP and FASTAMethods in

Enzymology 183:63 - 98). Another useful program for the calculation of homologies of different sequences is the standard blast program, which is included in the Biomax pedant software (Biomax, Munich, Federal Republic of Germany). This leads unfortunately sometimes to suboptimal results since blast does not always include complete sequences of the subject and the querry. Nevertheless as this program is very efficient it can be used for the comparison of a huge number of sequences.

The following settings are typically used for such a comparisons of sequences: -p Program Name [String]; -d Database [String]; default = nr; -i Query File [File In]; default = stdin; -e Expectation value (E) [Real]; default = 10.0; -m alignment view options: 0 = pairwise; 1 = query-anchored showing identities; 2 = query-anchored no identities; 3 = flat query-anchored, show identities; 4 = flat query-anchored, no identities; 5 = query-anchored no identities and blunt ends; 6 = flat query-anchored, no identities and blunt ends; 7 = XML Blast output; 8 = tabular; 9 tabular with comment lines [Integer]; default = 0; -o BLAST report Output File [File Out] Optional; default = stdout; -F Filter query sequence (DUST with blastn, SEG with others) [String]; default = T; -G Cost to open a gap (zero invokes default behavior) [Integer]; default = 0; -E Cost to extend a gap (zero invokes default behavior) [Integer]; default = 0; -X X dropoff value for gapped alignment (in bits) (zero invokes default behavior); blastn 30, megablast 20, tblastx 0, all others 15 [Integer]; default = 0; -I Show GI's in deflines [T/F]; default = F; -q Penalty for a nucleotide mismatch (blastn only) [Integer]; default = -3; -r Reward for a nucleotide match (blastn only) [Integer]; default = 1; -v Number of database sequences to show one-line descriptions for (V) [Integer]; default = 500; -b Number of database sequence to show alignments for (B) [Integer]; default = 250; -f Threshold for extending hits, default if zero; blastp 11, blastn 0, blastx 12, tblastn 13; tblastx 13, megablast 0 [Integer]; default = 0; -g Perfom gapped alignment (not available with tblastx) [T/F]; default = T; -Q Query Genetic code to use [Integer]; default = 1; -D DB Genetic code (for tblast[nx] only) [Integer]; default = 1; -a Number of processors to use [Integer]; default = 1; -O SeqAlign file [File Out] Optional; -J Believe the query defline [T/F]; default = F; -M Matrix [String]; default = BLO-SUM62; -W Word size, default if zero (blastn 11, megablast 28, all others 3) [Integer]; default = 0; -z Effective length of the database (use zero for the real size) [Real]; default = 0; -K Number of best hits from a region to keep (off by default, if used a value of 100 is recommended) [Integer]; default = 0; -P 0 for multiple hit, 1 for single hit [Integer]; default = 0; -Y Effective length of the search space (use zero for the real size) [Real]; default = 0; -S Query strands to search against database (for blast[nx], and tblastx); 3 is both, 1 is top, 2 is bottom [Integer]; default = 3; -T Produce HTML output [T/F]; default = F; -l Restrict search of database to list of GI's [String] Optional; -U Use lower case filtering of FASTA sequence [T/F] Optional; default = F; -y X dropoff value for ungapped extensions in bits (0.0 invokes default behavior); blastn 20, megablast 10, all others 7 [Real]; default = 0.0; -Z X dropoff value for final gapped alignment in bits (0.0 invokes default behavior); blastn/megablast 50, tblastx 0, all others 25 [Integer]; default = 0; -R PSI-TBLASTN checkpoint file [File In] Optional; -n MegaBlast search [T/F]; default = F; -L Location on query sequence [String] Optional; -A Multiple Hits window size, default if zero (blastn/megablast 0, all others 40 [Integer]; default = 0; -w Frame shift penalty (OOF algorithm for blastx) [Integer]; default = 0; -t Length of the largest intron allowed in tblastn for linking HSPs (0 disables linking) [Integer]; default = 0.

Results of high quality are reached by using the algorithm of Needleman and Wunsch or Smith and Waterman. Therefore programs based on said algorithms are preferred. Advantageously the comparisons of sequences can be done with the program PileUp (J. Mol. Evolution., 25, 351-360, 1987, Higgins et al., CABIOS, 5 1989: 151-153) or preferably with the programs Gap and BestFit, which are respectively based on the algorithms of Needleman and Wunsch [J. Mol. Biol. 48; 443-453 (1970)] and Smith and Waterman [Adv. Appl. Math. 2; 482-489 (1981)]. Both programs are part of the GCG software-package [Genetics Computer Group, 575 Science Drive, Madison, Wisconsin, USA 53711 (1991); Altschul et al. (1997) Nucleic Acids Res. 25:3389 et seq.]. Therefore preferably the calculations to determine the perentages of sequence homology are done with the program Gap over the whole range of the sequences. The following standard adjustments for the comparison of nucleic acid sequences were used: gap weight: 50, length weight: 3, average match: 10.000, average mismatch: 0.000.

Preferred transgenicplants are, for example, selected from the families Aceraceae, Anacardiaceae, Apiaceae, Asteraceae, Brassicaceae, Cactaceae, Cucurbitaceae, Euphorbiaceae, Fabaceae, Malvaceae, Nymphaeaceae, Papaveraceae, Rosaceae, Salicaceae, Solanaceae, Arecaceae, Bromeliaceae, Cyperaceae, Iridaceae, Liliaceae, Orchidaceae, Gentianaceae, Labiaceae, Magnoliaceae, Ranunculaceae, Carifolaceae, Rubiaceae, Scrophulariaceae, Caryophyllaceae, Ericaceae, Polygonaceae, Violaceae, Juncaceae or Poaceae and preferably from a plant selected from the group of the families Apiaceae, Asteraceae, Brassicaceae, Cucurbitaceae, Fabaceae, Papaveraceae, Rosaceae, Solanaceae, Liliaceae or Poaceae. Preferred are crop plants such as plants advantageously selected from the group of the genus peanut, oilseed rape, canola, cotton, sunflower, sugar cane, safflower, olive, sesame, hazelnut, almond, avocado, bay, pumpkin/squash, linseed, soybeans, pistachio, borage, maize, wheat, rye, oats, sorghum and millet, triticale, rice, barley, cassava, potato, sugar beet, egg plant, alfalfa, and perennial grasses and forage plants, oil palm, vegetables (brassicas, root vegetables, tuber vegetables, pod vegetables, fruiting vegetables, onion vegetables, leafy vegetables and stem vegetables), buckwheat, Jerusalem artichoke, broad bean, vetches, lentil, dwarf bean, lupin, clover, potato, tomato, lettuce, onions and Lucerne.

In one prefered embodiment, the transgenic plant is selected from the families Aceraceae, Anacardiaceae,

Apiaceae, Asteraceae, Brassicaceae, Cactaceae, Cucurbitaceae, Euphorbiaceae, Fabaceae, Malvaceae, Nymphaeaceae, Papaveraceae, Rosaceae, Salicaceae, Solanaceae, Arecaceae, Bromeliaceae, Cyperaceae, Iridaceae, Liliaceae, Orchidaceae, Gentianaceae, Labiaceae, Magnoliaceae, Ranunculaceae, Carifolaceae, Rubiaceae, Scrophulariaceae, Caryophyllaceae, Ericaceae, Polygonaceae, Violaceae, Juncaceae or Poaceae and preferably from a plant selected from the group of the families Apiaceae, Asteraceae, Brassicaceae, Cucurbitaceae, Fabaceae, Papaveraceae, Rosaceae, Solanaceae, Liliaceae or Poaceae. Preferred are crop plants and in particular plants mentioned herein above as host plants such as the families and genera mentioned above for example preferred the species *Anacardium occidentale, Calendula officinalis, Carthamus tinctorius, Cichorium intybus, Cynara scolymus, Helianthus annus, Tagetes lucida, Tagetes erecta, Tagetes tenuifolia; Daucus carota; Corylus avellana, Corylus colurna, Borago officinalis; Brassica napus, Brassica rapa* ssp., *Sinapis arvensis Brassica juncea, Brassica juncea var. juncea, Brassica juncea var. crispifolia, Brassica juncea var. foliosa, Brassica nigra, Brassica sinapioides, Melanosinapis communis, Brassica oleracea,* Arabidopsis thaliana, *Anana comosus, Ananas ananas, Bromelia comosa, Carica papaya, Cannabis sative, Ipomoea batatus, Ipomoea pandurata, Convolvulus batatas, Convolvulus tiliaceus, Ipomoea fastigiata, Ipomoea tiliacea, Ipomoea triloba, Convolvulus panduratus, Beta vulgaris, Beta vulgaris var. altissima, Beta vulgaris var. vulgaris, Beta maritima, Beta vulgaris var. perennis, Beta vulgaris var. conditiva, Beta vulgaris var. esculenta, Cucurbita maxima, Cucurbita mixta, Cucurbita pepo, Cucurbita moschata, Olea europaea, Manihot utilissima, Janipha manihot" Jatropha manihot., Manihot aipil, Manihot dulcis, Manihot manihot, Manihot melanobasis, Manihot esculenta, Ricinus communis, Pisum sativum, Pisum arvense, Pisum humile, Medicago sativa, Medicago falcata, Medicago varia, Glycine max Dolichos soja, Glycine gracilis, Glycine hispida, Phaseolus max, Soja hispida, Soja max, Cocos nucifera, Pelargonium grossularioides, Oleum cocoas, Laurus nobilis, Persea americana, Arachis hypogaea, Linum usitatissimum, Linum humile, Linum austriacum, Linum bienne, Linum angustifolium, Linum catharticum, Linum flavum, Linum grandiflorum, Adenolinum grandiflorum, Linum lewisii, Linum narbonense, Linum perenne, Linum perenne var. lewisii, Linum pratense, Linum trigynum, Punica granatum, Gossypium hirsutum, Gossypium arboreum, Gossypium barbadense, Gossypium herbaceum, Gossypium thurberi, Musa nana, Musa acuminata, Musa paradisiaca, Musa* spp., *Elaeis guineensis, Papaver orientale, Papaver rhoeas, Papaver dubium, Sesamum indicum, Piper aduncum, Piper amalago, Piper angustifolium, Piper auritum, Piper betel, Piper cubeba, Piper longum, Piper nigrum, Piper retrofractum, Artanthe adunca, Artanthe elongata, Peperomia elongata, Piper elongatum, Steffensia elongata,* , *Hordeum vulgare, Hordeum jubatum, Hordeum murinum, Hordeum secalinum, Hordeum distichon Hordeum aegiceras, Hordeum hexastichon., Hordeum hexastichum, Hordeum irregulare, Hordeum sativum, Hordeum secalinum, Avena sativa, Avena fatua, Avena byzantina, Avena fatua var. sativa, Avena hybrida, Sorghum bicolor, Sorghum halepense, Sorghum saccharatum, Sorghum vulgare, Andropogon drummondii, Holcus bicolor, Holcus sorghum, Sorghum aethiopicum, Sorghum arundinaceum, Sorghum caffrorum, Sorghum cernuum, Sorghum dochna, Sorghum drummondii, Sorghum durra, Sorghum guineense, Sorghum lanceolatum, Sorghum nervosum, Sorghum saccharatum, Sorghum subglabrescens, Sorghum verticilliflorum, Sorghum vulgare, Holcus halepensis, Sorghum miliaceum millet, Panicum militaceum, Zea mays, Triticum aestivum, Triticum durum, Triticum turgidum, Triticum hybernum, Triticum macha, Triticum sativum* or *Triticum vulgare,* Cofea *spp.,* Coffea *arabica,* Coffea *canephora,* Coffea *liberica, Capsicum annuum, Capsicum annuum var. glabriusculum, Capsicum frutescens, Capsicum annuum, Nicotiana tabacum, Solanum tuberosum, Solanum melongena, Lycopersicon esculentum, Lycopersicon lycopersicum., Lycopersicon pyriforme, Solanum integrifolium, Solanum lycopersicum Theobroma* cacao or *Camellia sinensis.* Anacardiaceae such as the genera Pistacia, Mangifera, Anacardium e.g. the species *Pistacia vera* [pistachios, Pistazie], *Mangifer indica* [Mango] or *Anacardium occidentale* [Cashew]; Asteraceae such as the genera Calendula, Carthamus, Centaurea, Cichorium, Cynara, Helianthus, Lactuca, Locusta, Tagetes, Valeriana e.g. the species *Calendula officinalis* [Marigold], *Carthamus tinctorius* [safflower], *Centaurea cyanus* [cornflower], *Cichorium intybus* [blue daisy], *Cynara scolymus* [Artichoke], *Helianthus annus* [sunflower], Lactuca sativa, *Lactuca crispa, Lactuca esculenta, Lactuca scariola L. ssp. sativa, Lactuca scariola L. var. integrata, Lactuca scariola L. var. integrifolia, Lactuca sativa subsp. romana, Locusta communis, Valeriana locusta* [lettuce], *Tagetes lucida, Tagetes erecta or Tagetes tenuifolia* [Marigold]; Apiaceae such as the genera Daucus e.g. the species *Daucus carota* [carrot]; Betulaceae such as the genera Corylus e.g. the species *Corylus avellana* or *Corylus colurna* [hazelnut]; Boraginaceae such as the genera Borago e.g. the species *Borago officinalis* [borage]; Brassicaceae such as the genera Brassica, Melanosinapis, Sinapis, Arabadopsis e.g. the species *Brassica napus, Brassica rapa* ssp. [canola, oilseed rape, turnip rape], *Sinapis arvensis Brassica juncea, Brassica juncea var. juncea, Brassica juncea var. crispifolia, Brassica juncea var. foliosa, Brassica nigra, Brassica sinapioides, Melanosinapis communis* [mustard], *Brassica oleracea* [fodder beet] or *Arabidopsis thaliana*; Bromeliaceae such as the genera Anana, Bromelia e.g. the species *Anana comosus, Ananas ananas* or *Bromelia comosa* [pineapple]; Caricaceae such as the genera Carica e.g. the species *Carica papaya* [papaya]; Cannabaceae such as the genera Cannabis e.g. the species *Cannabis sative* [hemp], Convolvulaceae such as the genera Ipomea, Convolvulus e.g. the species *Ipomoea batatus, Ipomoea pandurata, Convolvulus batatas, Convolvulus tiliaceus, Ipomoea fastigiata, Ipomoea tiliacea, Ipomoea triloba* or *Convolvulus panduratus* [sweet potato, Man of the Earth, wild potato], Chenopodiaceae such as the genera Beta, i.e. the species *Beta vulgaris, Beta vulgaris var. altissima*, *Beta vulgaris var. Vulgaris, Beta maritima, Beta vulgaris var. perennis, Beta vulgaris var. conditiva* or *Beta vulgaris var.*

*esculenta* [sugar beet]; Cucurbitaceae such as the genera Cucubita e.g. the species *Cucurbita maxima, Cucurbita mixta, Cucurbita pepo* or *Cucurbita moschata* [pumpkin, squash]; Elaeagnaceae such as the genera Elaeagnus e.g. the species *Olea europaea* [olive]; Ericaceae such as the genera Kalmia e.g. the species *Kalmia latifolia, Kalmia angustifolia, Kalmia microphylla, Kalmia polifolia, Kalmia occidentalis, Cistus chamaerhodendros* or *Kalmia lucida* [American laurel, broad-leafed laurel, calico bush, spoon wood, sheep laurel, alpine laurel, bog laurel, western bog-laurel, swamp-laurel]; Euphorbiaceae such as the genera Manihot, Janipha, Jatropha, Ricinus e.g. the species *Manihot utilissima, Janipha manihot" Jatropha mani-hot., Manihot aipil, Manihot dulcis, Manihot manihot, Manihot melanobasis, Manihot esculenta* [manihot, arrowroot, tapioca, cassava] or *Ricinus communis* [castor bean, Castor Oil Bush, Castor Oil Plant, Palma Christi, Wonder Tree]; Fabaceae such as the genera Pisum, Albizia, Cathormion, Feuillea, Inga, Pithecolobium, Acacia, Mimosa, Medicajo, Glycine, Dolichos, Phaseolus, Soja e.g. the species *Pisum sativum, Pisum arvense, Pisum humile* [pea], *Albizia berteriana, Albizia julibrissin, Albizia lebbeck, Acacia berteriana, Acacia littoralis, Albizia berteriana, Albizzia berteriana, Cathormion berteriana, Feuillea berteriana, Inga fragrans, Pithecellobium berterianum, Pithecellobium fragrans, Pithecolobium berterianum, Pseudalbizzia berte-riana, Acacia julibrissin, Acacia nemu, Albizia nemu, Feuilleea julibrissin, Mimosa julibrissin, Mimosa speciosa, Sericanrda julibrissin, Acacia lebbeck, Acacia macrophylla, Albizia lebbek, Feuilleea lebbeck, Mimosa lebbeck, Mimosa speciosa* [bastard logwood, silk tree, East Indian Walnut], *Medicago sativa, Medicago falcata, Medicago varia* [alfalfa] *Glycine max Dolichos soja, Glycine gracilis, Glycine hispida, Phaseolus max, Soja hispida* or *Soja max* [soybean]; Geraniaceae such as the genera Pelargonium, Cocos, Oleum e.g. the species *Cocos nucifera, Pelargonium grossularioides* or *Oleum cocois* [coconut]; Gramineae such as the genera Saccharum e.g. the species *Saccharum officinarum;* Juglandaceae such as the genera Juglans, Wallia e.g. the species *Juglans regia, Juglans ailanthifolia, Juglans sieboldiana, Juglans cinerea, Wallia cinerea, Juglans bixbyi, Juglans californica, Juglans hindsii, Juglans intermedia, Juglans jamaicensis, Juglans major, Juglans microcarpa, Juglans nigra* or *Vllallia nigra* [walnut, black walnut, common walnut, persian walnut, white walnut, butternut, black walnut]; Lau-raceae such as the genera Persea, Laurus e.g. the species laurel *Laurus nobilis* [bay, laurel, bay laurel, sweet bay], *Persea americana Persea americana, Persea gratissima* or *Persea persea* [avocado]; Leguminosae such as the genera Arachis e.g. the species *Arachis hypogaea* [peanut]; Linaceae such as the genera Linum, Adenolinum e.g. the species *Linum usitatissimum, Linum humile, Linum austriacum, Linum bienne, Linum angustifolium, Linum catharticum, Linum flavum, Linum grandiflorum, Adenolinum grandiflorum, Linum lewisii, Linum narbonense, Linum perenne, Linum perenne var. lewisii, Linum pratense* or *Linum trigynum* [flax, linseed]; Lythrarieae such as the genera Punica e.g. the species *Punica granatum* [pomegranate]; Malvaceae such as the genera Gossypium e.g. the species *Gossypium hirsutum, Gossypium arboreum, Gossypium barbadense, Gossypium herbaceum* or *Gossypium thurberi* [cotton]; Musaceae such as the genera Musa e.g. the species *Musa nana, Musa acuminata, Musa paradisiaca, Musa* spp. [banana]; Onagraceae such as the genera Camissonia, Oenothera e.g. the species *Oenothera biennis* or *Camissonia brevipes* [primrose, evening primrose]; Palmae such as the genera Elacis e.g. the species *Elaeis guineensis* [oil plam]; Papaveraceae such as the genera Papaver e.g. the species *Papaver orientale, Papaver rhoeas, Papaver dubium* [poppy, oriental poppy, corn poppy, field poppy, shirley poppies, field poppy, long-headed poppy, long-pod poppy]; Pedaliaceae such as the genera Sesamum e.g. the species *Sesamum indicum* [sesame]; Piperaceae such as the genera Piper, Artanthe, Peperomia, Steffensia e.g. the species *Piper aduncum, Piper amalago, Piper angustifolium, Piper auritum, Piper betel, Piper cubeba, Piper longum, Piper nigrum, Piper retrofractum, Artanthe adunca, Artanthe elongata, Peperomia elongata, Piper elongatum, Steffensia elongata.* [Cayenne pepper, wild pepper]; Poaceae such as the genera Hordeum, Secale, Avena, Sorghum, Andropogon, Holcus, Panicum, Oryza, Zea, Triticum e.g. the species *Hordeum vulgare, Hordeum jubatum, Hordeum murinum, Hordeum secalinum, Hordeum distichon Hordeum aegiceras, Hordeum hexastichon., Hordeum hexastichum, Hordeum irregulare, Hordeum sativum, Hordeum secalinum* [barley, pearl barley, foxtail barley, wall barley, meadow barley], *Secale cereale* [rye], Avena sativa, *Avena fatua, Avena byzantina, Avena fatua var. sativa, Avena hybrida* [oat], *Sorghum bicolor, Sorghum halepense, Sorghum saccharatum, Sorghum vulgare, Andropogon drummondii, Holcus bicolor, Holcus sorghum, Sorghum aethiopicum, Sorghum arundinaceum, Sorghum caffrorum, Sorghum cernuum, Sorghum dochna, Sorghum drummondii, Sorghum durra, Sorghum guineense, Sorghum lanceolatum, Sorghum nervosum, Sorghum saccharatum, Sorghum subglabrescens, Sorghum verticilliflorum, Sorghum vulgare, Holcus halepensis, Sorghum miliaceum* millet, *Panicum militaceum [Sorghum, millet], Oryza sativa, Oryza latifolia* [rice], *Zea mays* [corn, maize] *Triticum aestivum, Triticum durum, Triticum turgidum, Triticum hybernum, Triticum macha, Triticum sativum* or *Triticum vulgare* [wheat, bread wheat, common wheat], Proteaceae such as the genera Macadamia e.g. the species *Macadamia intergrifolia* [macadamia]; Rubiaceae such as the genera Coffea e.g. the species Cofea spp., *Coffea arabica, Coffea canephora* or *Coffea liberica* [coffee]; Scrophulariaceae such as the genera Verbascum e.g. the species *Verbascum blattaria, Verbascum chaixii, Verbascum densiflorum, Verbascum lagurus, Verbascum longifolium, Verbascum lychnitis, Verbascum nigrum, Verbascum olympicum, Verbascum phlomoides, Verbascum phoenicum, Verbascum pulverulentum* or *Verbascum thapsus* [mullein, white moth mullein, nettle-leaved mullein, dense-flowered mullein, silver mullein, long-leaved mullein, white mullein, dark mullein, greek mullein, orange mullein, purple mullein, hoary mullein, great mullein]; Solanaceae such as the genera Capsicum, Nicotiana, Solanum, Lycopersicon e.g. the species *Capsicum annuum, Capsicum annuum var. glabriusculum, Capsicum frutescens* [pepper], *Capsicum annuum* [paprika], *Nicotiana*

*tabacum, Nicotiana alata*, *Nicotiana attenuata, Nicotiana glauca, Nicotiana langsdorffii, Nicotiana obtusifolia, Nicotiana quadrivalvis, Nicotiana repanda, Nicotiana rustica, Nicotiana sylvestris* [tobacco], *Solanum tuberosum* [potato], *Solanum melongena* [egg-plant] *(Lycopersicon esculentum, Lycopersicon lycopersicum., Lycopersicon pyriforme, Solanum integrifolium* or *Solanum lycopersicum* [*tomato*]; Sterculiaceae such as the genera Theobroma e.g. the species *Theobroma* cacao [cacao]; Theaceae such as the genera Camellia e.g. the species *Camellia sinensis*) [tea].

In a further embodiment of the invention the plant with the transgenic modification is derived from a monocotyledonous plant.

In a further embodiment of the invention the plant with the transgenic modification is derived from a dicotyledonous plant.

In a further embodiment of the invention the plant with the transgenic modification is derived from a gymnosperm plant, preferably selected from the group of spruce, pine and fir.

In a further embodiment the process of the invention comprises treating the plant with an effective amount of a chemical composition comprising at least one active ingredient.

In a further embodiment of the invention the active ingredient (B) is an active compound selected from the group consisting of

B1) an active compound that inhibits the mitochondrial respiration (breathing) chain at the level of the b/c1 complex: famoxadone and strobilurins selected from the group consisting of pyraclostrobin, kresoxim-methyl, dimoxystrobin, picoxystrobin, ZJ 0712, trifloxystrobin, enestroburin, orysastrobin, metominostrobin, azoxystrobin, fluoxastrobin, metominostrobin, orysastrobin, pyribencarb, trifloxystrobin, 2-(2-(6-(3-chloro-2-methyl-phenoxy)-5-fluoro-pyrimidin-4-yloxy)-phenyl)-2-methoxyimino-N-methyl-acetamide, 3-methoxy-2-(2-(N-(4-methoxy-phenyl)-cyclopropane-carboximidoylsulfanylmethyl)-phenyl)-acrylic acid methyl ester, methyl (2-chloro-5-[1-(3-methylbenzyloxyimino)ethyl] benzyl)carbamate and 2-(2-(3-(2,6-dichlorophenyl)-1-methyl-allylideneaminooxymethyl)-phenyl)-2-methoxyimino-N-methyl-

preferably famoxadone, pyraclostrobin, kresoxim-methyl, dimoxystrobin, picoxystrobin, ZJ 0712, trifloxystrobin, orysastrobin, azoxystrobin, fluoxastrobin

B2) carboxylic amides selected from benalaxyl, benodanil, boscalid, carboxin, mepronil, fenfuram, fenhexamid, futolanil, furametpyr, metalaxyl, ofurace, oxadixyl, oxycarboxin, penthiopyrad, thifluzamid, tiadinil, 4-difluoromethyl-2-methyl-thiazol-5-carboxylic acid-(4'-bromo-biphenyl-2-yl)-amide, 4-difluoromethyl-2-methyl-thiazol-5-carboxylic acid-(4'-trifluoromethyl-biphenyl-2-yl)-amide, 4-difluoromethyl-2-methyl-thiazol-5-carboxylic acid-(4'-chloro-3'-fluoro-biphenyl-2-yl)-amide, 3-difluoromethyl-1-methyl-pyrazol-4-carboxylic acid-(3',4'-dichloro-4-fluoro-biphenyl-2-yl)-amide, 3,4-dichloro-isothiazol-5-carboxylic acid-(2-cyano-phenyl)-amide, dimethomorph, flumorph, flumetover, fluopicolide (picobenzamid), zoxamide, carpropamide, diclocymet, mandipropamid, N-(2-(4-[3-(4-chlorophenyl)-prop-2-inyloxy]-3-methoxy-phenyl)-ethyl)-2-methanesulfonylamino-3-methyl-butyramid and N-(2-(4-[3-(4-chloro-phenyl)-prop-2-inyloxy]-3-methoxy-phenyl)-ethyl)-2-ethanesulfonylamino-3-methyl-butyramide;

preferably benalaxyl, benodanil, boscalid, carboxin, mepronil, fenfuram, fenhexamid, futolanil, furametpyr, metalaxyl, ofurace, oxadixyl, oxycarboxin, penthiopyrad, thifluzamid, tiadinil, (picobenzamid), diclocymet

B3) azoles selected from bitertanole, bromuconazole, cyproconazole, difenoconazole, diniconazole, enilconazole, epoxiconazole, fenbuconazole, flusilazole, fluquinconazole, flutriafol, hexaconazole, imibenconazole, ipconazole, metconazole, myclobutanil, penconazole, propiconazole, prothioconazole, simeconazole, tebuconazole, tetraconazole, triadimenol, triadimefon, triticonazole, cyazofamid, imazalil, pefurazoate, prochloraz, triflumizol, benomyl, carbendazim, fuberidazole, thiabendazole, ethaboxam, etridiazole and hymexazole; paclobutrazol, uniconazole-P;

preferably cyproconazole, epoxiconazole, fenbuconazole, fluquinconazole, flutriafol, hexaconazole, ipconazole, metconazole, propiconazole, prothioconazole, ebuconazole, tetraconazole, triadimenol, triadimefon, triticonazole, cyazofamid, imazalil, prochloraz, triflumizol, benomyl, carbendazim, thiabendazole, ethaboxam, hymexazole

B4) nitrogen-containing heterocyclic compounds selected from fluazinam, pyrifenox, 3-[5-(4-chloro-phenyl)-2,3-dimethyl-isoxazolidin-3-yl]-pyridine, bupirimat, cyprodinil, ferimzon, fenarimol, mepanipyrim, nuarimol, pyrimethanil, triforin, fludioxonil, fenpiclonil, aldimorph, dodemorph, fenpropimorph, tridemorph, iprodion, procymidon, vinclozolin, acibenzolar-S-methyl, anilazin, captan, captafol, dazomet, diclomezine, fenoxanil, folpet, fenpropidin, famoxadone, fenamidone, octhilinon, probenazol, proquinazid, pyroquilon, quinoxyfen, tricyclazol, 2-butoxy-6-iodo-3-propyl-chromen-4-one, 3-(3-bromo-6-fluoro-2-methyl-indole-1-sulfonyl)-[1,2,4]triazole-1-sulfonic acid dimethylamide, ancymidol, flurprimidol, inabenfide, tetcyclacis;

preferably pyrimethanil, fludioxonil, fenpiclonil, aldimorph, dodemorph, fenpropimorph, tridemorph, iprodion, procymidon, captan, captafol, dazomet, diclomezine, fenoxanil, probenazol, proquinazid, pyroquilon, quinoxyfen, tricyclazol,

B5) carbamates and dithiocarbamates selected from ferbam, mancozeb, metiram, metam, propineb, thiram, zineb, ziram, diethofencarb, flubenthiavalicarb, iprovalicarb, propamocarb, 3-(4-chloro-phenyl)-3-(2-isopropoxycarbonylamino-3-methyl-butyrylamino)-propionic acid methylester and N-(1-(1-(4-cyanophenyl)ethanesulfonyl)-but-2-yl)

carbamic acid -(4-fluorophenyl)ester;
preferably erbam, mancozeb, metiram, metam, propineb, thiram, zineb, ziram, diethofencarb, iprovalicarb, propamocarb,
B6) guanidines selected from dodin, iminoctadine and guazatin; preferably guazatin
B7) antibiotics selected from kasugamycin, polyoxine, streptomycin, oxytetracyclin and validamycin A; preferably streptomycin, oxytetracyclin and validamycin A
B8) fentin salts; preferably fentin salts;
B9) sulfur-containing heterocyclic compounds selected from isoprothiolan and dithianon; preferably sulfur-containing heterocyclic compounds selected from isoprothiolan and dithianon;
B10) organophosphorous compounds selected from edifenphos, fosetyl, fosetyl-aluminium, iprobenfos, pyrazophos, tolclofos-methyl, phosphoric acid and the salts thereof;
preferably edifenphos, fosetyl, iprobenfos, pyrazopho, phosphoric acid and the salts thereof;
B11) organo-chloro compounds selected from thiophanate methyl, chlorothalonil, dichlofluanid, tolylfluanid, flusulfamid, phthalide, hexachlorbenzene, pencycuron, quintozen;
preferably thiophanate methyl, chlorothalonil, dichlofluanid, flusulfamid, phthalide, quintozen;
B12) nitrophenyl derivatives selected from binapacryl, dinocap and dinobuton;
preferably binapacryl, dinocap and dinobuton
B13) inorganic active ingredients selected from Bordeaux composition, copper acetate, copper hydroxide, copper oxychloride, basic copper sulfate and sulfur;
preferably copper acetate, copper hydroxide, copper oxychloride, basic copper sulfate and sulfur;
B14) spiroxamine;
B15) cyflufenamide;
B16) cymoxanil;
B17) metrafenone;
B18) organo(thio)phosphates selected from acephate, azamethiphos, azinphos-methyl, chlorpyrifos, chlorpyrifos-methyl, chlorfenvinphos, diazinon, dichlorvos, dicrotophos, dimethoate, disulfoton, ethion, fenitrothion, fenthion, isoxathion, malathion, methamidophos, methidathion, methyl-parathion, mevinphos, monocrotophos, oxydemeton-methyl, paraoxon, parathion, phenthoate, phosalone, phosmet, phosphamidon, phorate, phoxim, pirimiphos-methyl, profenofos, prothiofos, sulprophos, tetrachlorvinphos, terbufos, triazophos and trichlorfon;
preferably metrafenone, chlorpyrifos, chlorpyrifos-methyl, chlorfenvinphos, diazinon, dichlorvos, dimethoate, disulfoton, ethion, methidathion, methyl-parathion, paraoxon, parathion, phenthoate, phosalone, phosmet, phosphamidon, phorate, phoxim, profenofos, prothiofos, sulprophos, tetrachlorvinphos, terbufos, triazophos and trichlorfon;
B19) carbamates selected from alanycarb, aldicarb, bendiocarb, benfuracarb, carbaryl, carbofuran, carbosulfan, fenoxycarb, furathiocarb, methiocarb, methomyl, oxamyl, pirimicarb, propoxur, thiodicar and triazamate;
preferably aldicarb, bendiocarb, benfuracarb, carbaryl, carbofuran, carbosulfan, fenoxycarb, furathiocarb, methiocarb, methomyl, oxamyl, pirimicarb, propoxur, thiodicar and triazamate;
B20) pyrethroids selected from allethrin, bifenthrin, cyfluthrin, cyhalothrin, cyphenothrin, cypermethrin, alpha-cypermethrin, beta-cypermethrin, zeta-cypermethrin, deltamethrin, esfenvalerate, etofenprox, fenpropathrin, fenvalerate, imiprothrin, lambda-cyhalothrin, permethrin, prallethrin, pyrethrin I and II, resmethrin, silafluofen, tau-fluvalinate, tefluthrin, tetramethrin, tralomethrin, transfluthrin and profluthrin, dimefluthrin;
preferably bifenthrin, cyfluthrin, cyphenothrin, cypermethrin, alpha-cypermethrin, beta-cypermethrin, zeta-cypermethrin, deltamethrin, etofenprox, fenpropathrin, fenvalerate, imiprothrin, lambda-cyhalothrin, permethrin, prallethrin, pyrethrin I and II, resmethrin, tefluthrin, tetramethrin, tralomethrin, transfluthrin and profluthrin, dimefluthrin;
B21) growth regulators selected from a) chitin synthesis inhibitors that are selected from the benzoylureas chlorfluazuron, diflubenzuron, flucycloxuron, flufenoxuron, hexaflumuron, lufenuron, novaluron, teflubenzuron, triflumuron; buprofezin, diofenolan, hexythiazox, etoxazole and clofentazine; b) ecdysone antagonists that are selceted from halofenozide, methoxyfenozide, tebufenozide and azadirachtin; c) juvenoids that are selected from pyriproxyfen, methoprene and fenoxycarb and d) lipid biosynthesis inhibitors that are selected from spirodiclofen, spiromesifen and spirotetramat;
preferably flufenoxuron, hexaflumuron, teflubenzuron, triflumuron; azadirachtin, methoprene and fenoxycarb, spirodiclofen, spiromesifen and spirotetramat;
B22) nicotinic receptor agonists/antagonists compounds selected from clothianidin, dinotefuran, imidacloprid, thiamethoxam, nitenpyram, acetamiprid, thiacloprid, AKD1022, bensultap, cartap hydrochloride;
preferably clothianidin, dinotefuran, imidacloprid, thiamethoxam, nitenpyram, acetamiprid, thiacloprid, AKD1022B23)
GABA antagonist compoundsselected from acetoprole, endosulfan, ethiprole, fipronil, vaniliprole, pyrafluprole, pyriprole and the phenylpyrazole compound of formula Γ1

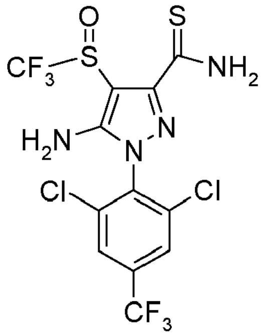

($\Gamma^1$)

,

preferably acetoprole, endosulfan, ethiprole, fipronil, vaniliprole, pyrafluprole, pyriprole and the phenylpyrazole
B24) METI I compounds selected from fenazaquin, pyridaben, tebufenpyrad, tolfenpyrad and flufenerim;
preferably fenazaquin, pyridaben, tebufenpyrad, tolfenpyrad and flufenerim;
B25) METI II and III compounds selected from acequinocyl, fluacyprim and hydramethylnon;
preferably acequinocyl, fluacyprim and hydramethylnon;
B26) chlorfenapyr;;
B27) oxidative phosphorylation inhibitor compounds selected from cyhexatin, diafenthiuron, fenbutatin oxide and propargite;
preferably diafenthiuron, fenbutatin oxid
B28) cyromazine;
B29) piperonyl butoxide
B30) indoxacarb
B31) a compound selected from benclothiaz, bifenazate, cartap, flonicamid, pyridalyl, pymetrozine, sulfur, thiocyclam, flubendiamide, cyenopyrafen, flupyrazofos, cyflumetofen, amidoflumet, pyrifluquinazon;
preferably benclothiaz, bifenazate, cartap, flonicamid, pyridalyl, pymetrozine, sulfur, thiocyclam, flubendiamide, cyenopyrafen, flupyrazofos, cyflumetofen, amidoflumet
B.32) Anthranilamides: chloranthraniliprole, the compound of formula $\Gamma^2$

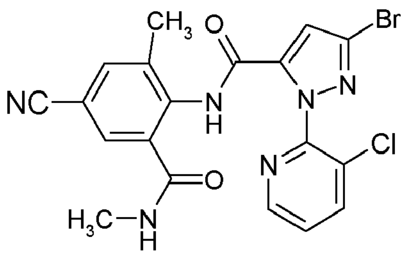

($\Gamma^2$)

;

B33) Acibenzolar-S-methyl;
B34) Plant bioregulators: trinexapac-ethyl, prohexadione-calcium, chlormequat chloride, mepiquat chloride, 16,17-dihydro gibberellin $A_5$, 1-methylcyclopropene, 2,5-norbornadiene, 3-amino-1,2,4-triazole;
preferably trinexapac-ethyl, prohexadione-calcium, chlormequat chloride, mepiquat chloride, 1-methylcyclopropene,
B35) neonicotinoid.

In a further embodiment of the invention the active ingredient (B) is an active compound that inhibits the mitochondrial respiration (breathing) chain at the level of the b/$c_1$ complex.

In a further embodiment of the invention the active ingredient (B) is a strobilurin selected from pyraclostrobin, kresoxim-methyl, dimoxystrobin, 2-(ortho-((2,5-Dimethylphenyl-oxymethylene)phenyl)-3-methoxy-acrylic acid methyl ester, picoxystrobin, trifloxystrobin, enestroburin, orysastrobin, metominostrobin, azoxystrobin and fluoxastrobin.

In a further embodiment of the invention the active ingredient (B) is selected from (*EZ*)-3-(2-chloro-1,3-thiazol-5-ylmethyl)-5-methyl-1,3,5-oxadiazinan-4-ylidene(nitro)amine (thiamethoxam), 5-amino-1-(2,6-dichloro-α,α,α-trifluoro-*p*-tolyl)-4-trifluoromethylsulfinylpyrazole-3-carbonitrile (fipronil) and (*EZ*)-1-(6-chloro-3-pyridylmethyl)-*N*-nitroimidazolidin-2-ylideneamine (imidacloprid).

In a further embodiment of the invention the active ingredient (B) is a plant bioregulator.

In one embodiment of the invention the plant bioregulator is selected from the group consisting of:

i) Plant bioregulators of the acylcyclohexanedione-type known to induce defence mechanisms against bacterial and fungal pathogens and against insect pests are: prohexadione and trinexapac, as free acids, esters (C1-C3) or salts (in particular: trinexapac-ethyl and prohexadione-calcium).
ii) Plant bioregulators known to reduce shoot length and leaf surface and to increase root growth, thereby diminishing the vulnerability of plants to abiotic stress (e.g. lodging as a result of wind and severe rainfall; dehydration as a result of water shortage and high evaporative demand; cell, tissue or whole-plant death as a result of too high or too low temperatures; root anoxia as a result of intense rainfall or flooding) are: (i) Quaternary ammonium compounds, in particular chlormequat and mepiquat as chlorides, borates, sulfates, phosphates or other agriculturally useful salts; (ii) compounds with a nitrogen-containing heterocycle, in particular paclobutrazol, uniconazole-P, metconazole, tebuconazole, ancymidol, flurprimidol, inabenfide, tetcyclacis; (iii) acylcyclohexanediones, in particular prohexadione and trinexapac, as free acids, esters (C1-C3) or salts (in particular: trinexapac-ethyl and prohexadione-calcium) (iiii) derivatives of 16, 17-dihydro gibberellin A5.
iii) Plant bioregulators diminishing the responsiveness of plants to abiotic and biotic stresses (thereby avoiding yield-reducing over-reactions), in particular ethylene modulators are:
ethylene biosynthesis inhibitors which inhibit the conversion of S-adenosyl-L-methionine into 1-aminocyclopropane-1-carboxylic acid (ACC), such as derivatives of vinylglycine, hydroxylamines, oxime ether derivatives;
ethylene biosynthesis inhibitors which block the conversion of ACC into ethylene, selected from the group consisting of: Co++ or Ni++ ions in plant-available form; phenolic radical scavengers such as n-propyl gallate; polyamines, such as putrescine, spermine or sper-midine; structural analogs of ACC, such as α-aminoisobutyric acid or L-aminocyclopropene-1-carboxylic acid; salicylic acid or acibenzolar-S-methyl; structural analogs of ascorbic acid which act as inhibitors of ACC oxidase, such as prohexadione-Ca or trinexapac-ethyl; and triazolyl compounds such as paclobutrazole or uniconazole as inhibitors of cytochrome P-450-dependent monooxygenases whose main action is to block the metabolism of gibberel-lins;
inhibitors of the action of ethylene selected from the group consisting of: structural analogs of ethylene such as 1-methylcyclopropene or 2,5-norbornadiene and 3-amino-1,2,4-triazole or Ag++ ions in a weight ratio of I to II of from 20 : 1 to 0.05 : 1

Plant bioregulators can also be involved in endogenous defense mechanisms against biotic (e.g. jasmonic acid and methyl jasmonate) and abiotic stress (e.g. abscisic acid and also its synthetic analogs).
In a further embodiment of the invention the chemical composition comprises at least one active ingredient (B) and a component (A) which is a glucan or a glucan derivative.
Component (A) according to the present invention is a glucan or a glucan derivative. "Glucans" are a class of homopolysaccharides which contain glucose units as monomer building blocks, wherein the glucose molecule may be linked by alpha- or beta-glycosidic bonds and may be branched or straight chain. Specific examples for suitable glucans according to the present invention are beta-glucans, more specifically beta -1,3-glucans such as, for example, laminarin and curdlan. Beta-1-3 glucans, for example, have various origins. They can be extracted from bacteria (for example *Alcaligenes faecalis* which leads to curdlan), fungi, yeasts and from various plants, particularly from algae and cereals.
"Glucan derivatives" according to the present invention are glucans that are modified, for example by sulfatation or by hydrolysis. Specific examples for suitable glucan derivatives are sulfated glucans, particularly sulfated beta -glucans, more specifically beta -1,3-glucans such as sulfated laminarin or sulfated curdlan. Furthermore, also laminaribiose, cellobiose, nigerose, laminaritriose, laminaritetrose and laminaripentose are suitable glucan derivatives according to the present invention.
Further derivates of glucans are described in US 6,979,665, Alban S. et al., Synthesis of laminarin sulfates with anticoagulant activity, Arzneim. Forsch. (1992) 42; 1005-1008; US 6,979,665; US 6,387,847; .US 6,303,587; US 6,303,587; Black et al., Appl. Chem. (1951), volume 1, pages 505 to 517; US 5,750,472 and references cited therein), US 5,750,472, FR 92 08387; which are included by reference.
According to one preferred embodiment of the present invention, component (A) is a beta - glucan, in particular a beta -1,3-glucan. Specifically, component (A) is laminarin or curdlan. According to another preferred embodiment, component (A) is selected from sulfated glucan, Laminaribiose, Cellobiose, Nigerose, Laminaritriose, Laminaritetrose and Laminaripentose. The active compounds of groups B1) to B17) that can be used as the active ingredient (B), their preparation and their action against harmful fungi are generally known; they are commercially available. In most of the cases, they can also be found in The Pesticide Manual, 13th Edition, British Crop Protection Council (2003) among other publications.

- benalaxyl, methyl *N*-(phenylacetyl)-*N*-(2,6-xylyl)-DL-alaninate (DE 29 03 612),

- boscalid, 2-chloro-*N*-(4'-chlorbiphenyl-2-yl)nicotinamide (EP-A 545 099);

- carboxin, 5,6-dihydro-2-methyl-*N*-phenyl-1,4-oxathiin-3-carboxamide (US 3 249 499),

- mepronil, 3'-isopropoxy-o-toluanilide (US 3 937 840),

- fenhexamid, N-(2,3-dichloro-4-hydroxyphenyl)-1-methylcyclohexanecarboxamide (Proc. Br. Crop Prot. Conf. - Pests Dis., 1998, Vol. 2, p. 327);

- flutolanil, α,α,α-trifluoro-3'-isopropoxy-*o*-toluanilide (JP 1104514),

- furametpyr, 5-chloro-*N*-(1,3-dihydro-1,1,3-trimethyl-4-isobenzofuranyl)-1,3-dimethyl-1*H*-pyrazole-4-carboxamide [CAS RN 123572-88-3],

- metalaxyl, methyl *N*-(methoxyacetyl)-*N*-(2,6-xylyl)-DL-alaninate (GB 15 00 581);

- ofurace, (*RS*)-α-(2-chloro-*N*-2,6-xylylacetamido)-γ-butyrolactone [CAS RN 58810-48-3];

- oxadixyl; *N*-(2,6-dimethylphenyl)-2-methoxy-*N*-(2-oxo-3-oxazolidinyl)acetamide (GB 20 58 059),

- oxycarboxin, 5,6-dihydro-2-methyl-1,4-oxathiin-3-carboxanilide 4,4-dioxide (US 3 399 214),

- penthiopyrad, *N*-[2-(1,3-dimethylbutyl)-3-thienyl]-1-methyl-3-(trifluoromethyl)-1*H*-pyrazole-4-carboxamide (JP 10130268),

- thifluzamide, *N*-[2,6-dibromo-4-(trifluoromethoxy)phenyl]-2-methyl-4-(trifluoromethyl)-5-thiazolecarboxamide;

- tiadinil, 3'-chloro-4,4'-dimethyl-1,2,3-thiadiazole-5-carboxanilide [CAS RN 223580-51-6],

- dimethomorph, 3-(4-chlorophenyl)-3-(3,4-dimethoxyphenyl)-1-morpholin-4-ylpropenone (EP-A 120 321);

- flumorph, 3-(4-fluorophenyl)-3-(3,4-dimethoxyphenyl)-1-morpholin-4-ylpropenone (EP-A 860 438);

- flumetover, 2-(3,4-dimethoxyphenyl)-*N*-ethyl-a,a,a-trifluoro-*N*-methyl-*p*-toluamide [AGROW No. 243, 22 (1995)],

- fluopicolide (picobenzamid), 2,6-dichloro-N-(3-chloro-5-trifluoromethylpyridin-2-ylmethyl)benzamide (WO 99/42447);

- zoxamide, (*RS*)-3,5-dichloro-N-(3-chloro-1-ethyl-1-methyl-2-oxopropyl)-*p*-toluamide [CAS RN 156052-68-5];

- carpropamid, 2,2-dichloro-*N*-[1-(4-chlorophenyl)ethyl]-1-ethyl-3-methylcyclopropane-carboxamide [CAS RN 104030-54-8],

- diclocymet, 2-cyano-*N*-[(1*R*)-1-(2,4-dichlorophenyl)ethyl]-3,3-dimethyl butanamide;

- mandipropamid, (*RS*)-2-(4-chlorophenyl)-*N*-[3-methoxy-4-(prop-2-ynyloxy)phenethyl]-2-(prop-2-ynyloxy)acetamide [CAS RN 374726-62-2];

- bitertanole, prop-2-'-biphenyl]-4-yloxy)-2-ynyloxy)phenethyl]-2-*H*-1,2,4-triazole-1-ethanol (DE 23 24 020),

- bromuconazole, 1-[[4-bromo-2-(2,4-dichlorophenyl)tetrahydro-2-furanyl]methyl]-1*H*-1,2,4-triazole (Proc. 1990 Br. Crop. Prot. Conf. - Pests Dis. Vol. 1, p. 459);

- cyproconazole, 2-(4-chlorophenyl)-3-cyclopropyl-1-[1,2,4]triazol-1-ylbutan-2-ol (US 4 664 696);

- difenoconazole, 1-{2-[2-chloro-4-(4-chlorophenoxy)phenyl]-4-methyl-[1,3]dioxolan-2-ylmethyl}-1H-[1,2,4]triazole (GB-A 2 098 607);

- diniconazole, (β*E*)-β-[(2,4-dichlorophenyl)methylene]-α-(1,1-dimethylethyl)-1*H*-1,2,4-triazole-1-ethanol (Noyaku Kagaku, 1983, Vol. 8, p. 575);

- enilconazole (imazalil), 1-[2-(2,4-dichlorphenyl)-2-(2-propenyloxy)ethyl]-1*H*-imidazole (Fruits, 1973, Vol. 28, p. 545);

- epoxiconazole, (2*RS*,3*SR*)-1-[3-(2-chlorophenyl)-2,3-epoxy-2-(4-fluorophenyl)propyl]-1*H*-1,2,4-triazole (EP-A 196 038);

- fenbuconazole, α-[2-(4-chlorophenyl)ethyl]-α-phenyl-1*H*-1,2,4-triazole-1-propanenitrile (Proc. 1988 Br. Crop Prot. Conf. - Pests Dis. Vol. 1, p. 33);

- flusilazole, 1-{[bis-(4-fluorophenyl)methylsilanyl]methyl}-1H-[1,2,4]triazole (Proc. Br. Crop Prot. Conf.-Pests Dis., 1, 413 (1984));

- fluquinconazole, 3-(2,4-dichlorophenyl)-6-fluoro-2-[1,2,4]-triazol-1-yl-3H-quinazolin-4-one (Proc. Br. Crop Prot. Conf.-Pests Dis., 5-3, 411 (1992));

- flutriafol, α-(2-fluorophenyl)-α-(4-fluorophenyl)-1*H*-1,2,4-triazole-1-ethanol (EP 15 756);

- hexaconazole, 2-(2,4-dichlorophenyl)-1-[1,2,4]triazol-1-ylhexan-2-ol (CAS RN 79983-71-4);

- imibenconazole, (4-chlorophenyl)methyl N-(2,4-dichlorophenyl)-1*H*-1,2,4-triazole-1-ethan-imidothioate ((Proc. 1988 Br. Crop Prot. Conf. - Pests Dis. Vol. 2, p. 519),

- ipconazole, 2-[(4-chlorophenyl)methyl]-5-(1-methylethyl)-1-(1*H*-1,2,4-triazol-1-yl-methyl)cyclopentanol (EP 267 778),

- metconazole, 5-(4-chlorobenzyl)-2,2-dimethyl-1-[1,2,4]triazol-1-ylmethylcyclopentanol (GB 857 383);

- myclobutanil, 2-(4-chlorophenyl)-2-[1,2,4]triazol-1-ylmethylpentanenitrile (CAS RN 88671-89-0);

- penconazole, 1-[2-(2,4-dichlorophenyl)pentyl]-1 H-[1,2,4]triazole (Pesticide Manual, 12th Ed. (2000), p.712);

- propiconazole, 1-[[2-(2,4-dichlorophenyl)-4-propyl-1,3-dioxolan-2-yl]methyl]-1*H*-1,2,4-triazole (BE 835 579);

- prothioconazole, 2-[2-(1-chlorocyclopropyl)-3-(2-chlorophenyl)-2-hydroxypropyl]-2,4-dihydro-[1,2,4]triazole-3-thione (WO 96/16048);

- simeconazole, α-(4-fluorophenyl)-α-[(trimethylsilyl)methyl]-1*H*-1,2,4-triazole-1-ethanol [CAS RN 149508-90-7],

- tebuconazole, 1-(4-chlorophenyl)-4,4-dimethyl-3-[1,2,4]triazol-1-ylmethylpentan-3-ol (EP-A 40 345);

- tetraconazole, 1-[2-(2,4-dichlorophenyl)-3-(1,1,2,2-tetrafluoroethoxy)propyl]-1*H*-1,2,4-triazole (EP 234 242);

- triadimenol, β-(4-chlorophenoxy)-α-(1,1-dimethylethyl)-1*H*-1,2,4-triazole-1-ethanol;

- triadimefon, 1-(4-chlorophenoxy)-3,3-dimethyl-1-(1*H*-1,2,4-triazol-1-yl)-2-butanone;

- triticonazole, (5E)-5-[(4-chlorophenyl)methylene]-2,2-dimethyl-1-(1*H*-1,2,4-triazol-1-ylmethyl)cyclopentanol (FR 26 41 277);

- cyazofamid, 4-chloro-2-cyano-*N,N*-dimethyl-5-(4-methylphenyl)-1*H*-imidazole-1-sulfonamide (CAS RN 120116-88-3],

- pefurazoate, 4-pentenyl 2-[(2-furanylmethyl)(1*H*-imidazol-1-ylcarbonyl)amino]butanoate [CAS RN 101903-30-4],

- prochloraz, N-{propyl-[2-(2,4,6-trchlorophenoxy)ethyl]}imidazole-1-carboxamide (US 3 991 071);

- triflumizole, (4-chloro-2-trifluoromethylphenyl)-(2-propoxy-1-[1,2,4]triazol-1-ylethylidene)amine (JP-A 79/119 462)

- benomyl, N-butyl-2-acetylaminobenzoimidazol-1-carboxamide (US 3 631 176);

- carbendazim, methyl (1H-benzoimidazol-2-yl)-carbamate (US 3 657 443);
- fuberidazole, 2-(2-furanyl)-1*H*-benzimidazole (DE 12 09 799),
- thiabendazole, 2-(1,3-thiazol-4-yl)benzimidazole (US 3 017 415),
- ethaboxam, *N*-(cyano-2-thienylmethyl)-4-ethyl-2-(ethylamino)-5-thiazolcarboxamide (EP-A 639 574),
- etridiazole,
- hymexazole, 5-methyl-1,2-oxazol-3-ol (JP 518249, JP 532202),
- fluazinam, 3-chloro-N-[3-chloro-2,6-dinitro-4-(trifluoromethyl)phenyl]-5-(trifluoromethyl)-2-pyridinamine (The Pesticide Manual, publ. The British Crop Protection Council, 10th ed. (1995), p. 474);
- pyrifenox, 1-(2,4-dichlorophenyl)-2-(3-pyridinyl)ethanone *O*-methyloxime (EP-A 49 854);
- bupirimate, 5-butyl-2-ethylamino-6-methylpyrimidin-4-yldimethylsulfamate [CAS RN 41483-43-6];
- cyprodinil, (4-cyclopropyl-6-methylpyrimidin-2-yl)phenylamine (EP-A 310 550);
- ferimzone, (Z)-2'-methylacetophenone 4,6-dimethylpyrimidin-2-ylhydrazone [CAS RN 89269-64-7];
- fenarimol, (4-chlorophenyl) (2-chlorophenyl) pyrimidin-5-ylmethanol (GB 12 18 623);
- mepanipyrim, (4-methyl-6-prop-1-ynylpyrimidin-2-yl)phenylamine (EP-A 224 339);
- nuarimol, alpha-(2-chlorophenyl)-alpha-(4-fluorophenyl)-5-pyrimidinemethanol (GB 12 18 623);
- pyrimethanil, 4,6-dimethylpyrimidin-2-ylphenylamine (DD-A 151 404);
- triforine, *N,N'*-{piperazine-1,4-diylbis[(trichloromethyl)methylene]}diformamide (DE 19 01 421);
- fludioxonil, 4-(2,2-difluorobenzo[1,3]dioxol-4-yl)-1H-pyrrole-3-carbonitrile (The Pesticide Manual, publ. The British Crop Protection Council, 10th ed. (1995), p. 482);
- fenpiclonil, 4-(2,3-dichlorophenyl)-1H-pyrrole-3-carbonitrile (Proc. 1988 Br. Crop Prot. Conf.-Pests Dis., Vol. 1, p. 65);
- aldimorph, 4-alkyl-2,5(or 2,6)-dimethylmorpholine, comprising 65-75% of 2,6-dimethylmorpholine and 25-35% of 2,5-dimethylmorpholine, comprising more than 85% of 4-dodecyl-2,5(or 2,6)-dimethylmorpholine, where "alkyl" may also include octyl, decyl, tetradecyl or hexadecyl and where the cis/trans ratio is 1:1;
- dodemorph, 4-cyclododecyl-2,6-dimethylmorpholine (DE 1198125);
- fenpropimorph, (*RS*)-*cis*-4-[3-(4-tert-butylphenyl)-2-methylpropyl]-2,6-dimethylmorpholine (DE 27 52 096);
- tridemorph, 2,6-dimethyl-4-tridecylmorpholine (DE 11 64 152);
- iprodione, N-isopropyl-3-(3,5-dichlorophenyl)-2,4-dioxoimidazolidine-1-carboxamide (GB 13 12 536);
- procymidone, *N*-(3,5-dichlorophenyl)-1,2-dimethylcyclopropane-1,2-dicarboximide (US 3 903 090);
- vinclozolin, 3-(3,5-dichlorophenyl)-5-methyl-5-vinyloxazolidine-2,4-dione (DE-OS 22 07 576);
- acibenzolar-S-methyl, methyl benzo[1,2,3]thiadiazole-7-carbothionate;
- anilazine, 4,6-dichloro-*N*-(2-chlorophenyl)-1,3,5-triazine-2-amine (US 2 720 480);

- captan, 2-trichloromethylsulfanyl-3a,4,7,7a-tetrahydroisoindole-1,3-dione (US 2 553 770);
- captafol, *N*-(1,1,2,2-tetrachloroethylthio)cyclohex-4-ene-1,2-dicarboximide (Phytopathology 52, S. 754 (1962));
- dazomet, 3,5-dimethyl-1,3,5-thiadiazinane-2-thione (Bull. Soc. Chim. Fr. Vol. 15, p. 891 (1897));
- diclomezine, 6-(3,5-dichlorophenyl)-*p*-tolyl)pyridazin-3(2*H*)-one (US 4,052,395);
- fenoxanil, *N*-(1-cyano-1,2-dimethylpropyl)-2-(2,4-dichlorophenoxy) propanamide (EP-A 262 393);
- folpet, 2-trichloromethylsulfanylisoindole-1,3-dione (US 2 553 770);
- fenpropidin, (*RS*)-1-[3-(4-tert-butylphenyl)-2-methylpropyl]piperidine (DE 27 52 096);
- famoxadone, (*RS*)-3-anilino-5-methyl-5-(4-phenoxyphenyl)-1,3-oxazolidine-2,4-dione [CAS RN 131807-57-3];
- fenamidone, (*S*)-1-anilino-4-methyl-2-methylthio-4-phenylimidazolin-5-one [CAS RN 161326-34-7];
- octhilinone,
- probenazole, 3-allyloxy-1,2-benzothiazole 1,1-dioxide;
- proquinazid, 6-iodo-2-propoxy-3-propylquinazolin-4(3*H*)-one (WO 97/48684);
- pyroquilon, 1,2,5,6-tetrahydropyrrolo[3,2,1-*ij*]quinolin-4-on (GB 139 43 373)
- quinoxyfen, 5,7-dichloro-4-(4-fluorophenoxy)quinoline (US 5 240 940);
- tricyclazole, 5-methyl-1,2,4-triazolo[3,4-*b*]benzothiazole (GB 14 19 121);
- ferbam, iron(3+) dimethyldithiocarbamate (US 1 972 961);
- mancozeb, manganese ethylenebis(dithiocarbanate) zinc complex (US 3 379 610);
- maneb, manganese ethylenebis(dithiocarbamate) (US 2 504 404);
- metiram, zinc ammoniate ethylenebis(dithiocarbamate) (US 3 248 400);
- metam, methyldithiocarbaminic acid (US 2 791 605);
- propineb, zinc propylenebis(dithiocarbamate) polymer (BE 611 960);
- thiram, bis(dimethylthiocarbamoyl) disulfide (DE 642 532);
- zineb, zinc ethylenebis(dithiocarbamate) (US 2 457 674);
- ziram, dimethyldithiocarbamate [CAS RN 137-30-4];
- diethofencarb, isopropyl 3,4-diethoxycarbanilate (EP-A 78 663);
- flubenthiavalicarb (benthiavalicarb), isopropyl {(S)-1-[(1R)-1-(6-fluorobenzothiazol-2-yl)-ethylcarbamoyl]-2-methylpropyl}carbamate (JP-A 09/323 984);
- iprovalicarb, isopropyl [(1S)-2-methyl-1-(1-p-tolylethylcarbamoyl)propyl]carbamate (EP-A 472 996);
- propamocarb, propyl 3-(dimethylamino)propylcarbamate (DE 16 43 040);
- dodine, (2,4-dichlorophenoxy)acetic acid (US 2 867 562);

- iminoctadine, bis(8-guanidinooctyl)amine (GB 11 14 155);
- guazatine, mixture of products from the amidation of iminodi(octamethylene)diamine, mainly iminoctadine [CAS RN 108173-90-6];
- kasugamycin, 1L-1,3,4/2,5,6-1-deoxy-2,3,4,5,6-pentahydroxycyclohexyl 2-amino-2,3,4,6-tetradeoxy-4-(alpha-iminoglycino)-alpha-D-arabino-hexopyranoside [CAS RN 6980-18-3];
- polyoxine, 5-(2-amino-5-O-carbamoyl-2-deoxy-L-xylonamido)-1-(5-carboxy-1,2,3,4-tetrahydro-2,4-dioxopyrimidin-1-yl)-1,5-dideoxy-beta-D-allofuranuronic acid and the salts thereof [CAS RN 22976-86-9];
- streptomycin, *O*-2-deoxy-2-methylamino-alpha-L-glucopyranosyl-(1□2)-*O*-5-deoxy-3-C-formyl-alpha-L-lyxofuranosyl-(1□4)$N^1$,$N^3$-diamidino-D-streptamine (J. Am. Chem. Soc. 69, S.1234 (1947));
- validamycin A,- fentin acetate, triphenyltin acetate (US 3 499 086);
- isoprothiolan, diisopropyl 1,3-dithiolan-2-ylidenemalonat (Proc. Insectic. Fungic. Conf. 8. Bd. 2, S. 715 (1975));
- dithianon, 5,10-Dioxo-5,10-dihydronaphtho[2,3-b][1,4]dithiin-2,3-dicarbonitril (GB 857 383);
- edifenphos, *O*-ethyl *S,S*-diphenyl phosphorodithioate (DE-A 14 93 736);
- fosetyl, fosetyl-aluminum, (aluminum) ethylphosphonate (FR 22 54 276);
- iprobenfos, *S*-benzyl *O,O*-diisopropyl phosphorothioate (Jpn. Pesticide Inf., No. 2, S. 11 (1970));
- pyrazophos, ethyl 2-diethoxyphosphinothioyloxy-5-methylpyrazolo[1,5-a]pyrimidine-6-carboxylate (DE 15 45 790);
- tolclofos-methyl, *O*-2,6-dichloro-*p*-tolyl *O*,*O*-dimethyl phosphorothioate (GB 14 67 561);
- thiophanate-methyl, 1,2-phenylenebis(iminocarbonothioyl)bis(dimethylcarbamate) (DE-OS 19 30 540);
- chlorothalonil, 2,4,5,6-tetrachloroisophthalonitrile (US 3 290 353);
- dichlofluanid, N-dichlorofluoromethylthio-*N',N'*-dimethyl-*N*-phenylsulfamide (DE 11 93 498);
- tolylfluanid, *N*-dichlorofluoromethylthio-*N',N'*-dimethyl-*N*-*p*-tolylsulfamide (DE 11 93 498);
- flusulfamide, 2',4-dichloro-α,α,α-trifluoro-4'-nitro-*m*-toluenesulfanilide (EP-A 199 433);
- phthalide (DE 16 43 347);
- hexachlorobenzene (C. R. Seances Acad. Agric. Fr., Vol. 31, p. 24 (1945));
- pencycuron, 1-(4-chlorobenzyl)-1-cyclopentyl-3-phenylurea (DE 27 32 257);
- quintozene, pentachloronitrobenzene (DE 682 048);
- binapacryl, (*RS*)-2-*sec*-Butyl-4,6-dinitrophenyl 3-methylcrotonat [CAS RN 485-31-4];
- dinocap, mixture of 2,6-dinitro-4-octylphenylcrotonate and 2,4-dinitro-6-octylphenylcrotonate, wherein "octyl" is a mixture of 1-methylheptyl, 1-ethylhexyl and 1-propylpentyl (US 2,526,660);
- dinobuton, (*RS*)-2-*sec*-Butyl-4,6-dinitrophenyl isopropyl carbonat [CAS RN 973-21-7];
- Bordeaux composition, mixture of $CuS0_4$ x $3Cu(OH)_2$ x $3CaSO_4$ [CAS RN 8011-63-0]
- copper acetate, $Cu(OCOCH_3)_2$ [CAS RN 8011-63-0];

- copper oxychloride, $Cu_2Cl(OH)_3$ [CAS RN 1332-40-7];
- basic copper sulfate, $CuSO_4$ [CAS RN 1344-73-6];
- spiroxamine, (8-tert-butyl-1,4-dioxaspiro[4.5]dec-2-yl)diethylamine (EP-A 281 842).
- cyflufenamid, (*Z*)-*N*-[α-(cyclopropylmethoxyimino)-2,3-difluoro-6-(trifluoromethyl)benzyl]-2-phenylacetamide (WO 96/19442);
- cymoxanil, 1-(2-cyano-2-methoxyiminoacetyl)-3-ethylurea (US 3 957 847);
- metrafenone, 3'-bromo-2,3,4,6'-tetramethoxy-2',6-dimethylbenzophenone (US 5 945 567);

The compounds are named according to IUPAC, their preparation and their fungicidal actions are likewise known:

N-(4'-bromobiphenyl-2-yl)-4-difluoromethyl-2-methylthiazole-5-carboxamide, N-(4'-trifluoromethylbiphenyl-2-yl)-4-difluoromethyl-2-methylthiazole-5-carboxamide, N-(4'-chloro-3'-fluorobiphenyl-2-yl)-4-difluoromethyl-2-methylthiazole-5-carboxamide, N-(3',4'-dichloro-4-fluorobiphenyl-2-yl)-3-difluoromethyl-1-methylpyrazole-4-carboxamide (WO 03/066609), 3,4-dichloro-isothiazol-5-carboxylic acid (2-cyanophenyl) amide (WO 99/24413);

N-(2-(4-[3-(4-Chlor-phenyl)-prop-2-ynyloxy]-3-methoxy-phenyl)-ethyl)-2-methansulfonylamino-3-methyl-butyramid, N-(2-(4-[3-(4-Chlor-phenyl)-prop-2-ynyloxy]-3-methoxy-phenyl)-ethyl)-2-ethanesulfonylamino-3-methyl-butyramid (WO 04/49804);

3-[5-(4-chlorophenyl)-2,3-dimethylisoxazolidin-3-yl]pyridine (EP-A 10 35 122); 2-butoxy-6-iodo-3-propylchromen-4-one (WO 03/14103),

methyl 3-(4-chlorophenyl)-3-(2-isopropoxycarbonylamino-3-methylbutyrylamino)propanoate (EP-A 1028125)

Furthermore, the commercially available compounds of groups B18) to B32) of the active ingredient (B) may be found in The Pesticide Manual, 13th Edition, British Crop Protection Council (2003) among other publications.

Thiamides of formula Γ (gamma)[1] and their preparation have been described in WO199828279.

Lepimection is known from Agro Project, PJB Publications Ltd, November 2004. Benclothiaz and its preparation have been described in EP-A1 454621. Methidathion and Paraoxon and their preparation have been described in Farm Chemicals Handbook, Volume 88, Meister Publishing Company, 2001. Acetoprole and its preparation have been described in WO199828277. Flupyrazofos has been described in Pesticide Science 54, 1988, p. 237-243 and in US 4822779. Pyrafluprole and its preparation have been described in JP 2002193709 and in W0200100614. Pyriprole and its preparation have been described in WO199845274 and in US 6335357. Amidoflumet and its preparation have been described in US 6221890 and in JP 21010907. Flufenerim and its preparation have been described in W02003007717 and in W02003007718. Cyflumetofen and its preparation have been described in W02004080180. Anthranilamides of formula Γ (gamma)[5] and their preparation have been described in W0200170671; W0200248137; W0200324222, W0200315518, W0200467528; W0200433468; and W02005118552.

According to one embodiment of the invention, the active ingredient (B) is an active compound that inhibits the mitochondrial breathing chain at the level of the $b/c_1$ complex.

Active compounds that inhibit the mitochondrial breathing chain at the level of the $b/c_1$ complex are known as fungicides from the literature [see for example Dechema-Monographien Bd. 129, 27-38, VCH Verlagsgemeinschaft Weinheim 1993; Natural Product Reports 1993, 565-574; Biochem. Soc. Trans. 22, 63S (1993)].

A particularly important class of active compounds that inhibit the mitochondrial breathing chain at the level of the $b/c_1$ complex are strobilurins. Strobilurins are generally known as fungicides since a long time and have, in some cases, also been described as insecticides (EP-A 178 826; EP-A 253 213; WO 93/15046; WO 95/18789; WO 95/21153; WO 95/21154; WO 95/24396; WO 96/01256; WO 97/15552; WO 97/27189). A further example of an active compound hat inhibits the mitochondrial breathing chain at the level of the $b/c_1$ complex is famoxadone (5-methyl-5-(4-phenoxyphenyl)-3-(phenylamino)-2,4-oxazolidinedione).

In a further preferred embodiment of the present invention, strobilurins are used as the active ingredient (B). According to the present invention, strobilurins which have proven particularly suitable are selected from

1) compounds of formula I

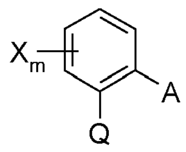

I

in which
X is halogen, $C_1$-$C_4$-alkyl or trifluoromethyl;
m is 0 or 1;
Q is $C(=CH-CH_3)-COOCH_3$, $C(=CH-OCH_3)-COOCH_3$, $C(=N-OCH_3)-CONHCH_3$, $C(=N-OCH_3)-COOCH_3$, $N(-OCH_3)-COOCH_3$, or a group Q1

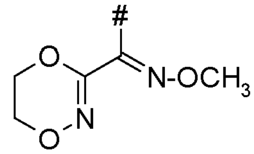

Q1

wherein # denotes the bond to the phenyl ring;

A is -O-B, $-CH_2O-B$, $-OCH_2-B$, $-CH_2S-B$, -CH=CH-B, -C≡C-B, $-CH_2O-N=C(R^1)-B$, $-CH_2S-N=C(R^1)-B$, $-CH_2O-N=C(R^1)-CH=CH-B$, or $-CH_2O-N=C(R^1)-C(R^2)=N-OR^3$, where

B is phenyl, naphthyl, 5-membered or 6-membered heteroaryl or 5-membered or 6-membered heterocyclyl, containing one, two or three N atoms and/or one O or S atom or one or two O and/or S atoms, the ring systems being unsubstituted or substituted by one, two or three radicals $R^a$:
$R^a$ is independently cyano, nitro, amino, aminocarbonyl, aminothiocarbonyl, halogen, $C_1$-$C_6$-alkyl, $C_1$-$C_6$-haloalkyl, $C_1$-$C_6$-alkylcarbonyl, $C_1$-$C_6$-alkylsulfonyl, $C_1$-$C_6$-alkyl-sulfinyl, $C_3$-$C_6$-cycloalkyl, $C_1$-$C_6$-alkoxy, $C_1$-$C_6$-haloalkoxy, $C_1$-$C_6$-alkyloxycarbonyl, $C_1$-$C_6$-alkylthio, $C_1$-$C_6$-alkylamino, di-$C_1$-$C_6$-alkylamino, $C_1$-$C_6$-alkylaminocarbonyl, di-$C_1$-$C_6$-alkylaminocarbonyl, $C_1$-$C_6$-alkylaminothiocarbonyl, di-$C_1$-$C_6$-alkylaminothiocarbonyl, $C_2$-$C_6$-alkenyl, $C_2$-$C_6$-alkenyloxy, phenyl, phenoxy, benzyl, benzyloxy, 5- or 6-membered heterocyclyl, 5- or 6-membered heteroaryl, 5- or 6-membered heteroaryloxy, $C(=NOR^a)-R^b$ or $OC(R^a)_2-C(R^b)=NOR^b$, the cyclic radicals, in turn, being unsubstituted or substituted by one, two or three radicals $R^b$:
$R^b$ is independently cyano, nitro, halogen, amino, aminocarbonyl, aminothiocarbonyl, $C_1$-$C_6$-alkyl, $C_1$-$C_6$-haloalkyl, $C_1$-$C_6$-alkylsulfonyl, $C_1$-$C_6$-alkylsulfinyl, $C_3$-$C_6$-cycloalkyl, $C_1$-$C_6$-alkoxy, $C_1$-$C_6$-haloalkoxy, $C_1$-$C_6$-alkoxycarbonyl, $C_1$-$C_6$-alkylthio, $C_1$-$C_6$-alkylamino, di-$C_1$-$C_6$-alkylamino, $C_1$-$C_6$-alkylaminocarbonyl, di-$C_1$-$C_6$-alkylaminocarbonyl, $C_1$-$C_6$-alkylaminothiocarbonyl, di-$C_i$-$C_6$-alkylaminothiocarbonyl, $C_2$-$C_6$-alkenyl, $C_2$-$C_6$-alkenyloxy, $C_3$-$C_6$-cycloalkyl, $C_3$-$C_6$-cycloalkenyl, phenyl, phenoxy, phenylthio, benzyl, benzyloxy, 5- or 6-membered heterocyclyl, 5- or 6-membered heteroaryl, 5- or 6-membered heteroaryloxy or $C(=NOR^A)-R^B$;
$R^A$, $R^B$ are independently hydrogen or $C_1$-$C_6$-alkyl;

$R^1$ is hydrogen, cyano, $C_1$-$C_4$-alkyl, $C_1$-$C_4$-haloalkyl, $C_3$-$C_6$-cycloalkyl, $C_1$-$C_4$-alkoxy, or $C_1$-$C_4$-alkylthio;

$R^2$ is phenyl, phenylcarbonyl, phenylsulfonyl, 5- or 6-membered heteroaryl, 5- or 6-membered heteroarylcarbonyl or 5- or 6-membered heteroarylsulfonyl, the ring systems being unsubstituted or substituted by one, two or three radicals $R^a$, $C_1$-$C_{10}$-alkyl, $C_3$-$C_6$-cycloalkyl, $C_2$-$C_{10}$-alkenyl, $C_2$-$C_{10}$-alkynyl, $C_1$-$C_{10}$-alkylcarbonyl, $C_2$-$C_{10}$-alkenylcarbonyl, $C_3$-$C_{10}$-alkynylcarbonyl, $C_1$-$C_{10}$-alkylsulfonyl, or $C(=NOR^a)-R^b$, the hydrocarbon radicals of these groups being unsubstituted or substituted by one, two or three radicals $R^c$:
$R^c$ is independently cyano, nitro, amino, aminocarbonyl, aminothiocarbonyl, halogen, $C_1$-$C_6$-alkyl, $C_1$-$C_6$-haloalkyl, $C_1$-$C_6$-alkylsulfonyl, $C_1$-$C_6$-alkylsulfinyl, $C_1$-$C_6$-alkoxy, $C_1$-$C_6$-haloalkoxy, $C_1$-$C_6$-alkoxycarbonyl, $C_1$-$C_6$-alkylthio, $C_1$-$C_6$-alkylamino, di-$C_1$-$C_6$-alkylamino, $C_1$-$C_6$-alkylaminocarbonyl, di-$C_1$-$C_6$-alkylaminocarbonyl, $C_1$-$C_6$-alkylaminothiocarbonyl, di-$C_1$-$C_6$-alkylaminothiocarbonyl, $C_2$-$C_6$-alkenyl, $C_2$-$C_6$-alkenyloxy, C3-C6-cycloalkyl, C3-C6-cycloalkyloxy, 5- or 6-membered heterocyclyl, 5- or 6-membered heterocyclyloxy, benzyl, benzyloxy, phenyl, phenoxy, phenylthio, 5- or 6-membered heteroaryl, 5- or 6-membered heteroaryloxy and heteroarylthio, it being possible for the cyclic groups, in turn, to be partially or fully halogenated

or to have attached to them one, two or three radicals Ra; and

$R^3$ is hydrogen, $C_1$-$C_6$-alkyl, $C_2$-$C_6$-alkenyl, $C_2$-$C_6$-alkynyl, the hydrocarbon radicals of these groups being unsubstituted or substituted by one, two or three radicals $R^c$; and

2) the strobilurins (2-chloro-5-[1-(3-methyl-benzyloxyimino)-ethyl]-benzyl)-carbamic acid methyl ester, (2-chloro-5-[1-(6-methyl-pyridine-2-ylmethoxyimino)-ethyl]-benzyl)-carbamic acid methyl ester and 2-(ortho-((2,5-dimethylphenyl-oxymethylene)phenyl)-3-methoxy-acrylic acid methyl ester.

Compounds of formula I are generally known as fungicides since a long time (see references above).

The publications cited above describe synthesis routes for the preparation of strobilurins used in the method according to the invention, the disclosure of which is hereby incorporated. Especially preferred according to the invention are strobilurins with the following meanings of the substituents, in each case alone or in combination, the disclosure of the publications cited being hereby incorporated.

In one preferred embodiment of the present invention, preferred strobilurins of formula I wherein Q is N(-$OCH_3$)-$COOCH_3$ are the compounds described in the publications WO 93/15046 and WO 96/01256.

In another preferred embodiment of the present invention, preferred strobilurins of formula I, wherein Q is C(=CH-$OCH_3$)-$COOCH_3$ are the compounds described in the publications EP-A 178 826 and EP-A 278 595.

In another preferred embodiment of the present invention, preferred strobilurins of formula I, wherein Q is C(=N-$OCH_3$)-$COOCH_3$ are the compounds described in the publications EP-A 253 213 and EP-A 254 426.

In another preferred embodiment of the present invention, preferred strobilurins of formula I, wherein Q is C(=N-$OCH_3$)-$CONHCH_3$ are the compounds described in the publications EP-A 398 692, EP-A 477 631 and EP-A 628 540.

In another preferred embodiment of the present invention, preferred strobilurins of formula I, wherein Q is C(=CH-$CH_3$)-$COOCH_3$ are the compounds described in the publications EP-A 280 185 and EP-A 350 691.

In another preferred embodiment of the present invention, preferred strobilurins of formula I, wherein Q is -$CH_2$O-N=C($R^1$)-B are the compounds described in the publications EP-A 460 575 and EP-A 463 488.

In another preferred embodiment of the present invention, preferred strobilurins of formula I, wherein A is -O-B are the compounds described in the publications EP-A 382 375 and EP-A 398 692.

In another preferred embodiment of the present invention, preferred strobilurins of formula I, wherein A is -$CH_2$O-N=C($R^1$)-C($R^2$)=N-$OR^3$ are the compounds described in the publications WO 95/18789, WO 95/21153, WO 95/21154, WO 97/05103 and WO 97/06133.

Especially preferred are the strobilurins of the formula I in which
Q is N(-$OCH_3$)-$COOCH_3$,
A is $CH_2$-O- and
B is 3-pyrazolyl or 1,2,4-triazolyl, where B has attached to it one or two substituents selected from the group of

- halogen, methyl and trifluoromethyl and
- phenyl and pyridyl, in particular 2-pyridyl, substituted by 1 to 3 radicals $R^b$.

These active ingredients are described by formula II,

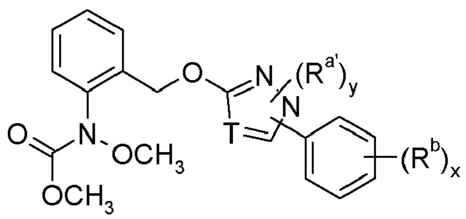

II

in which T is a carbon or a nitrogen atom, $R^{a'}$ is independently selected from halogen, methyl and trifluoromethyl, y is zero, 1 or 2, $R^b$ is as defined for formula I, x is zero, 1, 2, 3 or 4. More preferred active ingredients are those of formula II':

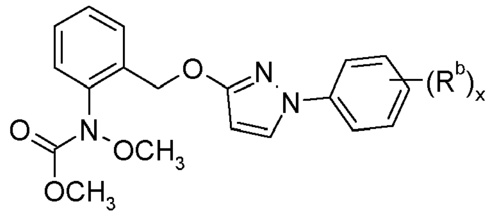

II'

in which $R^b$ is as defined for formula I.

According to another embodiment of the present invention, the active ingredient (B) is a carboxylic amide selected from the group B2).

According to another embodiment of the present invention, the active ingredient (B) is an azole selected from the group B3).

According to another embodiment of the present invention, the active ingredient (B) is a nitrogen-containing heterocyclic compound selected from the group B4).

According to another embodiment of the present invention, the active ingredient (B) is a carbamate or thiocarbamate selected from the group B5).

According to another embodiment of the present invention, the active ingredient (B) is a guanidine selected from the group B6).

According to another embodiment of the present invention, the active ingredient (B) is an antibiotic selected from the group B7).

According to another embodiment of the present invention, the active ingredient (B) is a fentin salt.

According to another embodiment of the present invention, the active ingredient (B) is isoprothiolan or dithianon.

According to another embodiment of the present invention, the active ingredient (B) is an organophosphorous compound selected from the group B10).

According to another embodiment of the present invention, the active ingredient (B) is an organo-chloro compound selected from the group B11).

According to another embodiment of the present invention, the active ingredient (B) is a nitrophenyl derivative selected from the group B12).

According to another embodiment of the present invention, the active ingredient (B) is an inorganic ingredient selected from the group B13).

According to another embodiment of the present invention, the active ingredient (B) is spiroxamine.

According to another embodiment of the present invention, the active ingredient (B) is cyflufenamide.

According to another embodiment of the present invention, the active ingredient (B) is cymoxanil.

According to another embodiment of the present invention, the active ingredient (B) is metrafenone.

According to still another preferred embodiment of the present ivention, the active ingredient (B) is (*EZ*)-3-(2-chloro-1,3-thiazol-5-ylmethyl)-5-methyl-1,3,5-oxadiazinan-4-ylidene(nitro)amine (thiamethoxam).

According to still another preferred embodiment of the present ivention, the active ingredient (B) is 5-amino-1-(2,6-dichloro-α,α,α-trifluoro-*p*-tolyl)-4-trifluoromethylsulfinylpyrazole-3-carbonitrile (fipronil).

According to still another preferred embodiment of the present ivnention, the active ingredient (B) is (*EZ*)-1-(6-chloro-3-pyridylmethyl)-*N*-nitroimidazolidin-2-ylideneamine (imidacloprit). According to still another preferred embodiment of the present ivnention, the active ingredient (B) is selected from the group consisting of:

Fungicide:

Anilide, preferably Isopyrazam, N-(2-bicyclopropyl-2-yl-phenyl)-3-difluoromethyl-1-methyl-1H-pyrazole-4-carboxylic, acid amide, N-(trans-2-bicyclopropyl-2-yl-phenyl)-3-difluoromethyl-1-methyl-1 H-pyrazole-4-carboxylic acid amide, N-(cis-2-bicyclopropyl-2-yl-phenyl)-3-difluoromethyl-1-methyl-1H-pyrazole-4-carboxylic acid amide, N-(2-(1,3-dimethyl-butyl)-phenyl)-1,3-dimethyl-5-fluoro-1H-pyrazole-4-carboxylic acid amide, 3-Difluoromethyl-1-methyl-1 H-pyrazole-4-carboxylic acid , (2',4'-difluorobiphenyl-2-yl)-amide, 3-Difluoromethyl-1-methyl-1 H-pyrazole-4-carboxylic acid , (2',4'-dichlorobiphenyl-2-yl)-amide, 3-Difluoromethyl-1-methyl-1H-pyrazole-4-carboxylic acid , (3',4'-difluorobiphenyl-2-yl)-amide, 3-Difluoromethyl-1-methyl-1 H-pyrazole-4-carboxylic acid , (3',4'-dichlorobiphenyl-2-yl)-amide, 3-Difluoromethyl-1-methyl-1H-pyrazole-4-carboxylic acid , (3',5'-difluorobiphenyl-2-yl)-amide, 3-Difluoromethyl-1-methyl-1H-pyrazole-4-carboxylic acid , (3',5'-dichlorobiphenyl-2-yl)-amide, 3-Difluoromethyl-1-methyl-1H-pyrazole-4-carboxylic acid , (3',4',5'-trifluorobiphenyl-2-yl)-amide

Fluopyram, 5-Amino-2-isopropyl-3-oxo-4-ortho-tolyl-2,3-dihydro-pyrazole-1-carbothioic acid S-allyl ester, N'-(4-(4-chloro-3-trifluoromethyl-phenoxy)-2,5-dimethyl-phenyl)-N-ethyl-N-methyl

formamidine, N'-(4-(4-fluoro-3-trifluoromethyl-phenoxy)-2,5-dimethyl-phenyl)-N-ethyl-N-methyl formamidine, N'-(2-methyl-5-trifluormethyl-4-(3-trimethylsilanyl-propoxy)-phenyl)-N-ethyl-N-methyl formamidine, N'-(5-difluormethyl-2-methyl-4-(3-trimethylsilanyl-propoxy)-phenyl)-N-ethyl-N-methyl formamidine,

According to still another preferred embodiment of the present ivnention, the active ingredient (B) is selected from the group consisting of: PGR's: abscisic acid, Amidochlor, Ancymidol, 6-Benzylaminopurine, Brassinolide, Butralin, Choline chloride, Cyclanilide, Daminozide, Dikegu-lac, Dimethipin, 2,6-Dimethylpuridine, Ethephon, Flumetralin, Flurprimidol, Fluthiacet, Forchlor-fenuron, Gibberellic acid, Inabenfide, indole-3-acetic acid, Maleic hydrazide, Mefluidide, naphthaleneacetic acid, N-6 benzyladenine, Paclobutrazol, Prohydrojasmon, Thidiazuron, Triapen-thenol, Tributyl phosphorotrithioate, 2,3,5-tri-iodobenzoic acid, Uniconazole,

According to still another preferred embodiment of the present ivnention, the active ingredient (B) is selected from the group consisting of: insecticides:acephate, chlorpyrifos, diazinon, dichlorvos, dimethoate, fenitrothion, methamidophos, methidathion, methyl-parathion, monocrotophos, phorate, profenofos, terbufos, aldicarb, carbaryl, carbofuran, carbosulfan, methomyl, thiodicarb, bifenthrin, cyfluthrin, cypermethrin, alpha-cypermethrin, zeta-cypermethrin, deltamethrin, esfenvalerate, lambda-cyhalothrin, permethrin, tefluthrin, diflubenzuron, flufenoxuron, lufenuron, teflubenzuron, spirotetramat; clothianidin, dinotefuran, imidacloprid, thiamethoxam, acetamiprid, thiacloprid; endosulfan, fipronil, abamectin, emamectin, spinosad, spinetoram, hydramethylnon; chlorfenapyr; fenbutatin oxide, indoxacarb, metaflumizone, flonicamid, flubendiamide, chlorantraniliprole, cyazypyr (HGW86), cyflumetofenmost preferred: acephate, chlorpyrifos, dimethoate, methamidophos, terbufos, aldicarb, carbofuran, bifenthrin, cypermethrin, alpha-cypermethrin, deltamethrin, lambda-cyhalothrin, tefluthrin, flufenoxuron, teflubenzuron, spirotetramat; clothianidin, imidacloprid, thiamethoxam, endosulfan, fipronil, abamectin, spinosad, spinetoram, hydramethylnon; chlorfenapyr; indoxacarb, metaflumizone, flubendiamide, chlorantraniliprole, cyazypyr (HGW86), cyflumetofen.

According to still another preferred embodiment of the present ivnention, the active ingredient (B) is selected from the group consisting of: fungicides:
azoxystrobin, Dimoxystrobin, Kresoxim-methyl, Orysastrobin, Pyraclostrobin, Trifloxystrobin, Bixafen, Boscalid, Isopyrazam, Metalaxyl, Penthiopyrad, 3-Difluoromethyl-1-methyl-1H-pyrazole-4-carboxylic acid (2',4',5'-trifluorobiphenyl-2-yl)-amide , N-(2-bicyclopropyl-2-yl-phenyl)-3-difluoromethyl-1-methyl-1 H-pyrazole-4-carboxylic acid amide, Dimethomorph, Fluopicolide, Difenoconazole, Epoxiconazole, Fluquinconazole, Flusilazole, Flutriafol, Metconazol, Myclobutanil, Propiconazole, Prothioconazole, Tebuconazole, Tetraconazole, Triticonazole, Prochloraz, Carbendazim , Fluazinam, Cyprodinil, Pyrimethanil, Fludioxonil, Dodemorph, Fenpropimorph, Tridemorph, Fenpropidin, Iprodione, Vinclozolin, Famoxadone, Probenazole, Captan, Folpet, 5-ethyl-6-octyl-[1,2,4]triazolo[1,5-a]pyrimidine-7-ylamine, Mancozeb, Maneb, Metiram, Thiram, Dithianon, Fosetyl, Fosetyl-aluminium, Chlorothalonil, Thiophanate Methyl, Cymoxanil, Metrafenone, Spiroxamine,most preferred: Azoxystrobin, Dimoxystrobin, Kresoxim-methyl, Orysastrobin, Pyraclostrobin, Trifloxystrobin, Bixafen, Boscalid, Isopyrazam, Metalaxyl, Penthiopyrad, 3-Difluoromethyl-1-methyl-1 H-pyrazole-4-carboxylic acid (2',4',5'-trifluoro-biphenyl-2-yl)-amide , Dimethomorph, Difenoconazole, Epoxiconazole, Fluquinconazole, Metconazol, Propiconazole, Prothioconazole, Tebuconazole, Triticonazole, Prochloraz, Carbendazim, Cyprodinil, Pyrimethanil, Fenpropimorph, Tridemorph, Iprodione, 5-ethyl-6-octyl-[1,2,4]triazolo[1,5-a]pyrimidine-7-ylamine, Mancozeb, Maneb, Metiram, Dithianon, Chlorothalonil, Thiophanate Methyl, Cymoxanil, Metrafenone.According to still another preferred embodiment of the present ivnention, the active ingredient (B) is selected from the group consisting of: N-(3',4',5'-trifluorobiphenyl-2-yl)-3-difluoromethyl-1-methyl-1H-pyrazole-4-carboxamide, N-[2-(4'-trifluoromethylthio)-biphenyl]-3-difluoromethyl-1-methyl-1H-pyrazole-4-carboxamide, N-[2-(1,3-dimethylbutyl)-phenyl]-1,3-dimethyl-5-fluoro-1H-pyrazole-4-carboxamide, N-(2-bicyclopropyl-2-yl-phenyl)-3-difluoromethyl-1-methyl-1H-pyrazole-4-carboxamide, N-(cis-2-bicyclopropyl-2-yl-phenyl)-3-difluoromethyl-1-methyl-1 H-pyrazole-4-carboxamide, N-(trans-2-bicyclopropyl-2-yl-phenyl)-3-difluoromethyl-1-methyl-1H-pyrazole-4-carboxamide, N-[1,2,3,4-tetrahydro-9-(1-methylethyl)-1,4-methanonaphthalen-5-yl]-3-(difluoromethyl)-1-methyl-1 H-pyrazole-4-carboxamide.

According to still another preferred embodiment of the present invention, the active ingredient (B) is selected from the group consisting of herbicides:
Acetochlor, Dimethenamid, Metolachlor, Metazachlor, Glyphosate, Glufosinate, Sulfosate, Clodinafop, Fenoxaprop, Fluazifop, Haloxyfop, Paraquat, Phenmedipham, Clethodim, Cycloxydim, Profoxydim, Sethoxydim, Tepraloxydim, Pendimethalin, Trifluralin, Acifluorfen, Bromoxynil, Imazamethabenz, Imazamox, Imazapic, Imazapyr, Imazaquin, Imazethapyr, 2,4-D, Chloridazon, Clopyralid, Fluroxypyr, Picloram, Picolinafen, Bensulfuron, Chlorimuron ethyl, Cyclosulfamuron, Iodosulfuron, Mesosulfuron, Metsulfuron-methyl, Nicosulfuron, Rimsulfuron, Triflusulfuron, Atrazine, Hexazinone, Diuron, Florasulam, Pyroxasulfone, Bentazone, Cinidon-ethly, Cinmethylin, Dicamba, Diflufenzopyr, Quinclorac, Quinmerac, Mesotrione, Saflufenacil, Topramezone;
most preferred:
Acetochlor, Dimethenamid, Metolachlor, Metazachlor, Glyphosate, Glufosinate, Sulfosate, Fenoxaprop, Paraquat, Cycloxydim, Profoxydim, Sethoxydim, Tepraloxydim, Pendimethalin, Acifluorfen, Imazamethabenz, Imazamox, Imazapic, Imazapyr, Imazaquin, Imazethapyr, 2,4-D, Chloridazon, Picloram, Picolinafen, Cyclosulfamuron, Triflusulfuron, Atrazine, Pyroxasulfone, Bentazone, Cinidon-ethly, Cinmethylin, Dicamba, Diflufenzopyr, Quinclorac, Quinmerac, Mesotrione,

Saflufenacil, Topramezone

In one embodiment a plant selected from the group of transgenic plants with at least one transgenic modification as compared to a corresponding wild-type plant with an increased or generated activities of a polypeptide with an activity as indicated in table B, column 5, line No 1, 5, 7,10, 11, 12, 16, 19, 20, 21, 23, 25, 27, 28, 30, 31, 33, 37, 39, 40, 42, 43, 91, 105, 107, 112, 150, 159, 164, 165, 166, 167, 168, 169, 170, 171, 172, 173, 174, 175, 176, 177, 178,182, 186, 187, 188, 189, 190, 194, 196, 203, 204, 205, 206, 207, 209, 210, 211, 213, respectively plants with an increased or generated activities of a polypeptide according to the sequence number as depicted in table B, column 3, line No 1, 5, 7,10, 11, 12, 16, 19, 20, 21, 23, 25, 27, 28, 30, 31, 33, 37, 39, 40, 42, 43, 91, 105, 107, 112, 150, 159, 164, 165, 166, 167, 168, 169, 170, 171, 172, 173, 174, 175, 176, 177, 178,182, 186, 187, 188, 189, 190, 194, 196, 203, 204, 205, 206, 207, 209, 210, 211, 213, respectively plants with an increased or generated expression of the gene as idepicted in table B, column 1, line No 1, 5, 7,10, 11, 12, 16, 19, 20, 21, 23, 25, 27, 28, 30, 31, 33, 37, 39, 40, 42, 43, 91, 105, 107, 112, 150, 159, 164, 165, 166, 167, 168, 169, 170, 171, 172, 173, 174, 175, 176, 177, 178,182, 186, 187, 188, 189, 190, 194, 196, 203, 204, 205, 206, 207, 209, 210, 211, 213;

in combination with the treatment with an effective amount of any of the active ingredients selected from the group consisting of

acephate, chlorpyrifos, diazinon, dichlorvos, dimethoate, fenitrothion, methamidophos, methidathion, methyl-parathion, monocrotophos, phorate, profenofos, terbufos, aldicarb, carbaryl, carbofuran, carbosulfan, methomyl, thiodicarb, bifenthrin, cyfluthrin, cypermethrin, alpha-cypermethrin, zeta-cypermethrin, deltamethrin, esfenvalerate, lambda-cyhalothrin, permethrin, tefluthrin, diflubenzuron, flufenoxuron, lufenuron, teflubenzuron, spirotetramat; clothianidin, dinotefuran, imidacloprid, thiamethoxam, acetamiprid, thiacloprid; endosulfan, fipronil, abamectin, emamectin, spinosad, spinetoram, hydramethylnon; chlorfenapyr; fenbutatin oxide, indoxacarb, metaflumizone, flonicamid, flubendiamide, chlorantraniliprole, cyazypyr (HGW86), cyflumetofen,

Azoxystrobin, Dimoxystrobin, Kresoxim-methyl, Orysastrobin, Pyraclostrobin, Trifloxystrobin, Bixafen, Boscalid, Isopyrazam, Metalaxyl, Penthiopyrad, 3-Difluoromethyl-1-methyl-1H-pyrazole-4-carboxylic acid (2',4',5'-trifluorobiphenyl-2-yl)-amide , N-(2-bicyclopropyl-2-yl-phenyl)-3-difluoromethyl-1-methyl-1 H-pyrazole-4-carboxylic acid amide, Dimethomorph, Fluopicolide, Difenoconazole, Epoxiconazole, Fluquinconazole, Flusilazole, Flutriafol, Metconazol, Myclobutanil, Propiconazole, Prothioconazole, Tebuconazole, Tetraconazole, Triticonazole, Prochloraz, Carbendazim , Fluazinam, Cyprodinil, Pyrimethanil, Fludioxonil, Dodemorph, Fenpropimorph, Tridemorph, Fenpropidin, Iprodione, Vinclozolin, Famoxadone, Probenazole, Captan, Folpet, 5-ethyl-6-octyl-[1,2,4]triazolo[1,5-a]pyrimidine-7-ylamine, Mancozeb, Maneb, Metiram, Thiram, Dithianon, Fosetyl, Fosetyl-aluminium, Chlorothalonil, Thiophanate Methyl, Cymoxanil, Metrafenone and Spiroxamine,

as depicted detailed in Table C.

In one embodiment of the invention the combination of the transgenic modification, e.g. the increased or generated activity in the plant and the active ingredient is depicted in table C.

In one embodiment 2, 3, 4, 5, 6, 7, 8, 9, 10 ore more of the above mentioned active ingredients are used simultaneously for the treatment.

In one embodiment a plant selected from the group of transgenic plants with at least one transgenic modification as compared to a corresponding wild-type plant with an increased or generated activities of a polypeptide with an activity as indicated in table B, column 5, line No 1, 5, 7, 10, 11, 12, 16, 19, 20, 21, 23, 25, 27, 28, 30, 31, 33, 37, 39, 40, 42, 43, 91, 105, 107, 112, 150, 159, 164, 165, 166, 167, 168, 169, 170, 171, 172, 173, 174, 175, 176, 177, 178,182, 186, 187, 188, 189, 190, 194, 196, 203, 204, 205, 206, 207, 209, 210, 211, 213, respectively plants with an increased or generated activities of a polypeptide according to the sequence number as depicted in table B, column 3, line No 1, 5, 7,10, 11, 12, 16, 19, 20, 21, 23, 25, 27, 28, 30, 31, 33, 37, 39, 40, 42, 43, 91, 105, 107, 112, 150, 159, 164, 165, 166, 167, 168, 169, 170, 171, 172, 173, 174, 175, 176, 177, 178,182, 186, 187, 188, 189, 190, 194, 196, 203, 204, 205, 206, 207, 209, 210, 211, 213, respectively plants with an increased or generated expression of the gene as idepicted in table B, column 1, line No 1, 5, 7,10, 11, 12, 16, 19, 20, 21, 23, 25, 27, 28, 30, 31, 33, 37, 39, 40, 42, 43, 91, 105, 107, 112, 150, 159, 164, 165, 166, 167, 168, 169, 170, 171, 172, 173, 174, 175, 176, 177, 178,182, 186, 187, 188, 189, 190, 194, 196, 203, 204, 205, 206, 207, 209, 210, 211, 213;

in combination with the treatment with an effective amount of any of the active ingredients selected from the group consisting of fipronil and pyraclostrobin.

If the component (A) is a sulfated glucan as defined above, the active ingredient (B) can also be the active ingredient maneb.

In another aspect, the present invention relates to a composition comprising an extract from seaweed and a pesticide. The seaweed extract is preferably obtained according to the methods as described in WO 93/06730, which is hereby incorporated by reference. Particularly, the extract is obtained from brown algae, wherein the brown algae is specifically from the *Phaeo-phyceae* type (in particular *Fucales* or *Laminariales*). In general, the extraction can be accomplished by basic hydrolysis of the respective seaweed in the presence of a reducing agent such as an alkali metal borhydride. Then, the resulting hydrolysate is neutralised to a pH of about 6 to 8, e.g. by the addition of a strong acid.

The product is filtrated and optionally diafil-trated or an electrodialysis may be carried out (cf. WO 93/06730).

The following list M of pesticides together with one or more of the above mentioned active ingredients (B) according to the invention can be used and potential synergistic effects might be produced, whereby the list is intended to illustrate the possible combinations, but not to impose any limitation:

M.1. Organo(thio)phosphates: acephate, azamethiphos, azinphos-ethyl, azinphos-methyl, chlorethoxyfos, chlorfenvinphos, chlormephos, chlorpyrifos, chlorpyrifos-methyl, coumaphos, cyanophos, demeton-S-methyl, diazinon, dichlorvos/ DDVP, dicrotophos, dimethoate, di-methylvinphos, disulfoton, EPN, ethion, ethoprophos, famphur, fenamiphos, fenitrothion, fenthion, flupyrazophos, fosthiazate, heptenophos, isoxathion, malathion, mecarbam, methamidophos, methidathion, mevinphos, monocrotophos, naled, omethoate, oxydemeton-methyl, parathion, parathion-methyl, phenthoate, phorate, phosalone, phosmet, phosphamidon, phoxim, pirimiphos-methyl, profenofos, propetamphos, prothiofos, pyraclofos, pyridaphenthion, quinalphos, sulfotep, tebupirimfos, temephos, terbufos, tetrachlorvinphos, thiometon, triazophos, trichlorfon, vamidothion;

M.2. Carbamates: aldicarb, alanycarb, bendiocarb, benfuracarb, butocarboxim, butoxycar-boxim, carbaryl, carbofuran, carbosulfan, ethiofencarb, fenobucarb, formetanate, furathiocarb, isoprocarb, methiocarb, methomyl, metolcarb, oxamyl, pirimicarb, propoxur, thiodicarb, thio-fanox, trimethacarb, XMC, xylylcarb, triazamate;

M.3. Pyrethroids: acrinathrin, allethrin, d-cis-trans allethrin, d-trans allethrin, bifenthrin, bioallethrin, bioallethrin S-cylclopentenyl, bioresmethrin, cycloprothrin, cyfluthrin, beta-, yfluthrin, cyhalothrin, lambda-cyhalothrin, gamma-cyhalothrin, cypermethrin, alpha-cypermethrin, beta-cypermethrin, theta-cypermethrin, zeta-cypermethrin, cyphenothrin, deltamethrin, empenthrin, esfenvalerate, etofenprox, fenpropathrin, fenvalerate, flucythrinate, flumethrin, tau-fluvalinate, halfenprox, imiprothrin, permethrin, phenothrin, prallethrin, resmethrin, RU 15525, silafluofen, tefluthrin, tetramethrin, tralomethrin, transfluthrin, ZXI 8901;

M.4. Juvenile hormone mimics: hydroprene, kinoprene, methoprene, fenoxycarb, pyriproxyfen;

M.5. Nicotinic receptor agonists/antagonists compounds: acetamiprid, bensultap, cartap hydrochloride, clothianidin, dinotefuran, imidacloprid, thiamethoxam, nitenpyram, nicotine, spinosad (allosteric agonist), thiacloprid, thiocyclam, thiosultap-sodium and AKD1022.

M.6. GABA gated chloride channel antagonist compounds: chlordane, endosulfan, gamma-HCH (lindane); acetoprole, ethiprole, fipronil, pyrafluprole, pyriprole, vaniliprole, the phenylpyrazole compound of formula $M^{6.1}$

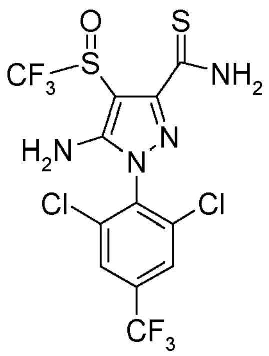

($M^{6.1}$)

,

M.7. Chloride channel activators: abamectin, emamectin benzoate, milbemectin, lepimectin;

M.8. METI I compounds: fenazaquin, fenpyroximate, pyrimidifen, pyridaben, tebufenpyrad, tolfenpyrad, flufenerim, rotenone;

M.9. METI II and III compounds: acequinocyl, fluacyprim, hydramethylnon;

M.10. Uncouplers of oxidative phosphorylation: chlorfenapyr, DNOC;

M.11. Inhibitors of oxidative phosphorylation: azocyclotin, cyhexatin, diafenthiuron, fenbutatin oxide, propargite,

tetradifon;

M.12. Moulting disruptors: cyromazine, chromafenozide, halofenozide, methoxyfenozide, tebufenozide;

M.13. Synergists: piperonyl butoxide, tribufos;

M.14. Sodium channel blocker compounds: indoxacarb, metaflumizone;

M.15. Fumigants: methyl bromide, chloropicrin sulfuryl fluoride;

M.16. Selective feeding blockers: crylotie, pymetrozine, flonicamid;

M.17. Mite growth inhibitors: clofentezine, hexythiazox, etoxazole;

M.18. Chitin synthesis inhibitors: buprofezin, bistrifluron, chlorfluazuron, diflubenzuron, flucycloxuron, flufenoxuron, hexaflumuron, lufenuron, novaluron, noviflumuron, teflubenzuron, triflumuron;

M.19. Lipid biosynthesis inhibitors: spirodiclofen, spiromesifen, spirotetramat;

M.20. octapaminergic agonsits: amitraz;

M.21. ryanodine receptor modulators: flubendiamide;

M.22. Various: aluminium phosphide, amidoflumet, benclothiaz, benzoximate, bifenazate, borax, bromopropylate, cyanide, cyenopyrafen, cyflumetofen, chinomethionate, dicofol, fluoroacetate, phosphine, pyridalyl, pyrifluquinazon, sulfur, organic sulfur compounds, tartar emetic; pyrimidinyl alkynylether compounds $M^{22.1}$ or thiadiazolyl alkynylether compounds $M^{22.2}$:

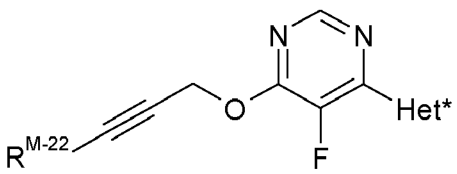 $M^{22.1}$ 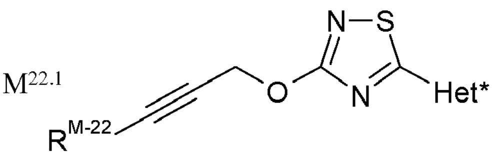 $M^{22.2}$

wherein $R^{M-22}$ is methyl or ethyl and Het* is 3,3-dimethylpyrrolidin-1-yl, 3-methylpiperidin-1-yl, 3,5-dimethylpiperidin-1-yl, 3-trifluormethylpiperidin-1-yl, hexahydroazepin-1-yl, 2,6-dimethylhexahydroazepin-1-yl or 2,6-dimethylmorpholin-4-yl.

M.23. N-R'-2,2-dihalo-1-R"cyclo-propanecarboxamide-2-(2,6-dichloro- alpha, alpha, alpha -tri-fluoro-p-tolyl)hydrazone or N-R'-2,2-di(R"')propionamide-2-(2,6-dichloro- alpha, alpha, alpha - trifluoro-p-tolyl)-hydrazone, wherein R' is methyl or ethyl, halo is chloro or bromo, R" is hydrogen or methyl and R"' is methyl or ethyl;

M.24. Anthranilamides: chloranthraniliprole, the compound of formula $M^{24}$ 1

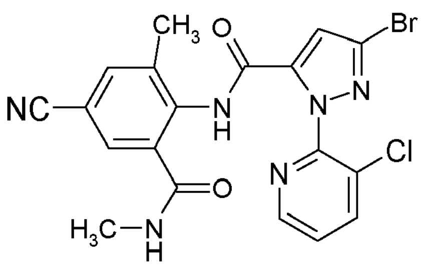 ($M^{24.1}$)

;

M.25. Malononitrile compounds: $CF_2HCF_2CF_2CF_2CH_2C(CN)_2CH_2CH_2CF_3$ (2-(2,2,3,3,4,4,5,5-octafluoropentyl)-2-(3,3,3-trifluoro-propyl)malononitrile), $CF_3(CH_2)_2C(CN)_2CH_2(CF_2)_5CF_2H$, (2-(2,2,3,3,4,4,5,5,6,6,7,7-Dodecafluoro-heptyl)-2-(3,3,3-trifluoro-propyl)-malononitrile), $CF_3(CH_2)_2C(CN)_2(CH_2)_2C(CF_3)_2F$ (2-(3,4,4,4-Tetrafluoro-3-trifluoromethyl-butyl)-2-(3,3,3-trifluoro-propyl)-malononitrile), $CF_3(CH_2)_2C(CN)_2(CH_2)_2(CF_2)_3CF_3$ (2-(3,3,4,4,5,5,6,6,6-Nonafluoro-hexyl)-2-(3,3,3-trifluoro-propyl)-malononitrile), $CF_2H(CF_2)_3CH_2C(CN)_2CH_2(CF_2)_3CF_2H$ (2,2-Bis-(2,2,3,3,4,4,5,5-octafluoro-pentyl)-malononitrile), $CF_3(CH_2)_2C(CN)_2CH_2(CF_2)_3CF_3$ (2-(2,2,3,3,4,4,5,5,5-Nonafluoro-pentyl)-2-(3,3,3-trifluoro-propyl)-malononitrile), $CF_3(CF_2)_2CH_2C(CN)_2CH_2(CF_2)_3CF_2H$ (2-(2,2,3,3,4,4,4-Heptafluoro-butyl)-2-(2,2,3,3,4,4,5,5-octafluoro-pentyl)-malononitrile), $CF_3CF_2CH_2C(CN)_2CH_2(CF_2)_3CF_2H$ (2-(2,2,3,3,4,4,5,5-Octafluoro-pentyl)-2-(2,2,3,3,3-pentafluoro-propyl)-malononitrile), $CF_2HCF_2CF_2CF_2CH_2C(CN)_2CH_2CH_2CF_2CF_3$ (2-(2,2,3,3,4,4,5,5-octafluoropentyl)-2-(3,3,4,4,4-pentafluorobutyl)-malonodinitrile), $CF_3(CH_2)_2C(CN)_2CH_2(CF_2)_3CF_2H$ (2-(2,2,3,3,4,4,5,5-octafluoropentyl)-2-(3,3,3-trifluoro-butyl)-malononitrile);

M.26. Microbial disruptors: *Bacillus thuringiensis* subsp. *Israelensi, Bacillus sphaericus, Bacillus thuringiensis* subsp. *Aizawai, Bacillus thuringiensis* subsp. *Kurstaki, Bacillus thuringiensis* subsp. *Tenebrionis;*

The commercially available compounds of the group M may be found in The Pesticide Manual, 13th Edition, British Crop Protection Council (2003) among other publications.

Thioamides of formula $M^{6.1}$ and their preparation have been described in WO 98/28279. Lepimectin is known from Agro Project, PJB Publications Ltd, November 2004. Benclothiaz and its preparation have been described in EP-A1 454621. Methidathion and Paraoxon and their preparation have been described in Farm Chemicals Handbook, Volume 88, Meister Publishing Company, 2001. Acetoprole and its preparation have been described in WO 98/28277. Metaflumizone and its preparation have been described in EP-A1 462 456. Flupyrazofos has been described in Pesticide Science 54, 1988, p.237-243 and in US 4822779. Pyrafluprole and its preparation have been described in JP 2002193709 and in WO 01/00614. Pyriprole and its preparation have been described in WO 98/45274 and in US 6335357. Amidoflumet and its preparation have been described in US 6221890 and in JP 21010907. Flufenerim and its preparation have been described in WO 03/007717 and in WO 03/007718. AKD 1022 and its preparation have been described in US 6300348. Chloranthraniliprole has been described in WO 01/70671, WO 03/015519 and WO 05/118552. Anthranilamide derivatives of formula $M^{24.1}$ have been described in WO 01/70671, WO 04/067528 and WO 05/118552. Cyflumetofen and its preparation have been described in WO 04/080180. The aminoquinazolinone compound pyrifluquinazon has been described in EP A 109 7932. The alkynylether compounds $M^{22.1}$ and $M^{22.2}$ are described e.g. in JP 2006131529. Organic sulfur compounds have been described in WO 2007060839. The malononitrile compounds have been described in WO 02/089579, WO 02/090320, WO 02/090321, WO 04/006677, WO 05/068423, WO 05/068432 and WO 05/063694.

The pesticide that can be used according to this aspect of the invention may be selected from fungicides, herbices and insecticides. Also maneb is a suitable pesticide. Particularly, the pesticide is selected from the active ingredient (B) and preferred the active ingredients (B) as indicated above.

Thus, it was surprisingly found within the framework of the present invention that the combination of chemical agents, e.g. at least one active ingredient, and at least one trangenic modification, which does not confer a resistance against the active ingredient, leads to a synergistic effect, i.e. the effectiveness of the combination is higher compared to the use of the individual components. Thereby, synergistic effects confering plant productivity enhancement and/or plant health enhancement and/or pest control can be obtained.

Consequently, according to another aspect, the present invention provides the use of a composition as defined according to the present invention for increasing the health, the productivity, the yield and/or improving the vigor of a transgenic plant of the invention, preferably an agricultural transgenic plant.

The present invention further provides a method for increasing productivity of a transgenic plant, which comprises treating the location where the plant is growing or is expected to grow, and/or the seeds from which the plant grows with an effective amount of the active ingredient (B) as defined herein.

According to a further embodiment, the present invention provides a method for increasing the productivity of a transgenic plant, which comprises treating the plant, the location where the plant is growing or is expected to grow, and/or the seeds from which the plant grows with an effective amount of the active ingredient (B) as defined herein.

According to the present invention, "increased yield" of an agricultural plant means that the yield of a product of the respective plant is increased by a measurable amount over the yield of the same product of the plant produced under the same conditions, but without the application of the present invention. According to the present invention, it is preferred that the yield be increased by at least 0,5 %, more preferred at least 1 %, even more preferred at least 2 %, still more preferred at least 4 %, preferably 5% or even more.

According to the present invention, "improved plant vigor" means that certain crop characteristics are increased or improved by a measurable or noticeable amount over the same factor of the plant produced under the same conditions, but without the application of the present invention, such as: delay of senescence, root growth, longer panicles, increased or improved plant stand, the plant weight, plant height, emergence, improved visual appearance, improved protein content, more developed root system, tillering increase, increase in plant height, bigger leaf blade, less dead basal leaves, stronger tillers, greener leaf color, pigment content, photosynthetic activity, less fertilizers needed, less seeds

needed, more productive tillers, earlier flowering, early grain maturity, less plant verse (lodging), increased shoot growth, early and improved germination, improved vitality of the plant, improved quality of the plant, improved quality of the fruits or vegetables (or other products produced by the plant), improved self defence mechanism of the plant such as induced tolerance against fungi, bacteria, viruses and/or insects.

The improvement of the plant vigor according to the present invention particularly means that the improvement of any one or several or all of the above mentioned plant characteristics are improved independently of the pesticidal action of the composition or active ingredients. According to one embodiment of the present invention, the inventive compositions are used for yield increase of an agricultural transgenic plant.

According to a further embodiment of the present invention, the inventive compositions are used for stimulating the natural defensive reactions of a plant against a pathogen and/or a pest. Thereby, the plant can be protected against unwanted microorganisms such as phytopathogenic fungi, bacteria, viruses and insects and it has been found that the inventive compositions result in plant strengthening effects. Therefore, they are useful for mobilizing the plant's defense mechanisms against the attack of unwanted microorganisms. Consequently, the plant becomes tolerant or resistant towards these microorganisms. Unwanted microorganisms in this context are phytopathogenic fungi and/or bacteria and/or viruses and/or insects, preferably phytopathogenic fungi, bacteria and/or viruses, wherein, according to the present invention, the treated plant may develop increased defense mechanism against one of these pathogens/pests or against two, three or all of these pathogens/pests.

According to one embodiment of the described methods of the invention the treatment is made to transgenic vegetables and transgenic field crops. According to a further embodiment of the described methods of the invention the treatment is made to transgenic cereals such as for example wheat, barley or rye. In one specific embodiment, the method of the invention can be applied to transgenic field crops, such as soybeans, corn, cotton, wheat, barley, rye, rice, sugar beets, sugar cane, sunflower and/or oilseed rape/canola, in particular soybeans, corn, cotton, sugar cane, oilseed rape/canola, tobacco, common beans, wheat, barley, rye, peas, and others. In relation to these crops the method is preferably applied by treating the seeds or the plants. In this embodiment it may be preferred that the plants are treated with two to three applications per season.

According to another embodiment of the described methods of the invention the treatment is made to transgenic potatoes, tomatoes, cucurbits, cucumbers, melons, watermelons, garlic, onions, bananas, peanuts, carrots, cabage, peppers, common beans, peas, lentils and/or lettuce, in particular potatoes, tomatoes, cucurbits, cucumbers, melons, watermelons, garlic, onions, and/or lettuce.

According to another embodiment of the described methods of the invention the treatment is made to transgenic apples, pears, stone fruits, or citrus, in particular apples, stone fruits, citrus, pines, snip grass.

According to another embodiment of the described methods of the invention, the treatment is made to transgenic strawberries, cherries, almonds, mango, papaya, blueberries and/or grapes in particular strawberries and/or cherries.

According to another embodiment of the described methods of the invention, the treatment is made to transgenic turf and/or ornamentals.

According to another embodiment of the described methods of the invention, the treatment is made to transgenic tea, tobacco and/or coffee.

According to one embodiment of the described methods of the invention, two to ten, three to eight or four to six treatments with the compounds are made during a season.

According to one embodiment of the invention, the inventive composition is applied via the leaves or to the soil. According to another embodiment of the described methods of the invention, the treatment(s) are carried out as foliar application. In one preferred embodiment of the present invention the method according to the invention is carried out as foliar application or spray application, respectively. Preferably, one, two, three, four, five and up to ten applications during one season are carried out, specifically more than two applications, and up to 10 applications. Also preferred more than two applications, and up to 5 applications during a season are carried out.

The inventive compositions are also suitable for dressing applications on plant propagation material. The latter term embraces seeds of all kinds (fruit, tubers, grains), cuttings, cut shoots and the like. One particular field of application is the treatment of all kinds of seeds.

The method according to the invention is preferably carried out as foliar application when applied to transgenic fruit and vegetables, such as potatoes, tomatoes, cucurbits, preferably cucumbers, melons, watermelons, garlic, onions, and lettuce. Preferably more than two applications and up to 5 or up to 10 applications during a season are carried out.

The application rates are usually between 0.01 and 2.0 kg, preferably up to 1.0 kg of active ingredient per hectare.

In one embodiment the application rates are usually between 0.0001 and 2.0 kg, preferably between 0.0001 and 1.0 kg of active ingredient per hectare.

According to a further aspect, the present invention relates to seed, comprising one of the inventive copositions as defined herein in an amount of from 0.1 g to 1 kg per 100 kg of seeds. Furthermore, it has been found that the inventive compositions achieve markedly enhanced action against plant pathogens (insects and harmful fungi).

In general, if laminarin is used, it can be preferred to use it in doses of between 0.005 g and 100 g per liter for

treating the leaves, and of between 1 g and 100 g per 100 kg for treating the seeds.

The active ingredient mixtures of the invention can be used in the form of premix formulations or the active ingredients can be applied to the area, plant or seed to be treated simultaneously or in immediate succession, if desired together with further carriers, surfactants or other application-promoting adjuvants customarily employed in formulation technology. Besides an effective amount of the active ingredients, the inventive composition can contain an agriculturally acceptable carrier and/or vehicle. The composition may be in solid form, for example in the form of a powder or granules, or in liquid form, for example in the form of an aqueous solution. The active ingredients or compositions used according to the present invention can be converted into the formulations conventionally used for pesticides, for example solutions, emulsions, suspensions, dusts, powders, pastes and granules. The use form depends on the particular purpose; in any case, it should ensure fine and uniform distribution of the compound according to the invention.

The present invention furthermore provides a composition as described above with at least one one solid or liquid carrier.

Best results are obtained when a formulation is used which supports the transport of the active compounds into the plants, and the distribution within the entire plant.

The compositions generally comprise between 0.1 and 95%, preferably between 0.5 and 90%, by weight of active ingredient.

When employed in plant protection, the amounts applied are, depending on the kind of effect desired, between 0.01 and 2.0 kg, preferably between 0.1 and 1 kg of active ingredient per ha. In one embodiment the application the amounts applied are, depending on the kind of effect desired, between 0.0001 and 2.0 kg, preferably between 0.1 and 1 kg of active ingredient per ha.

Seed can be treated by methods known to the person skilled in the art, such as, for example, seed dressing, seed coating, seed dusting, seed soaking and seed pelleting.

In the treatment of seed, the amounts of active ingredient employed are generally from 1 to 1000 g/100 kg of seed, preferably from 1 to 200 g/100 kg, in particular from 1 to 100 g/100 kg. When used in the protection of materials or stored products, the amount of active ingredient applied depends on the kind of application area and on the desired effect. Amounts customarily applied in the protection of materials are, for example, 0.001 g to 2 kg, preferably 0.005 g to 1 kg, of active compound per cubic meter of treated material.

The formulations are prepared in a known manner, for example by extending the active compound with solvents and/or carriers, if desired using emulsifiers and dispersants. Solvents/auxiliaries which are suitable are essentially:

- water, aromatic solvents (for example Solvesso products, xylene), paraffins (for example mineral oil fractions), alcohols (for example methanol, butanol, pentanol, benzyl alcohol), ketones (for example cyclohexanone, gamma-butyrolactone), pyrrolidones (NMP, NOP), acetates (glycol diacetate), glycols, fatty acid dimethylamides, fatty acids and fatty acid esters. In principle, solvent mixtures may also be used,

- carriers such as ground natural minerals (for example kaolins, clays, talc, chalk) and ground synthetic minerals (for example finely divided silica, silicates); emulsifiers such as nonionic and anionic emulsifiers (for example polyoxyethylene fatty alcohol ethers, alkylsulfonates and arylsulfonates) and dispersants such as lignosulfite waste liquors and methylcellulose.

Suitable surfactants are alkali metal, alkaline earth metal and ammonium salts of lignosulfonic acid, naphthalenesulfonic acid, phenolsulfonic acid, dibutylnaphthalenesulfonic acid, alkylarylsulfonates, alkyl sulfates, alkylsulfonates, fatty alcohol sulfates, fatty acids and sulfated fatty alcohol glycol ethers, furthermore condensates of sulfonated naphthalene and naphthalene derivatives with formaldehyde, condensates of naphthalene or of naphthalenesulfonic acid with phenol and formaldehyde, polyoxyethylene octylphenol ether, ethoxylated isooctylphenol, octylphenol, nonylphenol, alkylphenol polyglycol ethers, tributylphenyl polyglycol ether, tristearylphenyl polyglycol ether, alkylaryl polyether alcohols, alcohol and fatty alcohol/ethylene oxide condensates, ethoxylated castor oil, polyoxyethylene alkyl ethers, ethoxylated polyoxypropylene, lauryl alcohol polyglycol ether acetal, sorbitol esters, lignosulfite waste liquors and methylcellulose.

Suitable for the preparation of directly sprayable solutions, emulsions, pastes or oil dispersions are mineral oil fractions of medium to high boiling point, such as kerosene or diesel oil, furthermore coal tar oils and oils of vegetable or animal origin, aliphatic, cyclic and aromatic hydrocarbons, for example toluene, xylene, paraffin, tetrahydronaphthalene, alkylated naphthalenes or their derivatives, methanol, ethanol, propanol, butanol, cyclohexanol, cyclohexanone, isophorone, strongly polar solvents, for example dimethyl sulfoxide, N-methylpyrrolidone and water.

Powders, materials for spreading and dustable products can be prepared by mixing or concomitantly grinding the active substances with a solid carrier.

Granules, for example coated granules, impregnated granules and homogeneous granules, can be prepared by binding the active compounds to solid carriers. Examples of solid carriers are mineral earths such as silica gels,

silicates, talc, kaolin, attaclay, limestone, lime, chalk, bole, loess, clay, dolomite, diatomaceous earth, calcium sulfate, magnesium sulfate, magnesium oxide, ground synthetic materials, fertilizers, such as, for example, ammonium sulfate, ammonium phosphate, ammonium nitrate, ureas, and products of vegetable origin, such as cereal meal, tree bark meal, wood meal and nutshell meal, cellulose powders and other solid carriers.

Formulations for seed treatment may further comprise binders and/or gelants and if appropriate dyes.

Binders can be added to increase the adhesion of the active compounds to the seed after the treatment. Suitable binders are for example EO/PO block copolymer surfactants, but also polyvinyl alcohols, polyvinylpyrrolidones, polyacrylates, polymethacrylates, polybutenes, polyisobutylenes, polystyrenes, polyethyleneamines, polyethyleneamides, polyethyleneimines (Lupa-sol®, Polymin®), polyethers, polyurethanes, polyvinyl acetates, Tylose and copolymers of these polymers. A suitable gelant is for example carrageen (Satiagel®).

In general, the formulations comprise from 0.01 to 95% by weight, preferably from 0.1 to 90% by weight, of the active compound. The active compounds are employed in a purity of from 90% to 100%, preferably 95% to 100% (according to NMR spectrum).

The concentrations of active compound in the ready-to-use preparations can be varied within relatively wide ranges. In general, they are between 0.0001 and 10%, preferably between 0.01 and 1%.

The active compounds can also be used with great success in the ultra-low-volume (ULV) process, it being possible to apply formulations with more than 95% by weight of active compound or even the active compound without additives.

For the treatment of seed, the formulations in question give, after two-to-tenfold dilution, active compound concentrations of from 0.01 to 60% by weight, preferably from 0.1 to 40% by weight, in the ready-to-use preparations.

The following are examples of formulations according to the invention: Products for dilution with water

A Water-soluble concentrates (SL, LS)
10 parts by weight of active ingredient are dissolved with 90 parts by weight of water or with a water- soluble solvent. As an alternative, wetters or other auxiliaries are added. The active compound dissolves upon dilution with water. This gives a formulation having an active compound content of 10% by weight.

B Dispersible concentrates (DC)
20 parts by weight of active ingredient are dissolved in 70 parts by weight of cyclohexanone with addition of 10 parts by weight of a dispersant, for example polyvinylpyrrolidone. Dilution with water gives a dispersion. The active compound content is 20% by weight.

C Emulsifiable concentrates (EC)
15 parts by weight of active ingredient are dissolved in 75 parts by weight of xylene with addition of calcium dodecylbenzenesulfonate and castor oil ethoxylate (in each case 5 parts by weight). Dilution with water gives an emulsion. The formulation has an active compound content of 15% by weight.

D Emulsions (EW, EO, ES)
25 parts by weight of active ingredient are dissolved in 35 parts by weight of xylene with addition of calcium dodecylbenzenesulfonate and castor oil ethoxylate (in each case 5 parts by weight). This mixture is added to 30 parts by weight of water by means of an emulsifying machine (e.g. Ultraturrax) and made into a homogeneous emulsion. Dilution with water gives an emulsion. The formulation has an active compound content of 25% by weight.

E Suspensions (SC, OD, FS)
In an agitated ball mill, 20 parts by weight of active ingredient are comminuted with addition of 10 parts by weight of dispersants and wetters and 70 parts by weight of water or an organic solvent to give a fine active compound suspension. Dilution with water gives a stable suspension of the active compound. The active compound content in the formulation is 20% by weight.

F Water-dispersible granules and water-soluble granules (WG, SG)
50 parts by weight of active ingredient are ground finely with addition of 50 parts by weight of dispersants and wetters and made into water-dispersible or water-soluble granules by means of technical appliances (for example extrusion, spray tower, fluidized bed). Dilution with water gives a stable dispersion or solution of the active compound. The formulation has an active compound content of 50% by weight.

G Water-dispersible powders and water-soluble powders (WP, SP, SS, WS)
75 parts by weight of active ingredient are ground in a rotor-stator mill with addition of 25 parts by weight of dispersants, wetters and silica gel. Dilution with water gives a stable dispersion or solution of the active compound. The active

compound content of the formulation is 75% by weight.
Products to be applied undiluted

H Dustable powders (DP, DS)
5 parts by weight of active ingredient are ground finely and mixed intimately with 95 parts by weight of finely divided kaolin. This gives a dustable product with an active compound content of 5% by weight.

I Granules (GR, FG, GG, MG)
0.5 part by weight of active ingredient is ground finely and associated with 99.5 parts by weight of carriers. Current methods are extrusion, spray-drying or the fluidized bed. This gives granules with an active compound content of 0.5% by weight to be applied undiluted.

J ULV solutions (UL)
10 parts by weight of active ingredient are dissolved in 90 parts by weight of an organic solvent, for example xylene. This gives a product with an active compound content of 10% by weight to be applied undiluted.

Seed treatment typically utilizes water-soluble concentrates (LS), suspensions (FS), dusts (DS), water-dispersible and water-soluble powders (WS, SS), emulsions (ES), emulsifiable concentrates (EC) and gel formulations (GF). These formulations can be applied neat or preferably diluted to the seed. The application can take place prior to sowing.

Preference is given to using FS formulations for seed treatment. Such formulations typically comprise from 1 to 800 g/l of active compound, from 1 to 200 g/l of surfactants, from 0 to 200 g/l of antifreeze, from 0 to 400 g/l of binder, from 0 to 200 g/l of dyes and solvent, preferably water.

The active compounds can be used as such, in the form of their formulations or the use forms prepared therefrom, for example in the form of directly sprayable solutions, powders, suspensions or dispersions, emulsions, oil dispersions, pastes, dustable products, materials for spreading, or granules, by means of spraying, atomizing, dusting, spreading or pouring. The use forms depend entirely on the intended purposes; the intention is to ensure in each case the finest possible distribution of the active compounds used according to the invention. Aqueous use forms can be prepared from emulsion concentrates, pastes or wettable powders (sprayable powders, oil dispersions) by adding water. To prepare emulsions, pastes or oil dispersions, the substances, as such or dissolved in an oil or solvent, can be homogenized in water by means of a wetting agent, tackifier, dispersant or emulsifier. Alternatively, it is also possible to prepare concentrates composed of active substance, wetter, tackifier, dispersant or emulsifier and, if appropriate, solvent or oil, and such concentrates are suitable for dilution with water.

The active compound concentrations in the ready-to-use preparations can be varied within relatively wide ranges. In general, they are from 0.0001 to 10%, preferably from 0.01 to 1%.

If not otherwise specified, the solutions are prepared as follow:

The active ingredient is dissolved at the desired concentration in a mixture of 1:1 (vol/vol) distilled water: acteon. The test solution is prepared at the day of use.

Test solutions are prepared in general at concentrations of 1000 ppm, 500 ppm, 300 ppm, 100 ppm and 30 ppm (wt/vol).

The active compounds may also be used successfully in the ultra-low-volume process (ULV), by which it is possible to apply formulations comprising over 95% by weight of active compound, or even to apply the active compound without additives.

Various types of oils, wetters, adjuvants, may be added to the active compounds, if appropriate not until immediately prior to use (tank mix). These agents can be admixed with the agents according to the invention in a weight ratio of 1:100 to 100:1, preferably 1:10 to 10:1.

Suitable adjuvants in this sense are in particular: organically modified polysiloxanes, for example Break Thru S 240®; alcohol alkoxylates, for example Atplus 245®, Atplus MBA 1303®, Plu-rafac LF 300® and Lutensol ON 30®; EO/PO block polymers, for example Pluronic RPE 2035® and Genapol B®; alcohol ethoxylates, for example Lutensol XP 80®; and sodium dioctylsulfo-succinate, for example Leophen RA®.

In one embodiment, the method of the invention is directed to the transgenic plant according to table A, preferably A1 by increasing or generating the activity as depicted in table B, column 5, preferably a polypeptide as depicted in SEQ ID NO:1 to 270, preferably a polypeptide encoded by a nucleic acid molecule as depicted in table B, column 1 or 3 and the active ingredient (B) is selected from the group consisting of the groups B1, B2, B3, B4, B5, B6, B7, B8, B9, B10, B11, B12, B13, B14, B15, B16, B17, B18, B19, B20, B21, B22, B23, B24, B25, B26, B27, B28, B29, B30, B31, B32, B33, B34 and B35. The present examples illustrate the basic invention without being intended as limiting the subject of the invention.

The content of all of the references, patent applications, patents and published patent applications cited in the present patent application is herewith incorporated by reference.

In one embodiment the present invention is characterized by the following items:

Item 1. A method for increasing plant health and/or controlling pests in plants with at least one transgenic modification related to yield increase as compared to a corresponding wild-type plant
comprising treating the location where the plant with at least one transgenic modification is growing or is expected to grow and/or the transgenic plant with at least one transgenic modification or propagation material of the plant with at least one transgenic modification with an effective amount of a chemical composition comprising at least one active ingredient.

Item 2. The method of item 1 whereby at least one transgenic modification of said plant does not confer resistance to the active ingredient which is employed.

Item 3. The method of item 1 whereby the method is a method for increasing plant health. Item 4. The method of item 1 whereby the method is a method for controlling pests.

Item 5. The method of any one of the preceding items wherein the active ingredient is selected from the group consisting of insecticides, fungicides or herbicides.

Item 6. The method of any one of the preceding items wherein the active ingredient is a pesticide selected from the group consisting of insecticides and fungicides.

Item 7. The method of any one of the preceding items wherein the active ingredient is an insecticide selected from the group consisting of acephate, chlorpyrifos, diazinon, dichlorvos, dimethoate, fenitrothion, methamidophos, methidathion, methyl-parathion, monocrotophos, phorate, profenofos, terbufos, aldicarb, carbaryl, carbofuran, carbosulfan, methomyl, thiodicarb, bifenthrin, cyfluthrin, cypermethrin, alpha-cypermethrin, zeta-cypermethrin, deltamethrin, esfenvalerate, lambda-cyhalothrin, permethrin, tefluthrin, diflubenzuron, flufenoxuron, lufenuron, teflubenzuron, spirotetramat; clothianidin, dinotefuran, imidacloprid, thiamethoxam, acetamiprid, thiacloprid; endosulfan, fipronil, abamectin, emamectin, spinosad, spinetoram, hydramethylnon; chlorfenapyr; fenbutatin oxide, indoxacarb, metaflumizone, flonicamid, flubendiamide, chlorantraniliprole, cyazypyr (HGW86), cyflumetofen.

Item 8. The method of any one of the preceding items wherein the active ingredient is a fungicide selected from the group consisting of azoxystrobin, dimoxystrobin, kresoxim-methyl, orysastrobin, pyraclostrobin, trifloxystrobin, bixafen, boscalid, isopyrazam, metalaxyl, penthiopyrad, 3-difluoromethyl-1-methyl-1 H-pyrazole-4-carboxylic acid (2',4',5'-trifluorobiphenyl-2-yl)-amide, N-(2-bicyclopropyl-2-yl-phenyl)-3-difluoromethyl-1-methyl-1H-pyrazole-4-carboxylic acid amide, dimethomorph, fluopicolide, difenoconazole, epoxiconazole, fluquinconazole, flusilazole, flutriafol, metconazol, myclobutanil, propiconazole, prothioconazole, tebuconazole, tetraconazole, triticonazole, prochloraz, carbendazim, fluazinam, cyprodinil, pyrimethanil, fludioxonil, dodemorph, fenpropimorph, tridemorph, fenpropidin, iprodione, vinclozolin, famoxadone, probenazole, captan, folpet, 5-ethyl-6-octyl-[1,2,4]triazolo[1,5-a]pyrimidine-7-ylamine, mancozeb, maneb, metiram, thiram, dithianon, fosetyl, fosetyl-aluminium, chlorothalonil, thiophanate methyl, cymoxanil, metrafenone, spiroxamine, bixafen, N-(3',4',5'-trifluorobiphenyl-2-yl)-3-difluoromethyl-1-methyl-1H-pyrazole-4-carboxamide, N-[2-(4'-trifluoromethylthio)-biphenyl]-3-difluoromethyl-1-methyl-1H-pyrazole-4-carboxamide, N-[2-(1,3-dimethylbutyl)-phenyl]-1,3-dimethyl-5-fluoro-1H-pyrazole-4-carboxamide, N-(2-bicyclopropyl-2-yl-phenyl)-3-difluoromethyl-1-methyl-1H-pyrazole-4-carboxamide, N-(cis-2-bicyclopropyl-2-yl-phenyl)-3-difluoromethyl-1-methyl-1H-pyrazole-4-carboxamide, N-(trans-2-bicyclopropyl-2-yl-phenyl)-3-difluoromethyl-1-methyl-1H-pyrazole-4-carboxamide , N-[1,2,3,4-tetrahydro-9-(1-methylethyl)-1,4-methanonaphthalen-5-yl]-3-(difluoromethyl)-1-methyl-1 H-pyrazole-4-carboxamide.

Item 9. The method of any one of the preceding items wherein the active ingredient is a herbicide selected from the group consisting of acetochlor, dimethenamid, metolachlor, metazachlor, glyphosate, glufosinate, sulfosate, clodinafop, fenoxaprop, fluazifop, haloxyfop, paraquat, phenmedipham, clethodim, cycloxydim, profoxydim, sethoxydim, tepraloxydim, pendimethalin, trifluralin, acifluorfen, bromoxynil, imazamethabenz, imazamox, imazapic, imazapyr, imazaquin, imazethapyr, 2,4-D, chloridazon, clopyralid, fluroxypyr, picloram, picolinafen, bensulfuron, chlorimuron ethyl, cyclosulfamuron, iodosulfuron, mesosulfuron, metsulfuron-methyl, nicosulfuron, rimsulfuron, triflusulfuron, atrazine, hexazinone, diuron, florasulam, pyroxasulfone, bentazone, cinidon-ethly, cinmethylin, dicamba, diflufenzopyr, quinclorac, quinmerac, mesotrione, saflufenacil, topramezone.

Item 10. The method of any one of the preceding items, wherein the active ingredient (B) is a strobilurin selected from pyraclostrobin, kresoxim-methyl, dimoxystrobin, 2-(ortho-((2,5-dimethylphenyl-oxymethylene)phenyl)-3-methoxy-acrylic acid methyl ester, picoxystrobin, trifloxystrobin, enestroburin, orysastrobin, metominostrobin, azoxystrobin and fluoxastrobin.

Item 11. The method of any one of the preceding items, wherein the active ingredient (B) is selected from thiamethoxam, fipronil and imidacloprid and clothianidin

Item 12. The method of any one of the preceding items, wherein the chemical composition comprises at least one active ingredient (B) and a component (A) which is a glucan or a glucan derivative.

Item 13. The method of any one of the preceding items wherein at least one transgenic modification is related to yield increase as compared to a corresponding wild-type plant

Item 14. The method of any one of the preceding items wherein at least one transgenic modification related to yield increase as compared to a corresponding wild-type plant is a transgenic modification conferring one or more increased or generated activities selected from the group consisting of the polypeptides encoded by:

a) a nucleic acid molecule encoding the polypeptide as depicted in any of the SEQ ID NO: 1 to 270 or the homologs as depicted in SEQ ID NO: 271 to 273;

b) a nucleic acid molecule as depicted in table B, column 1 or 3;

c) a nucleic acid molecule, which, as a result of the degeneracy of the genetic code, can be derived from a polypeptide sequence as depicted in table B, column 1 or 3 and/or in SEQ ID NO: 1 to 270;

d) a nucleic acid molecule having at least 30 % identity with the nucleic acid molecule sequence of a polynucleotide comprising the nucleic acid molecule according to a) to c);

e) a nucleic acid molecule encoding a polypeptide having at least 30 % identity with the amino acid sequence of the polypeptide encoded by the nucleic acid molecule of (a) to (c) and having the activity represented by a nucleic acid molecule of (a) to (c) ;

f) nucleic acid molecule which hybridizes with a nucleic acid molecule of (a) to (c) under stringent hybridization conditions;

g) a nucleic acid molecule encoding a polypeptide which can be isolated with the aid of monoclonal or polyclonal antibodies made against a polypeptide encoded by one of the nucleic acid molecules of (a) to (e) and having the activity represented by the nucleic acid molecule comprising a polynucleotide of (a) to (c);

h) a nucleic acid molecule encoding a polypeptide comprising the consensus sequence or one or more polypeptide motifs resulting from 2 ore more of the polypeptides as depicted in table B, column 1 or 3 and/or in SEQ ID NO: 1 to 270 and/or a consensus sequence as depicted in SEQ ID NO: 274 to 277;

h) a nucleic acid molecule encoding a polypeptide having the activity represented by a protein as depicted in table B, column 1 or 3 or 5 and/or SEQ ID NO: 1 to 270;

i) nucleic acid molecule which comprises a polynucleotide, which is obtained by amplifying a cDNA library or a genomic library using the primers for the polynucleotides as depicted in table B, column 1 or 3 and/or SEQ ID NO: 1 to 270 which do not start at their 5'-end with the nucleotides ATA;
and

j) a nucleic acid molecule which is obtainable by screening a suitable nucleic acid library under stringent hybridization conditions with a probe comprising a complementary sequence of a nucleic acid molecule of (a) or (b) or with a fragment thereof, having at least 15 nt, preferably 20 nt, 30 nt, 50 nt, 100 nt, 200 nt or 500 nt of a nucleic acid molecule complementary to a nucleic acid molecule sequence characterized in (a) to (e) and encoding a polypeptide having the activity represented by a protein comprising a polypeptide as depicted in table B, column 1 or 3 and/or SEQ ID NO: 1 to 270.

Item 15. The method of any one of the preceding items wherein at least one transgenic modification related to yield

increase as compared to a corresponding wild-type plant is a transgenic modification conferring one or more increased or generated activities selected from the group consisting of polypeptides as depicted in SEQ ID NO: 1, 2, 3, 4, 5, 6, 7, 8, 9, 10, 11 1 ,12 ,13 ,14 ,15 ,16 ,17 ,18, 19, 20 ,21 ,22, 23 ,24, 25, 26, 27, 28, 29, 30 ,31 ,32, 46, 53, 266.

Item 16. The method of any one of the preceding items wherein the propagation material is seed.

Item 17. The method of any one of the preceding items wherein the transgenic seed or transgenic plant contains one or more genes which lead to an increase in plant health.

Item 18. The method of any one of the preceding items wherein the transgenic seed or transgenic plant contains one or more genes which lead to an increase in stress resistance.

Item 19. The method of any one of the preceding items wherein the transgenic seed or transgenic plant contains one or more genes which lead to an increase in pest control.

Item 20. The method of any one of the preceding items wherein the transgenic seed or transgenic plant contains one or more genes which lead to an increase in yield.

Item 21. The method of any one of the preceding items, wherein the treatment(s) are carried out as foliar application.

Item 22. The method of any one of the preceding items, wherein the treatment(s) are carried out in furrow.

Item 23. The method of any one of the preceding items, wherein the application rate of the active compound is in the range from 0.0001 to 2000 g/ha.

Item 24. The method of any one of the preceding items, wherein the active compound is applied to seed.

Item 25. The method of any one of the preceding items, wherein the transgenic plant is a monocotyledonous plant.

Item 26. The method of any one of the preceding items, wherein the transgenic plant is a dicotyledonous plant.

Item 27. The method of any one of the preceding items, wherein the plant with the transgenic modification is selected from the group comprising maize, wheat, rye, oat, triticale, rice, barley, soybean, peanut, cotton, oilseed rape, canola/OSR, manihot, pepper, sunflower, flax, borage, sugar cane, safflower, linseed, primrose, rapeseed, turnip rape, tagetes, solanaceous plants, potato, tobacco, eggplant, tomato, lettuce, Vicia species, pea, alfalfa, coffee, cacao, tea, Salix species, oil palm, coconut, perennial grass, snip grass and forage crops.

Item 28. The method of any one of the preceding items, wherein the plant with the transgenic modification is selected from the group comprising maize, soybean, cotton, canola/OSR and snip grass.

Examples

## Materials and Methods

### *Engineering Productivity-Enhanced Corn Plants by Expressing EST163 Genes*

Transformation of maize (*Zea Mays L.*) was performed using the construct NC027 (Figure 1). Immature embryos were co-cultivated with *Agrobacterium tumefaciens* that carry "super binary" vectors, and transgenic plants were recovered through organogenesis (Ishida et al., 1996, Nature Biotech 14745-50). This procedure provided a transformation efficiency of between 2.5% and 20%. The transgenic plants were then screened for improved plant biomass demonstrating that transgene expression confers productivity performance.
Figure 1. Map of the construct NC027 of the gene of interest, EST163. The expression was driven by the promoter, ScBv, and AHAS was chosen as the selectable marker. Agrobacterium cells harboring the genes and the maize ahas gene on the same plasmid were grown in YP medium supplemented with appropriate antibiotics for 1-3 days. A loop of Agrobacterium cells was collected and suspended in 2 ml M-LS-002 medium (LS-inf), and the tube containing Agrobactium cells was kept on a shaker for 1-3 hrs at 1,200 rpm.
Corncobs [genotype J553x (HIIIAxA188)] were harvested at 7-12 days after pollination. The cobs were sterilized in a 20% Clorox solution for 15 min followed by a thorough rinse with sterile water. Immature embryos between 0.8 and

2.0 mm in size were dissected into the tube containing Agrobacterium cells in LS-inf solution.

Agro-infection was carried out by keeping the tube horizontally in the laminar hood at room temperature for 30 min. Mixture of the agro infection was poured onto a plate containing the co-cultivation medium (M-LS-011). After the liquid agro-solution was piped out, the embryos were plated on the co-cultivation medium with scutellum side up and cultured in the dark at 22C for 2-4 days.

Embryos were transferred to M-MS-101 medium without selection. Seven to ten days later, the embryos were transferred to M-LS-401 medium containing 0.75 uM imazethapyr where they were grown for 4 weeks to select for transformed callus cells.

Plant regeneration was initiated by transferring resistant calli to M-LS-504 medium supplemented with 0.75 μM imazethapyr and grown under light at 26°C for 2 to 3 weeks. Regenerated shoots were then transferred to a rooting box with M-MS-607 medium (0.5 μM imazethapyr).

Plantlets with roots were transferred to potting mixture and grown in a growth chamber for 1 week, then transplanted to larger pots and maintained in greenhouse until maturity. The seed was harvested, and the presence of the transgene was determined by resistance to imi herbicide or by the Taqman molecular analysis technique.

*Seed Treatments*

J553x(HIIIAxA188) null and transgenic corn seeds of the T2 generation were treated with deionized water (Blank), 200 grams fipronil, 10 grams pyraclostrobin (F500), and 200 grams fipronil + 10 grams pyraclostrobin; all formulation rates were grams /100 kg seed. Every formulation was applied to approximately 80 seeds. The formulation was pipetted into a 125 ml flask along the sides and bottom of the flask before adding the seeds and shaking the flask for 30 seconds. The coated seeds were then removed from the flask and placed in a plastic dish for drying.

*Plant Management - WUE Study of Plants with the NC027 Construct*

Transgene positive and negative corn seedlings for the transformation events, SDM-23881, SDM-23885, SDM-23889, SDM-23890, and SDM-23891 were transplanted into a pot 5-L pots. The pots were covered with lids that permit the seedlings to grow through but minimize water loss. Each pot was weighed periodically and water added to maintain the initial water content (50% full water capacity). At the end of the experiment, the fresh and dry weights of each plant were measured, the water consumed by each plant was determined, and WUE of each plant was computed. Plant growth and physiological traits such as WUE, height, fresh weight, and dry weight were measured during the experiment. A comparison was made for every phenotype between the transgene positive and negative plants.

*Plant Management - fipronil/pyraclostrobin Study*

Seventy-five 3-L pots per treatment (Blank, fipronil, *pyraclostrobin,* and fipronil + *pyraclostrobin*) were filled with potting media, labeled with colored stakes, and given a unique barcode. One seed per pot was planted at a depth of approximately 2 cm and covered with media. The media was lightly watered to imbibe the seeds, while allowing for ample oxygen exchange and so that the chemical coatings on the seeds remained intact. After planting, the pots were randomly distributed into three replicate blocks (1 bench = 1 block), each with 25 plants of every treatment.

The plants were maintained in a greenhouse under optimal, well-watered conditions (80-90% field capacity) upon emergence. Supplemental nutrients were administered every third day during watering. The greenhouse temperature was maintained at 30°C, relative humidity at 75%, and light at 350 μmol $m^{-2}s^{-1}$, in a 15-hour day / 9-hour night photoperiod. Supplemental lighting was provided using metal-halide lights. Once per week, the pots were randomly mixed within each block.

On day 21, the plants were imaged to collect the phenotypic data as described in the *Imaging Procedures* section. Leaves were sampled for transgene copy number on day 25 to identify the null and transgenic plants. On day 28, the plants were imaged and then harvested to collect fresh weight. The presence of the transgene was determined by the Taqman molecular analysis technique. Differences in the phenotypic data collected during the imaging process and in fresh weight among the four treatments and between the null and transgenic plants were assessed. The chemical by transgenic interactions were determined.

*Imaging Procedures*

Imaging of the plants was facilitated using a LemnaTec Scanalyzer (Würselen, Germany), which includes a conveyor belt, an imaging station, a watering station, and computers for collecting, processing, and storing the image data. The cameras have a resolution of 1280 X 960 pixels and operate in 24-bit color.

The plants were manually transferred by block onto the conveyor system of the scanalyzer. Each plant was

then moved through the imager automatically. At the imaging station, cameras collected the top view image and two side view images. The side view images were the transverse view and the view parallel with the plane of the plant.

As described in the formula (see Appendix A), the scanalyzer calculates plant volume as a number of pixels. To assign a physiologically meaningful value to the volume measurements, a separate experiment was conducted. The plants were scanned with the scanalyzer then harvested. The plant material was placed into a graduated cylinder filled to a known volume with water. The difference between the water plus plant material and water alone provided a known plant volume in mL. The number of LemnaTec calculated volume pixels divided by the number of mL per plant indicated that about 36,000 pixels is equivalent to 1 mL of plant volume. Using the LemnaTec scanalyzer, we collected stem width; stalk volume; top, transverse, and parallel view plant areas; top, transverse, and parallel view green and yellow pixel counts; stem length; leaf angles and internode lengths through leaf 5; plant width and height; total leaf length; and total plant length.

**Results (format of the following text needs to be corrected)**

***Testing Enhancement of NC027***

As shown in Table 1, the transgenic plants exhibited significant improvements in fresh weight (FW), dry weight, and plant volume. On average, fresh weight increased 6.5%, dry weight increased 7%, and plant volume increased 10.7%. All three were significant at 95% level of probability according to Analysis of Variance.

Table 1. Average percent change in growth between transgenic plants and their null siblings for 5 NC027 events in two experiments. Asterisks (*) indicate significance at 95%.

| Experiment | Event | Lemnatec FW | Lemnatec Plant Volume | Lemnatec Plant Length | Measured FW | Measured Dry Wt |
|---|---|---|---|---|---|---|
| 1 | SDM-23881 | 8.5 | 18.2 | -5.7* | 6.3 | 8.1 |
| 1 | SDM-23885 | 0.3 | -0.2 | -10.5* | 2.2 | 2.1 |
| 1 | SDM-23889 | -2.0 | -1.3 | 1.7 | 1.2 | 4.0 |
| 1 | SDM-23890 | 0.1 | 9.2 | 1.3 | 3.2 | 3.0 |
| 1 | SDM-23891 | 11.8* | 8.1 | 3.5 | 7.3 | 8.5* |
| 1 | Average | 5.2* | 10.7* | -2.0 | 6.5* | 7.0* |

*Testing Enhancement of Chemical Amendments to Trangenic Plants*

In an initial trial, the phenotypes that showed treatment by genotype interactions at 95% significance included internode length and green vs. yellow plant material (Tables 2-5). At 80% significance, leaf angle and plant area were also included (Tables 6-7). fipronil enhanced greenness, internode length, leaf angle, and plant area relative to all other treatments. Other phenotypes showing significance for transgenic and treatment effects included fresh weight, plant and volume, number of fully expanded leaves, stem length, and View 3 size (Tables 8-10).

Table 2. Values for internode length in all treatments, measured in cm. The single asterisk (*) indicates a significant treatment by genotype effect at 95%. Small letters indicate significant differences between treatments at 95% confidence, and capital letters indicate differences at 99%.

| | Internode1* | | | | Internode2 | | | | Internode3 | | | |
|---|---|---|---|---|---|---|---|---|---|---|---|---|
| | Null | Trans | Avg | Test | Null | Trans | Avg | Test | Null | Trans | Avg | Test |
| Blank | 34.1 | 35.0 | 34.6 | a | 40.3 | 36.8 | 38.5 | a | 35.4 | 40.0 | 37.8 | a |
| F500 | 32.5 | 35.9 | 34.3 | ac | 36.0 | 40.4 | 38.4 | ac | 38.8 | 42.1 | 40.7 | ac |
| Fipronil | 35.3 | 36.6 | 35.9 | ac | 45.9 | 42.5 | 44.2 | BD | 42.4 | 37.7 | 40.0 | ac |
| Fipronil + F500 | 33.4 | 27.6 | 30.7 | BDE | 38.1 | 36.6 | 37.4 | acE | 32.6 | 32.7 | 32.6 | bDE |
| Average | 33.8 | 33.9 | 33.9 | | 40.1 | 39.2 | 39.7 | | 37.2 | 38.3 | 37.8 | |
| Test | a | a | | | a | a | | | a | a | | |

Table 3. Values for yellow, medium green, and dark green pixels counts in all treatments in the parallel view. The single asterisk (*) indicates a significant treatment by genotype effect at 95%. Small letters indicate significant differences between treatments at 95% confidence, and capital letters indicate differences at 99%.

| | Yellow Count* | | | | Medium Green Count | | | | Dark Green Count* | | | |
|---|---|---|---|---|---|---|---|---|---|---|---|---|
| | Null | Trans | Avg | Test | Null | Trans | Avg | Test | Null | Trans | Avg | Test |
| Blank | 1777.7 | 1956.1 | 1866.9 | A | 5648.8 | 5876.9 | 5762.8 | a | 16259.9 | 15850.4 | 16055.2 | a |
| F500 | 2006.6 | 2088.1 | 2051.9 | A | 5691.8 | 6522.1 | 6153.1 | a | 14727.7 | 15010.4 | 14884.7 | B |
| Fipronil | 1609.1 | 1806.0 | 1708.9 | Ac | 5470.9 | 5848.7 | 5662.4 | ac | 15955.0 | 16812.3 | 16389.5 | aC |
| Fipronil + F500 | 2496.2 | 3486.3 | 2970.9 | BCD | 6605.8 | 7106.0 | 6845.6 | BCD | 13381.2 | 11886.3 | 12664.5 | BCD |
| Average | 1981.7 | 2321.1 | 2154.9 | | 5872.6 | 6341.2 | 6111.8 | | 15049.9 | 14913.7 | 14980.4 | |
| Test | a | b | | | a | b | | | a | a | | |

Table 4. Values for yellow and medium green pixels counts in all treatments in the transverse view. Small letters indicate significant differences between treatments at 95% confidence, and capital letters indicate differences at 99%.

| | Medium Green Count | | | | Yellow Count | | | |
|---|---|---|---|---|---|---|---|---|
| | Null | Trans | Avg | Test | Null | Trans | Avg | Test |
| Blank | 8861.5 | 9198.8 | 9030.2 | a | 3625.4 | 3837.4 | 3731.4 | a |
| F500 | 9303.7 | 9809.2 | 9584.5 | bc | 4081.7 | 4343.5 | 4227.2 | b |
| Fipronil | 9132.8 | 9046.4 | 9089.0 | ade | 3268.7 | 3455.9 | 3363.6 | aC |
| Fipronil+ F500 | 9591.0 | 9251.6 | 9428.3 | ace | 4959.3 | 5865.9 | 5394.0 | BCD |
| Average | 9230.4 | 9340.1 | 9286.4 | | 4000.0 | 4365.7 | 4186.7 | |
| Test | a | a | | | a | b | | |

Table 5. Values for medium green pixels counts in all treatments in the top view. Small letters indicate significant differences between treatments at 95% confidence, and capital letters indicate differences at 99%.

| | Medium Green Count | | | |
|---|---|---|---|---|
| | Null | Trans | Avg | Test |
| Blank | 17563 | 17866 | 17715 | a |
| F500 | 17893 | 19062 | 18542 | a |
| Fipronil | 17038 | 17881 | 17465 | ac |
| Fipronil + F500 | 19152 | 19720 | 19424 | Bd |
| Average | 17935 | 18642 | 18296 | |
| Test | a | a | | |

Table 6. Values for leaf angle in all treatments, measured in degrees. This phenotype was significant at 80% confidence. Small letters indicate significant differences between treatments at 95% confidence, and capital letters indicate differences at 99%.

| | Leaf Angle 0 | | | | Leaf Angle 1 | | | |
|---|---|---|---|---|---|---|---|---|
| | Null | Trans | Avg | Test | Null | Trans | Avg | Test |
| Blank | 62.7 | 69.6 | 66.2 | a | 51.3 | 33.6 | 42.4 | a |
| F500 | 39.2 | 52.5 | 46.6 | bc | 36.5 | 38.2 | 37.4 | ab |
| Fipronil | 57.5 | 68.7 | 63.2 | acd | 63.6 | 54.4 | 58.9 | acd |
| Fipronil+ F500 | 43.9 | 36.6 | 40.5 | BcE | 47.8 | 16.8 | 33.2 | abE |
| Average | 50.8 | 56.9 | 53.9 | | 50.1 | 36.2 | 43.0 | |
| Test | a | a | | | a | b | | |

Table 7. Values for plant area in the parallel view in all treatments, measured in pixels. This phenotype was significant at 80% confidence. Small letters indicate significant differences between treatments at 95% confidence, and capital letters indicate differences at 99%.

| | Plant Area | | | |
|---|---|---|---|---|
| | Null | Trans | Avg | Test |
| Blank | 23686.4 | 23683.3 | 23684.8 | a |
| F500 | 22426.2 | 23620.6 | 23089.7 | a |
| Fipronil | 23035.0 | 24467.0 | 23760.8 | ac |
| Fipronil + F500 | 22483.1 | 22478.7 | 22481.0 | Bd |
| Average | 22904.2 | 23576.0 | 23247.1 | |

(continued)

| | Plant Area | | | |
|---|---|---|---|---|
| | Null | Trans | Avg | Test |
| Test | a | b | | |

Table 8. Values for whole plant phenotypes in all treatments. Fresh weight was hand-collected and measured in grams. Plant height was LemnaTec calculated and measured in cm. Plant volume was measured in pixels. Small letters indicate significant differences between treatments at 95% confidence, and capital letters indicate differences at 99%.

| | Fresh Weight | | | | Plant Height | | | | Plant Volume | | | |
|---|---|---|---|---|---|---|---|---|---|---|---|---|
| | Null | Trans | Avg | Test | Null | Trans | Avg | Test | Null | Trans | Avg | Test |
| Blank | 162.9 | 155.2 | 159.0 | a | 477.4 | 479.4 | 478.4 | a | 6.58E+06 | 6.60E+06 | 6.59E+06 | a |
| F500 | 155.7 | 155.0 | 155.3 | ac | 469.9 | 481.7 | 476.5 | a | 6.18E+06 | 6.63E+06 | 6.43E+06 | a |
| Fipronil | 160.9 | 164.7 | 162.8 | ace | 478.7 | 486.1 | 482.5 | a | 6.53E+06 | 6.86E+06 | 6.70E+06 | ac |
| Fipronil + F500 | 144.0 | 152.8 | 148.2 | BdF | 478.3 | 481.1 | 479.7 | a | 6.19E+06 | 6.10E+06 | 6.15E+06 | BD |
| Average | 155.6 | 157.0 | 156.3 | | 476.3 | 482.2 | 479.3 | | 6.37E+06 | 6.55E+06 | 6.46E+06 | |
| Test | a | a | | | a | b | | | a | a | | |

Table 9. Values for leaf number and stem length in all treatments. Stem length was measured in cm. Small letters indicate significant differences between treatments at 95% confidence, and capital letters indicate differences at 99%.

| | # Fully Expanded Leaves | | | | Stem Length | | | |
|---|---|---|---|---|---|---|---|---|
| | Null | Trans | Avg | Test | Null | Trans | Avq | Test |
| Blank | 3.2 | 3.4 | 3.3 | a | 107.3 | 117.6 | 112.5 | a |
| F500 | 2.9 | 3.1 | 3.0 | a | 97.2 | 110.2 | 104.4 | ac |
| Fipronil | 3.1 | 3.0 | 3.0 | a | 110.4 | 107.1 | 108.8 | ac |
| Fipronil+ F500 | 2.9 | 2.5 | 2.7 | B | 86.7 | 77.4 | 82.2 | BD |
| Average | 3.0 | 3.0 | 3.0 | | 100.2 | 103.3 | 101.8 | |
| Test | a | a | | | a | a | | |

Table 10. Values for stalk area from the transverse view. Small letters indicate significant differences between treatments at 95% confidence, and capital letters indicate differences at 99%.

| | Pixel Count | | | |
|---|---|---|---|---|
| | Null | Trans | Avg | Test |
| Blank | 4881 | 5138 | 5010 | a |
| F500 | 4725 | 4723 | 4724 | ac |
| Fipronil | 5081 | 4744 | 4910 | ac |
| Fipronil + F500 | 4375 | 4196 | 4289 | BdE |
| Average | 4760 | 4699 | 4729 | |
| Test | a | a | | |

Longer internode length may have represented better plant growth. A higher percentage of green pixels per plant area implies that the plant had greater photosynthetic capacity. Similarly, when the preponderance of yellow pixels may have indicated that the plant was experiencing some measure of stress causing chlorosis.

Larger leaf angles implies that leaves were laying flatter and thus received more photosynthetic radiation, and larger plant area suggests that there was more leaf surface area for photosynthesis.

In general, fipronil enhanced fresh mass, plant volume, plant height, and plant width as well.

The effect of chemical amendments was enhanced when applied to transgenic plants indicating a significant chemical by trangene interaction, as exemplified by the effects on internode length and changes in leaf angle.

| Variable | Interaction | SS | MS | FValue | ProbF |
|---|---|---|---|---|---|
| Internode | treatment*Copy_Call | 1805.79 | 601.9298 | 3.56 | 0.0142 |
| Sideview-yellow | treatment*Copy_Call | 13579567 | 4526522 | 2.81 | 0.0389 |
| Sideview-darkgreen_ | treatment*Copy_Call | 96292593 | 32097531 | 2.6 | 0.0516 |

In a second trial, the phenotypes that showed treatment by genotype interactions at 95% significance included plant volume, calculated fresh weight, side plant area, and dark green pixel count (Table 11). Plant volume and dark green pixel count were significant at 99% for the pyraclostrobin treatment. In general, pyraclostrobin enhanced plant size and photosynthetic health. The effect of chemical amendments was enhanced when applied to transgenic plants indicating a significant chemical by trangene interaction.

Increases in plant volume, fresh weight, and plant area show an enhancement of plant growth. A higher percentage of green pixels per plant area may imply increased photosynthetic capacity. Similarly, a preponderance of yellow pixels indicates some measure of stress, causing chlorosis.

A larger plant area suggests that more leaf surface area is available for photosynthesis.

Results from both days 21 and 28 followed the same trends.

Table 11. Values for plant volume, fresh weight, and plant greenness. Small letters indicate significant differences between treatments at 95% confidence, and capital letters indicate differences at 99%.

| | Plant Volume (M pixels) | | | | CalcFreshWeight (g) | | | | Broad Side Plnt Area (pixels) | | | |
|---|---|---|---|---|---|---|---|---|---|---|---|---|
| Treatment | Null | Trans | Avg | Test | Null | Trans | Avg | Test | Null | Trans | Avg | Test |
| Blank | 7.28 | 9 43 | 8 36 | a | 83.28 | 101 45 | 92 37 | a | 29782.18 | 34714 96 | 32248 57 | a |
| Control | 8 42 | 8 65 | 8 53 | ac | 99 33 | 94 37 | 96 85 | ac | 32106 05 | 31843 49 | 31974 77 | ac |
| F500 | 9 90 | **11.04** | 10 47 | Bde | 100 53 | **110.41** | 105 47 | bcd | 36740 30 | **39599.17** | 38169 74 | bde |
| Fip + F500 | 9 56 | 9 94 | 9 75 | acef | 99 33 | 105 42 | 102 38 | acde | 37117 46 | 37053 24 | 37085 35 | adef |
| Fipronil | 8 89 | 9 50 | 9 19 | acef | 99 88 | 101 65 | 100 77 | acde | 36398 18 | 34428 06 | 35413 12 | acef |
| Average | 8 82 | 9 68 | 9 25 | | 96 22 | 102 41 | 99 31 | | 34443 87 | 35397 68 | 34920 78 | |
| Test | a | a | | | a | a | | | a | a | | |

| | Top Drk Grn (pixels) | | | | Broad Side Drk Grn (pixels) | | | | Narrow Side Drk Grn (pix) | | | |
|---|---|---|---|---|---|---|---|---|---|---|---|---|
| Treatment | Null | Trans | Avg | Test | Null | Trans | Avg | Test | Null | Trans | Avg | Test |
| Blank | 50195.35 | 55954 94 | 53075 14 | a | 20741.88 | 23051 09 | 21896 48 | a | 21603.48 | 25317 00 | 23460 24 | a |
| Control | 58874 73 | 56391 61 | 57633 17 | ac | 22733 68 | 21788 39 | 22261 04 | ac | 26482 95 | 25010 75 | 25746 85 | ac |
| F500 | 57676 78 | **62307.07** | 59991 92 | Bcd | 26088 87 | **27881.04** | 26984 96 | Bde | 26394 93 | **28392.28** | 27393 61 | bcd |
| Fip + F500 | 56384 17 | 56031 02 | 56207 60 | acde | 26769 63 | 26003 90 | 26386 77 | bdef | 26203 26 | 27419 26 | 26811 26 | acde |
| Fipronil | 56608 71 | 57655 69 | 57132 20 | acde | 26176 97 | 23958 33 | 25067 65 | acef | 24703 55 | 27235 14 | 25969 34 | acde |
| Average | 55800 19 | 57661 27 | 56730 73 | | 24512 31 | 24435 34 | 24473 82 | | 25053 73 | 26633 30 | 25843 52 | |
| Test | a | a | | | a | a | | | a | a | | |

| Variable | Interaction | SS | MS | FValue | ProbF |
|---|---|---|---|---|---|
| Internode | treatment*Copy_Call | 1805.79 | 601.9298 | 3.56 | 0.0142 |
| Sideview-yellow | treatment*Copy_Call | 13579567 | 4526522 | 2.81 | 0.0389 |
| Sideview-darkgreen_ | treatment*Copy_Call | 96292593 | 32097531 | 2.6 | 0.0516 |

Conclusions

Chemical amendments enhance plant performance of transgenic and non-transgenic plants through manipulation of whole plant architecture and size, leaf color, and leaf morphology, among others. Administering combinations of chemical amendments carefully with transgenic plants may stimulate an even greater effect. The benefits will include greater plant performance in stress conditions, whether biotic or abiotic in nature, leading to increased yield and economic benefit.

Appendix A

Dry Weight - A measurement of the dry weight of the plant in grams. After harvest, the plants are placed into a drying oven set at 65°C, dried for a minimum of 72 hours, then weighed.

Fresh Weight - A measurement of the fresh weight of the plant in grams. Hand-collected fresh weight is measured by harvesting the plant and weighing it on a balance. LemnaTec fresh weight is calculated by the scanalyzer based on plant volume and plant age. The zoom angles of the camera lenses are normalized using a calibration table.

Internode length - Internode length is measured from leaf collar to leaf collar up the stem. Internode 1, for example, is measured from Leaf 0 to Leaf 1. Reported in cm.

Leaf Angle - A measurement of the angle of the leaf. The vectors for the angle are from the base of the leaf horizontally to the leaf tip and vertically up the plant stalk. The measured angle is from the stalk above the leaf to the leaf tip, reported in degrees.

Number of Fully Expanded Leaves - A count of the number of leaves with a recognizable leaf collar.

Parallel View Plant Area - A count of the total number of pixels of the plant as imaged from the parallel side.

Parallel View Dark Green Pixels - A count of the total number of user-defined dark green pixels as imaged from the parallel side. Indicates photosynthetic health of the plant.

Parallel View Medium Green Pixels - A count of the total number of user-defined dark green pixels as imaged from the parallel side. Indicates initial onset of chlorosis in the plant.

Parallel View Yellow Pixels - A count of the total number of user-defined yellow pixels as imaged from the parallel side. Indicates chlorosis in the plant.

Plant Height - A measurement from the base of the stem to the collar of the last fully expanded leaf, reported in cm.

Plant Volume - Calculated from the pixel counts of the three images and reported in pixels according to the formula:

$$\text{Volume} = (\text{top view x transverse view x parallel view pixels})^{0.5}$$

Plant Width - A measurement of the breadth of the plant from left-most leaf tip to right-most leaf tip.

Stalk Volume - Measured from the base of the plant to the last expanded leaf. Incorporates the pixel counts from the transverse and parallel view images, reported in pixels.

Stem Length - A measurement of the stem length from the base of the plant to the last fully expanded leaf, reported in cm.

Stem Width - A measurement of the stem at the base of the plant in the transverse view, reported in mm.

Top View Plant Area - A count of the total number of pixels of the plant as imaged from above.

Top View Dark Green Pixels - A count of the total number of user-defined dark green pixels as imaged from above. Indicates photosynthetic health of the plant.

Top View Medium Green Pixels - A count of the total number of user-defined dark green pixels as imaged from the parallel side. Indicates initial onset of chlorosis in the plant.

Top View Yellow Pixels - A count of the total number of user-defined yellow pixels as imaged from above. Indicates chlorosis in the plant.

Total Leaf Length - A sum of all leaves as measured from the base to the tip of each leaf, reported in cm.

Total Plant Length - A measurement from the base of the stem to the tip of the highest most leaf, reported in cm.

Transverse View Plant Area - A count of the total number of pixels of the plant as imaged from the transverse side.

Transverse View Dark Green Pixels - A count of the total number of user-defined dark green pixels as imaged from the transverse side. Indicates photosynthetic health of the plant.

Transverse View Medium Green Pixels - A count of the total number of user-defined dark green pixels as imaged from the parallel side. Indicates initial onset of chlorosis in the plant.

Transverse View Yellow Pixels - A count of the total number of user-defined yellow pixels as imaged from the transverse side. Indicates chlorosis in the plant.

Transverse View Stalk Area - A count of the total number of pixels of the stalk from the transverse view.

Those of ordinary skill in the art will recognize, or will be able to ascertain using no more than routine experimentation, many equivalents to the specific embodiments of the invention described herein. Such equivalents are intended to be encompassed by the following claims.

Table B

| Line/C olum | 1 | 2 | 3 | 4 | 5 |
|---|---|---|---|---|---|
| | Gene Name/ Locus ID | Publication No | SEQ ID NO. in the resp. publication | Source organism | Gene Annotation/Function/Activity |
| 1. | ATACCD | WO20070 11625 | 2 | Arabidopsis thaliana | ATACCD |
| 2. | ATAGR1 | WO20070 11771 | 2 | Arabidopsis thaliana | ATAGR1 |
| 3. | ATHLS | WO20070 11681 | 2,6 | Arabidopsis thaliana | N-ACETYL TRANSFERASE PROTEIN FROM ARABIDOPSIS THALIANA |
| 4. | ATSHMT4 | US2006/0 27384 | 2 | Arabidopsis thaliana | GLYCINE HYDROXYMETHYL TRANSFERASE |
| 5. | ATSTE24 | WO2002/ 16625 | 3,4 | Arabidopsis thaliana | ARABIDOPSIS THALIANA PRENYLPEPTIDASE |
| 6. | b2699 | WO20070 20198 | 159 | E. coli | (0/1061)DNA STRAND EXCHANGE, RENATURATION |
| 7. | b2965 | WO20060 69610 | 797, 91636 | E. coli | ORNITHINE DECARBOXYLASE |
| 8. | BN45412825 | WO2004/ 092398 | 517 | Brassica napus | RHO1PS HOMOLOG/RAC-LIKE PROTEIN |
| 9. | BN51473779 | WO2004/ 092398 | | Brassica napus | ASPARTYLAMINO PEPTIDASE |
| 10. | B0730 | WO2006/ 069610 | 34229, 29163, 24084, 4465,1 7105,2 2610,1 9785,4 18, 2049 | E. coli | TRANSCRIPTION REGULATOR |
| 11. | b1830 | WO2004/ 092398 | 259, 260 | E. coli | CARBOXY-TERMINALPROTEASE FOR PENICILLIN-BINDING PROTEIN3 |
| 12. | b2664 | WO2004/ 092398 | 263, 264 | E. coli | PUTATIVE TRANSCRIPTIONAL REGULATOR |
| 13. | b2082 | WO2004/ 092398 | 253, 254 | E. coli | PROPHAGE P2OGR PROTEIN |
| 14. | b2799 | WO2004/ 092398 | 265, 266 | E. coli | L-1 |
| 15. | b2148 | WO2004/ 092398 | 249, 250 | E. coli | METHYL-GALACTOSIDE TRANSPORT, GALACTOSETAXIS |
| 16. | b2796 | WO2004/ 092398 | 251, 252 | E. coli | PROBABLE SERINE TRANSPORTER |

(continued)

| Line/C olum | 1 | 2 | 3 | 4 | 5 |
|---|---|---|---|---|---|
| 17. | b3116 | WO20060 69610 | 258 | E. coli | ANAEROBICALLY INDUCIBLE LTHREONINE |
| 18. | b0124 | WO2004/ 092398 | 255, 256 | E. coli | GLUCOSE DEHYDROGENASE |
| 19. | EST102 | US20070 028333 | 016,5, 6 | Physcomitrella patens | |
| 20. | EST12 | US20070 157343 | 883, 11, 18 | Physcomitrella patens | |
| 21. | EST134 | US20070 261131 | 688,5, 8 | Physcomitrella patens | |
| 22. | PpPK_EST142 | US7,176, 026 | 176, 026,5, 6 | | |
| 23. | PpAPS_EST16 3 | US20070 192908 | 282,9, 17 | Physcomitrella patens | TRANSCRIPTION FACTOR |
| 24. | PpGBP1_EST203 | US2004/0 194163 | 6, 11 | Physcomitrella patens | SMALL GTP-BINDING PROTEIN SAR1 BNT |
| 25. | EST217 | US20070 157344 | 246,8, 13 | Physcomitrella patens | |
| 26. | EST255 | US6,677, 504 | 677, 504, 10, 18 | | |
| 27. | EST266 | US7,166, 767 | 166, 767,6, 11 | | |
| 28. | EST268 | WO20061 33983 | 8,477, 7, 12 | | |
| 29. | PpCK3_EST289 | WO20060 20717 | 105, 723, 11, 12 | | |
| 30. | EST293 | US20070 157334 | 902, 20,33 | Physcomitrella patens | |
| 31. | EST295 | W020030 40171 | 12 | Physcomitrellapatens | LEUCINE-RICH REPEAT TRANSMEMBRANE PROTEIN KINASE1 |
| 32. | PpZF_EST307 | US2004/0 107463 | 12,20 | Physcomitrella patens | ZINC-FINGERPROTEIN |
| 33. | EST335 | US2004/0 128721 | 5,8 | Physcomitrella patens | PUTATIVE CDC21 PROTEIN |
| 34. | PpSCL_EST38 6 | WO2006/ 044912 | 1,2 | Physcomitrella patens | SCARE CROW-LIKE TRANSCRIP-TION FACTOR 14(SCL14) |
| 35. | PpCBF_EST39 | US7,164, 057 | 164, 057, 10, 17 | | |
| 36. | EST391 | US20070 079400 | 646, 129, 130 | Physcomitrella patens | |
| 37. | EST4 | US7,091, 402 | 091, 402,2, 3 | | |
| 38. | pPP2A4_EST430 | WO20024 6442 | 8, 13 | Physcomitrella patens | TYPE 2A PROTEIN PHOSPHATASE-1 |
| 39. | EST443 | US20070 157345 | 353,8, 9 | Physcomitrella patens | |

(continued)

| Line/C olum | 1 | 2 | 3 | 4 | 5 |
|---|---|---|---|---|---|
| 40. | EST46 | US7,125, 719 | 125, 719,5, 6 | | |
| 41. | EST472 | US20040 148658 | 750, 1, 2 | Physcomitrella patens | |
| 42. | EST512 | WO2006/ 044912 | 5,6 | Physcomitrella patens | PHYTOCHROME ASIGNAL TRANSDUCTION1(PAT1) |
| 43. | PpVTP_EST51 3 | WO2006/ 050038 | 1,2 | Physcomitrella patens | RAB1C |
| 44. | PpLLPK_EST5 57 | WO20061 34162 | 2. | Physcomitrella patens | PUTATIVE LECTIN-LIKE PROTEIN KINASE |
| 45. | EST65 | US2003/0 172408 | 11, 12 | Physcomitrella patens | TRANSCRIPTIONAL REGULATOR |
| 46. | GM52570278 | WO20060 32708 | 968 | Glycine max | VESICLE TRANSPORT V-SNARE PROTEIN |
| 47. | GM59556757 | WO2006/ 044912 | 7,8 | Glycine max | (124/2079)SCARE CROW-LIKE TRANSCRIPTION FACTOR8(SCL8) |
| 48. | GM59587863 | WO20060 32708 | 834 | Glycine max | PUTATIVE PROTEINKINASE |
| 49. | GM59594319 | W020030 20914 | 18,20,2 2 | Glycine max | (186/1106)TYPE2APROTEINPHOSPHATA SE-1 |
| 50. | GM59629961 | WO20060 32708 | 914 | Glycine max | PUTATIVE CASEINKINASEI |
| 51. | At3g24570 | WO20040 74440 | 2 | Arabidopsis thaliana | ARABIDOPSIS THALIANA AT3G24570 PROTEIN |
| 52. | YDR071C | WO2004/ 092398 | 289, 290 | Saccharomyces cerevisiae | POLYAMINE ACETYL TRANSFERASE |
| 53. | YGL239c | WO2004/ 092398 | 7,8 | Saccharomyces cerevisiae | HYPOTHETICAL PROTEIN |
| 54. | YBR288C | WO2004/ 092398 | 59,60 | Saccharomyces cerevisiae | MU3-LIKE SUBUNIT OF THE CLATHRIN ASSOCIATED PROTEIN COMPLEX(AP-3) |
| 55. | YBR258C | WO2004/ 092398 | 61,62 | Saccharomyces cerevisiae | SUBUNIT OF THE COMPASS(SET1C) COMPLEX |
| 56. | YCL001w-a | US2004/0 11888 | 63,64 | Saccharomyces cerevisiae | DOM34 PROTEIN HOMOLOG |
| 57. | YBR274w | WO2004/ 092398 | 65,66 | Saccharomyces cerevisiae | DNA DAMAGE CHECKPOINT EFFECTORKINASE |
| 58. | YCL027w | WO2004/ 092398 | 273, 274 | Saccharomyces cerevisiae | CELL FUSION PROTEIN |
| 59. | YBL060w | WO2004/092398 | 9, 10 | Saccharomyces cerevisiae | HYPOTHETICAL PROTEIN |

(continued)

| Line/C olum | 1 | 2 | 3 | 4 | 5 |
|---|---|---|---|---|---|
| 60. | YHR090c | WO2004/ 092398 | 67,68 | Saccharomyces cerevisiae | SUBUNIT OF THE NUA4 HISTONE ACETYLTRANSFERASE COMPLEX THAT ACETYLATES HISTONE H4, H2A; HAS SIMILARITY TO THE HUMAN TUMOR SUPPRESSORING1 |
| 61. | YGR121c | WO2004/ 092398 | 69, 70 | Saccharomyces cerevisiae | AMMONIUM TRANSPOR TPROTEINMEP1 |
| 62. | YGR127w | WO2004/ 092398 | 71,72 | Saccharomyces cerevisiae | HYPOTHETICAL PROTEIN |
| 63. | YGR161c | WO2004/ 092398 | 287, 288 | Saccharomyces cerevisiae | PROTEIN PHOSPHATASE TYPE2A |
| 64. | YKL051w | WO2004/ 092398 | 77, 78 | Saccharomyces cerevisiae | PLASMA MEMBRANE PROTEIN THAT MAY ACT TO GET HER WITH OR UPSTREAM OF STT4P TO GENERATE NORMAL LEVEL SOFTHE ESSENTIAL PHOSPHOLIPID PI4P |
| 65. | YKL103C | WO2004/ 092398 | 283, 284 | Saccharomy ces cerevisiae | AMINOPEPTIDASE YSCI |
| 66. | YKL017c | WO2004/ 092398 | 83,84 | Saccharomyces cerevisiae | DNA HELICASE1; HCS1 P |
| 67. | YKL132c | WO2004/ 092398 | 87, 88 | Saccharomyces cerevisiae | SIMILAR TO FOLYL-POLYGLUTAMATE SYNTHASE RMA1P |
| 68. | YGR126w | WO2004/ 092398 | 89,90 | Saccharomyces cerevisiae | PUTATIVE PROTEIN OF UNKNOWN FUNCTION |
| 69. | YKL058w | WO2004/ 092398 | 93, 94 | Saccharomyces cerevisiae | TOA2TFIIA SUBUNIT (TRANSCRIPTION INITIATION FACTOR) |
| 70. | YIL023c | WO2004/ 092398 | 243, 244 | Saccharomyces cerevisiae | YEAST KE4 |
| 71. | YHR195w | WO2004/ 092398 | 99, 100 | Saccharomyces cerevisiae | NUCLEAR ENVELOPEP ROTEIN THAT INTERACTS WITH THE VACUOLARMEMBRANE PROTEIN VAC8P TO PROMOTE FORMATION OF NUCLEUS-VACUOLE JUNCTIONS DURING PIECEMEAL MICRO AUTOPHAGYOFTHENUCLEUS |
| 72. | YIR022w | WO2004/ 092398 | 101, 102 | Saccharomyces cerevisiae | SIGNAL PEPTIDASE SUBUNIT SEC11 P |
| 73. | YJL172w | WO2004/ 092398 | 105, 106 | Saccharomyces cerevisiae | VACUOLAR CARBOXYPEPTIDASE YSCS |

(continued)

| Line/C olum | 1 | 2 | 3 | 4 | 5 |
|---|---|---|---|---|---|
| 74. | YHR113W | WO2004/ 092398 | 107, 108 | Saccharomyces cerevisiae | CYTOPLASMIC ASPARTYLAMINOPEPTIDASE] |
| 75. | YGR141w | WO20060 32708 | 84 | Saccharomyces cerevisiae | VACUOLAR PROTEIN SORTING (VPS) PROTEIN REQUIRED FOR CYTOPLASM TO VACUOLE TARGETING OF PROTEINS |
| 76. | YHR175w | WO2004/ 092398 | 109, 110 | Saccharomyces cerevisiae | PUTATIVE LOW-AFFINITY COPPERTRANSPORTER OF THE VACUOLARMEMBRANE |
| 77. | YGR212w | WO2004/ 092398 | 111, 112 | Saccharomyces cerevisiae | N-ACETYLTRANSFERASE |
| 78. | YGR180c | WO2004/ 092398 | 115, 116 | Saccharomyces cerevisiae | RNR4 RIBONUCLEOTIDEREDUCTASE |
| 79. | YJL179W | WO2004/ 092398 | 117, 118 | Saccharomyces cerevisiae | PREFOLDIN SUBUNIT1; PUTATIVE HOMOLOG OF SUBUNIT 1 OF BOVINE PREFOLDIN |
| 80. | YJL001W | WO2004/ 092398 | 119, 120 | Saccharomyces cerevisiae | 20S PROTEASOME BETA-TYPE SUBUNIT |
| 81. | YJL208c | WO2004/ 092398 | 121, 122 | Saccharomyces cerevisiae | NUC1_YEAST MITOCHONDRIAL NUCLEASE |
| 82. | YJL152W | WO2004/ 092398 | 123, 124 | Saccharomyces cerevisiae | HYPOTHETICALPROTEIN |
| 83. | YGL166w | WO2004/ 092398 | 11, 12 | Saccharomyces cerevisiae | CUP2 COPPER-DEPENDENT TRANSCRIPTION FACTOR |
| 84. | YJL151c | WO2004/ 092398 | 127, 128 | Saccharomyces cerevisiae | SNA3 INTEGRAL MEMBRANE PROTEIN LOCALIZED TO VACUOLAR INTRALUMENAL VESICLE |
| 85. | YLR441c | WO2004/ 092398 | 129, 130 | Saccharomyces | RPS1A RIBOSOMALPROTEIN S3A.E |
| 86. | YLR029C | WO2004/ 092398 | 135, 136 | Saccharomyces cerevisiae | PROTEIN COMPONENT OF THE LARGE (60S) RIBOSOMAL SUBUNIT |
| 87. | YFR042W | WO2004/ 092398 | 279, 280 | Saccharomyces cerevisiae | PUTATIVE PROTEIN OF UNKNOWN FUNCTION |
| 88. | YLR210w | WO20060 32708 | | Saccharomyces cerevisiae | B-TYPE CYCLIN INVOLVED IN CELLCYCLE PROGRESSION |

(continued)

| Line/C olum | 1 | 2 | 3 | 4 | 5 |
|---|---|---|---|---|---|
| 89. | YLR173w | WO20060 32708 | | Saccharomyces cerevisiae | HYPOTHETICAL PROTEIN |
| 90. | YLL041 c | WO2004/ 092398 | 137, 138 | Saccharomyces cerevisiae | IRON-SULFUR PROTEIN SUBUNIT OF SUCCINATE DEHYDROGENASE (SDH1P |
| 91. | YKL150w | WO20060 32708 | | Saccharomyces cerevisiae | MCR1 CYTOCHROME-B5 REDUCTASE |
| 92. | YLR105c | WO2004/ 092398 | 139, 140 | Saccharomyces cerevisiae | SUBUNIT OF THE TRNASPLICING ENDONUCLEASE |
| 93. | YIL136w | WO2004/ 092398 | 141, 142 | Saccharomyces cerevisiae | OM45 PROTEIN OF THE OUTER MITOCHONDRIAL MEMBRANE |
| 94. | YLR224W | WO2004/ 092398 | 223, 224 | Saccharomyces cerevisiae | F-BOX PROTEIN, COMPONENT OF SCF UBIQUITIN LIGASECOMPLEXES INVOLVED INUBIQUIT IN DEPENDENT PROTEINCATABOLISM; READILY MONO UBIQUITIN ATED INVITRO BY SCF-UBC4 COMPLEXES |
| 95. | YGL106W | WO2004/ 092398 | 239, 240 | Saccharomyces cerevisiae | ESSENTIAL LIGHT CHAINFORMYOSIN MYO2P; MAY STABILIZE MYO2P BY BINDING TO THE NECK REGION; MAY INTERACT WITH MYO1P |
| 96. | YLR215c | WO2004/ 092398 | 143, 144 | Saccharomyces cerevisiae | PROTEIN INVOLVED IN NUTRITIONAL CONTROL OF THE CELLCYCLE; REGULATES ABUNDANCE OF THE TRANSLATION INITIATION FACTOR EIF2; ORTHOLOG OF HUMAN D123 PROTEIN |
| 97. | YLR274w | WO20060 32708 | | Saccharomyces cerevisiae | COMPONENT OF THE HEXAMERIC MCM COMPLEX |
| 98. | YM R260C | WO2004/ 092398 | 147, 148 | Saccharomyces cerevisiae | TRANSLATION INITIATION FACTOR EIF1A |
| 99. | YNL120C | WO2004/ 092398 | 149, 150 | Saccharomyces cerevisiae | DUBIOUS OPENREADINGFRAME UNLIKELY TO ENCODE A PROTEIN |
| 100. | YMR154c | WO2004/ 092398 | 227, 228 | Saccharomyces cerevisiae | CALPAIN-LIKE PROTEASE INVOLVED IN PROTEOLYTIC ACTIVATION OF RIM101 P IN RESPONSE TO ALKALINE PH |

(continued)

| Line/C olum | 1 | 2 | 3 | 4 | 5 |
|---|---|---|---|---|---|
| 101. | YNL090W | WO2004/ 092398 | 285, 286 | Saccharomyces cerevisiae | NON-ESSENTIAL SMALL GTPASE OF THE RHO/RAC SUBFAMILY OF RAS-LIKE PROTEINS |
| 102. | YNL108c | WO20060 32708 | | Saccharomyces cerevisiae | PROTEIN OF UNKNOWN FUNCTION LOCALISED TO CYTOPLASM, NUCLEUS |
| 103. | YNL141w | WO20060 32708 | | Saccharomyces cerevisiae | AAH1 ADENOSINE DEAMINASE |
| 104. | YLR407W | WO2004/ 092398 | 151, 152 | Saccharomyces cerevisiae | PUTATIVE PROTEIN OF UNKNOWN FUNCTION |
| 105. | YN L079C | WO2004/ 092398 | 277, 278 | Saccharomyces cerevisiae | TPM1TROPOMYOSIN1 |
| 106. | YMR197c | WO2004/ 092398 | 154 | Saccharomyces cerevisiae | PROTEIN INVOLVED IN CIS-GOLGI MEMBRANE TRAFFIC; V-SNARE THAT INTERACTS WITHT WOT-SNARES |
| 107. | YMR100W | WO2004/ 092398 | 155, 156 | Saccharomyces cerevisiae | PROTEIN OF UNKNOWN FUNCTION |
| 108. | YMR210w | WO2004/ 092398 | 157, 158 | Saccharomyces cerevisiae | PUTATIVE ACYLTRANSFERASE WITH SIMILARITY TO EEB1 PANDEHT1 P |
| 109. | YMR069w | WO2004/ 092398 | 161, 162 | Saccharomyces cerevisiae | NAT4N-ALPHAACETYL TRANSFERASE (N-TERMINAL RESIDUES OF HISTONE SH4, H2A) |
| 110. | YMR118c | WO2004/ 092398 | 219, 220 | Saccharomyces cerevisiae | PROTEIN OF UNKNOWN FUNCTION WITH SIMILARITY TO SUCCINATE DEHYDROGENASE CYTOCHROMEB SUBUNIT;YMR118C IS NOT AN ESSENTIAL GENE |
| 111. | YN L076w | WO2004/ 092398 | 163, 164 | Saccharomyces cerevisiae | GI\|1301965\|EMB\|CAA95950.1\|ORFYNL076 W |
| 112. | YN L024c | WO2004/ 092398 | 165, 166 | Saccharomyces cerevisiae | PUTATIVE PROTEIN OF UNKNOWN FUNCTION WITH SEVEN BETA-STRAND METHYLTRANSFERASE MOTIF |
| 113. | YNL125c | WO2004/ 092398 | 167, 168 | Saccharomyces cerevisiae | PROTEIN WITH SIMILARITY TO MONOCARBOXYLATE PERMEASES |

(continued)

| Line/C olum | 1 | 2 | 3 | 4 | 5 |
|---|---|---|---|---|---|
| 114. | YNL029c | WO2004/ 092398 | 169, 170 | Saccharomyces cerevisiae | PUTATIVE MANNOSYL TRANSFERASE INVOLVED IN PROTEINGLYCOSYLATION; MEMBER OF THE KRE2/MNT1 MANNOSYLTRANSFERASE FAMILY |
| 115. | YMR115w | WO2004/ 092398 | 171, 172 | Saccharomyces cerevisiae | HYPOTHETICAL ORF |
| 116. | YNL282W | WO2006032708 | 166 | Saccharomyces cerevisiae | SUBUNIT OF BOTH RNASE MRP |
| 117. | YAL046c | WO2004/ 092398 | 15, 16 | Saccharomyces cerevisiae | HYPOTHETICAL PROTEIN |
| 118. | YN L244c | WO2004/ 092398 | 173, 174 | Saccharomyces cerevisiae | TRANSLATION INITIATION FACTOR EIF1; COMPONENT OF A COMPLEX INVOLVED IN RECOGNITION OF THE INITIATOR CODON; MODULATES TRANSLATION ACCURACY AT THE INITIATION PHASE |
| 119. | YN L334c | WO2004/ 092398 | 175, 176 | Saccharomyces cerevisiae | SNO2 PROTEIN IS RELATED WITH B(1) BIOSYNTHESIS DURING THE EXPONENTIAL PHASE |
| 120. | YNR029c | WO20060 32708 | | Saccharomyces cerevisiae | HYPOTHETICAL PROTEIN |
| 121. | YNR018w | WO2004/ 092398 | 177, 178 | Saccharomyces cerevisiae | PROTEIN OF UNKNOWN FUNCTION LOCALISED TO MITOCHONDRIA |
| 122. | YNL277w | WO2004/ 092398 | 179, 180 | Saccharomyces cerevisiae | L-HOMOSERINE-OACETYLTRANSFERASE |
| 123. | YOL118c | WO2004/ 092398 | 181, 182 | Saccharomyces cerevisiae | HYPOTHETICAL PROTEIN |
| 124. | YOL123w | WO2004/ 092398 | 183, 184 | Saccharomyces cerevisiae | SUBUNIT OF CLEAVAGE FACTORI |
| 125. | YOR020c | WO2004/ 092398 | 185, 186 | Saccharomyces cerevisiae | 10KDA HEAT SHOCK PROTEIN |
| 126. | YOR154w | WO20060 32708 | | Saccharomyces cerevisiae | HYPOTHETICAL PROTEIN |
| 127. | YOL116w | WO2004/ 092398 | 187, 188 | Saccharomyces cerevisiae | TRANSCRIPTIONAL ACTIVATOR |
| 128. | YOR266W | WO20060 32708 | | Saccharomyces cerevisiae | MITOCHONDRIAL INNER MEMBRANE PROTEIN INVOLVED IN EXPORT OF PROTEINS FROM THE MITOCHONDRIAL MATRIX |

(continued)

| Line/C olum | 1 | 2 | 3 | 4 | 5 |
|---|---|---|---|---|---|
| 129. | YPL103c | WO20060 32708 | | Saccharomyces cerevisiae | UNCHARACTERIZED ORF |
| 130. | | WO2004/ 092398 | 21,22 | Saccharomyces cerevisiae | HYPOTHETICAL PROTEIN |
| 131. | YOR305w | WO2004/ 092398 | 189, 190 | Saccharomyces cerevisiae | PROTEIN OF UNKNOWN FUNCTION |
| 132. | YKL057c | WO20060 32708 | | Saccharomyces cerevisiae | SUBUNIT OF THE NUP84P SUBCOMPLEX OF THE NUCLEARPORECOMPLEX(NPC) |
| 133. | YDR205w | WO2004/ 092398 | 229, 230 | Saccharomyces cerevisiae | MEMBER OF THE CATION DIFFUSION FACILITATOR FAMILY |
| 134. | YPL267w | WO2004/ 092398 | 191, 192 | Saccharomyces cerevisiae | PUTATIVE TRANSCRIPTION FACTOR |
| 135. | YPL229w | WO2004/ 092398 | 193, 194 | Saccharomyces cerevisiae | PUTATIVEP ROTEIN OF UNKNOWN FUNCTION |
| 136. | YPL038w | WO2004/ 092398 | 195, 196 | Saccharomyces cerevisiae | ZINC-FINGERDNA-BINDINGPROTEIN |
| 137. | YPR047w | WO2004/092398 | 197, 198 | Saccharomyces cerevisiae | ALPHASUBUNIT OF YEAST MITOCHONDRIAL PHENYL ALANYLTRNASYNTHETASE;MSF1 P |
| 138. | YPR052C | WO2004/ 092398 | 221, 222 | Saccharomyces cerevisiae | HOMOLOGOUS TO MAMMALIA NHIGHMOBILITY GROUP PROTEINS1 AND2; FUNCTIONS REDUNDANTLY WITH THE HIGHLY HOMOLOGOUS GENE |
| 139. | YN R008w | WO2004/ 092398 | 233, 234 | Saccharomyces cerevisiae | ACYLTRANSFERASE THAT CATALYZES DIACYLGLYCEROL ESTERIFICATION |
| 140. | YOL103w | WO2004/ 092398 | 203, 204 | Saccharomyces cerevisiae | MYO-INOSITOLTRANSPORTER WITH STRONG SIMILARITY TO THE MAJOR MYO-INOSITOL TRANSPORTER ITR1P P |
| 141. | YOR260w | WO2004/ 092398 | 209, 210 | Saccharomyces cerevisiae | GAMMA SUBUNIT OF THE TRANSLATION INITIATION FACTOREIF 2B |
| 142. | YOR360c | WO2004/ 092398 | 211, 212 | Saccharomyces cerevisiae | HIGH-AFFINITY CYCLIC AMP PHOSPHODIESTERASE |
| 143. | YOR084w | WO2004/ 092398 | 235, 236 | Saccharomyces cerevisiae | PUTATIVE LIPASE OF THE PEROXISOMALMATRIX |

(continued)

| Line/C olum | 1 | 2 | 3 | 4 | 5 |
|---|---|---|---|---|---|
| 144. | YPL030w | WO20060 32708 | | Saccharomyces cerevisiae | HYPOTHETICAL PROTEIN |
| 145. | YGL263W | WO2004/ 092398 | 1,2 | Saccharomyces cerevisiae | PUTATIVE MEMBRANE TRAFFICKING PROTEIN |
| 146. | YDL057w | WO20060 13010 | 129 | Saccharomyces cerevisiae | WEAK SIMILARITY TO HYPOTHETICAL PROTEIN A.THALIANA |
| 147. | YGR054W | WO2004/ 092398 | 237, 238 | Saccharomyces cerevisiae | EUKARYOTIC INITIATION FACTOR (EIF)2A |
| 148. | YBR064w | WO2004/ 092398 | 245, 246 | Saccharomyces cerevisiae | PROBABLE MEMBRANE PROTEIN |
| 149. | YAL067C | WO2004/ 092398 | 241, 242 | Saccharomyces cerevisiae | PUTATIVE PERMEASE |
| 150. | YER174c | WO2004/ 092398 | 33,34 | Saccharomyces cerevisiae | HYDROPEROXIDE, SUPEROXIDERADICAL RESPONSIVE GLUTATHIONEDEPENDENT OXIDOREDUCTASE; MONOTHIOLGLUTAREDOXINSUBFAMILY MEMBER ALONG WITH GRX3P, GRX5P; PROTECTS CELLS FROMOXIDATIVE DAMAGE |
| 151. | YBR051w | WO2004/ 092398 | 35,36 | Saccharomyces cerevisiae | PROBABLE MEMBRANE PROTEIN |
| 152. | YER175c | WO2004/ 092398 | 37, 38 | Saccharomyces cerevisiae | TRANS-ACONITATE METHYLTRANSFERASE |
| 153. | YDR521w | WO2004/ 092398 | 39,40 | Saccharomyces cerevisiae | HYPOTHETICAL PROTEIN |
| 154. | YER123w | WO2004/ 092398 | 41 | Saccharomyces cerevisiae | CASEIN KINASEI |
| 155. | YER137C | WO2004/ 092398 | 281, 282 | Saccharomyces cerevisiae | HYPOTHETICAL PROTEIN |
| 156. | YEL052w | WO2004/ 092398 | 47,48 | Saccharomyces cerevisiae | PUTATIVEATPASE OF THE CDC48/PAS1/SEC18(AAA) FAMILY |
| 157. | YDR536w | WO2004/ 092398 | 49,50 | Saccharomyces cerevisiae | STL1P:PLASMA MEMBRANE SUGAR TRANSPORTER |
| 158. | YDR513w | WO2004/ 092398 | 51,52 | Saccharomyces cerevisiae | TTR1P:GLUTAREDOXIN |
| 159. | YGR060w | WO20060 32708 | | Saccharomyces cerevisiae | ERG25C-4STEROLMETHYLOXIDASE |

(continued)

| Line/C olum | 1 | 2 | 3 | 4 | 5 |
|---|---|---|---|---|---|
| 160. | YEL041w | WO2004/ 092398 | 55, 56 | Saccharomyces cerevisiae | ATP-NADH KINASE;PHOSOPHORYLATES BOTH NAD, NADH |
| 161. | YEL001C | WO20060 32708 | 54 | Saccharomyces cerevisiae | PUTATIVE PROTEIN OF UNKNOWN FUNCTION |
| 162. | YDR415c | WO2004/ 092398 | 45,46 | Saccharomyces cerevisiae | STRONG SIMILARITY TO BACTERIAL LEUCYL AMINOPEPTIDASE |
| 163. | YDL238c | WO2004/ 092398 | 57,58 | Saccharomyces cerevisiae | GUANINEDEAMINASE |
| 164. | ROB5 | US2006/0 122375 | 1,2 | BI | LATE EMBRYOGENESIS ABUNDANT TYPE PROTEIN ROB5 |
| 165. | GOICDS_CDS 0012 | WO92/09 685 | Claim3 | Arabidopsis thaliana | CYCLIN-DEPENDENT PROTEINKINASE |
| 166. | GOICDS_CDS 0045 | WO2005/ 024029 | 4 | Arabidopsis thaliana | CYCLIN-DEPENDENT PROTEIN KINASECCDC2B2);CELLCYCLE REGULATION NOTE:THIS IS A TRUNCATED PROTEIN |
| 167. | GOICDS_CDS 0185 | WO20070 64724 | 167 | Arabidopsis thaliana | PUTATIVE TRANSCRIPTION FACTOR |
| 168. | | WO20050 94562 | 2 | Arabidopsis thaliana | PUTATIVE PP2A INHIBITOR; INVOLVED IN NUCLEOSOME ASSEMBLY |
| 169. | | WO20040 38027 | Claim 8 | Arabidopsis thaliana | CYTOKININOXIDASE; DEGRADATION OF CYTOKININ |
| 170. | GOICDS_CDS 0647 | WO20050 83094 | 2 | Arabidopsis thaliana | CYCLIN-DEPENDENT PROTEINKINASE; D-TYPE (SER/THRPROTEINKINASE); CELLCYCLE REGULATION |
| 171. | | WO2006/ 008271 | 2 | Arabidopsis thaliana | TYROSINEKINASE |
| 172. | GOICDS_CDS 1499_2 | WO01/96 580 | 2,4,6,8, , 10,12,3 2or35 | Arabidopsis thaliana | CYTOKININOXIDASE; DEGRADATION OF CYTOKININ |
| 173. | GOICDS_CDS 1532 | WO20060 45829 | 2 | Arabidopsis thaliana | SODIUMTRANSPORTER |
| 174. | GOICDS_CDS 3325 | WO20070 64724 | 199 | Arabidopsis thaliana | TRANSCRIPTION FACTOR |

(continued)

| Line/C olum | 1 | 2 | 3 | 4 | 5 |
|---|---|---|---|---|---|
| 175. | GOICDS_CDS 0218 | WO20071 13237 | 2 | Arabidopsis thaliana | CATALYZES THE DECARBOXYLATION OF 4-PHOSPHOPANTOTHENOYL CYSTEINETO 4-PHOSPHOPANTETHEINE |
| 176. | GOICDS_CDS 1536 | WO2004/ 058980 | 2 | Arabidopsis thaliana | SALT INDUCIBLE TRANSCRIPTIONAL REGULATOR (CYS-2/HIS-2-TYPE ZINC FINGER) |
| 177. | | WO2004/ 058980 | 27 | Arabidopsis thaliana | TRANSCRIPTION FACTOR; CYS2/HIS2TYPE ZINC FINGER PROTEIN |
| 178. | GOICDS_CDS 3074 | WO2006/ 079655 | 8 | Arabidopsis thaliana | SYTprotein |
| 179. | DPprotein | WO2005/ 117568 | 2 | Arabidopsis thaliana | TRANSCRIPTION FACTOR;CELL CYCLEREGULATION |
| 180. | GOICDS_CDS 0078 | WO20060 18432 | 2 | Arabidopsis thaliana | RNA BINDING;CELLP ROLIFERATION |
| 181. | GOICDS_CDS 0827 | WO20060 05771 | 2 | Arabidopsis thaliana | SERINE/THREONINE-PROTEINKINASE |
| 182. | | WO2004/ 090142 | 2 | Arabidopsis thaliana | METAL-BINDING;RESISTANCE TO METALS;DETOXIFICATION OF HYDROXYL RADICALS |
| 183. | GOICDS_CDS 1522 | WO2006/ 005751 | 2 | Arabidopsis thaliana | PUTATIVE ZINC BINDINGPROTEIN |
| 184. | GOICDS_CDS 2591 | WO20040 87755 | 2 | Arabidopsis thaliana | NON-SYMBIOTIC HEMOGLOBIN2 |
| 185. | GOICDS_CDS 3142 | WO2006/ 131547 | 14 | Arabidopsis thaliana | RECEPTOR-LIKE KINASE;SERINE/ THREONINEPROTEINKINASE;LEUCINE-RICH REPEAT(LRR)CONTAINING KINASE |
| 186. | | WO2006/ 079655 | 804 | Arabidopsis thaliana | |
| 187. | | WO2007/ 138070 | 14 | | |
| 188. | | WO20070 64724 | 132 | Arabidopsis thaliana | CELLCYCLE REGULATION |
| 189. | CDS3298 | WO2007/ 113237 | 602 | Arabidopsis thaliana | |
| 190. | | WO2004/ 090141 | 10 | BV | PUTATIVE UBIQUITIN-PROTEINLIGASE |
| 191. | GOICDS_CDS 2767 | WO20040 87755 | 2 | BV | NON-SYMBIOTIC HEMOGLOBIN2 |

(continued)

| Line/C olum | 1 | 2 | 3 | 4 | 5 |
|---|---|---|---|---|---|
| 192. | YIL076w | WO20060 32708 | 98 | Saccharomyces cerevisiae | EPSILON-COP SUBUNIT OF THE COATOMER;REGULATES RETROGRADEGOLGI-TO-ER PROTEIN TRAFFIC;STABILIZE SCOP1 P |
| 193. | YIL156W | WO20060 32708 | | Saccharomyces cerevisiae | UBIQUITIN-SPECIFIC PROTEASE THAT CLEAVE SUBIQUITIN-PROTEIN FUSIONS |
| 194. | YIL172C | WO20070 20198 | 225 | Saccharomyces cerevisiae | PUTATIVE PROTEIN OF UNKNOWN FUNCTION WITH SIMILARITY TO GLUCOSIDASES |
| 195. | YMR095C | WO20060 13010 | 2 | Saccharomyces cerevisiae | PUTATIVE GLUTAMINE AMIDOTRANSFERASE |
| 196. | YMR217W | WO20070 20198 | 244 | Saccharomyces cerevisiae | GMP SYNTHASE |
| 197. | GOICDS_CDS 0689 | WO20050 49646 | 2 | Nicotianatabacum | UNKNOWN (UNDEFINED ROLEIN CELLCYCLE) |
| 198. | GOICDS_CDS 0701 | WO20060 18432 | 15 | Nicotianatabacum | RNA BINDING;PROBABLY INVOLVED IN RNA PRCESSING(I.E.SPLICING) |
| 199. | EF-Tu | WO2006/ 067232 | 2 | Nicotianatabacum | TRANSLATION ELONGATION FACTOR; TRNA BINDING |
| 200. | GOICDS_CDS 0669 | WO20050 59147 | 2 | Nicotianatabacum | FUNCTION UNKNOWN (HYPOTHETICALLY GROWTH RELATED) |
| 201. | GOICDS_CDS 0671 | WO20040 87927 | 2 | Nicotianatabacum | AAA-ATPASE;THIS IS A TRUNCATED PROTEIN THAT CONTAINS ONLY THE ATPASE DOMAIN OFATOB3LIKEPROTEIN |
| 202. | GOICDS | WO2007/ 0647249 | 042 | Oryza sativa | |
| 203. | GOICDS_CDS 1608 | WO2004/ 106528 | 2 | Oryza sativa | VACUOLAR(NA |
| 204. | | WO20060 56590 | 2 | Oryza sativa | TRANSCRIPTION FACTOR;MAY PROMOTE FLORAL TRANSITION PAHSE, DIFFERENTIATION PROGRAM OF THE VEGETATIVE SHOOT |
| 205. | GOICDS_CDS 0644_7 | WO20060 58897 | 2 | Oryza sativa | CDKA |

(continued)

| Line/C olum | 1 | 2 | 3 | 4 | 5 |
|---|---|---|---|---|---|
| 206. | | WO20070 99096 | 2 | Oryza sativa | DNA BINDING PROTEIN;INVOLVED IN SEEDMATURATION(PUTATIVE) |
| 207. | GOICDS_CDS 2447 | WO2007/ 051866 | 2 | Oryza sativa | TRANSCRIPTION FACTOR;HOME O DOMAIN LEUCINE ZIPPERPROTEIN |
| 208. | GOICDS_CDS 3027 | WO2007/ 113237 | 2 | Oryza sativa | |
| 209. | GOISCDS_CD S3159 | WO2007/ 0647249 | 042 | Oryza sativa | |
| 210. | GOISCDS | WO2006/ 008271 | 2 | Oryza sativa | TRANSCRIPTIONFACTOR |
| 211. | GOICDS_CDS 0644_7 | WO20060 58897 | 2 | Oryza sativa | CYCLIN-DEPENDENT KINASE |
| 212. | GOICDS_CDS 1877 | WO2006/ 067236 | 2 | Oryza sativa | CO-CHAPERONE;PROTECTION OF CELLS AGAINST STRESS |
| 213. | | WO2007/ 003409 | 2 | Oryza sativa | TRANSCRIPTIONAL REGULATOR (GASIGNALLING REPRESSORINKERNELS) |
| 214. | YPL211w | WO20060 32708 | 218 | Saccharomyces cerevisiae | NUCLEOLAR PROTEIN REQUIRED FOR 60SRIBOSOME SUBUNIT BIOGENESIS |
| 215. | YIL121w | WO20060 32708 | 106 | Saccharomyces cerevisiae | MULTIDRUG TRANSPORTER REQUIRED FOR RESISTANCE TO QUINIDINE |

Table C

| Line in Table B | Gene Name/ LocusID | Active ingredientB | Line in Table B | Gene Name/ LocusID | Active ingredientB |
|---|---|---|---|---|---|
| 1 | ATACCD | acephate | 112 | YNL024c | Cyprodinil |
| 1 | ATACCD | chlorpyrifos | 112 | YNL024c | Pyrimethanil |
| 1 | ATACCD | dimethoate | 112 | YNL024c | Fenpropimorph |
| 1 | ATACCD | methamidophos | 112 | YNL024c | Tridemorph |
| 1 | ATACCD | terbufos | 112 | YNL024c | Iprodione |
| 1 | ATACCD | aldicarb | 112 | YNL024c | 5-ethyl-6-octyl-[1,2,4] triazolo [1,5-a]pyrimidine-7-ylamine |
| 1 | ATACCD | carbofuran | 112 | YNL024c | Mancozeb |
| 1 | ATACCD | bifenthrin | 112 | YNL024c | Maneb |
| 1 | ATACCD | cypermethrin | 112 | YNL024c | Metiram |
| 1 | ATACCD | alpha-cypermethrin | 112 | YNL024c | Dithianon |
| 1 | ATACCD | deltamethrin | 112 | YNL024c | Chlorothalonil |
| 1 | ATACCD | lambda-cyhalothrin | 112 | YNL024c | ThiophanateMethyl |
| 1 | ATACCD | tefluthrin | 112 | YNL024c | Cymoxanil |
| 1 | ATACCD | flufenoxuron | 112 | YNL024c | Metrafenone |
| 1 | ATACCD | teflubenzuron | 112 | YNL024c | Acetochlor |
| 1 | ATACCD | spirotetramat; | 112 | YNL024c | Dimethenamid |
| 1 | ATACCD | clothianidin | 112 | YNL024c | Metolachlor |
| 1 | ATACCD | imidacloprid | 112 | YNL024c | Metazachlor |
| 1 | ATACCD | thiamethoxam | 112 | YNL024c | Glyphosate |
| 1 | ATACCD | endosulfan | 112 | YNL024c | Glufosinate |
| 1 | ATACCD | fipronil | 112 | YNL024c | Sulfosate |
| 1 | ATACCD | abamectin | 112 | YNL024c | Fenoxaprop |
| 1 | ATACCD | spinosad | 112 | YNL024c | Paraquat |
| 1 | ATACCD | spinetoram | 112 | YNL024c | Cycloxydim |
| 1 | ATACCD | hydramethylnon; | 112 | YNL024c | Profoxydim |
| 1 | ATACCD | chlorfenapyr; | 112 | YNL024c | Sethoxydim |
| 1 | ATACCD | indoxacarb | 112 | YNL024c | Tepraloxydim |
| 1 | ATACCD | metaflumizone | 112 | YNL024c | Pendimethalin |
| 1 | ATACCD | flubendiamide | 112 | YNL024c | Acifluorfen |
| 1 | ATACCD | chlorantraniliprole | 112 | YNL024c | Imazamethabenz |
| 1 | ATACCD | cyazypyr(HGW86) | 112 | YNL024c | Imazamox |
| 1 | ATACCD | Azoxystrobin | 112 | YNL024c | Imazapic |
| 1 | ATACCD | Dimoxystrobin | 112 | YNL024c | Imazapyr |
| 1 | ATACCD | Kresoxim-methyl | 112 | YNL024c | Imazaquin |

(continued)

| Line in Table B | Gene Name/ LocusID | Active ingredientB | Line in Table B | Gene Name/ LocusID | Active ingredientB |
|---|---|---|---|---|---|
| 1 | ATACCD | Orysastrobin | 112 | YNL024c | Imazethapyr |
| 1 | ATACCD | Pyraclostrobin | 112 | YNL024c | 2,4-D |
| 1 | ATACCD | Trifloxystrobin | 112 | YNL024c | Chloridazon |
| 1 | ATACCD | Bixafen | 112 | YNL024c | Picloram |
| 1 | ATACCD | Boscalid | 112 | YNL024c | Picolinafen |
| 1 | ATACCD | Isopyrazam | 112 | YNL024c | Cyclosulfamuron |
| 1 | ATACCD | Metalaxyl | 112 | YNL024c | Triflusulfuron |
| 1 | ATACCD | Penthiopyrad | 112 | YNL024c | Atrazine |
| 1 | ATACCD | 3-Difluoromethyl-1-methyl-1 H-pyrazole-4-carboxylic acid(2', 4',5'-trifluorobiphenyl-2-yl)-amide | 112 | YNL024c | Pyroxasulfone |
| 1 | ATACCD | Dimethomorph | 112 | YNL024c | Bentazone |
| 1 | ATACCD | Difenoconazole | 112 | YNL024c | Cinidon-ethly |
| 1 | ATACCD | Epoxiconazole | 112 | YNL024c | Cinmethylin |
| 1 | ATACCD | Fluquinconazole | 112 | YNL024c | Dicamba |
| 1 | ATACCD | Metconazol | 112 | YNL024c | Diflufenzopyr |
| 1 | ATACCD | Propiconazole | 112 | YNL024c | Quinclorac |
| 1 | ATACCD | Prothioconazole | 112 | YNL024c | Quinmerac |
| 1 | ATACCD | Tebuconazole | 112 | YNL024c | Mesotrione |
| 1 | ATACCD | Triticonazole | 112 | YNL024c | Saflufenacil |
| 1 | ATACCD | Prochloraz | 112 | YNL024c | Topramezone; |
| 1 | ATACCD | Carbendazim | 150 | YER174c | acephate |
| 1 | ATACCD | Cyprodinil | 150 | YER174c | chlorpyrifos |
| 1 | ATACCD | Pyrimethanil | 150 | YER174c | dimethoate |
| 1 | ATACCD | Fenpropimorph | 150 | YER174c | methamidophos |
| 1 | ATACCD | Tridemorph | 150 | YER174c | terbufos |
| 1 | ATACCD | Iprodione | 150 | YER174c | aldicarb |
| 1 | ATACCD | 5-ethyl-6-octyl-[1,2,4] triazolo [1,5-a]pyrimidine-7-ylamine | 150 | YER174c | carbofuran |
| 1 | ATACCD | Mancozeb | 150 | YER174c | bifenthrin |
| 1 | ATACCD | Maneb | 150 | YER174c | cypermethrin |
| 1 | ATACCD | Metiram | 150 | YER174c | alpha-cypermethrin |
| 1 | ATACCD | Dithianon | 150 | YER174c | deltamethrin |
| 1 | ATACCD | Chlorothalonil | 150 | YER174c | lambda-cyhalothrin |

(continued)

| Line in Table B | Gene Name/ LocusID | Active ingredientB | Line in Table B | Gene Name/ LocusID | Active ingredientB |
|---|---|---|---|---|---|
| 1 | ATACCD | ThiophanateMethyl | 150 | YER174c | tefluthrin |
| 1 | ATACCD | Cymoxanil | 150 | YER174c | flufenoxuron |
| 1 | ATACCD | Metrafenone | 150 | YER174c | teflubenzuron |
| 1 | ATACCD | Acetochlor | 150 | YER174c | spirotetramat; |
| 1 | ATACCD | Dimethenamid | 150 | YER174c | clothianidin |
| 1 | ATACCD | Metolachlor | 150 | YER174c | imidacloprid |
| 1 | ATACCD | Metazachlor | 150 | YER174c | thiamethoxam |
| 1 | ATACCD | Glyphosate | 150 | YER174c | endosulfan |
| 1 | ATACCD | Glufosinate | 150 | YER174c | fipronil |
| 1 | ATACCD | Sulfosate | 150 | YER174c | abamectin |
| 1 | ATACCD | Fenoxaprop | 150 | YER174c | spinosad |
| 1 | ATACCD | Paraquat | 150 | YER174c | spinetoram |
| 1 | ATACCD | Cycloxydim | 150 | YER174c | hydramethylnon; |
| 1 | ATACCD | Profoxydim | 150 | YER174c | chlorfenapyr; |
| 1 | ATACCD | Sethoxydim | 150 | YER174c | indoxacarb |
| 1 | ATACCD | Tepraloxydim | 150 | YER174c | metaflumizone |
| 1 | ATACCD | Pendimethalin | 150 | YER174c | flubendiamide |
| 1 | ATACCD | Acifluorfen | 150 | YER174c | chlorantraniliprole |
| 1 | ATACCD | Imazamethabenz | 150 | YER174c | cyazypyr(HGW86) |
| 1 | ATACCD | Imazamox | 150 | YER174c | Azoxystrobin |
| 1 | ATACCD | Imazapic | 150 | YER174c | Dimoxystrobin |
| 1 | ATACCD | Imazapyr | 150 | YER174c | Kresoxim-methyl |
| 1 | ATACCD | Imazaquin | 150 | YER174c | Orysastrobin |
| 1 | ATACCD | Imazethapyr | 150 | YER174c | Pyraclostrobin |
| 1 | ATACCD | 2,4-D | 150 | YER174c | Trifloxystrobin |
| 1 | ATACCD | Chloridazon | 150 | YER174c | Bixafen |
| 1 | ATACCD | Picloram | 150 | YER174c | Boscalid |
| 1 | ATACCD | Picolinafen | 150 | YER174c | Isopyrazam |
| 1 | ATACCD | Cyclosulfamuron | 150 | YER174c | Metalaxyl |
| 1 | ATACCD | Triflusulfuron | 150 | YER174c | Penthiopyrad |
| 1 | ATACCD | Atrazine | 150 | YER174c | 3-Difluoromethyl-1-methyl-1H-pyrazole-4-carboxylic acid(2',4',5'-trifluorobiphenyl-2-yl)-amide |
| 1 | ATACCD | Pyroxasulfone | 150 | YER174c | Dimethomorph |
| 1 | ATACCD | Bentazone | 150 | YER174c | Difenoconazole |

(continued)

| Line in Table B | Gene Name/ LocusID | Active ingredientB | Line in Table B | Gene Name/ LocusID | Active ingredientB |
|---|---|---|---|---|---|
| 1 | ATACCD | Cinidon-ethly | 150 | YER174c | Epoxiconazole |
| 1 | ATACCD | Cinmethylin | 150 | YER174c | Fluquinconazole |
| 1 | ATACCD | Dicamba | 150 | YER174c | Metconazol |
| 1 | ATACCD | Diflufenzopyr | 150 | YER174c | Propiconazole |
| 1 | ATACCD | Quinclorac | 150 | YER174c | Prothioconazole |
| 1 | ATACCD | Quinmerac | 150 | YER174c | Tebuconazole |
| 1 | ATACCD | Mesotrione | 150 | YER174c | Triticonazole |
| 1 | ATACCD | Saflufenacil | 150 | YER174c | Prochloraz |
| 1 | ATACCD | Topramezone; | 150 | YER174c | Carbendazim |
| 5 | ATSTE24 | acephate | 150 | YER174c | Cyprodinil |
| 5 | ATSTE24 | chlorpyrifos | 150 | YER174c | Pyrimethanil |
| 5 | ATSTE24 | dimethoate | 150 | YER174c | Fenpropimorph |
| 5 | ATSTE24 | methamidophos | 150 | YER174c | Tridemorph |
| 5 | ATSTE24 | terbufos | 150 | YER174c | Iprodione |
| 5 | ATSTE24 | aldicarb | 150 | YER174c | 5-ethyl-6-octyl-[1,2,4] triazolo [1,5-a]pyrimidine-7-ylamine |
| 5 | ATSTE24 | carbofuran | 150 | YER174c | Mancozeb |
| 5 | ATSTE24 | bifenthrin | 150 | YER174c | Maneb |
| 5 | ATSTE24 | cypermethrin | 150 | YER174c | Metiram |
| 5 | ATSTE24 | alpha-cypermethrin | 150 | YER174c | Dithianon |
| 5 | ATSTE24 | deltamethrin | 150 | YER174c | Chlorothalonil |
| 5 | ATSTE24 | lambda-cyhalothrin | 150 | YER174c | ThiophanateMethyl |
| 5 | ATSTE24 | tefluthrin | 150 | YER174c | Cymoxanil |
| 5 | ATSTE24 | flufenoxuron | 150 | YER174c | Metrafenone |
| 5 | ATSTE24 | teflubenzuron | 150 | YER174c | Acetochlor |
| 5 | ATSTE24 | spirotetramat; | 150 | YER174c | Dimethenamid |
| 5 | ATSTE24 | clothianidin | 150 | YER174c | Metolachlor |
| 5 | ATSTE24 | imidacloprid | 150 | YER174c | Metazachlor |
| 5 | ATSTE24 | thiamethoxam | 150 | YER174c | Glyphosate |
| 5 | ATSTE24 | endosulfan | 150 | YER174c | Glufosinate |
| 5 | ATSTE24 | fipronil | 150 | YER174c | Sulfosate |
| 5 | ATSTE24 | abamectin | 150 | YER174c | Fenoxaprop |
| 5 | ATSTE24 | spinosad | 150 | YER174c | Paraquat |
| 5 | ATSTE24 | spinetoram | 150 | YER174c | Cycloxydim |
| 5 | ATSTE24 | hydramethylnon; | 150 | YER174c | Profoxydim |
| 5 | ATSTE24 | chlorfenapyr; | 150 | YER174c | Sethoxydim |

(continued)

| Line in Table B | Gene Name/ LocusID | Active ingredientB | Line in Table B | Gene Name/ LocusID | Active ingredientB |
|---|---|---|---|---|---|
| 5 | ATSTE24 | indoxacarb | 150 | YER174c | Tepraloxydim |
| 5 | ATSTE24 | metaflumizone | 150 | YER174c | Pendimethalin |
| 5 | ATSTE24 | flubendiamide | 150 | YER174c | Acifluorfen |
| 5 | ATSTE24 | chlorantraniliprole | 150 | YER174c | Imazamethabenz |
| 5 | ATSTE24 | cyazypyr(HGW86) | 150 | YER174c | Imazamox |
| 5 | ATSTE24 | Azoxystrobin | 150 | YER174c | Imazapic |
| 5 | ATSTE24 | Dimoxystrobin | 150 | YER174c | Imazapyr |
| 5 | ATSTE24 | Kresoxim-methyl | 150 | YER174c | Imazaquin |
| 5 | ATSTE24 | Orysastrobin | 150 | YER174c | Imazethapyr |
| 5 | ATSTE24 | Pyraclostrobin | 150 | YER174c | 2,4-D |
| 5 | ATSTE24 | Trifloxystrobin | 150 | YER174c | Chloridazon |
| 5 | ATSTE24 | Bixafen | 150 | YER174c | Picloram |
| 5 | ATSTE24 | Boscalid | 150 | YER174c | Picolinafen |
| 5 | ATSTE24 | Isopyrazam | 150 | YER174c | Cyclosulfamuron |
| 5 | ATSTE24 | Metalaxyl | 150 | YER174c | Triflusulfuron |
| 5 | ATSTE24 | Penthiopyrad | 150 | YER174c | Atrazine |
| 5 | ATSTE24 | 3-Difluoromethyl-1-methyl-1 H-pyrazole-4-carboxylic acid(2', 4',5'-trifluorobiphenyl-2-yl)-amide | 150 | YER174c | Pyroxasulfone |
| 5 | ATSTE24 | Dimethomorph | 150 | YER174c | Bentazone |
| 5 | ATSTE24 | Difenoconazole | 150 | YER174c | Cinidon-ethly |
| 5 | ATSTE24 | Epoxiconazole | 150 | YER174c | Cinmethylin |
| 5 | ATSTE24 | Fluquinconazole | 150 | YER174c | Dicamba |
| 5 | ATSTE24 | Metconazol | 150 | YER174c | Diflufenzopyr |
| 5 | ATSTE24 | Propiconazole | 150 | YER174c | Quinclorac |
| 5 | ATSTE24 | Prothioconazole | 150 | YER174c | Quinmerac |
| 5 | ATSTE24 | Tebuconazole | 150 | YER174c | Mesotrione |
| 5 | ATSTE24 | Triticonazole | 150 | YER174c | Saflufenacil |
| 5 | ATSTE24 | Prochloraz | 150 | YER174c | Topramezone; |
| 5 | ATSTE24 | Carbendazim | 159 | YGR060w | acephate |
| 5 | ATSTE24 | Cyprodinil | 159 | YGR060w | chlorpyrifos |
| 5 | ATSTE24 | Pyrimethanil | 159 | YGR060w | dimethoate |
| 5 | ATSTE24 | Fenpropimorph | 159 | YGR060w | methamidophos |
| 5 | ATSTE24 | Tridemorph | 159 | YGR060w | terbufos |
| 5 | ATSTE24 | Iprodione | 159 | YGR060w | aldicarb |

(continued)

| Line in Table B | Gene Name/ LocusID | Active ingredientB | Line in Table B | Gene Name/ LocusID | Active ingredientB |
|---|---|---|---|---|---|
| 5 | ATSTE24 | 5-ethyl-6-octyl-[1,2,4] triazolo [1,5-a]pyrimidine-7-ylamine | 159 | YGR060w | carbofuran |
| 5 | ATSTE24 | Mancozeb | 159 | YGR060w | bifenthrin |
| 5 | ATSTE24 | Maneb | 159 | YGR060w | cypermethrin |
| 5 | ATSTE24 | Metiram | 159 | YGR060w | alpha-cypermethrin |
| 5 | ATSTE24 | Dithianon | 159 | YGR060w | deltamethrin |
| 5 | ATSTE24 | Chlorothalonil | 159 | YGR060w | lambda-cyhalothrin |
| 5 | ATSTE24 | ThiophanateMethyl | 159 | YGR060w | tefluthrin |
| 5 | ATSTE24 | Cymoxanil | 159 | YGR060w | flufenoxuron |
| 5 | ATSTE24 | Metrafenone | 159 | YGR060w | teflubenzuron |
| 5 | ATSTE24 | Acetochlor | 159 | YGR060w | spirotetramat; |
| 5 | ATSTE24 | Dimethenamid | 159 | YGR060w | clothianidin |
| 5 | ATSTE24 | Metolachlor | 159 | YGR060w | imidacloprid |
| 5 | ATSTE24 | Metazachlor | 159 | YGR060w | thiamethoxam |
| 5 | ATSTE24 | Glyphosate | 159 | YGR060w | endosulfan |
| 5 | ATSTE24 | Glufosinate | 159 | YGR060w | fipronil |
| 5 | ATSTE24 | Sulfosate | 159 | YGR060w | abamectin |
| 5 | ATSTE24 | Fenoxaprop | 159 | YGR060w | spinosad |
| 5 | ATSTE24 | Paraquat | 159 | YGR060w | spinetoram |
| 5 | ATSTE24 | Cycloxydim | 159 | YGR060w | hydramethylnon; |
| 5 | ATSTE24 | Profoxydim | 159 | YGR060w | chlorfenapyr; |
| 5 | ATSTE24 | Sethoxydim | 159 | YGR060w | indoxacarb |
| 5 | ATSTE24 | Tepraloxydim | 159 | YGR060w | metaflumizone |
| 5 | ATSTE24 | Pendimethalin | 159 | YGR060w | flubendiamide |
| 5 | ATSTE24 | Acifluorfen | 159 | YGR060w | chlorantraniliprole |
| 5 | ATSTE24 | Imazamethabenz | 159 | YGR060w | cyazypyr(HGW86) |
| 5 | ATSTE24 | Imazamox | 159 | YGR060w | Azoxystrobin |
| 5 | ATSTE24 | Imazapic | 159 | YGR060w | Dimoxystrobin |
| 5 | ATSTE24 | Imazapyr | 159 | YGR060w | Kresoxim-methyl |
| 5 | ATSTE24 | Imazaquin | 159 | YGR060w | Orysastrobin |
| 5 | ATSTE24 | Imazethapyr | 159 | YGR060w | Pyraclostrobin |
| 5 | ATSTE24 | 2,4-D | 159 | YGR060w | Trifloxystrobin |
| 5 | ATSTE24 | Chloridazon | 159 | YGR060w | Bixafen |
| 5 | ATSTE24 | Picloram | 159 | YGR060w | Boscalid |
| 5 | ATSTE24 | Picolinafen | 159 | YGR060w | Isopyrazam |
| 5 | ATSTE24 | Cyclosulfamuron | 159 | YGR060w | Metalaxyl |

(continued)

| Line in Table B | Gene Name/ LocusID | Active ingredientB | Line in Table B | Gene Name/ LocusID | Active ingredientB |
|---|---|---|---|---|---|
| 5 | ATSTE24 | Triflusulfuron | 159 | YGR060w | Penthiopyrad |
| 5 | ATSTE24 | Atrazine | 159 | YGR060w | 3-Difluoromethyl-1-methyl-1H-pyrazole-4-carboxylic acid(2',4',5'-trifluorobiphenyl-2-yl)-amide |
| 5 | ATSTE24 | Pyroxasulfone | 159 | YGR060w | Dimethomorph |
| 5 | ATSTE24 | Bentazone | 159 | YGR060w | Difenoconazole |
| 5 | ATSTE24 | Cinidon-ethly | 159 | YGR060w | Epoxiconazole |
| 5 | ATSTE24 | Cinmethylin | 159 | YGR060w | Fluquinconazole |
| 5 | ATSTE24 | Dicamba | 159 | YGR060w | Metconazol |
| 5 | ATSTE24 | Diflufenzopyr | 159 | YGR060w | Propiconazole |
| 5 | ATSTE24 | Quinclorac | 159 | YGR060w | Prothioconazole |
| 5 | ATSTE24 | Quinmerac | 159 | YGR060w | Tebuconazole |
| 5 | ATSTE24 | Mesotrione | 159 | YGR060w | Triticonazole |
| 5 | ATSTE24 | Saflufenacil | 159 | YGR060w | Prochloraz |
| 5 | ATSTE24 | Topramezone; | 159 | YGR060w | Carbendazim |
| 7 | b2965 | acephate | 159 | YGR060w | Cyprodinil |
| 7 | b2965 | chlorpyrifos | 159 | YGR060w | Pyrimethanil |
| 7 | b2965 | dimethoate | 159 | YGR060w | Fenpropimorph |
| 7 | b2965 | methamidophos | 159 | YGR060w | Tridemorph |
| 7 | b2965 | terbufos | 159 | YGR060w | Iprodione |
| 7 | b2965 | aldicarb | 159 | YGR060w | 5-ethyl-6-octyl-[1,2,4] triazolo [1,5-a]pyrimidine-7-ylamine |
| 7 | b2965 | carbofuran | 159 | YGR060w | Mancozeb |
| 7 | b2965 | bifenthrin | 159 | YGR060w | Maneb |
| 7 | b2965 | cypermethrin | 159 | YGR060w | Metiram |
| 7 | b2965 | alpha-cypermethrin | 159 | YGR060w | Dithianon |
| 7 | b2965 | deltamethrin | 159 | YGR060w | Chlorothalonil |
| 7 | b2965 | lambda-cyhalothrin | 159 | YGR060w | ThiophanateMethyl |
| 7 | b2965 | tefluthrin | 159 | YGR060w | Cymoxanil |
| 7 | b2965 | flufenoxuron | 159 | YGR060w | Metrafenone |
| 7 | b2965 | teflubenzuron | 159 | YGR060w | Acetochlor |
| 7 | b2965 | spirotetramat; | 159 | YGR060w | Dimethenamid |
| 7 | b2965 | clothianidin | 159 | YGR060w | Metolachlor |
| 7 | b2965 | imidacloprid | 159 | YGR060w | Metazachlor |

(continued)

| Line in Table B | Gene Name/ LocusID | Active ingredientB | Line in Table B | Gene Name/ LocusID | Active ingredientB |
|---|---|---|---|---|---|
| 7 | b2965 | thiamethoxam | 159 | YGR060w | Glyphosate |
| 7 | b2965 | endosulfan | 159 | YGR060w | Glufosinate |
| 7 | b2965 | fipronil | 159 | YGR060w | Sulfosate |
| 7 | b2965 | abamectin | 159 | YGR060w | Fenoxaprop |
| 7 | b2965 | spinosad | 159 | YGR060w | Paraquat |
| 7 | b2965 | spinetoram | 159 | YGR060w | Cycloxydim |
| 7 | b2965 | hydramethylnon; | 159 | YGR060w | Profoxydim |
| 7 | b2965 | chlorfenapyr; | 159 | YGR060w | Sethoxydim |
| 7 | b2965 | indoxacarb | 159 | YGR060w | Tepraloxydim |
| 7 | b2965 | metaflumizone | 159 | YGR060w | Pendimethalin |
| 7 | b2965 | flubendiamide | 159 | YGR060w | Acifluorfen |
| 7 | b2965 | chlorantraniliprole | 159 | YGR060w | Imazamethabenz |
| 7 | b2965 | cyazypyr(HGW86) | 159 | YGR060w | Imazamox |
| 7 | b2965 | Azoxystrobin | 159 | YGR060w | Imazapic |
| 7 | b2965 | Dimoxystrobin | 159 | YGR060w | Imazapyr |
| 7 | b2965 | Kresoxim-methyl | 159 | YGR060w | Imazaquin |
| 7 | b2965 | Orysastrobin | 159 | YGR060w | Imazethapyr |
| 7 | b2965 | Pyraclostrobin | 159 | YGR060w | 2,4-D |
| 7 | b2965 | Trifloxystrobin | 159 | YGR060w | Chloridazon |
| 7 | b2965 | Bixafen | 159 | YGR060w | Picloram |
| 7 | b2965 | Boscalid | 159 | YGR060w | Picolinafen |
| 7 | b2965 | Isopyrazam | 159 | YGR060w | Cyclosulfamuron |
| 7 | b2965 | Metalaxyl | 159 | YGR060w | Triflusulfuron |
| 7 | b2965 | Penthiopyrad | 159 | YGR060w | Atrazine |
| 7 | b2965 | 3-Difluoromethyl-1-methyl-1H-pyrazole-4-carboxylic acid(2',4',5'-trifluorobiphenyl-2-yl)-amide | 159 | YGR060w | Pyroxasulfone |
| 7 | b2965 | Dimethomorph | 159 | YGR060w | Bentazone |
| 7 | b2965 | Difenoconazole | 159 | YGR060w | Cinidon-ethly |
| 7 | b2965 | Epoxiconazole | 159 | YGR060w | Cinmethylin |
| 7 | b2965 | Fluquinconazole | 159 | YGR060w | Dicamba |
| 7 | b2965 | Metconazol | 159 | YGR060w | Diflufenzopyr |
| 7 | b2965 | Propiconazole | 159 | YGR060w | Quinclorac |
| 7 | b2965 | Prothioconazole | 159 | YGR060w | Quinmerac |
| 7 | b2965 | Tebuconazole | 159 | YGR060w | Mesotrione |

(continued)

| Line in Table B | Gene Name/ LocusID | Active ingredientB | Line in Table B | Gene Name/ LocusID | Active ingredientB |
|---|---|---|---|---|---|
| 7 | b2965 | Triticonazole | 159 | YGR060w | Saflufenacil |
| 7 | b2965 | Prochloraz | 159 | YGR060w | Topramezone; |
| 7 | b2965 | Carbendazim | 164 | ROB5 | acephate |
| 7 | b2965 | Cyprodinil | 164 | ROB5 | chlorpyrifos |
| 7 | b2965 | Pyrimethanil | 164 | ROB5 | dimethoate |
| 7 | b2965 | Fenpropimorph | 164 | ROB5 | methamidophos |
| 7 | b2965 | Tridemorph | 164 | ROB5 | terbufos |
| 7 | b2965 | Iprodione | 164 | ROB5 | aldicarb |
| 7 | b2965 | 5-ethyl-6-octyl-[1,2,4] triazolo [1,5-a]pyrimidine-7-ylamine | 164 | ROB5 | carbofuran |
| 7 | b2965 | Mancozeb | 164 | ROB5 | bifenthrin |
| 7 | b2965 | Maneb | 164 | ROB5 | cypermethrin |
| 7 | b2965 | Metiram | 164 | ROB5 | alpha-cypermethrin |
| 7 | b2965 | Dithianon | 164 | ROB5 | deltamethrin |
| 7 | b2965 | Chlorothalonil | 164 | ROB5 | lambda-cyhalothrin |
| 7 | b2965 | ThiophanateMethyl | 164 | ROB5 | tefluthrin |
| 7 | b2965 | Cymoxanil | 164 | ROB5 | flufenoxuron |
| 7 | b2965 | Metrafenone | 164 | ROB5 | teflubenzuron |
| 7 | b2965 | Acetochlor | 164 | ROB5 | spirotetramat; |
| 7 | b2965 | Dimethenamid | 164 | ROB5 | clothianidin |
| 7 | b2965 | Metolachlor | 164 | ROB5 | imidacloprid |
| 7 | b2965 | Metazachlor | 164 | ROB5 | thiamethoxam |
| 7 | b2965 | Glyphosate | 164 | ROB5 | endosulfan |
| 7 | b2965 | Glufosinate | 164 | ROB5 | fipronil |
| 7 | b2965 | Sulfosate | 164 | ROB5 | abamectin |
| 7 | b2965 | Fenoxaprop | 164 | ROB5 | spinosad |
| 7 | b2965 | Paraquat | 164 | ROB5 | spinetoram |
| 7 | b2965 | Cycloxydim | 164 | ROB5 | hydramethylnon; |
| 7 | b2965 | Profoxydim | 164 | ROB5 | chlorfenapyr; |
| 7 | b2965 | Sethoxydim | 164 | ROB5 | indoxacarb |
| 7 | b2965 | Tepraloxydim | 164 | ROB5 | metaflumizone |
| 7 | b2965 | Pendimethalin | 164 | ROB5 | flubendiamide |
| 7 | b2965 | Acifluorfen | 164 | ROB5 | chlorantraniliprole |
| 7 | b2965 | Imazamethabenz | 164 | ROB5 | cyazypyr(HGW86) |
| 7 | b2965 | Imazamox | 164 | ROB5 | Azoxystrobin |
| 7 | b2965 | Imazapic | 164 | ROB5 | Dimoxystrobin |

(continued)

| Line in Table B | Gene Name/ LocusID | Active ingredientB | Line in Table B | Gene Name/ LocusID | Active ingredientB |
|---|---|---|---|---|---|
| 7 | b2965 | Imazapyr | 164 | ROB5 | Kresoxim-methyl |
| 7 | b2965 | Imazaquin | 164 | ROB5 | Orysastrobin |
| 7 | b2965 | Imazethapyr | 164 | ROB5 | Pyraclostrobin |
| 7 | b2965 | 2,4-D | 164 | ROB5 | Trifloxystrobin |
| 7 | b2965 | Chloridazon | 164 | ROB5 | Bixafen |
| 7 | b2965 | Picloram | 164 | ROB5 | Boscalid |
| 7 | b2965 | Picolinafen | 164 | ROB5 | Isopyrazam |
| 7 | b2965 | Cyclosulfamuron | 164 | ROB5 | Metalaxyl |
| 7 | b2965 | Triflusulfuron | 164 | ROB5 | Penthiopyrad |
| 7 | b2965 | Atrazine | 164 | ROB5 | 3-Difluoromethyl-1-methyl-1H-pyrazole-4-carboxylic acid(2',4',5'-trifluorobiphenyl-2-yl)-amide |
| 7 | b2965 | Pyroxasulfone | 164 | ROB5 | Dimethomorph |
| 7 | b2965 | Bentazone | 164 | ROB5 | Difenoconazole |
| 7 | b2965 | Cinidon-ethly | 164 | ROB5 | Epoxiconazole |
| 7 | b2965 | Cinmethylin | 164 | ROB5 | Fluquinconazole |
| 7 | b2965 | Dicamba | 164 | ROB5 | Metconazol |
| 7 | b2965 | Diflufenzopyr | 164 | ROB5 | Propiconazole |
| 7 | b2965 | Quinclorac | 164 | ROB5 | Prothioconazole |
| 7 | b2965 | Quinmerac | 164 | ROB5 | Tebuconazole |
| 7 | b2965 | Mesotrione | 164 | ROB5 | Triticonazole |
| 7 | b2965 | Saflufenacil | 164 | ROB5 | Prochloraz |
| 7 | b2965 | Topramezone; | 164 | ROB5 | Carbendazim |
| 10 | B0730 | acephate | 164 | ROB5 | Cyprodinil |
| 10 | B0730 | chlorpyrifos | 164 | ROB5 | Pyrimethanil |
| 10 | B0730 | dimethoate | 164 | ROB5 | Fenpropimorph |
| 10 | B0730 | methamidophos | 164 | ROB5 | Tridemorph |
| 10 | B0730 | terbufos | 164 | ROB5 | Iprodione |
| 10 | B0730 | aldicarb | 164 | ROB5 | 5-ethyl-6-octyl-[1,2,4] triazolo [1,5-a]pyrimidine-7-ylamine |
| 10 | B0730 | carbofuran | 164 | ROB5 | Mancozeb |
| 10 | B0730 | bifenthrin | 164 | ROB5 | Maneb |
| 10 | B0730 | cypermethrin | 164 | ROB5 | Metiram |
| 10 | B0730 | alpha-cypermethrin | 164 | ROB5 | Dithianon |

(continued)

| Line in Table B | Gene Name/ LocusID | Active ingredientB | Line in Table B | Gene Name/ LocusID | Active ingredientB |
|---|---|---|---|---|---|
| 10 | B0730 | deltamethrin | 164 | ROB5 | Chlorothalonil |
| 10 | B0730 | lambda-cyhalothrin | 164 | ROB5 | ThiophanateMethyl |
| 10 | B0730 | tefluthrin | 164 | ROB5 | Cymoxanil |
| 10 | B0730 | flufenoxuron | 164 | ROB5 | Metrafenone |
| 10 | B0730 | teflubenzuron | 164 | ROB5 | Acetochlor |
| 10 | B0730 | spirotetramat; | 164 | ROB5 | Dimethenamid |
| 10 | B0730 | clothianidin | 164 | ROB5 | Metolachlor |
| 10 | B0730 | imidacloprid | 164 | ROB5 | Metazachlor |
| 10 | B0730 | thiamethoxam | 164 | ROB5 | Glyphosate |
| 10 | B0730 | endosulfan | 164 | ROB5 | Glufosinate |
| 10 | B0730 | fipronil | 164 | ROB5 | Sulfosate |
| 10 | B0730 | abamectin | 164 | ROB5 | Fenoxaprop |
| 10 | B0730 | spinosad | 164 | ROB5 | Paraquat |
| 10 | B0730 | spinetoram | 164 | ROB5 | Cycloxydim |
| 10 | B0730 | hydramethylnon; | 164 | ROB5 | Profoxydim |
| 10 | B0730 | chlorfenapyr; | 164 | ROB5 | Sethoxydim |
| 10 | B0730 | indoxacarb | 164 | ROB5 | Tepraloxydim |
| 10 | B0730 | metaflumizone | 164 | ROB5 | Pendimethalin |
| 10 | B0730 | flubendiamide | 164 | ROB5 | Acifluorfen |
| 10 | B0730 | chlorantraniliprole | 164 | ROB5 | Imazamethabenz |
| 10 | B0730 | cyazypyr(HGW86) | 164 | ROB5 | Imazamox |
| 10 | B0730 | Azoxystrobin | 164 | ROB5 | Imazapic |
| 10 | B0730 | Dimoxystrobin | 164 | ROB5 | Imazapyr |
| 10 | B0730 | Kresoxim-methyl | 164 | ROB5 | Imazaquin |
| 10 | B0730 | Orysastrobin | 164 | ROB5 | Imazethapyr |
| 10 | B0730 | Pyraclostrobin | 164 | ROB5 | 2,4-D |
| 10 | B0730 | Trifloxystrobin | 164 | ROB5 | Chloridazon |
| 10 | B0730 | Bixafen | 164 | ROB5 | Picloram |
| 10 | B0730 | Boscalid | 164 | ROB5 | Picolinafen |
| 10 | B0730 | Isopyrazam | 164 | ROB5 | Cyclosulfamuron |
| 10 | B0730 | Metalaxyl | 164 | ROB5 | Triflusulfuron |
| 10 | B0730 | Penthiopyrad | 164 | ROB5 | Atrazine |
| 10 | B0730 | 3-Difluoromethyl-1-methyl-1 H-pyrazole-4-carboxylic acid(2',4',5'-trifluorobiphenyl-2-yl)-amide | 164 | ROB5 | Pyroxasulfone |

(continued)

| Line in Table B | Gene Name/ LocusID | Active ingredientB | Line in Table B | Gene Name/ LocusID | Active ingredientB |
|---|---|---|---|---|---|
| 10 | B0730 | Dimethomorph | 164 | ROB5 | Bentazone |
| 10 | B0730 | Difenoconazole | 164 | ROB5 | Cinidon-ethly |
| 10 | B0730 | Epoxiconazole | 164 | ROB5 | Cinmethylin |
| 10 | B0730 | Fluquinconazole | 164 | ROB5 | Dicamba |
| 10 | B0730 | Metconazol | 164 | ROB5 | Diflufenzopyr |
| 10 | B0730 | Propiconazole | 164 | ROB5 | Quinclorac |
| 10 | B0730 | Prothioconazole | 164 | ROB5 | Quinmerac |
| 10 | B0730 | Tebuconazole | 164 | ROB5 | Mesotrione |
| 10 | B0730 | Triticonazole | 164 | ROB5 | Saflufenacil |
| 10 | B0730 | Prochloraz | 164 | ROB5 | Topramezone; |
| 10 | B0730 | Carbendazim | 165 | GOICDS_CDS0012 | acephate |
| 10 | B0730 | Cyprodinil | 165 | GOICDS_CDS0012 | chlorpyrifos |
| 10 | B0730 | Pyrimethanil | 165 | GOICDS_CDS0012 | dimethoate |
| 10 | B0730 | Fenpropimorph | 165 | GOICDS_CDS0012 | methamidophos |
| 10 | B0730 | Tridemorph | 165 | GOICDS_CDS0012 | terbufos |
| 10 | B0730 | Iprodione | 165 | GOICDS_CDS0012 | aldicarb |
| 10 | B0730 | 5-ethyl-6-octyl-[1,2,4] triazolo [1,5-a]pyrimidine-7-ylamine | 165 | GOICDS_CDS0012 | carbofuran |
| 10 | B0730 | Mancozeb | 165 | GOICDS_CDS0012 | bifenthrin |
| 10 | B0730 | Maneb | 165 | GOICDS_CDS0012 | cypermethrin |
| 10 | B0730 | Metiram | 165 | GOICDS_CDS0012 | alpha-cypermethrin |
| 10 | B0730 | Dithianon | 165 | GOICDS_CDS0012 | deltamethrin |
| 10 | B0730 | Chlorothalonil | 165 | GOICDS_CDS0012 | lambda-cyhalothrin |
| 10 | B0730 | ThiophanateMethyl | 165 | GOICDS_CDS0012 | tefluthrin |
| 10 | B0730 | Cymoxanil | 165 | GOICDS_CDS0012 | flufenoxuron |
| 10 | B0730 | Metrafenone | 165 | GOICDS_CDS0012 | teflubenzuron |

(continued)

| Line in Table B | Gene Name/ LocusID | Active ingredientB | Line in Table B | Gene Name/ LocusID | Active ingredientB |
|---|---|---|---|---|---|
| 10 | B0730 | Acetochlor | 165 | GOICDS_C DS0012 | spirotetramat; |
| 10 | B0730 | Dimethenamid | 165 | GOICDS_C DS0012 | clothianidin |
| 10 | B0730 | Metolachlor | 165 | GOICDS_C DS0012 | imidacloprid |
| 10 | B0730 | Metazachlor | 165 | GOICDS_C DS0012 | thiamethoxam |
| 10 | B0730 | Glyphosate | 165 | GOICDS_C DS0012 | endosulfan |
| 10 | B0730 | Glufosinate | 165 | GOICDS_C DS0012 | fipronil |
| 10 | B0730 | Sulfosate | 165 | GOICDS_C DS0012 | abamectin |
| 10 | B0730 | Fenoxaprop | 165 | GOICDS_C DS0012 | spinosad |
| 10 | B0730 | Paraquat | 165 | GOICDS_C DS0012 | spinetoram |
| 10 | B0730 | Cycloxydim | 165 | GOICDS_C DS0012 | hydramethylnon; |
| 10 | B0730 | Profoxydim | 165 | GOICDS_C DS0012 | chlorfenapyr; |
| 10 | B0730 | Sethoxydim | 165 | GOICDS_C DS0012 | indoxacarb |
| 10 | B0730 | Tepraloxydim | 165 | GOICDS_C DS0012 | metaflumizone |
| 10 | B0730 | Pendimethalin | 165 | GOICDS_C DS0012 | flubendiamide |
| 10 | B0730 | Acifluorfen | 165 | GOICDS_C DS0012 | chlorantraniliprole |
| 10 | B0730 | Imazamethabenz | 165 | GOICDS_C DS0012 | cyazypyr(HGW86) |
| 10 | B0730 | Imazamox | 165 | GOICDS_C DS0012 | Azoxystrobin |
| 10 | B0730 | Imazapic | 165 | GOICDS_C DS0012 | Dimoxystrobin |
| 10 | B0730 | Imazapyr | 165 | GOICDS_C DS0012 | Kresoxim-methyl |
| 10 | B0730 | Imazaquin | 165 | GOICDS_C DS0012 | Orysastrobin |
| 10 | B0730 | Imazethapyr | 165 | GOICDS_C DS0012 | Pyraclostrobin |
| 10 | B0730 | 2,4-D | 165 | GOICDS_C DS0012 | Trifloxystrobin |

(continued)

| Line in Table B | Gene Name/ LocusID | Active ingredientB | Line in Table B | Gene Name/ LocusID | Active ingredientB |
|---|---|---|---|---|---|
| 10 | B0730 | Chloridazon | 165 | GOICDS_C DS0012 | Bixafen |
| 10 | B0730 | Picloram | 165 | GOICDS_C DS0012 | Boscalid |
| 10 | B0730 | Picolinafen | 165 | GOICDS_C DS0012 | Isopyrazam |
| 10 | B0730 | Cyclosulfamuron | 165 | GOICDS_C DS0012 | Metalaxyl |
| 10 | B0730 | Triflusulfuron | 165 | GOICDS_C DS0012 | Penthiopyrad |
| 10 | B0730 | Atrazine | 165 | GOICDS_C DS0012 | 3-Difluoromethyl-1-methyl-1H-pyrazole-4-carboxylic acid(2',4',5'-trifluorobiphenyl-2-yl)-amide |
| 10 | B0730 | Pyroxasulfone | 165 | GOICDS_C DS0012 | Dimethomorph |
| 10 | B0730 | Bentazone | 165 | GOICDS_C DS0012 | Difenoconazole |
| 10 | B0730 | Cinidon-ethly | 165 | GOICDS_C DS0012 | Epoxiconazole |
| 10 | B0730 | Cinmethylin | 165 | GOICDS_C DS0012 | Fluquinconazole |
| 10 | B0730 | Dicamba | 165 | GOICDS_C DS0012 | Metconazol |
| 10 | B0730 | Diflufenzopyr | 165 | GOICDS_C DS0012 | Propiconazole |
| 10 | B0730 | Quinclorac | 165 | GOICDS_C DS0012 | Prothioconazole |
| 10 | B0730 | Quinmerac | 165 | GOICDS_C DS0012 | Tebuconazole |
| 10 | B0730 | Mesotrione | 165 | GOICDS_C DS0012 | Triticonazole |
| 10 | B0730 | Saflufenacil | 165 | GOICDS_C DS0012 | Prochloraz |
| 10 | B0730 | Topramezone; | 165 | GOICDS_C DS0012 | Carbendazim |
| 11 | b1830 | acephate | 165 | GOICDS_C DS0012 | Cyprodinil |
| 11 | b1830 | chlorpyrifos | 165 | GOICDS_C DS0012 | Pyrimethanil |
| 11 | b1830 | dimethoate | 165 | GOICDS_C DS0012 | Fenpropimorph |

(continued)

| Line in Table B | Gene Name/ LocusID | Active ingredientB | Line in Table B | Gene Name/ LocusID | Active ingredientB |
|---|---|---|---|---|---|
| 11 | b1830 | methamidophos | 165 | GOICDS_C DS0012 | Tridemorph |
| 11 | b1830 | terbufos | 165 | GOICDS_C DS0012 | Iprodione |
| 11 | b1830 | aldicarb | 165 | GOICDS_C DS0012 | 5-ethyl-6-octyl-[1,2,4] triazolo [1,5-a]pyrimidine-7-ylamine |
| 11 | b1830 | carbofuran | 165 | GOICDS_C DS0012 | Mancozeb |
| 11 | b1830 | bifenthrin | 165 | GOICDS_C DS0012 | Maneb |
| 11 | b1830 | cypermethrin | 165 | GOICDS_C DS0012 | Metiram |
| 11 | b1830 | alpha-cypermethrin | 165 | GOICDS_C DS0012 | Dithianon |
| 11 | b1830 | deltamethrin | 165 | GOICDS_C DS0012 | Chlorothalonil |
| 11 | b1830 | lambda-cyhalothrin | 165 | GOICDS_C DS0012 | ThiophanateMethyl |
| 11 | b1830 | tefluthrin | 165 | GOICDS_C DS0012 | Cymoxanil |
| 11 | b1830 | flufenoxuron | 165 | GOICDS_C DS0012 | Metrafenone |
| 11 | b1830 | teflubenzuron | 165 | GOICDS_C DS0012 | Acetochlor |
| 11 | b1830 | spirotetramat; | 165 | GOICDS_C DS0012 | Dimethenamid |
| 11 | b1830 | clothianidin | 165 | GOICDS_C DS0012 | Metolachlor |
| 11 | b1830 | imidacloprid | 165 | GOICDS_C DS0012 | Metazachlor |
| 11 | b1830 | thiamethoxam | 165 | GOICDS_C DS0012 | Glyphosate |
| 11 | b1830 | endosulfan | 165 | GOICDS_C DS0012 | Glufosinate |
| 11 | b1830 | fipronil | 165 | GOICDS_C DS0012 | Sulfosate |
| 11 | b1830 | abamectin | 165 | GOICDS_C DS0012 | Fenoxaprop |
| 11 | b1830 | spinosad | 165 | GOICDS_C DS0012 | Paraquat |
| 11 | b1830 | spinetoram | 165 | GOICDS_C DS0012 | Cycloxydim |

(continued)

| Line in Table B | Gene Name/ LocusID | Active ingredientB | Line in Table B | Gene Name/ LocusID | Active ingredientB |
|---|---|---|---|---|---|
| 11 | b1830 | hydramethylnon; | 165 | GOICDS_C DS0012 | Profoxydim |
| 11 | b1830 | chlorfenapyr; | 165 | GOICDS_C DS0012 | Sethoxydim |
| 11 | b1830 | indoxacarb | 165 | GOICDS_C DS0012 | Tepraloxydim |
| 11 | b1830 | metaflumizone | 165 | GOICDS_C DS0012 | Pendimethalin |
| 11 | b1830 | flubendiamide | 165 | GOICDS_C DS0012 | Acifluorfen |
| 11 | b1830 | chlorantraniliprole | 165 | GOICDS_C DS0012 | Imazamethabenz |
| 11 | b1830 | cyazypyr(HGW86) | 165 | GOICDS_C DS0012 | Imazamox |
| 11 | b1830 | Azoxystrobin | 165 | GOICDS_C DS0012 | Imazapic |
| 11 | b1830 | Dimoxystrobin | 165 | GOICDS_C DS0012 | Imazapyr |
| 11 | b1830 | Kresoxim-methyl | 165 | GOICDS_C DS0012 | Imazaquin |
| 11 | b1830 | Orysastrobin | 165 | GOICDS_C DS0012 | Imazethapyr |
| 11 | b1830 | Pyraclostrobin | 165 | GOICDS_C DS0012 | 2,4-D |
| 11 | b1830 | Trifloxystrobin | 165 | GOICDS_C DS0012 | Chloridazon |
| 11 | b1830 | Bixafen | 165 | GOICDS_C DS0012 | Picloram |
| 11 | b1830 | Boscalid | 165 | GOICDS_C DS0012 | Picolinafen |
| 11 | b1830 | Isopyrazam | 165 | GOICDS_C DS0012 | Cyclosulfamuron |
| 11 | b1830 | Metalaxyl | 165 | GOICDS_C DS0012 | Triflusulfuron |
| 11 | b1830 | Penthiopyrad | 165 | GOICDS_C DS0012 | Atrazine |
| 11 | b1830 | 3-Difluoromethyl-1-methyl-1 H-pyrazole-4-carboxylic acid(2',4',5'-trifluorobiphenyl-2-yl)-amide | 165 | GOICDS_C DS0012 | Pyroxasulfone |
| 11 | b1830 | Dimethomorph | 165 | GOICDS_C DS0012 | Bentazone |

(continued)

| Line in Table B | Gene Name/ LocusID | Active ingredientB | Line in Table B | Gene Name/ LocusID | Active ingredientB |
|---|---|---|---|---|---|
| 11 | b1830 | Difenoconazole | 165 | GOICDS_CDS0012 | Cinidon-ethly |
| 11 | b1830 | Epoxiconazole | 165 | GOICDS_CDS0012 | Cinmethylin |
| 11 | b1830 | Fluquinconazole | 165 | GOICDS_CDS0012 | Dicamba |
| 11 | b1830 | Metconazol | 165 | GOICDS_CDS0012 | Diflufenzopyr |
| 11 | b1830 | Propiconazole | 165 | GOICDS_CDS0012 | Quinclorac |
| 11 | b1830 | Prothioconazole | 165 | GOICDS_CDS0012 | Quinmerac |
| 11 | b1830 | Tebuconazole | 165 | GOICDS_CDS0012 | Mesotrione |
| 11 | b1830 | Triticonazole | 165 | GOICDS_CDS0012 | Saflufenacil |
| 11 | b1830 | Prochloraz | 165 | GOICDS_CDS0012 | Topramezone; |
| 11 | b1830 | Carbendazim | 166 | GOICDS_CDS0045 | acephate |
| 11 | b1830 | Cyprodinil | 166 | GOICDS_CDS0045 | chlorpyrifos |
| 11 | b1830 | Pyrimethanil | 166 | GOICDS_CDS0045 | dimethoate |
| 11 | b1830 | Fenpropimorph | 166 | GOICDS_CDS0045 | methamidophos |
| 11 | b1830 | Tridemorph | 166 | GOICDS_CDS0045 | terbufos |
| 11 | b1830 | Iprodione | 166 | GOICDS_CDS0045 | aldicarb |
| 11 | b1830 | 5-ethyl-6-octyl-[1,2,4] triazolo [1,5-a]pyrimidine-7-ylamine | 166 | GOICDS_CDS0045 | carbofuran |
| 11 | b1830 | Mancozeb | 166 | GOICDS_CDS0045 | bifenthrin |
| 11 | b1830 | Maneb | 166 | GOICDS_CDS0045 | cypermethrin |
| 11 | b1830 | Metiram | 166 | GOICDS_CDS0045 | alpha-cypermethrin |
| 11 | b1830 | Dithianon | 166 | GOICDS_CDS0045 | deltamethrin |
| 11 | b1830 | Chlorothalonil | 166 | GOICDS_CDS0045 | lambda-cyhalothrin |

(continued)

| Line in Table B | Gene Name/ LocusID | Active ingredientB | Line in Table B | Gene Name/ LocusID | Active ingredientB |
|---|---|---|---|---|---|
| 11 | b1830 | ThiophanateMethyl | 166 | GOICDS_C DS0045 | tefluthrin |
| 11 | b1830 | Cymoxanil | 166 | GOICDS_C DS0045 | flufenoxuron |
| 11 | b1830 | Metrafenone | 166 | GOICDS_C DS0045 | teflubenzuron |
| 11 | b1830 | Acetochlor | 166 | GOICDS_C DS0045 | spirotetramat; |
| 11 | b1830 | Dimethenamid | 166 | GOICDS_C DS0045 | clothianidin |
| 11 | b1830 | Metolachlor | 166 | GOICDS_C DS0045 | imidacloprid |
| 11 | b1830 | Metazachlor | 166 | GOICDS_C DS0045 | thiamethoxam |
| 11 | b1830 | Glyphosate | 166 | GOICDS_C DS0045 | endosulfan |
| 11 | b1830 | Glufosinate | 166 | GOICDS_C DS0045 | fipronil |
| 11 | b1830 | Sulfosate | 166 | GOICDS_C DS0045 | abamectin |
| 11 | b1830 | Fenoxaprop | 166 | GOICDS_C DS0045 | spinosad |
| 11 | b1830 | Paraquat | 166 | GOICDS_C DS0045 | spinetoram |
| 11 | b1830 | Cycloxydim | 166 | GOICDS_C DS0045 | hydramethylnon; |
| 11 | b1830 | Profoxydim | 166 | GOICDS_C DS0045 | chlorfenapyr; |
| 11 | b1830 | Sethoxydim | 166 | GOICDS_C DS0045 | indoxacarb |
| 11 | b1830 | Tepraloxydim | 166 | GOICDS_C DS0045 | metaflumizone |
| 11 | b1830 | Pendimethalin | 166 | GOICDS_C DS0045 | flubendiamide |
| 11 | b1830 | Acifluorfen | 166 | GOICDS_C DS0045 | chlorantraniliprole |
| 11 | b1830 | Imazamethabenz | 166 | GOICDS_C DS0045 | cyazypyr(HGW86) |
| 11 | b1830 | Imazamox | 166 | GOICDS_C DS0045 | Azoxystrobin |
| 11 | b1830 | Imazapic | 166 | GOICDS_C DS0045 | Dimoxystrobin |
| 11 | b1830 | Imazapyr | 166 | GOICDS_C DS0045 | Kresoxim-methyl |

(continued)

| Line in Table B | Gene Name/ LocusID | Active ingredientB | Line in Table B | Gene Name/ LocusID | Active ingredientB |
|---|---|---|---|---|---|
| 11 | b1830 | Imazaquin | 166 | GOICDS_C DS0045 | Orysastrobin |
| 11 | b1830 | Imazethapyr | 166 | GOICDS_C DS0045 | Pyraclostrobin |
| 11 | b1830 | 2,4-D | 166 | GOICDS_C DS0045 | Trifloxystrobin |
| 11 | b1830 | Chloridazon | 166 | GOICDS_C DS0045 | Bixafen |
| 11 | b1830 | Picloram | 166 | GOICDS_C DS0045 | Boscalid |
| 11 | b1830 | Picolinafen | 166 | GOICDS_C DS0045 | Isopyrazam |
| 11 | b1830 | Cyclosulfamuron | 166 | GOICDS_C DS0045 | Metalaxyl |
| 11 | b1830 | Triflusulfuron | 166 | GOICDS_C DS0045 | Penthiopyrad |
| 11 | b1830 | Atrazine | 166 | GOICDS_C DS0045 | 3-Difluoromethyl-1-methyl-1H-pyrazole-4-carboxylic acid(2',4',5'-trifluorobiphenyl-2-yl)-amide |
| 11 | b1830 | Pyroxasulfone | 166 | GOICDS_C DS0045 | Dimethomorph |
| 11 | b1830 | Bentazone | 166 | GOICDS_C DS0045 | Difenoconazole |
| 11 | b1830 | Cinidon-ethly | 166 | GOICDS_C DS0045 | Epoxiconazole |
| 11 | b1830 | Cinmethylin | 166 | GOICDS_C DS0045 | Fluquinconazole |
| 11 | b1830 | Dicamba | 166 | GOICDS_C DS0045 | Metconazol |
| 11 | b1830 | Diflufenzopyr | 166 | GOICDS_C DS0045 | Propiconazole |
| 11 | b1830 | Quinclorac | 166 | GOICDS_C DS0045 | Prothioconazole |
| 11 | b1830 | Quinmerac | 166 | GOICDS_C DS0045 | Tebuconazole |
| 11 | b1830 | Mesotrione | 166 | GOICDS_C DS0045 | Triticonazole |
| 11 | b1830 | Saflufenacil | 166 | GOICDS_C DS0045 | Prochloraz |
| 11 | b1830 | Topramezone; | 166 | GOICDS_C DS0045 | Carbendazim |

(continued)

| Line in Table B | Gene Name/ LocusID | Active ingredientB | Line in Table B | Gene Name/ LocusID | Active ingredientB |
|---|---|---|---|---|---|
| 12 | b2664 | acephate | 166 | GOICDS_C DS0045 | Cyprodinil |
| 12 | b2664 | chlorpyrifos | 166 | GOICDS_C DS0045 | Pyrimethanil |
| 12 | b2664 | dimethoate | 166 | GOICDS_C DS0045 | Fenpropimorph |
| 12 | b2664 | methamidophos | 166 | GOICDS_C DS0045 | Tridemorph |
| 12 | b2664 | terbufos | 166 | GOICDS_C DS0045 | Iprodione |
| 12 | b2664 | aldicarb | 166 | GOICDS_C DS0045 | 5-ethyl-6-octyl-[1,2,4] triazolo [1,5-a]pyrimidine-7-ylamine |
| 12 | b2664 | carbofuran | 166 | GOICDS_C DS0045 | Mancozeb |
| 12 | b2664 | bifenthrin | 166 | GOICDS_C DS0045 | Maneb |
| 12 | b2664 | cypermethrin | 166 | GOICDS_C DS0045 | Metiram |
| 12 | b2664 | alpha-cypermethrin | 166 | GOICDS_C DS0045 | Dithianon |
| 12 | b2664 | deltamethrin | 166 | GOICDS_C DS0045 | Chlorothalonil |
| 12 | b2664 | lambda-cyhalothrin | 166 | GOICDS_C DS0045 | ThiophanateMethyl |
| 12 | b2664 | tefluthrin | 166 | GOICDS_C DS0045 | Cymoxanil |
| 12 | b2664 | flufenoxuron | 166 | GOICDS_C DS0045 | Metrafenone |
| 12 | b2664 | teflubenzuron | 166 | GOICDS_C DS0045 | Acetochlor |
| 12 | b2664 | spirotetramat; | 166 | GOICDS_C DS0045 | Dimethenamid |
| 12 | b2664 | clothianidin | 166 | GOICDS_C DS0045 | Metolachlor |
| 12 | b2664 | imidacloprid | 166 | GOICDS_C DS0045 | Metazachlor |
| 12 | b2664 | thiamethoxam | 166 | GOICDS_C DS0045 | Glyphosate |
| 12 | b2664 | endosulfan | 166 | GOICDS_C DS0045 | Glufosinate |
| 12 | b2664 | fipronil | 166 | GOICDS_C DS0045 | Sulfosate |

(continued)

| Line in Table B | Gene Name/ LocusID | Active ingredientB | Line in Table B | Gene Name/ LocusID | Active ingredientB |
|---|---|---|---|---|---|
| 12 | b2664 | abamectin | 166 | GOICDS_C DS0045 | Fenoxaprop |
| 12 | b2664 | spinosad | 166 | GOICDS_C DS0045 | Paraquat |
| 12 | b2664 | spinetoram | 166 | GOICDS_C DS0045 | Cycloxydim |
| 12 | b2664 | hydramethylnon; | 166 | GOICDS_C DS0045 | Profoxydim |
| 12 | b2664 | chlorfenapyr; | 166 | GOICDS_C DS0045 | Sethoxydim |
| 12 | b2664 | indoxacarb | 166 | GOICDS_C DS0045 | Tepraloxydim |
| 12 | b2664 | metaflumizone | 166 | GOICDS_C DS0045 | Pendimethalin |
| 12 | b2664 | flubendiamide | 166 | GOICDS_C DS0045 | Acifluorfen |
| 12 | b2664 | chlorantraniliprole | 166 | GOICDS_C DS0045 | Imazamethabenz |
| 12 | b2664 | cyazypyr(HGW86) | 166 | GOICDS_C DS0045 | Imazamox |
| 12 | b2664 | Azoxystrobin | 166 | GOICDS_C DS0045 | Imazapic |
| 12 | b2664 | Dimoxystrobin | 166 | GOICDS_C DS0045 | Imazapyr |
| 12 | b2664 | Kresoxim-methyl | 166 | GOICDS_C DS0045 | Imazaquin |
| 12 | b2664 | Orysastrobin | 166 | GOICDS_C DS0045 | Imazethapyr |
| 12 | b2664 | Pyraclostrobin | 166 | GOICDS_C DS0045 | 2,4-D |
| 12 | b2664 | Trifloxystrobin | 166 | GOICDS_C DS0045 | Chloridazon |
| 12 | b2664 | Bixafen | 166 | GOICDS_C DS0045 | Picloram |
| 12 | b2664 | Boscalid | 166 | GOICDS_C DS0045 | Picolinafen |
| 12 | b2664 | Isopyrazam | 166 | GOICDS_C DS0045 | Cyclosulfamuron |
| 12 | b2664 | Metalaxyl | 166 | GOICDS_C DS0045 | Triflusulfuron |
| 12 | b2664 | Penthiopyrad | 166 | GOICDS_C DS0045 | Atrazine |

(continued)

| Line in Table B | Gene Name/ LocusID | Active ingredientB | Line in Table B | Gene Name/ LocusID | Active ingredientB |
|---|---|---|---|---|---|
| 12 | b2664 | 3-Difluoromethyl-1-methyl-1 H-pyrazole-4-carboxylic acid(2',4',5'-trifluorobiphenyl-2-yl)-amide | 166 | GOICDS_CDS0045 | Pyroxasulfone |
| 12 | b2664 | Dimethomorph | 166 | GOICDS_CDS0045 | Bentazone |
| 12 | b2664 | Difenoconazole | 166 | GOICDS_CDS0045 | Cinidon-ethly |
| 12 | b2664 | Epoxiconazole | 166 | GOICDS_CDS0045 | Cinmethylin |
| 12 | b2664 | Fluquinconazole | 166 | GOICDS_CDS0045 | Dicamba |
| 12 | b2664 | Metconazol | 166 | GOICDS_CDS0045 | Diflufenzopyr |
| 12 | b2664 | Propiconazole | 166 | GOICDS_CDS0045 | Quinclorac |
| 12 | b2664 | Prothioconazole | 166 | GOICDS_CDS0045 | Quinmerac |
| 12 | b2664 | Tebuconazole | 166 | GOICDS_CDS0045 | Mesotrione |
| 12 | b2664 | Triticonazole | 166 | GOICDS_CDS0045 | Saflufenacil |
| 12 | b2664 | Prochloraz | 166 | GOICDS_CDS0045 | Topramezone; |
| 12 | b2664 | Carbendazim | 167 | GOICDS_CDS0185 | acephate |
| 12 | b2664 | Cyprodinil | 167 | GOICDS_CDS0185 | chlorpyrifos |
| 12 | b2664 | Pyrimethanil | 167 | GOICDS_CDS0185 | dimethoate |
| 12 | b2664 | Fenpropimorph | 167 | GOICDS_CDS0185 | methamidophos |
| 12 | b2664 | Tridemorph | 167 | GOICDS_CDS0185 | terbufos |
| 12 | b2664 | Iprodione | 167 | GOICDS_CDS0185 | aldicarb |
| 12 | b2664 | 5-ethyl-6-octyl-[1,2,4] triazolo [1,5-a]pyrimidine-7-ylamine | 167 | GOICDS_CDS0185 | carbofuran |
| 12 | b2664 | Mancozeb | 167 | GOICDS_CDS0185 | bifenthrin |

(continued)

| Line in Table B | Gene Name/ LocusID | Active ingredientB | Line in Table B | Gene Name/ LocusID | Active ingredientB |
|---|---|---|---|---|---|
| 12 | b2664 | Maneb | 167 | GOICDS_C DS0185 | cypermethrin |
| 12 | b2664 | Metiram | 167 | GOICDS_C DS0185 | alpha-cypermethrin |
| 12 | b2664 | Dithianon | 167 | GOICDS_C DS0185 | deltamethrin |
| 12 | b2664 | Chlorothalonil | 167 | GOICDS_C DS0185 | lambda-cyhalothrin |
| 12 | b2664 | ThiophanateMethyl | 167 | GOICDS_C DS0185 | tefluthrin |
| 12 | b2664 | Cymoxanil | 167 | GOICDS_C DS0185 | flufenoxuron |
| 12 | b2664 | Metrafenone | 167 | GOICDS_C DS0185 | teflubenzuron |
| 12 | b2664 | Acetochlor | 167 | GOICDS_C DS0185 | spirotetramat; |
| 12 | b2664 | Dimethenamid | 167 | GOICDS_C DS0185 | clothianidin |
| 12 | b2664 | Metolachlor | 167 | GOICDS_C DS0185 | imidacloprid |
| 12 | b2664 | Metazachlor | 167 | GOICDS_C DS0185 | thiamethoxam |
| 12 | b2664 | Glyphosate | 167 | GOICDS_C DS0185 | endosulfan |
| 12 | b2664 | Glufosinate | 167 | GOICDS_C DS0185 | fipronil |
| 12 | b2664 | Sulfosate | 167 | GOICDS_C DS0185 | abamectin |
| 12 | b2664 | Fenoxaprop | 167 | GOICDS_C DS0185 | spinosad |
| 12 | b2664 | Paraquat | 167 | GOICDS_C DS0185 | spinetoram |
| 12 | b2664 | Cycloxydim | 167 | GOICDS_C DS0185 | hydramethylnon; |
| 12 | b2664 | Profoxydim | 167 | GOICDS_C DS0185 | chlorfenapyr; |
| 12 | b2664 | Sethoxydim | 167 | GOICDS_C DS0185 | indoxacarb |
| 12 | b2664 | Tepraloxydim | 167 | GOICDS_C DS0185 | metaflumizone |
| 12 | b2664 | Pendimethalin | 167 | GOICDS_C DS0185 | flubendiamide |
| 12 | b2664 | Acifluorfen | 167 | GOICDS_C DS0185 | chlorantraniliprole |

(continued)

| Line in Table B | Gene Name/ LocusID | Active ingredientB | Line in Table B | Gene Name/ LocusID | Active ingredientB |
|---|---|---|---|---|---|
| 12 | b2664 | Imazamethabenz | 167 | GOICDS_C DS0185 | cyazypyr(HGW86) |
| 12 | b2664 | Imazamox | 167 | GOICDS_C DS0185 | Azoxystrobin |
| 12 | b2664 | Imazapic | 167 | GOICDS_C DS0185 | Dimoxystrobin |
| 12 | b2664 | Imazapyr | 167 | GOICDS_C DS0185 | Kresoxim-methyl |
| 12 | b2664 | Imazaquin | 167 | GOICDS_C DS0185 | Orysastrobin |
| 12 | b2664 | Imazethapyr | 167 | GOICDS_C DS0185 | Pyraclostrobin |
| 12 | b2664 | 2,4-D | 167 | GOICDS_C DS0185 | Trifloxystrobin |
| 12 | b2664 | Chloridazon | 167 | GOICDS_C DS0185 | Bixafen |
| 12 | b2664 | Picloram | 167 | GOICDS_C DS0185 | Boscalid |
| 12 | b2664 | Picolinafen | 167 | GOICDS_C DS0185 | Isopyrazam |
| 12 | b2664 | Cyclosulfamuron | 167 | GOICDS_C DS0185 | Metalaxyl |
| 12 | b2664 | Triflusulfuron | 167 | GOICDS_C DS0185 | Penthiopyrad |
| 12 | b2664 | Atrazine | 167 | GOICDS_C DS0185 | 3-Difluoromethyl-1-methyl-1H-pyrazole-4-carboxylic acid(2',4',5'-trifluorobiphenyl-2-yl)-amide |
| 12 | b2664 | Pyroxasulfone | 167 | GOICDS_C DS0185 | Dimethomorph |
| 12 | b2664 | Bentazone | 167 | GOICDS_C DS0185 | Difenoconazole |
| 12 | b2664 | Cinidon-ethly | 167 | GOICDS_C DS0185 | Epoxiconazole |
| 12 | b2664 | Cinmethylin | 167 | GOICDS_C DS0185 | Fluquinconazole |
| 12 | b2664 | Dicamba | 167 | GOICDS_C DS0185 | Metconazol |
| 12 | b2664 | Diflufenzopyr | 167 | GOICDS_C DS0185 | Propiconazole |
| 12 | b2664 | Quinclorac | 167 | GOICDS_C DS0185 | Prothioconazole |

(continued)

| Line in Table B | Gene Name/ LocusID | Active ingredientB | Line in Table B | Gene Name/ LocusID | Active ingredientB |
|---|---|---|---|---|---|
| 12 | b2664 | Quinmerac | 167 | GOICDS_CDS0185 | Tebuconazole |
| 12 | b2664 | Mesotrione | 167 | GOICDS_CDS0185 | Triticonazole |
| 12 | b2664 | Saflufenacil | 167 | GOICDS_CDS0185 | Prochloraz |
| 12 | b2664 | Topramezone; | 167 | GOICDS_CDS0185 | Carbendazim |
| 16 | b2796 | acephate | 167 | GOICDS_CDS0185 | Cyprodinil |
| 16 | b2796 | chlorpyrifos | 167 | GOICDS_CDS0185 | Pyrimethanil |
| 16 | b2796 | dimethoate | 167 | GOICDS_CDS0185 | Fenpropimorph |
| 16 | b2796 | methamidophos | 167 | GOICDS_CDS0185 | Tridemorph |
| 16 | b2796 | terbufos | 167 | GOICDS_CDS0185 | Iprodione |
| 16 | b2796 | aldicarb | 167 | GOICDS_CDS0185 | 5-ethyl-6-octyl-[1,2,4] triazolo [1,5-a]pyrimidine-7-ylamine |
| 16 | b2796 | carbofuran | 167 | GOICDS_CDS0185 | Mancozeb |
| 16 | b2796 | bifenthrin | 167 | GOICDS_CDS0185 | Maneb |
| 16 | b2796 | cypermethrin | 167 | GOICDS_CDS0185 | Metiram |
| 16 | b2796 | alpha-cypermethrin | 167 | GOICDS_CDS0185 | Dithianon |
| 16 | b2796 | deltamethrin | 167 | GOICDS_CDS0185 | Chlorothalonil |
| 16 | b2796 | lambda-cyhalothrin | 167 | GOICDS_CDS0185 | ThiophanateMethyl |
| 16 | b2796 | tefluthrin | 167 | GOICDS_CDS0185 | Cymoxanil |
| 16 | b2796 | flufenoxuron | 167 | GOICDS_CDS0185 | Metrafenone |
| 16 | b2796 | teflubenzuron | 167 | GOICDS_CDS0185 | Acetochlor |
| 16 | b2796 | spirotetramat; | 167 | GOICDS_CDS0185 | Dimethenamid |
| 16 | b2796 | clothianidin | 167 | GOICDS_CDS0185 | Metolachlor |

(continued)

| Line in Table B | Gene Name/ LocusID | Active ingredientB | Line in Table B | Gene Name/ LocusID | Active ingredientB |
|---|---|---|---|---|---|
| 16 | b2796 | imidacloprid | 167 | GOICDS_C DS0185 | Metazachlor |
| 16 | b2796 | thiamethoxam | 167 | GOICDS_C DS0185 | Glyphosate |
| 16 | b2796 | endosulfan | 167 | GOICDS_C DS0185 | Glufosinate |
| 16 | b2796 | fipronil | 167 | GOICDS_C DS0185 | Sulfosate |
| 16 | b2796 | abamectin | 167 | GOICDS_C DS0185 | Fenoxaprop |
| 16 | b2796 | spinosad | 167 | GOICDS_C DS0185 | Paraquat |
| 16 | b2796 | spinetoram | 167 | GOICDS_C DS0185 | Cycloxydim |
| 16 | b2796 | hydramethylnon; | 167 | GOICDS_C DS0185 | Profoxydim |
| 16 | b2796 | chlorfenapyr; | 167 | GOICDS_C DS0185 | Sethoxydim |
| 16 | b2796 | indoxacarb | 167 | GOICDS_C DS0185 | Tepraloxydim |
| 16 | b2796 | metaflumizone | 167 | GOICDS_C DS0185 | Pendimethalin |
| 16 | b2796 | flubendiamide | 167 | GOICDS_C DS0185 | Acifluorfen |
| 16 | b2796 | chlorantraniliprole | 167 | GOICDS_C DS0185 | Imazamethabenz |
| 16 | b2796 | cyazypyr(HGW86) | 167 | GOICDS_C DS0185 | Imazamox |
| 16 | b2796 | Azoxystrobin | 167 | GOICDS_C DS0185 | Imazapic |
| 16 | b2796 | Dimoxystrobin | 167 | GOICDS_C DS0185 | Imazapyr |
| 16 | b2796 | Kresoxim-methyl | 167 | GOICDS_C DS0185 | Imazaquin |
| 16 | b2796 | Orysastrobin | 167 | GOICDS_C DS0185 | Imazethapyr |
| 16 | b2796 | Pyraclostrobin | 167 | GOICDS_C DS0185 | 2,4-D |
| 16 | b2796 | Trifloxystrobin | 167 | GOICDS_C DS0185 | Chloridazon |
| 16 | b2796 | Bixafen | 167 | GOICDS_C DS0185 | Picloram |
| 16 | b2796 | Boscalid | 167 | GOICDS_C DS0185 | Picolinafen |

(continued)

| Line in Table B | Gene Name/ LocusID | Active ingredientB | Line in Table B | Gene Name/ LocusID | Active ingredientB |
|---|---|---|---|---|---|
| 16 | b2796 | Isopyrazam | 167 | GOICDS_C DS0185 | Cyclosulfamuron |
| 16 | b2796 | Metalaxyl | 167 | GOICDS_C DS0185 | Triflusulfuron |
| 16 | b2796 | Penthiopyrad | 167 | GOICDS_C DS0185 | Atrazine |
| 16 | b2796 | 3-Difluoromethyl-1-methyl-1 H-pyrazole-4-carboxylic acid(2', 4',5'-trifluorobiphenyl-2-yl)-amide | 167 | GOICDS_C DS0185 | Pyroxasulfone |
| 16 | b2796 | Dimethomorph | 167 | GOICDS_C DS0185 | Bentazone |
| 16 | b2796 | Difenoconazole | 167 | GOICDS_C DS0185 | Cinidon-ethly |
| 16 | b2796 | Epoxiconazole | 167 | GOICDS_C DS0185 | Cinmethylin |
| 16 | b2796 | Fluquinconazole | 167 | GOICDS_C DS0185 | Dicamba |
| 16 | b2796 | Metconazol | 167 | GOICDS_C DS0185 | Diflufenzopyr |
| 16 | b2796 | Propiconazole | 167 | GOICDS_C DS0185 | Quinclorac |
| 16 | b2796 | Prothioconazole | 167 | GOICDS_C DS0185 | Quinmerac |
| 16 | b2796 | Tebuconazole | 167 | GOICDS_C DS0185 | Mesotrione |
| 16 | b2796 | Triticonazole | 167 | GOICDS_C DS0185 | Saflufenacil |
| 16 | b2796 | Prochloraz | 167 | GOICDS_C DS0185 | Topramezone; |
| 16 | b2796 | Carbendazim | 168 | | acephate |
| 16 | b2796 | Cyprodinil | 168 | | chlorpyrifos |
| 16 | b2796 | Pyrimethanil | 168 | | dimethoate |
| 16 | b2796 | Fenpropimorph | 168 | | methamidophos |
| 16 | b2796 | Tridemorph | 168 | | terbufos |
| 16 | b2796 | Iprodione | 168 | | aldicarb |
| 16 | b2796 | 5-ethyl-6-octyl-[1,2,4] triazolo [1,5-a]pyrimidine-7-ylamine | 168 | | carbofuran |
| 16 | b2796 | Mancozeb | 168 | | bifenthrin |

(continued)

| Line in Table B | Gene Name/ LocusID | Active ingredientB | Line in Table B | Gene Name/ LocusID | Active ingredientB |
|---|---|---|---|---|---|
| 16 | b2796 | Maneb | 168 | | cypermethrin |
| 16 | b2796 | Metiram | 168 | | alpha-cypermethrin |
| 16 | b2796 | Dithianon | 168 | | deltamethrin |
| 16 | b2796 | Chlorothalonil | 168 | | lambda-cyhalothrin |
| 16 | b2796 | ThiophanateMethyl | 168 | | tefluthrin |
| 16 | b2796 | Cymoxanil | 168 | | flufenoxuron |
| 16 | b2796 | Metrafenone | 168 | | teflubenzuron |
| 16 | b2796 | Acetochlor | 168 | | spirotetramat; |
| 16 | b2796 | Dimethenamid | 168 | | clothianidin |
| 16 | b2796 | Metolachlor | 168 | | imidacloprid |
| 16 | b2796 | Metazachlor | 168 | | thiamethoxam |
| 16 | b2796 | Glyphosate | 168 | | endosulfan |
| 16 | b2796 | Glufosinate | 168 | | fipronil |
| 16 | b2796 | Sulfosate | 168 | | abamectin |
| 16 | b2796 | Fenoxaprop | 168 | | spinosad |
| 16 | b2796 | Paraquat | 168 | | spinetoram |
| 16 | b2796 | Cycloxydim | 168 | | hydramethylnon; |
| 16 | b2796 | Profoxydim | 168 | | chlorfenapyr; |
| 16 | b2796 | Sethoxydim | 168 | | indoxacarb |
| 16 | b2796 | Tepraloxydim | 168 | | metaflumizone |
| 16 | b2796 | Pendimethalin | 168 | | flubendiamide |
| 16 | b2796 | Acifluorfen | 168 | | chlorantraniliprole |
| 16 | b2796 | Imazamethabenz | 168 | | cyazypyr(HGW86) |
| 16 | b2796 | Imazamox | 168 | | Azoxystrobin |
| 16 | b2796 | Imazapic | 168 | | Dimoxystrobin |
| 16 | b2796 | Imazapyr | 168 | | Kresoxim-methyl |
| 16 | b2796 | Imazaquin | 168 | | Orysastrobin |
| 16 | b2796 | Imazethapyr | 168 | | Pyraclostrobin |
| 16 | b2796 | 2,4-D | 168 | | Trifloxystrobin |
| 16 | b2796 | Chloridazon | 168 | | Bixafen |
| 16 | b2796 | Picloram | 168 | | Boscalid |
| 16 | b2796 | Picolinafen | 168 | | Isopyrazam |
| 16 | b2796 | Cyclosulfamuron | 168 | | Metalaxyl |
| 16 | b2796 | Triflusulfuron | 168 | | Penthiopyrad |

(continued)

| Line in Table B | Gene Name/ LocusID | Active ingredientB | Line in Table B | Gene Name/ LocusID | Active ingredientB |
|---|---|---|---|---|---|
| 16 | b2796 | Atrazine | 168 | | 3-Difluoromethyl-1-methyl-1H-pyrazole-4-carboxylic acid(2',4',5'-trifluorobiphenyl-2-yl)-amide |
| 16 | b2796 | Pyroxasulfone | 168 | | Dimethomorph |
| 16 | b2796 | Bentazone | 168 | | Difenoconazole |
| 16 | b2796 | Cinidon-ethly | 168 | | Epoxiconazole |
| 16 | b2796 | Cinmethylin | 168 | | Fluquinconazole |
| 16 | b2796 | Dicamba | 168 | | Metconazol |
| 16 | b2796 | Diflufenzopyr | 168 | | Propiconazole |
| 16 | b2796 | Quinclorac | 168 | | Prothioconazole |
| 16 | b2796 | Quinmerac | 168 | | Tebuconazole |
| 16 | b2796 | Mesotrione | 168 | | Triticonazole |
| 16 | b2796 | Saflufenacil | 168 | | Prochloraz |
| 16 | b2796 | Topramezone; | 168 | | Carbendazim |
| 19 | EST102 | acephate | 168 | | Cyprodinil |
| 19 | EST102 | chlorpyrifos | 168 | | Pyrimethanil |
| 19 | EST102 | dimethoate | 168 | | Fenpropimorph |
| 19 | EST102 | methamidophos | 168 | | Tridemorph |
| 19 | EST102 | terbufos | 168 | | Iprodione |
| 19 | EST102 | aldicarb | 168 | | 5-ethyl-6-octyl-[1,2,4] triazolo [1,5-a]pyrimidine-7-ylamine |
| 19 | EST102 | carbofuran | 168 | | Mancozeb |
| 19 | EST102 | bifenthrin | 168 | | Maneb |
| 19 | EST102 | cypermethrin | 168 | | Metiram |
| 19 | EST102 | alpha-cypermethrin | 168 | | Dithianon |
| 19 | EST102 | deltamethrin | 168 | | Chlorothalonil |
| 19 | EST102 | lambda-cyhalothrin | 168 | | ThiophanateMethyl |
| 19 | EST102 | tefluthrin | 168 | | Cymoxanil |
| 19 | EST102 | flufenoxuron | 168 | | Metrafenone |
| 19 | EST102 | teflubenzuron | 168 | | Acetochlor |
| 19 | EST102 | spirotetramat; | 168 | | Dimethenamid |
| 19 | EST102 | clothianidin | 168 | | Metolachlor |
| 19 | EST102 | imidacloprid | 168 | | Metazachlor |
| 19 | EST102 | thiamethoxam | 168 | | Glyphosate |

(continued)

| Line in Table B | Gene Name/ LocusID | Active ingredientB | Line in Table B | Gene Name/ LocusID | Active ingredientB |
|---|---|---|---|---|---|
| 19 | EST102 | endosulfan | 168 | | Glufosinate |
| 19 | EST102 | fipronil | 168 | | Sulfosate |
| 19 | EST102 | abamectin | 168 | | Fenoxaprop |
| 19 | EST102 | spinosad | 168 | | Paraquat |
| 19 | EST102 | spinetoram | 168 | | Cycloxydim |
| 19 | EST102 | hydramethylnon; | 168 | | Profoxydim |
| 19 | EST102 | chlorfenapyr; | 168 | | Sethoxydim |
| 19 | EST102 | indoxacarb | 168 | | Tepraloxydim |
| 19 | EST102 | metaflumizone | 168 | | Pendimethalin |
| 19 | EST102 | flubendiamide | 168 | | Acifluorfen |
| 19 | EST102 | chlorantraniliprole | 168 | | Imazamethabenz |
| 19 | EST102 | cyazypyr(HGW86) | 168 | | Imazamox |
| 19 | EST102 | Azoxystrobin | 168 | | Imazapic |
| 19 | EST102 | Dimoxystrobin | 168 | | Imazapyr |
| 19 | EST102 | Kresoxim-methyl | 168 | | Imazaquin |
| 19 | EST102 | Orysastrobin | 168 | | Imazethapyr |
| 19 | EST102 | Pyraclostrobin | 168 | | 2,4-D |
| 19 | EST102 | Trifloxystrobin | 168 | | Chloridazon |
| 19 | EST102 | Bixafen | 168 | | Picloram |
| 19 | EST102 | Boscalid | 168 | | Picolinafen |
| 19 | EST102 | Isopyrazam | 168 | | Cyclosulfamuron |
| 19 | EST102 | Metalaxyl | 168 | | Triflusulfuron |
| 19 | EST102 | Penthiopyrad | 168 | | Atrazine |
| 19 | EST102 | 3-Difluoromethyl-1-methyl-1H-oyrazole-4-carboxylic acic (2',4',5'-trifluorobiphenyl-2-yl)-amide | 168 | | Pyroxasulfone |
| 19 | EST102 | Dimethomorph | 168 | | Bentazone |
| 19 | EST102 | Difenoconazole | 168 | | Cinidon-ethly |
| 19 | EST102 | Epoxiconazole | 168 | | Cinmethylin |
| 19 | EST102 | Fluquinconazole | 168 | | Dicamba |
| 19 | EST102 | Metconazol | 168 | | Diflufenzopyr |
| 19 | EST102 | Propiconazole | 168 | | Quinclorac |
| 19 | EST102 | Prothioconazole | 168 | | Quinmerac |
| 19 | EST102 | Tebuconazole | 168 | | Mesotrione |
| 19 | EST102 | Triticonazole | 168 | | Saflufenacil |

(continued)

| Line in Table B | Gene Name/ LocusID | Active ingredientB | Line in Table B | Gene Name/ LocusID | Active ingredientB |
|---|---|---|---|---|---|
| 19 | EST102 | Prochloraz | 168 | | Topramezone; |
| 19 | EST102 | Carbendazim | 169 | | acephate |
| 19 | EST102 | Cyprodinil | 169 | | chlorpyrifos |
| 19 | EST102 | Pyrimethanil | 169 | | dimethoate |
| 19 | EST102 | Fenpropimorph | 169 | | methamidophos |
| 19 | EST102 | Tridemorph | 169 | | terbufos |
| 19 | EST102 | Iprodione | 169 | | aldicarb |
| 19 | EST102 | 5-ethyl-6-octyl-[1,2,4] triazolo [1,5-a]pyrimidine-7-ylamine | 169 | | carbofuran |
| 19 | EST102 | Mancozeb | 169 | | bifenthrin |
| 19 | EST102 | Maneb | 169 | | cypermethrin |
| 19 | EST102 | Metiram | 169 | | alpha-cypermethrin |
| 19 | EST102 | Dithianon | 169 | | deltamethrin |
| 19 | EST102 | Chlorothalonil | 169 | | lambda-cyhalothrin |
| 19 | EST102 | ThiophanateMethyl | 169 | | tefluthrin |
| 19 | EST102 | Cymoxanil | 169 | | flufenoxuron |
| 19 | EST102 | Metrafenone | 169 | | teflubenzuron |
| 19 | EST102 | Acetochlor | 169 | | spirotetramat; |
| 19 | EST102 | Dimethenamid | 169 | | clothianidin |
| 19 | EST102 | Metolachlor | 169 | | imidacloprid |
| 19 | EST102 | Metazachlor | 169 | | thiamethoxam |
| 19 | EST102 | Glyphosate | 169 | | endosulfan |
| 19 | EST102 | Glufosinate | 169 | | fipronil |
| 19 | EST102 | Sulfosate | 169 | | abamectin |
| 19 | EST102 | Fenoxaprop | 169 | | spinosad |
| 19 | EST102 | Paraquat | 169 | | spinetoram |
| 19 | EST102 | Cycloxydim | 169 | | hydramethylnon; |
| 19 | EST102 | Profoxydim | 169 | | chlorfenapyr; |
| 19 | EST102 | Sethoxydim | 169 | | indoxacarb |
| 19 | EST102 | Tepraloxydim | 169 | | metaflumizone |
| 19 | EST102 | Pendimethalin | 169 | | flubendiamide |
| 19 | EST102 | Acifluorfen | 169 | | chlorantraniliprole |
| 19 | EST102 | Imazamethabenz | 169 | | cyazypyr(HGW86) |
| 19 | EST102 | Imazamox | 169 | | Azoxystrobin |
| 19 | EST102 | Imazapic | 169 | | Dimoxystrobin |
| 19 | EST102 | Imazapyr | 169 | | Kresoxim-methyl |

(continued)

| Line in Table B | Gene Name/ LocusID | Active ingredientB | Line in Table B | Gene Name/ LocusID | Active ingredientB |
|---|---|---|---|---|---|
| 19 | EST102 | Imazaquin | 169 | | Orysastrobin |
| 19 | EST102 | Imazethapyr | 169 | | Pyraclostrobin |
| 19 | EST102 | 2,4-D | 169 | | Trifloxystrobin |
| 19 | EST102 | Chloridazon | 169 | | Bixafen |
| 19 | EST102 | Picloram | 169 | | Boscalid |
| 19 | EST102 | Picolinafen | 169 | | Isopyrazam |
| 19 | EST102 | Cyclosulfamuron | 169 | | Metalaxyl |
| 19 | EST102 | Triflusulfuron | 169 | | Penthiopyrad |
| 19 | EST102 | Atrazine | 169 | | 3-Difluoromethyl-1-methyl-1H-pyrazole-4-carboxylic acid(2',4',5'-trifluorobiphenyl-2-yl)-amide |
| 19 | EST102 | Pyroxasulfone | 169 | | Dimethomorph |
| 19 | EST102 | Bentazone | 169 | | Difenoconazole |
| 19 | EST102 | Cinidon-ethly | 169 | | Epoxiconazole |
| 19 | EST102 | Cinmethylin | 169 | | Fluquinconazole |
| 19 | EST102 | Dicamba | 169 | | Metconazol |
| 19 | EST102 | Diflufenzopyr | 169 | | Propiconazole |
| 19 | EST102 | Quinclorac | 169 | | Prothioconazole |
| 19 | EST102 | Quinmerac | 169 | | Tebuconazole |
| 19 | EST102 | Mesotrione | 169 | | Triticonazole |
| 19 | EST102 | Saflufenacil | 169 | | Prochloraz |
| 19 | EST102 | Topramezone; | 169 | | Carbendazim |
| 20 | EST12 | acephate | 169 | | Cyprodinil |
| 20 | EST12 | chlorpyrifos | 169 | | Pyrimethanil |
| 20 | EST12 | dimethoate | 169 | | Fenpropimorph |
| 20 | EST12 | methamidophos | 169 | | Tridemorph |
| 20 | EST12 | terbufos | 169 | | Iprodione |
| 20 | EST12 | aldicarb | 169 | | 5-ethyl-6-octyl-[1,2,4] triazolo [1,5-a]pyrimidine-7-ylamine |
| 20 | EST12 | carbofuran | 169 | | Mancozeb |
| 20 | EST12 | bifenthrin | 169 | | Maneb |
| 20 | EST12 | cypermethrin | 169 | | Metiram |
| 20 | EST12 | alpha-cypermethrin | 169 | | Dithianon |
| 20 | EST12 | deltamethrin | 169 | | Chlorothalonil |

(continued)

| Line in Table B | Gene Name/ LocusID | Active ingredientB | Line in Table B | Gene Name/ LocusID | Active ingredientB |
|---|---|---|---|---|---|
| 20 | EST12 | lambda-cyhalothrin | 169 | | ThiophanateMethyl |
| 20 | EST12 | tefluthrin | 169 | | Cymoxanil |
| 20 | EST12 | flufenoxuron | 169 | | Metrafenone |
| 20 | EST12 | teflubenzuron | 169 | | Acetochlor |
| 20 | EST12 | spirotetramat; | 169 | | Dimethenamid |
| 20 | EST12 | clothianidin | 169 | | Metolachlor |
| 20 | EST12 | imidacloprid | 169 | | Metazachlor |
| 20 | EST12 | thiamethoxam | 169 | | Glyphosate |
| 20 | EST12 | endosulfan | 169 | | Glufosinate |
| 20 | EST12 | fipronil | 169 | | Sulfosate |
| 20 | EST12 | abamectin | 169 | | Fenoxaprop |
| 20 | EST12 | spinosad | 169 | | Paraquat |
| 20 | EST12 | spinetoram | 169 | | Cycloxydim |
| 20 | EST12 | hydramethylnon; | 169 | | Profoxydim |
| 20 | EST12 | chlorfenapyr; | 169 | | Sethoxydim |
| 20 | EST12 | indoxacarb | 169 | | Tepraloxydim |
| 20 | EST12 | metaflumizone | 169 | | Pendimethalin |
| 20 | EST12 | flubendiamide | 169 | | Acifluorfen |
| 20 | EST12 | chlorantraniliprole | 169 | | Imazamethabenz |
| 20 | EST12 | cyazypyr(HGW86) | 169 | | Imazamox |
| 20 | EST12 | Azoxystrobin | 169 | | Imazapic |
| 20 | EST12 | Dimoxystrobin | 169 | | Imazapyr |
| 20 | EST12 | Kresoxim-methyl | 169 | | Imazaquin |
| 20 | EST12 | Orysastrobin | 169 | | Imazethapyr |
| 20 | EST12 | Pyraclostrobin | 169 | | 2,4-D |
| 20 | EST12 | Trifloxystrobin | 169 | | Chloridazon |
| 20 | EST12 | Bixafen | 169 | | Picloram |
| 20 | EST12 | Boscalid | 169 | | Picolinafen |
| 20 | EST12 | Isopyrazam | 169 | | Cyclosulfamuron |
| 20 | EST12 | Metalaxyl | 169 | | Triflusulfuron |
| 20 | EST12 | Penthiopyrad | 169 | | Atrazine |
| 20 | EST12 | 3-Difluoromethyl-1-methyl-1 H-pyrazole-4-carboxylic acid(2',4',5'-trifluorobiphenyl-2-yl)-amide | 169 | | Pyroxasulfone |
| 20 | EST12 | Dimethomorph | 169 | | Bentazone |

(continued)

| Line in Table B | Gene Name/ LocusID | Active ingredientB | Line in Table B | Gene Name/ LocusID | Active ingredientB |
|---|---|---|---|---|---|
| 20 | EST12 | Difenoconazole | 169 | | Cinidon-ethly |
| 20 | EST12 | Epoxiconazole | 169 | | Cinmethylin |
| 20 | EST12 | Fluquinconazole | 169 | | Dicamba |
| 20 | EST12 | Metconazol | 169 | | Diflufenzopyr |
| 20 | EST12 | Propiconazole | 169 | | Quinclorac |
| 20 | EST12 | Prothioconazole | 169 | | Quinmerac |
| 20 | EST12 | Tebuconazole | 169 | | Mesotrione |
| 20 | EST12 | Triticonazole | 169 | | Saflufenacil |
| 20 | EST12 | Prochloraz | 169 | | Topramezone; |
| 20 | EST12 | Carbendazim | 170 | GOICDS_C DS0647 | acephate |
| 20 | EST12 | Cyprodinil | 170 | GOICDS_C DS0647 | chlorpyrifos |
| 20 | EST12 | Pyrimethanil | 170 | GOICDS_C DS0647 | dimethoate |
| 20 | EST12 | Fenpropimorph | 170 | GOICDS_C DS0647 | methamidophos |
| 20 | EST12 | Tridemorph | 170 | GOICDS_C DS0647 | terbufos |
| 20 | EST12 | Iprodione | 170 | GOICDS_C DS0647 | aldicarb |
| 20 | EST12 | 5-ethyl-6-octyl-[1,2,4] triazolo [1,5-a]pyrimidine-7-ylamine | 170 | GOICDS_C DS0647 | carbofuran |
| 20 | EST12 | Mancozeb | 170 | GOICDS_C DS0647 | bifenthrin |
| 20 | EST12 | Maneb | 170 | GOICDS_C DS0647 | cypermethrin |
| 20 | EST12 | Metiram | 170 | GOICDS_C DS0647 | alpha-cypermethrin |
| 20 | EST12 | Dithianon | 170 | GOICDS_C DS0647 | deltamethrin |
| 20 | EST12 | Chlorothalonil | 170 | GOICDS_C DS0647 | lambda-cyhalothrin |
| 20 | EST12 | ThiophanateMethyl | 170 | GOICDS_C DS0647 | tefluthrin |
| 20 | EST12 | Cymoxanil | 170 | GOICDS_C DS0647 | flufenoxuron |
| 20 | EST12 | Metrafenone | 170 | GOICDS_C DS0647 | teflubenzuron |
| 20 | EST12 | Acetochlor | 170 | GOICDS_C DS0647 | spirotetramat; |

(continued)

| Line in Table B | Gene Name/ LocusID | Active ingredientB | Line in Table B | Gene Name/ LocusID | Active ingredientB |
|---|---|---|---|---|---|
| 20 | EST12 | Dimethenamid | 170 | GOICDS_C DS0647 | clothianidin |
| 20 | EST12 | Metolachlor | 170 | GOICDS_C DS0647 | imidacloprid |
| 20 | EST12 | Metazachlor | 170 | GOICDS_C DS0647 | thiamethoxam |
| 20 | EST12 | Glyphosate | 170 | GOICDS_C DS0647 | endosulfan |
| 20 | EST12 | Glufosinate | 170 | GOICDS_C DS0647 | fipronil |
| 20 | EST12 | Sulfosate | 170 | GOICDS_C DS0647 | abamectin |
| 20 | EST12 | Fenoxaprop | 170 | GOICDS_C DS0647 | spinosad |
| 20 | EST12 | Paraquat | 170 | GOICDS_C DS0647 | spinetoram |
| 20 | EST12 | Cycloxydim | 170 | GOICDS_C DS0647 | hydramethylnon; |
| 20 | EST12 | Profoxydim | 170 | GOICDS_C DS0647 | chlorfenapyr; |
| 20 | EST12 | Sethoxydim | 170 | GOICDS_C DS0647 | indoxacarb |
| 20 | EST12 | Tepraloxydim | 170 | GOICDS_C DS0647 | metaflumizone |
| 20 | EST12 | Pendimethalin | 170 | GOICDS_C DS0647 | flubendiamide |
| 20 | EST12 | Acifluorfen | 170 | GOICDS_C DS0647 | chlorantraniliprole |
| 20 | EST12 | Imazamethabenz | 170 | GOICDS_C DS0647 | cyazypyr(HGW86) |
| 20 | EST12 | Imazamox | 170 | GOICDS_C DS0647 | Azoxystrobin |
| 20 | EST12 | Imazapic | 170 | GOICDS_C DS0647 | Dimoxystrobin |
| 20 | EST12 | Imazapyr | 170 | GOICDS_C DS0647 | Kresoxim-methyl |
| 20 | EST12 | Imazaquin | 170 | GOICDS_C DS0647 | Orysastrobin |
| 20 | EST12 | Imazethapyr | 170 | GOICDS_C DS0647 | Pyraclostrobin |
| 20 | EST12 | 2,4-D | 170 | GOICDS_C DS0647 | Trifloxystrobin |
| 20 | EST12 | Chloridazon | 170 | GOICDS_C DS0647 | Bixafen |

(continued)

| Line in Table B | Gene Name/ LocusID | Active ingredientB | Line in Table B | Gene Name/ LocusID | Active ingredientB |
|---|---|---|---|---|---|
| 20 | EST12 | Picloram | 170 | GOICDS_C DS0647 | Boscalid |
| 20 | EST12 | Picolinafen | 170 | GOICDS_C DS0647 | Isopyrazam |
| 20 | EST12 | Cyclosulfamuron | 170 | GOICDS_C DS0647 | Metalaxyl |
| 20 | EST12 | Triflusulfuron | 170 | GOICDS_C DS0647 | Penthiopyrad |
| 20 | EST12 | Atrazine | 170 | GOICDS_C DS0647 | 3-Difluoromethyl-1-methyl-1H-pyrazole-4-carboxylic acid(2',4',5'-trifluorobiphenyl-2-yl)-amide |
| 20 | EST12 | Pyroxasulfone | 170 | GOICDS_C DS0647 | Dimethomorph |
| 20 | EST12 | Bentazone | 170 | GOICDS_C DS0647 | Difenoconazole |
| 20 | EST12 | Cinidon-ethly | 170 | GOICDS_C DS0647 | Epoxiconazole |
| 20 | EST12 | Cinmethylin | 170 | GOICDS_C DS0647 | Fluquinconazole |
| 20 | EST12 | Dicamba | 170 | GOICDS_C DS0647 | Metconazol |
| 20 | EST12 | Diflufenzopyr | 170 | GOICDS_C DS0647 | Propiconazole |
| 20 | EST12 | Quinclorac | 170 | GOICDS_C DS0647 | Prothioconazole |
| 20 | EST12 | Quinmerac | 170 | GOICDS_C DS0647 | Tebuconazole |
| 20 | EST12 | Mesotrione | 170 | GOICDS_C DS0647 | Triticonazole |
| 20 | EST12 | Saflufenacil | 170 | GOICDS_C DS0647 | Prochloraz |
| 20 | EST12 | Topramezone; | 170 | GOICDS_C DS0647 | Carbendazim |
| 21 | EST134 | acephate | 170 | GOICDS_C DS0647 | Cyprodinil |
| 21 | EST134 | chlorpyrifos | 170 | GOICDS_C DS0647 | Pyrimethanil |
| 21 | EST134 | dimethoate | 170 | GOICDS_C DS0647 | Fenpropimorph |
| 21 | EST134 | methamidophos | 170 | GOICDS_C DS0647 | Tridemorph |

(continued)

| Line in Table B | Gene Name/ LocusID | Active ingredientB | Line in Table B | Gene Name/ LocusID | Active ingredientB |
|---|---|---|---|---|---|
| 21 | EST134 | terbufos | 170 | GOICDS_C DS0647 | Iprodione |
| 21 | EST134 | aldicarb | 170 | GOICDS_C DS0647 | 5-ethyl-6-octyl-[1,2,4] triazolo [1,5-a]pyrimidine-7-ylamine |
| 21 | EST134 | carbofuran | 170 | GOICDS_C DS0647 | Mancozeb |
| 21 | EST134 | bifenthrin | 170 | GOICDS_C DS0647 | Maneb |
| 21 | EST134 | cypermethrin | 170 | GOICDS_C DS0647 | Metiram |
| 21 | EST134 | alpha-cypermethrin | 170 | GOICDS_C DS0647 | Dithianon |
| 21 | EST134 | deltamethrin | 170 | GOICDS_C DS0647 | Chlorothalonil |
| 21 | EST134 | lambda-cyhalothrin | 170 | GOICDS_C DS0647 | ThiophanateMethyl |
| 21 | EST134 | tefluthrin | 170 | GOICDS_C DS0647 | Cymoxanil |
| 21 | EST134 | flufenoxuron | 170 | GOICDS_C DS0647 | Metrafenone |
| 21 | EST134 | teflubenzuron | 170 | GOICDS_C DS0647 | Acetochlor |
| 21 | EST134 | spirotetramat; | 170 | GOICDS_C DS0647 | Dimethenamid |
| 21 | EST134 | clothianidin | 170 | GOICDS_C DS0647 | Metolachlor |
| 21 | EST134 | imidacloprid | 170 | GOICDS_C DS0647 | Metazachlor |
| 21 | EST134 | thiamethoxam | 170 | GOICDS_C DS0647 | Glyphosate |
| 21 | EST134 | endosulfan | 170 | GOICDS_C DS0647 | Glufosinate |
| 21 | EST134 | fipronil | 170 | GOICDS_C DS0647 | Sulfosate |
| 21 | EST134 | abamectin | 170 | GOICDS_C DS0647 | Fenoxaprop |
| 21 | EST134 | spinosad | 170 | GOICDS_C DS0647 | Paraquat |
| 21 | EST134 | spinetoram | 170 | GOICDS_C DS0647 | Cycloxydim |
| 21 | EST134 | hydramethylnon; | 170 | GOICDS_C DS0647 | Profoxydim |

(continued)

| Line in Table B | Gene Name/ LocusID | Active ingredientB | Line in Table B | Gene Name/ LocusID | Active ingredientB |
|---|---|---|---|---|---|
| 21 | EST134 | chlorfenapyr; | 170 | GOICDS_C DS0647 | Sethoxydim |
| 21 | EST134 | indoxacarb | 170 | GOICDS_C DS0647 | Tepraloxydim |
| 21 | EST134 | metaflumizone | 170 | GOICDS_C DS0647 | Pendimethalin |
| 21 | EST134 | flubendiamide | 170 | GOICDS_C DS0647 | Acifluorfen |
| 21 | EST134 | chlorantraniliprole | 170 | GOICDS_C DS0647 | Imazamethabenz |
| 21 | EST134 | cyazypyr(HGW86) | 170 | GOICDS_C DS0647 | Imazamox |
| 21 | EST134 | Azoxystrobin | 170 | GOICDS_C DS0647 | Imazapic |
| 21 | EST134 | Dimoxystrobin | 170 | GOICDS_C DS0647 | Imazapyr |
| 21 | EST134 | Kresoxim-methyl | 170 | GOICDS_C DS0647 | Imazaquin |
| 21 | EST134 | Orysastrobin | 170 | GOICDS_C DS0647 | Imazethapyr |
| 21 | EST134 | Pyraclostrobin | 170 | GOICDS_C DS0647 | 2,4-D |
| 21 | EST134 | Trifloxystrobin | 170 | GOICDS_C DS0647 | Chloridazon |
| 21 | EST134 | Bixafen | 170 | GOICDS_C DS0647 | Picloram |
| 21 | EST134 | Boscalid | 170 | GOICDS_C DS0647 | Picolinafen |
| 21 | EST134 | Isopyrazam | 170 | GOICDS_C DS0647 | Cyclosulfamuron |
| 21 | EST134 | Metalaxyl | 170 | GOICDS_C DS0647 | Triflusulfuron |
| 21 | EST134 | Penthiopyrad | 170 | GOICDS_C DS0647 | Atrazine |
| 21 | EST134 | 3-Difluoromethyl-1-methyl-1 H-pyrazole-4-carboxylic acid(2',4',5'-trifluorobiphenyl-2-yl)-amide | 170 | GOICDS_C DS0647 | Pyroxasulfone |
| 21 | EST134 | Dimethomorph | 170 | GOICDS_C DS0647 | Bentazone |
| 21 | EST134 | Difenoconazole | 170 | GOICDS_C DS0647 | Cinidon-ethly |

(continued)

| Line in Table B | Gene Name/ LocusID | Active ingredientB | Line in Table B | Gene Name/ LocusID | Active ingredientB |
|---|---|---|---|---|---|
| 21 | EST134 | Epoxiconazole | 170 | GOICDS_C DS0647 | Cinmethylin |
| 21 | EST134 | Fluquinconazole | 170 | GOICDS_C DS0647 | Dicamba |
| 21 | EST134 | Metconazol | 170 | GOICDS_C DS0647 | Diflufenzopyr |
| 21 | EST134 | Propiconazole | 170 | GOICDS_C DS0647 | Quinclorac |
| 21 | EST134 | Prothioconazole | 170 | GOICDS_C DS0647 | Quinmerac |
| 21 | EST134 | Tebuconazole | 170 | GOICDS_C DS0647 | Mesotrione |
| 21 | EST134 | Triticonazole | 170 | GOICDS_C DS0647 | Saflufenacil |
| 21 | EST134 | Prochloraz | 170 | GOICDS_C DS0647 | Topramezone; |
| 21 | EST134 | Carbendazim | 171 | | acephate |
| 21 | EST134 | Cyprodinil | 171 | | chlorpyrifos |
| 21 | EST134 | Pyrimethanil | 171 | | dimethoate |
| 21 | EST134 | Fenpropimorph | 171 | | methamidophos |
| 21 | EST134 | Tridemorph | 171 | | terbufos |
| 21 | EST134 | Iprodione | 171 | | aldicarb |
| 21 | EST134 | 5-ethyl-6-octyl-[1,2,4] triazolo [1,5-a]pyrimidine-7-ylamine | 171 | | carbofuran |
| 21 | EST134 | Mancozeb | 171 | | bifenthrin |
| 21 | EST134 | Maneb | 171 | | cypermethrin |
| 21 | EST134 | Metiram | 171 | | alpha-cypermethrin |
| 21 | EST134 | Dithianon | 171 | | deltamethrin |
| 21 | EST134 | Chlorothalonil | 171 | | lambda-cyhalothrin |
| 21 | EST134 | ThiophanateMethyl | 171 | | tefluthrin |
| 21 | EST134 | Cymoxanil | 171 | | flufenoxuron |
| 21 | EST134 | Metrafenone | 171 | | teflubenzuron |
| 21 | EST134 | Acetochlor | 171 | | spirotetramat; |
| 21 | EST134 | Dimethenamid | 171 | | clothianidin |
| 21 | EST134 | Metolachlor | 171 | | imidacloprid |
| 21 | EST134 | Metazachlor | 171 | | thiamethoxam |
| 21 | EST134 | Glyphosate | 171 | | endosulfan |
| 21 | EST134 | Glufosinate | 171 | | fipronil |

(continued)

| Line in Table B | Gene Name/ LocusID | Active ingredientB | Line in Table B | Gene Name/ LocusID | Active ingredientB |
|---|---|---|---|---|---|
| 21 | EST134 | Sulfosate | 171 | | abamectin |
| 21 | EST134 | Fenoxaprop | 171 | | spinosad |
| 21 | EST134 | Paraquat | 171 | | spinetoram |
| 21 | EST134 | Cycloxydim | 171 | | hydramethylnon; |
| 21 | EST134 | Profoxydim | 171 | | chlorfenapyr; |
| 21 | EST134 | Sethoxydim | 171 | | indoxacarb |
| 21 | EST134 | Tepraloxydim | 171 | | metaflumizone |
| 21 | EST134 | Pendimethalin | 171 | | flubendiamide |
| 21 | EST134 | Acifluorfen | 171 | | chlorantraniliprole |
| 21 | EST134 | Imazamethabenz | 171 | | cyazypyr(HGW86) |
| 21 | EST134 | Imazamox | 171 | | Azoxystrobin |
| 21 | EST134 | Imazapic | 171 | | Dimoxystrobin |
| 21 | EST134 | Imazapyr | 171 | | Kresoxim-methyl |
| 21 | EST134 | Imazaquin | 171 | | Orysastrobin |
| 21 | EST134 | Imazethapyr | 171 | | Pyraclostrobin |
| 21 | EST134 | 2,4-D | 171 | | Trifloxystrobin |
| 21 | EST134 | Chloridazon | 171 | | Bixafen |
| 21 | EST134 | Picloram | 171 | | Boscalid |
| 21 | EST134 | Picolinafen | 171 | | Isopyrazam |
| 21 | EST134 | Cyclosulfamuron | 171 | | Metalaxyl |
| 21 | EST134 | Triflusulfuron | 171 | | Penthiopyrad |
| 21 | EST134 | Atrazine | 171 | | 3-Difluoromethyl-1-methyl-1H-pyrazole-4-carboxylic acid(2',4',5'-trifluorobiphenyl-2-yl)-amide |
| 21 | EST134 | Pyroxasulfone | 171 | | Dimethomorph |
| 21 | EST134 | Bentazone | 171 | | Difenoconazole |
| 21 | EST134 | Cinidon-ethly | 171 | | Epoxiconazole |
| 21 | EST134 | Cinmethylin | 171 | | Fluquinconazole |
| 21 | EST134 | Dicamba | 171 | | Metconazol |
| 21 | EST134 | Diflufenzopyr | 171 | | Propiconazole |
| 21 | EST134 | Quinclorac | 171 | | Prothioconazole |
| 21 | EST134 | Quinmerac | 171 | | Tebuconazole |
| 21 | EST134 | Mesotrione | 171 | | Triticonazole |
| 21 | EST134 | Saflufenacil | 171 | | Prochloraz |
| 21 | EST134 | Topramezone; | 171 | | Carbendazim |

(continued)

| Line in Table B | Gene Name/ LocusID | Active ingredientB | Line in Table B | Gene Name/ LocusID | Active ingredientB |
|---|---|---|---|---|---|
| 23 | EST163 | acephate | 171 | | Cyprodinil |
| 23 | EST163 | chlorpyrifos | 171 | | Pyrimethanil |
| 23 | EST163 | dimethoate | 171 | | Fenpropimorph |
| 23 | EST163 | methamidophos | 171 | | Tridemorph |
| 23 | EST163 | terbufos | 171 | | Iprodione |
| 23 | EST163 | aldicarb | 171 | | 5-ethyl-6-octyl-[1,2,4] triazolo [1,5-a]pyrimidine-7-ylamine |
| 23 | EST163 | carbofuran | 171 | | Mancozeb |
| 23 | EST163 | bifenthrin | 171 | | Maneb |
| 23 | EST163 | cypermethrin | 171 | | Metiram |
| 23 | EST163 | alpha-cypermethrin | 171 | | Dithianon |
| 23 | EST163 | deltamethrin | 171 | | Chlorothalonil |
| 23 | EST163 | lambda-cyhalothrin | 171 | | ThiophanateMethyl |
| 23 | EST163 | tefluthrin | 171 | | Cymoxanil |
| 23 | EST163 | flufenoxuron | 171 | | Metrafenone |
| 23 | EST163 | teflubenzuron | 171 | | Acetochlor |
| 23 | EST163 | spirotetramat; | 171 | | Dimethenamid |
| 23 | EST163 | clothianidin | 171 | | Metolachlor |
| 23 | EST163 | imidacloprid | 171 | | Metazachlor |
| 23 | EST163 | thiamethoxam | 171 | | Glyphosate |
| 23 | EST163 | endosulfan | 171 | | Glufosinate |
| 23 | EST163 | fipronil | 171 | | Sulfosate |
| 23 | EST163 | abamectin | 171 | | Fenoxaprop |
| 23 | EST163 | spinosad | 171 | | Paraquat |
| 23 | EST163 | spinetoram | 171 | | Cycloxydim |
| 23 | EST163 | hydramethylnon; | 171 | | Profoxydim |
| 23 | EST163 | chlorfenapyr; | 171 | | Sethoxydim |
| 23 | EST163 | indoxacarb | 171 | | Tepraloxydim |
| 23 | EST163 | metaflumizone | 171 | | Pendimethalin |
| 23 | EST163 | flubendiamide | 171 | | Acifluorfen |
| 23 | EST163 | chlorantraniliprole | 171 | | Imazamethabenz |
| 23 | EST163 | cyazypyr(HGW86) | 171 | | Imazamox |
| 23 | EST163 | Azoxystrobin | 171 | | Imazapic |
| 23 | EST163 | Dimoxystrobin | 171 | | Imazapyr |
| 23 | EST163 | Kresoxim-methyl | 171 | | Imazaquin |
| 23 | EST163 | Orysastrobin | 171 | | Imazethapyr |

(continued)

| Line in Table B | Gene Name/ LocusID | Active ingredientB | Line in Table B | Gene Name/ LocusID | Active ingredientB |
|---|---|---|---|---|---|
| 23 | EST163 | Pyraclostrobin | 171 | | 2,4-D |
| 23 | EST163 | Trifloxystrobin | 171 | | Chloridazon |
| 23 | EST163 | Bixafen | 171 | | Picloram |
| 23 | EST163 | Boscalid | 171 | | Picolinafen |
| 23 | EST163 | Isopyrazam | 171 | | Cyclosulfamuron |
| 23 | EST163 | Metalaxyl | 171 | | Triflusulfuron |
| 23 | EST163 | Penthiopyrad | 171 | | Atrazine |
| 23 | EST163 | 3-Difluoromethyl-1-methyl-1H-pyrazole-4-carboxylic acic (2',4',5'-trifluorobiphenyl-2-yl)-amide | 171 | | Pyroxasulfone |
| 23 | EST163 | Dimethomorph | 171 | | Bentazone |
| 23 | EST163 | Difenoconazole | 171 | | Cinidon-ethly |
| 23 | EST163 | Epoxiconazole | 171 | | Cinmethylin |
| 23 | EST163 | Fluquinconazole | 171 | | Dicamba |
| 23 | EST163 | Metconazol | 171 | | Diflufenzopyr |
| 23 | EST163 | Propiconazole | 171 | | Quinclorac |
| 23 | EST163 | Prothioconazole | 171 | | Quinmerac |
| 23 | EST163 | Tebuconazole | 171 | | Mesotrione |
| 23 | EST163 | Triticonazole | 171 | | Saflufenacil |
| 23 | EST163 | Prochloraz | 171 | | Topramezone; |
| 23 | EST163 | Carbendazim | 172 | GOICDS_CDS1499_2 | acephate |
| 23 | EST163 | Cyprodinil | 172 | GOICDS_CDS1499_2 | chlorpyrifos |
| 23 | EST163 | Pyrimethanil | 172 | GOICDS_CDS1499_2 | dimethoate |
| 23 | EST163 | Fenpropimorph | 172 | GOICDS_CDS1499_2 | methamidophos |
| 23 | EST163 | Tridemorph | 172 | GOICDS_CDS1499_2 | terbufos |
| 23 | EST163 | Iprodione | 172 | GOICDS_CDS1499_2 | aldicarb |
| 23 | EST163 | 5-ethyl-6-octyl-[1,2,4] triazolo [1,5-a]pyrimidine-7-ylamine | 172 | GOICDS_CDS1499_2 | carbofuran |
| 23 | EST163 | Mancozeb | 172 | GOICDS_CDS1499_2 | bifenthrin |

(continued)

| Line in Table B | Gene Name/ LocusID | Active ingredientB | Line in Table B | Gene Name/ LocusID | Active ingredientB |
|---|---|---|---|---|---|
| 23 | EST163 | Maneb | 172 | GOICDS_C DS1499_2 | cypermethrin |
| 23 | EST163 | Metiram | 172 | GOICDS_C DS1499_2 | alpha-cypermethrin |
| 23 | EST163 | Dithianon | 172 | GOICDS_C DS1499_2 | deltamethrin |
| 23 | EST163 | Chlorothalonil | 172 | GOICDS_C DS1499_2 | lambda-cyhalothrin |
| 23 | EST163 | ThiophanateMethyl | 172 | GOICDS_C DS1499_2 | tefluthrin |
| 23 | EST163 | Cymoxanil | 172 | GOICDS_C DS1499_2 | flufenoxuron |
| 23 | EST163 | Metrafenone | 172 | GOICDS_C DS1499_2 | teflubenzuron |
| 23 | EST163 | Acetochlor | 172 | GOICDS_C DS1499_2 | spirotetramat; |
| 23 | EST163 | Dimethenamid | 172 | GOICDS_C DS1499_2 | clothianidin |
| 23 | EST163 | Metolachlor | 172 | GOICDS_C DS1499_2 | imidacloprid |
| 23 | EST163 | Metazachlor | 172 | GOICDS_C DS1499_2 | thiamethoxam |
| 23 | EST163 | Glyphosate | 172 | GOICDS_C DS1499_2 | endosulfan |
| 23 | EST163 | Glufosinate | 172 | GOICDS_C DS1499 2 | fipronil |
| 23 | EST163 | Sulfosate | 172 | GOICDS_C DS1499_2 | abamectin |
| 23 | EST163 | Fenoxaprop | 172 | GOICDS_C DS1499_2 | spinosad |
| 23 | EST163 | Paraquat | 172 | GOICDS_C DS1499_2 | spinetoram |
| 23 | EST163 | Cycloxydim | 172 | GOICDS_C DS1499_2 | hydramethylnon; |
| 23 | EST163 | Profoxydim | 172 | GOICDS_C DS1499_2 | chlorfenapyr; |
| 23 | EST163 | Sethoxydim | 172 | GOICDS_C DS1499_2 | indoxacarb |
| 23 | EST163 | Tepraloxydim | 172 | GOICDS_C DS1499_2 | metaflumizone |
| 23 | EST163 | Pendimethalin | 172 | GOICDS_C DS1499_2 | flubendiamide |
| 23 | EST163 | Acifluorfen | 172 | GOICDS_C DS1499_2 | chlorantraniliprole |

(continued)

| Line in Table B | Gene Name/ LocusID | Active ingredientB | Line in Table B | Gene Name/ LocusID | Active ingredientB |
|---|---|---|---|---|---|
| 23 | EST163 | Imazamethabenz | 172 | GOICDS_C DS1499_2 | cyazypyr(HGW86) |
| 23 | EST163 | Imazamox | 172 | GOICDS_C DS1499_2 | Azoxystrobin |
| 23 | EST163 | Imazapic | 172 | GOICDS_C DS1499_2 | Dimoxystrobin |
| 23 | EST163 | Imazapyr | 172 | GOICDS_C DS1499_2 | Kresoxim-methyl |
| 23 | EST163 | Imazaquin | 172 | GOICDS_C DS1499_2 | Orysastrobin |
| 23 | EST163 | Imazethapyr | 172 | GOICDS_C DS1499_2 | Pyraclostrobin |
| 23 | EST163 | 2,4-D | 172 | GOICDS_C DS1499_2 | Trifloxystrobin |
| 23 | EST163 | Chloridazon | 172 | GOICDS_C DS1499_2 | Bixafen |
| 23 | EST163 | Picloram | 172 | GOICDS_C DS1499_2 | Boscalid |
| 23 | EST163 | Picolinafen | 172 | GOICDS_C DS1499_2 | Isopyrazam |
| 23 | EST163 | Cyclosulfamuron | 172 | GOICDS_C DS1499_2 | Metalaxyl |
| 23 | EST163 | Triflusulfuron | 172 | GOICDS_C DS1499_2 | Penthiopyrad |
| 23 | EST163 | Atrazine | 172 | GOICDS_C DS1499_2 | 3-Difluoromethyl-1-methyl-1H-pyrazole-4-carboxylic acid(2',4',5'-trifluorobiphenyl-2-yl)-amide |
| 23 | EST163 | Pyroxasulfone | 172 | GOICDS_C DS1499_2 | Dimethomorph |
| 23 | EST163 | Bentazone | 172 | GOICDS_C DS1499_2 | Difenoconazole |
| 23 | EST163 | Cinidon-ethly | 172 | GOICDS_C DS1499_2 | Epoxiconazole |
| 23 | EST163 | Cinmethylin | 172 | GOICDS_C DS1499_2 | Fluquinconazole |
| 23 | EST163 | Dicamba | 172 | GOICDS_C DS1499_2 | Metconazol |
| 23 | EST163 | Diflufenzopyr | 172 | GOICDS_C DS1499_2 | Propiconazole |
| 23 | EST163 | Quinclorac | 172 | GOICDS_C DS1499_2 | Prothioconazole |

(continued)

| Line in Table B | Gene Name/ LocusID | Active ingredientB | Line in Table B | Gene Name/ LocusID | Active ingredientB |
|---|---|---|---|---|---|
| 23 | EST163 | Quinmerac | 172 | GOICDS_C DS1499_2 | Tebuconazole |
| 23 | EST163 | Mesotrione | 172 | GOICDS_C DS1499_2 | Triticonazole |
| 23 | EST163 | Saflufenacil | 172 | GOICDS_C DS1499_2 | Prochloraz |
| 23 | EST163 | Topramezone; | 172 | GOICDS_C DS1499_2 | Carbendazim |
| 25 | EST217 | acephate | 172 | GOICDS_C DS1499_2 | Cyprodinil |
| 25 | EST217 | chlorpyrifos | 172 | GOICDS_C DS1499_2 | Pyrimethanil |
| 25 | EST217 | dimethoate | 172 | GOICDS_C DS1499_2 | Fenpropimorph |
| 25 | EST217 | methamidophos | 172 | GOICDS_C DS1499_2 | Tridemorph |
| 25 | EST217 | terbufos | 172 | GOICDS_C DS1499_2 | Iprodione |
| 25 | EST217 | aldicarb | 172 | GOICDS_C DS1499_2 | 5-ethyl-6-octyl-[1,2,4] triazolo [1,5-a]pyrimidine-7-ylamine |
| 25 | EST217 | carbofuran | 172 | GOICDS_C DS1499_2 | Mancozeb |
| 25 | EST217 | bifenthrin | 172 | GOICDS_C DS1499_2 | Maneb |
| 25 | EST217 | cypermethrin | 172 | GOICDS_C DS1499_2 | Metiram |
| 25 | EST217 | alpha-cypermethrin | 172 | GOICDS_C DS1499_2 | Dithianon |
| 25 | EST217 | deltamethrin | 172 | GOICDS_C DS1499_2 | Chlorothalonil |
| 25 | EST217 | lambda-cyhalothrin | 172 | GOICDS_C DS1499_2 | ThiophanateMethyl |
| 25 | EST217 | tefluthrin | 172 | GOICDS_C DS1499_2 | Cymoxanil |
| 25 | EST217 | flufenoxuron | 172 | GOICDS_C DS1499_2 | Metrafenone |
| 25 | EST217 | teflubenzuron | 172 | GOICDS_C DS1499_2 | Acetochlor |
| 25 | EST217 | spirotetramat; | 172 | GOICDS_C DS1499_2 | Dimethenamid |
| 25 | EST217 | clothianidin | 172 | GOICDS_C DS1499_2 | Metolachlor |

(continued)

| Line in Table B | Gene Name/ LocusID | Active ingredientB | Line in Table B | Gene Name/ LocusID | Active ingredientB |
|---|---|---|---|---|---|
| 25 | EST217 | imidacloprid | 172 | GOICDS_C DS1499_2 | Metazachlor |
| 25 | EST217 | thiamethoxam | 172 | GOICDS_C DS1499_2 | Glyphosate |
| 25 | EST217 | endosulfan | 172 | GOICDS_C DS1499_2 | Glufosinate |
| 25 | EST217 | fipronil | 172 | GOICDS_C DS1499_2 | Sulfosate |
| 25 | EST217 | abamectin | 172 | GOICDS_C DS1499_2 | Fenoxaprop |
| 25 | EST217 | spinosad | 172 | GOICDS_C DS1499_2 | Paraquat |
| 25 | EST217 | spinetoram | 172 | GOICDS_C DS1499 2 | Cycloxydim |
| 25 | EST217 | hydramethylnon; | 172 | GOICDS_C DS1499_2 | Profoxydim |
| 25 | EST217 | chlorfenapyr; | 172 | GOICDS_C DS1499_2 | Sethoxydim |
| 25 | EST217 | indoxacarb | 172 | GOICDS_C DS1499_2 | Tepraloxydim |
| 25 | EST217 | metaflumizone | 172 | GOICDS_C DS1499_2 | Pendimethalin |
| 25 | EST217 | flubendiamide | 172 | GOICDS_C DS1499_2 | Acifluorfen |
| 25 | EST217 | chlorantraniliprole | 172 | GOICDS_C DS1499_2 | Imazamethabenz |
| 25 | EST217 | cyazypyr(HGW86) | 172 | GOICDS_C DS1499_2 | Imazamox |
| 25 | EST217 | Azoxystrobin | 172 | GOICDS_C DS1499_2 | Imazapic |
| 25 | EST217 | Dimoxystrobin | 172 | GOICDS_C DS1499_2 | Imazapyr |
| 25 | EST217 | Kresoxim-methyl | 172 | GOICDS_C DS1499_2 | Imazaquin |
| 25 | EST217 | Orysastrobin | 172 | GOICDS_C DS1499_2 | Imazethapyr |
| 25 | EST217 | Pyraclostrobin | 172 | GOICDS_C DS1499_2 | 2,4-D |
| 25 | EST217 | Trifloxystrobin | 172 | GOICDS_C DS1499_2 | Chloridazon |
| 25 | EST217 | Bixafen | 172 | GOICDS_C DS1499_2 | Picloram |
| 25 | EST217 | Boscalid | 172 | GOICDS_C DS1499_2 | Picolinafen |

(continued)

| Line in Table B | Gene Name/ LocusID | Active ingredientB | Line in Table B | Gene Name/ LocusID | Active ingredientB |
|---|---|---|---|---|---|
| 25 | EST217 | Isopyrazam | 172 | GOICDS_CDS1499_2 | Cyclosulfamuron |
| 25 | EST217 | Metalaxyl | 172 | GOICDS_CDS1499_2 | Triflusulfuron |
| 25 | EST217 | Penthiopyrad | 172 | GOICDS_CDS1499_2 | Atrazine |
| 25 | EST217 | 3-Difluoromethyl-1-methyl-1 H-pyrazole-4-carboxylic acid(2', 4',5'-trifluorobiphenyl-2-yl)-amide | 172 | GOICDS_CDS1499_2 | Pyroxasulfone |
| 25 | EST217 | Dimethomorph | 172 | GOICDS_CDS1499_2 | Bentazone |
| 25 | EST217 | Difenoconazole | 172 | GOICDS_CDS1499_2 | Cinidon-ethly |
| 25 | EST217 | Epoxiconazole | 172 | GOICDS_CDS1499_2 | Cinmethylin |
| 25 | EST217 | Fluquinconazole | 172 | GOICDS_CDS1499_2 | Dicamba |
| 25 | EST217 | Metconazol | 172 | GOICDS_CDS1499_2 | Diflufenzopyr |
| 25 | EST217 | Propiconazole | 172 | GOICDS_CDS1499_2 | Quinclorac |
| 25 | EST217 | Prothioconazole | 172 | GOICDS_CDS1499_2 | Quinmerac |
| 25 | EST217 | Tebuconazole | 172 | GOICDS_CDS1499_2 | Mesotrione |
| 25 | EST217 | Triticonazole | 172 | GOICDS_CDS1499_2 | Saflufenacil |
| 25 | EST217 | Prochloraz | 172 | GOICDS_CDS1499_2 | Topramezone; |
| 25 | EST217 | Carbendazim | 173 | GOICDS_CDS1532 | acephate |
| 25 | EST217 | Cyprodinil | 173 | GOICDS_CDS1532 | chlorpyrifos |
| 25 | EST217 | Pyrimethanil | 173 | GOICDS_CDS1532 | dimethoate |
| 25 | EST217 | Fenpropimorph | 173 | GOICDS_CDS1532 | methamidophos |
| 25 | EST217 | Tridemorph | 173 | GOICDS_CDS1532 | terbufos |
| 25 | EST217 | Iprodione | 173 | GOICDS_CDS1532 | aldicarb |

(continued)

| Line in Table B | Gene Name/ LocusID | Active ingredientB | Line in Table B | Gene Name/ LocusID | Active ingredientB |
|---|---|---|---|---|---|
| 25 | EST217 | 5-ethyl-6-octyl-[1,2,4] triazolo [1,5-a]pyrimidine-7-ylamine | 173 | GOICDS_C DS1532 | carbofuran |
| 25 | EST217 | Mancozeb | 173 | GOICDS_C DS1532 | bifenthrin |
| 25 | EST217 | Maneb | 173 | GOICDS_C DS1532 | cypermethrin |
| 25 | EST217 | Metiram | 173 | GOICDS_C DS1532 | alpha-cypermethrin |
| 25 | EST217 | Dithianon | 173 | GOICDS_C DS1532 | deltamethrin |
| 25 | EST217 | Chlorothalonil | 173 | GOICDS_C DS1532 | lambda-cyhalothrin |
| 25 | EST217 | ThiophanateMethyl | 173 | GOICDS_C DS1532 | tefluthrin |
| 25 | EST217 | Cymoxanil | 173 | GOICDS_C DS1532 | flufenoxuron |
| 25 | EST217 | Metrafenone | 173 | GOICDS_C DS1532 | teflubenzuron |
| 25 | EST217 | Acetochlor | 173 | GOICDS_C DS1532 | spirotetramat; |
| 25 | EST217 | Dimethenamid | 173 | GOICDS_C DS1532 | clothianidin |
| 25 | EST217 | Metolachlor | 173 | GOICDS_C DS1532 | imidacloprid |
| 25 | EST217 | Metazachlor | 173 | GOICDS_C DS1532 | thiamethoxam |
| 25 | EST217 | Glyphosate | 173 | GOICDS_C DS1532 | endosulfan |
| 25 | EST217 | Glufosinate | 173 | GOICDS_C DS1532 | fipronil |
| 25 | EST217 | Sulfosate | 173 | GOICDS_C DS1532 | abamectin |
| 25 | EST217 | Fenoxaprop | 173 | GOICDS_C DS1532 | spinosad |
| 25 | EST217 | Paraquat | 173 | GOICDS_C DS1532 | spinetoram |
| 25 | EST217 | Cycloxydim | 173 | GOICDS_C DS1532 | hydramethylnon; |
| 25 | EST217 | Profoxydim | 173 | GOICDS_C DS1532 | chlorfenapyr; |
| 25 | EST217 | Sethoxydim | 173 | GOICDS_C DS1532 | indoxacarb |

(continued)

| Line in Table B | Gene Name/ LocusID | Active ingredientB | Line in Table B | Gene Name/ LocusID | Active ingredientB |
|---|---|---|---|---|---|
| 25 | EST217 | Tepraloxydim | 173 | GOICDS_C DS1532 | metaflumizone |
| 25 | EST217 | Pendimethalin | 173 | GOICDS_C DS1532 | flubendiamide |
| 25 | EST217 | Acifluorfen | 173 | GOICDS_C DS1532 | chlorantraniliprole |
| 25 | EST217 | Imazamethabenz | 173 | GOICDS_C DS1532 | cyazypyr(HGW86) |
| 25 | EST217 | Imazamox | 173 | GOICDS_C DS1532 | Azoxystrobin |
| 25 | EST217 | Imazapic | 173 | GOICDS_C DS1532 | Dimoxystrobin |
| 25 | EST217 | Imazapyr | 173 | GOICDS_C DS1532 | Kresoxim-methyl |
| 25 | EST217 | Imazaquin | 173 | GOICDS_C DS1532 | Orysastrobin |
| 25 | EST217 | Imazethapyr | 173 | GOICDS_C DS1532 | Pyraclostrobin |
| 25 | EST217 | 2,4-D | 173 | GOICDS_C DS1532 | Trifloxystrobin |
| 25 | EST217 | Chloridazon | 173 | GOICDS_C DS1532 | Bixafen |
| 25 | EST217 | Picloram | 173 | GOICDS_C DS1532 | Boscalid |
| 25 | EST217 | Picolinafen | 173 | GOICDS_C DS1532 | Isopyrazam |
| 25 | EST217 | Cyclosulfamuron | 173 | GOICDS_C DS1532 | Metalaxyl |
| 25 | EST217 | Triflusulfuron | 173 | GOICDS_C DS1532 | Penthiopyrad |
| 25 | EST217 | Atrazine | 173 | GOICDS_C DS1532 | 3-Difluoromethyl-1-methyl-1H-pyrazole-4-carboxylic acid(2',4',5'-trifluorobiphenyl-2-yl)-amide |
| 25 | EST217 | Pyroxasulfone | 173 | GOICDS_C DS1532 | Dimethomorph |
| 25 | EST217 | Bentazone | 173 | GOICDS_C DS1532 | Difenoconazole |
| 25 | EST217 | Cinidon-ethly | 173 | GOICDS_C DS1532 | Epoxiconazole |
| 25 | EST217 | Cinmethylin | 173 | GOICDS_C DS1532 | Fluquinconazole |

(continued)

| Line in Table B | Gene Name/ LocusID | Active ingredientB | Line in Table B | Gene Name/ LocusID | Active ingredientB |
|---|---|---|---|---|---|
| 25 | EST217 | Dicamba | 173 | GOICDS_C DS1532 | Metconazol |
| 25 | EST217 | Diflufenzopyr | 173 | GOICDS_C DS1532 | Propiconazole |
| 25 | EST217 | Quinclorac | 173 | GOICDS_C DS1532 | Prothioconazole |
| 25 | EST217 | Quinmerac | 173 | GOICDS_C DS1532 | Tebuconazole |
| 25 | EST217 | Mesotrione | 173 | GOICDS_C DS1532 | Triticonazole |
| 25 | EST217 | Saflufenacil | 173 | GOICDS_C DS1532 | Prochloraz |
| 25 | EST217 | Topramezone; | 173 | GOICDS_C DS1532 | Carbendazim |
| 27 | EST266 | acephate | 173 | GOICDS_C DS1532 | Cyprodinil |
| 27 | EST266 | chlorpyrifos | 173 | GOICDS_C DS1532 | Pyrimethanil |
| 27 | EST266 | dimethoate | 173 | GOICDS_C DS1532 | Fenpropimorph |
| 27 | EST266 | methamidophos | 173 | GOICDS_C DS1532 | Tridemorph |
| 27 | EST266 | terbufos | 173 | GOICDS_C DS1532 | Iprodione |
| 27 | EST266 | aldicarb | 173 | GOICDS_C DS1532 | 5-ethyl-6-octyl-[1,2,4] triazolo [1,5-a]pyrimidine-7-ylamine |
| 27 | EST266 | carbofuran | 173 | GOICDS_C DS1532 | Mancozeb |
| 27 | EST266 | bifenthrin | 173 | GOICDS_C DS1532 | Maneb |
| 27 | EST266 | cypermethrin | 173 | GOICDS_C DS1532 | Metiram |
| 27 | EST266 | alpha-cypermethrin | 173 | GOICDS_C DS1532 | Dithianon |
| 27 | EST266 | deltamethrin | 173 | GOICDS_C DS1532 | Chlorothalonil |
| 27 | EST266 | lambda-cyhalothrin | 173 | GOICDS_C DS1532 | ThiophanateMethyl |
| 27 | EST266 | tefluthrin | 173 | GOICDS_C DS1532 | Cymoxanil |
| 27 | EST266 | flufenoxuron | 173 | GOICDS_C DS1532 | Metrafenone |

(continued)

| Line in Table B | Gene Name/ LocusID | Active ingredientB | Line in Table B | Gene Name/ LocusID | Active ingredientB |
|---|---|---|---|---|---|
| 27 | EST266 | teflubenzuron | 173 | GOICDS_C DS1532 | Acetochlor |
| 27 | EST266 | spirotetramat; | 173 | GOICDS_C DS1532 | Dimethenamid |
| 27 | EST266 | clothianidin | 173 | GOICDS_C DS1532 | Metolachlor |
| 27 | EST266 | imidacloprid | 173 | GOICDS_C DS1532 | Metazachlor |
| 27 | EST266 | thiamethoxam | 173 | GOICDS_C DS1532 | Glyphosate |
| 27 | EST266 | endosulfan | 173 | GOICDS_C DS1532 | Glufosinate |
| 27 | EST266 | fipronil | 173 | GOICDS_C DS1532 | Sulfosate |
| 27 | EST266 | abamectin | 173 | GOICDS_C DS1532 | Fenoxaprop |
| 27 | EST266 | spinosad | 173 | GOICDS_C DS1532 | Paraquat |
| 27 | EST266 | spinetoram | 173 | GOICDS_C DS1532 | Cycloxydim |
| 27 | EST266 | hydramethylnon; | 173 | GOICDS_C DS1532 | Profoxydim |
| 27 | EST266 | chlorfenapyr; | 173 | GOICDS_C DS1532 | Sethoxydim |
| 27 | EST266 | indoxacarb | 173 | GOICDS_C DS1532 | Tepraloxydim |
| 27 | EST266 | metaflumizone | 173 | GOICDS_C DS1532 | Pendimethalin |
| 27 | EST266 | flubendiamide | 173 | GOICDS_C DS1532 | Acifluorfen |
| 27 | EST266 | chlorantraniliprole | 173 | GOICDS_C DS1532 | Imazamethabenz |
| 27 | EST266 | cyazypyr(HGW86) | 173 | GOICDS_C DS1532 | Imazamox |
| 27 | EST266 | Azoxystrobin | 173 | GOICDS_C DS1532 | Imazapic |
| 27 | EST266 | Dimoxystrobin | 173 | GOICDS_C DS1532 | Imazapyr |
| 27 | EST266 | Kresoxim-methyl | 173 | GOICDS_C DS1532 | Imazaquin |
| 27 | EST266 | Orysastrobin | 173 | GOICDS_C DS1532 | Imazethapyr |
| 27 | EST266 | Pyraclostrobin | 173 | GOICDS_C DS1532 | 2,4-D |

(continued)

| Line in Table B | Gene Name/ LocusID | Active ingredientB | Line in Table B | Gene Name/ LocusID | Active ingredientB |
|---|---|---|---|---|---|
| 27 | EST266 | Trifloxystrobin | 173 | GOICDS_C DS1532 | Chloridazon |
| 27 | EST266 | Bixafen | 173 | GOICDS_C DS1532 | Picloram |
| 27 | EST266 | Boscalid | 173 | GOICDS_C DS1532 | Picolinafen |
| 27 | EST266 | Isopyrazam | 173 | GOICDS_C DS1532 | Cyclosulfamuron |
| 27 | EST266 | Metalaxyl | 173 | GOICDS_C DS1532 | Triflusulfuron |
| 27 | EST266 | Penthiopyrad | 173 | GOICDS_C DS1532 | Atrazine |
| 27 | EST266 | 3-Difluoromethyl-1-methyl-1 H-pyrazole-4-carboxylic acid(2',4',5'-trifluorobiphenyl-2-yl)-amide | 173 | GOICDS_C DS1532 | Pyroxasulfone |
| 27 | EST266 | Dimethomorph | 173 | GOICDS_C DS1532 | Bentazone |
| 27 | EST266 | Difenoconazole | 173 | GOICDS_C DS1532 | Cinidon-ethly |
| 27 | EST266 | Epoxiconazole | 173 | GOICDS_C DS1532 | Cinmethylin |
| 27 | EST266 | Fluquinconazole | 173 | GOICDS_C DS1532 | Dicamba |
| 27 | EST266 | Metconazol | 173 | GOICDS_C DS1532 | Diflufenzopyr |
| 27 | EST266 | Propiconazole | 173 | GOICDS_C DS1532 | Quinclorac |
| 27 | EST266 | Prothioconazole | 173 | GOICDS_C DS1532 | Quinmerac |
| 27 | EST266 | Tebuconazole | 173 | GOICDS_C DS1532 | Mesotrione |
| 27 | EST266 | Triticonazole | 173 | GOICDS_C DS1532 | Saflufenacil |
| 27 | EST266 | Prochloraz | 173 | GOICDS_C DS1532 | Topramezone; |
| 27 | EST266 | Carbendazim | 174 | _GOICDS_ CDS3325 | acephate |
| 27 | EST266 | Cyprodinil | 174 | _GOICDS_ CDS3325 | chlorpyrifos |
| 27 | EST266 | Pyrimethanil | 174 | _GOICDS_ CDS3325 | dimethoate |

(continued)

| Line in Table B | Gene Name/ LocusID | Active ingredientB | Line in Table B | Gene Name/ LocusID | Active ingredientB |
|---|---|---|---|---|---|
| 27 | EST266 | Fenpropimorph | 174 | _GOICDS_ CDS3325 | methamidophos |
| 27 | EST266 | Tridemorph | 174 | _GOICDS_ CDS3325 | terbufos |
| 27 | EST266 | Iprodione | 174 | _GOICDS_ CDS3325 | aldicarb |
| 27 | EST266 | 5-ethyl-6-octyl-[1,2,4] triazolo [1,5-a]pyrimidine-7-ylamine | 174 | _GOICDS_ CDS3325 | carbofuran |
| 27 | EST266 | Mancozeb | 174 | _GOICDS_ CDS3325 | bifenthrin |
| 27 | EST266 | Maneb | 174 | _GOICDS_ CDS3325 | cypermethrin |
| 27 | EST266 | Metiram | 174 | _GOICDS_ CDS3325 | alpha-cypermethrin |
| 27 | EST266 | Dithianon | 174 | _GOICDS_ CDS3325 | deltamethrin |
| 27 | EST266 | Chlorothalonil | 174 | _GOICDS_ CDS3325 | lambda-cyhalothrin |
| 27 | EST266 | ThiophanateMethyl | 174 | _GOICDS_ CDS3325 | tefluthrin |
| 27 | EST266 | Cymoxanil | 174 | _GOICDS_ CDS3325 | flufenoxuron |
| 27 | EST266 | Metrafenone | 174 | _GOICDS_ CDS3325 | teflubenzuron |
| 27 | EST266 | Acetochlor | 174 | _GOICDS_ CDS3325 | spirotetramat; |
| 27 | EST266 | Dimethenamid | 174 | _GOICDS_ CDS3325 | clothianidin |
| 27 | EST266 | Metolachlor | 174 | _GOICDS_ CDS3325 | imidacloprid |
| 27 | EST266 | Metazachlor | 174 | _GOICDS_ CDS3325 | thiamethoxam |
| 27 | EST266 | Glyphosate | 174 | _GOICDS_ CDS3325 | endosulfan |
| 27 | EST266 | Glufosinate | 174 | _GOICDS_ CDS3325 | fipronil |
| 27 | EST266 | Sulfosate | 174 | _GOICDS_ CDS3325 | abamectin |
| 27 | EST266 | Fenoxaprop | 174 | _GOICDS_ CDS3325 | spinosad |
| 27 | EST266 | Paraquat | 174 | _GOICDS_ CDS3325 | spinetoram |

(continued)

| Line in Table B | Gene Name/ LocusID | Active ingredientB | Line in Table B | Gene Name/ LocusID | Active ingredientB |
|---|---|---|---|---|---|
| 27 | EST266 | Cycloxydim | 174 | _GOICDS_ CDS3325 | hydramethylnon; |
| 27 | EST266 | Profoxydim | 174 | _GOICDS_ CDS3325 | chlorfenapyr; |
| 27 | EST266 | Sethoxydim | 174 | _GOICDS_ CDS3325 | indoxacarb |
| 27 | EST266 | Tepraloxydim | 174 | _GOICDS_ CDS3325 | metaflumizone |
| 27 | EST266 | Pendimethalin | 174 | _GOICDS_ CDS3325 | flubendiamide |
| 27 | EST266 | Acifluorfen | 174 | _GOICDS_ CDS3325 | chlorantraniliprole |
| 27 | EST266 | Imazamethabenz | 174 | _GOICDS_ CDS3325 | cyazypyr(HGW86) |
| 27 | EST266 | Imazamox | 174 | _GOICDS_ CDS3325 | Azoxystrobin |
| 27 | EST266 | Imazapic | 174 | _GOICDS_ CDS3325 | Dimoxystrobin |
| 27 | EST266 | Imazapyr | 174 | _GOICDS_ CDS3325 | Kresoxim-methyl |
| 27 | EST266 | Imazaquin | 174 | _GOICDS_ CDS3325 | Orysastrobin |
| 27 | EST266 | Imazethapyr | 174 | _GOICDS_ CDS3325 | Pyraclostrobin |
| 27 | EST266 | 2,4-D | 174 | _GOICDS_ CDS3325 | Trifloxystrobin |
| 27 | EST266 | Chloridazon | 174 | _GOICDS_ CDS3325 | Bixafen |
| 27 | EST266 | Picloram | 174 | _GOICDS_ CDS3325 | Boscalid |
| 27 | EST266 | Picolinafen | 174 | _GOICDS_ CDS3325 | Isopyrazam |
| 27 | EST266 | Cyclosulfamuron | 174 | _GOICDS_ CDS3325 | Metalaxyl |
| 27 | EST266 | Triflusulfuron | 174 | _GOICDS_ CDS3325 | Penthiopyrad |
| 27 | EST266 | Atrazine | 174 | _GOICDS_ CDS3325 | 3-Difluoromethyl-1-methyl-1H-pyrazole-4-carboxylic acid(2',4',5'-trifluorobiphenyl-2-yl)-amide |
| 27 | EST266 | Pyroxasulfone | 174 | _GOICDS_ CDS3325 | Dimethomorph |

(continued)

| Line in Table B | Gene Name/ LocusID | Active ingredientB | Line in Table B | Gene Name/ LocusID | Active ingredientB |
|---|---|---|---|---|---|
| 27 | EST266 | Bentazone | 174 | _GOICDS_ CDS3325 | Difenoconazole |
| 27 | EST266 | Cinidon-ethly | 174 | _GOICDS_ CDS3325 | Epoxiconazole |
| 27 | EST266 | Cinmethylin | 174 | _GOICDS_ CDS3325 | Fluquinconazole |
| 27 | EST266 | Dicamba | 174 | _GOICDS_ CDS3325 | Metconazol |
| 27 | EST266 | Diflufenzopyr | 174 | _GOICDS_ CDS3325 | Propiconazole |
| 27 | EST266 | Quinclorac | 174 | _GOICDS_ CDS3325 | Prothioconazole |
| 27 | EST266 | Quinmerac | 174 | _GOICDS_ CDS3325 | Tebuconazole |
| 27 | EST266 | Mesotrione | 174 | _GOICDS_ CDS3325 | Triticonazole |
| 27 | EST266 | Saflufenacil | 174 | _GOICDS_ CDS3325 | Prochloraz |
| 27 | EST266 | Topramezone; | 174 | _GOICDS_ CDS3325 | Carbendazim |
| 28 | EST268 | acephate | 174 | _GOICDS_ CDS3325 | Cyprodinil |
| 28 | EST268 | chlorpyrifos | 174 | _GOICDS_ CDS3325 | Pyrimethanil |
| 28 | EST268 | dimethoate | 174 | _GOICDS_ CDS3325 | Fenpropimorph |
| 28 | EST268 | methamidophos | 174 | _GOICDS_ CDS3325 | Tridemorph |
| 28 | EST268 | terbufos | 174 | _GOICDS_ CDS3325 | Iprodione |
| 28 | EST268 | aldicarb | 174 | _GOICDS_ CDS3325 | 5-ethyl-6-octyl-[1,2,4] triazolo [1,5-a]pyrimidine-7-ylamine |
| 28 | EST268 | carbofuran | 174 | _GOICDS_ CDS3325 | Mancozeb |
| 28 | EST268 | bifenthrin | 174 | _GOICDS_ CDS3325 | Maneb |
| 28 | EST268 | cypermethrin | 174 | _GOICDS_ CDS3325 | Metiram |
| 28 | EST268 | alpha-cypermethrin | 174 | _GOICDS_ CDS3325 | Dithianon |
| 28 | EST268 | deltamethrin | 174 | _GOICDS_ CDS3325 | Chlorothalonil |

(continued)

| Line in Table B | Gene Name/ LocusID | Active ingredientB | Line in Table B | Gene Name/ LocusID | Active ingredientB |
|---|---|---|---|---|---|
| 28 | EST268 | lambda-cyhalothrin | 174 | _GOICDS_ CDS3325 | ThiophanateMethyl |
| 28 | EST268 | tefluthrin | 174 | _GOICDS_ CDS3325 | Cymoxanil |
| 28 | EST268 | flufenoxuron | 174 | _GOICDS_ CDS3325 | Metrafenone |
| 28 | EST268 | teflubenzuron | 174 | _GOICDS_ CDS3325 | Acetochlor |
| 28 | EST268 | spirotetramat; | 174 | _GOICDS_ CDS3325 | Dimethenamid |
| 28 | EST268 | clothianidin | 174 | _GOICDS_ CDS3325 | Metolachlor |
| 28 | EST268 | imidacloprid | 174 | _GOICDS_ CDS3325 | Metazachlor |
| 28 | EST268 | thiamethoxam | 174 | _GOICDS_ CDS3325 | Glyphosate |
| 28 | EST268 | endosulfan | 174 | _GOICDS_ CDS3325 | Glufosinate |
| 28 | EST268 | fipronil | 174 | _GOICDS_ CDS3325 | Sulfosate |
| 28 | EST268 | abamectin | 174 | _GOICDS_ CDS3325 | Fenoxaprop |
| 28 | EST268 | spinosad | 174 | _GOICDS_ CDS3325 | Paraquat |
| 28 | EST268 | spinetoram | 174 | _GOICDS_ CDS3325 | Cycloxydim |
| 28 | EST268 | hydramethylnon; | 174 | _GOICDS_ CDS3325 | Profoxydim |
| 28 | EST268 | chlorfenapyr; | 174 | _GOICDS_ CDS3325 | Sethoxydim |
| 28 | EST268 | indoxacarb | 174 | _GOICDS_ CDS3325 | Tepraloxydim |
| 28 | EST268 | metaflumizone | 174 | _GOICDS_ CDS3325 | Pendimethalin |
| 28 | EST268 | flubendiamide | 174 | _GOICDS_ CDS3325 | Acifluorfen |
| 28 | EST268 | chlorantraniliprole | 174 | _GOICDS_ CDS3325 | Imazamethabenz |
| 28 | EST268 | cyazypyr(HGW86) | 174 | _GOICDS_ CDS3325 | Imazamox |
| 28 | EST268 | Azoxystrobin | 174 | _GOICDS_ CDS3325 | Imazapic |
| 28 | EST268 | Dimoxystrobin | 174 | _GOICDS_ CDS3325 | Imazapyr |

(continued)

| Line in Table B | Gene Name/ LocusID | Active ingredientB | Line in Table B | Gene Name/ LocusID | Active ingredientB |
|---|---|---|---|---|---|
| 28 | EST268 | Kresoxim-methyl | 174 | _GOICDS_ CDS3325 | Imazaquin |
| 28 | EST268 | Orysastrobin | 174 | _GOICDS_ CDS3325 | Imazethapyr |
| 28 | EST268 | Pyraclostrobin | 174 | _GOICDS_ CDS3325 | 2,4-D |
| 28 | EST268 | Trifloxystrobin | 174 | _GOICDS_ CDS3325 | Chloridazon |
| 28 | EST268 | Bixafen | 174 | _GOICDS_ CDS3325 | Picloram |
| 28 | EST268 | Boscalid | 174 | _GOICDS_ CDS3325 | Picolinafen |
| 28 | EST268 | Isopyrazam | 174 | _GOICDS_ CDS3325 | Cyclosulfamuron |
| 28 | EST268 | Metalaxyl | 174 | _GOICDS_ CDS3325 | Triflusulfuron |
| 28 | EST268 | Penthiopyrad | 174 | _GOICDS_ CDS3325 | Atrazine |
| 28 | EST268 | 3-Difluoromethyl-1-methyl-1 H-pyrazole-4-carboxylic acid(2',4',5'-trifluorobiphenyl-2-yl)-amide | 174 | _GOICDS_ CDS3325 | Pyroxasulfone |
| 28 | EST268 | Dimethomorph | 174 | _GOICDS_ CDS3325 | Bentazone |
| 28 | EST268 | Difenoconazole | 174 | _GOICDS_ CDS3325 | Cinidon-ethly |
| 28 | EST268 | Epoxiconazole | 174 | _GOICDS_ CDS3325 | Cinmethylin |
| 28 | EST268 | Fluquinconazole | 174 | _GOICDS_ CDS3325 | Dicamba |
| 28 | EST268 | Metconazol | 174 | _GOICDS_ CDS3325 | Diflufenzopyr |
| 28 | EST268 | Propiconazole | 174 | _GOICDS_ CDS3325 | Quinclorac |
| 28 | EST268 | Prothioconazole | 174 | _GOICDS_ CDS3325 | Quinmerac |
| 28 | EST268 | Tebuconazole | 174 | _GOICDS_ CDS3325 | Mesotrione |
| 28 | EST268 | Triticonazole | 174 | _GOICDS_ CDS3325 | Saflufenacil |
| 28 | EST268 | Prochloraz | 174 | _GOICDS_ CDS3325 | Topramezone; |

(continued)

| Line in Table B | Gene Name/ LocusID | Active ingredientB | Line in Table B | Gene Name/ LocusID | Active ingredientB |
|---|---|---|---|---|---|
| 28 | EST268 | Carbendazim | 175 | GOICDS_C DS0218 | acephate |
| 28 | EST268 | Cyprodinil | 175 | GOICDS_C DS0218 | chlorpyrifos |
| 28 | EST268 | Pyrimethanil | 175 | GOICDS_C DS0218 | dimethoate |
| 28 | EST268 | Fenpropimorph | 175 | GOICDS_C DS0218 | methamidophos |
| 28 | EST268 | Tridemorph | 175 | GOICDS_C DS0218 | terbufos |
| 28 | EST268 | Iprodione | 175 | GOICDS_C DS0218 | aldicarb |
| 28 | EST268 | 5-ethyl-6-octyl-[1,2,4] triazolo [1,5-a]pyrimidine-7-ylamine | 175 | GOICDS_C DS0218 | carbofuran |
| 28 | EST268 | Mancozeb | 175 | GOICDS_C DS0218 | bifenthrin |
| 28 | EST268 | Maneb | 175 | GOICDS_C DS0218 | cypermethrin |
| 28 | EST268 | Metiram | 175 | GOICDS_C DS0218 | alpha-cypermethrin |
| 28 | EST268 | Dithianon | 175 | GOICDS_C DS0218 | deltamethrin |
| 28 | EST268 | Chlorothalonil | 175 | GOICDS_C DS0218 | lambda-cyhalothrin |
| 28 | EST268 | ThiophanateMethyl | 175 | GOICDS_C DS0218 | tefluthrin |
| 28 | EST268 | Cymoxanil | 175 | GOICDS_C DS0218 | flufenoxuron |
| 28 | EST268 | Metrafenone | 175 | GOICDS_C DS0218 | teflubenzuron |
| 28 | EST268 | Acetochlor | 175 | GOICDS_C DS0218 | spirotetramat; |
| 28 | EST268 | Dimethenamid | 175 | GOICDS_C DS0218 | clothianidin |
| 28 | EST268 | Metolachlor | 175 | GOICDS_C DS0218 | imidacloprid |
| 28 | EST268 | Metazachlor | 175 | GOICDS_C DS0218 | thiamethoxam |
| 28 | EST268 | Glyphosate | 175 | GOICDS_C DS0218 | endosulfan |
| 28 | EST268 | Glufosinate | 175 | GOICDS_C DS0218 | fipronil |

(continued)

| Line in Table B | Gene Name/ LocusID | Active ingredientB | Line in Table B | Gene Name/ LocusID | Active ingredientB |
|---|---|---|---|---|---|
| 28 | EST268 | Sulfosate | 175 | GOICDS_C DS0218 | abamectin |
| 28 | EST268 | Fenoxaprop | 175 | GOICDS_C DS0218 | spinosad |
| 28 | EST268 | Paraquat | 175 | GOICDS_C DS0218 | spinetoram |
| 28 | EST268 | Cycloxydim | 175 | GOICDS_C DS0218 | hydramethylnon; |
| 28 | EST268 | Profoxydim | 175 | GOICDS_C DS0218 | chlorfenapyr; |
| 28 | EST268 | Sethoxydim | 175 | GOICDS_C DS0218 | indoxacarb |
| 28 | EST268 | Tepraloxydim | 175 | GOICDS_C DS0218 | metaflumizone |
| 28 | EST268 | Pendimethalin | 175 | GOICDS_C DS0218 | flubendiamide |
| 28 | EST268 | Acifluorfen | 175 | GOICDS_C DS0218 | chlorantraniliprole |
| 28 | EST268 | Imazamethabenz | 175 | GOICDS_C DS0218 | cyazypyr(HGW86) |
| 28 | EST268 | Imazamox | 175 | GOICDS_C DS0218 | Azoxystrobin |
| 28 | EST268 | Imazapic | 175 | GOICDS_C DS0218 | Dimoxystrobin |
| 28 | EST268 | Imazapyr | 175 | GOICDS_C DS0218 | Kresoxim-methyl |
| 28 | EST268 | Imazaquin | 175 | GOICDS_C DS0218 | Orysastrobin |
| 28 | EST268 | Imazethapyr | 175 | GOICDS_C DS0218 | Pyraclostrobin |
| 28 | EST268 | 2,4-D | 175 | GOICDS_C DS0218 | Trifloxystrobin |
| 28 | EST268 | Chloridazon | 175 | GOICDS_C DS0218 | Bixafen |
| 28 | EST268 | Picloram | 175 | GOICDS_C DS0218 | Boscalid |
| 28 | EST268 | Picolinafen | 175 | GOICDS_C DS0218 | Isopyrazam |
| 28 | EST268 | Cyclosulfamuron | 175 | GOICDS_C DS0218 | Metalaxyl |
| 28 | EST268 | Triflusulfuron | 175 | GOICDS_C DS0218 | Penthiopyrad |

(continued)

| Line in Table B | Gene Name/ LocusID | Active ingredientB | Line in Table B | Gene Name/ LocusID | Active ingredientB |
|---|---|---|---|---|---|
| 28 | EST268 | Atrazine | 175 | GOICDS_C DS0218 | 3-Difluoromethyl-1-methyl-1H-pyrazole-4-carboxylic acid(2', 4',5'-trifluorobiphenyl-2-yl)-amide |
| 28 | EST268 | Pyroxasulfone | 175 | GOICDS_C DS0218 | Dimethomorph |
| 28 | EST268 | Bentazone | 175 | GOICDS_C DS0218 | Difenoconazole |
| 28 | EST268 | Cinidon-ethly | 175 | GOICDS_C DS0218 | Epoxiconazole |
| 28 | EST268 | Cinmethylin | 175 | GOICDS_C DS0218 | Fluquinconazole |
| 28 | EST268 | Dicamba | 175 | GOICDS_C DS0218 | Metconazol |
| 28 | EST268 | Diflufenzopyr | 175 | GOICDS_C DS0218 | Propiconazole |
| 28 | EST268 | Quinclorac | 175 | GOICDS_C DS0218 | Prothioconazole |
| 28 | EST268 | Quinmerac | 175 | GOICDS_C DS0218 | Tebuconazole |
| 28 | EST268 | Mesotrione | 175 | GOICDS_C DS0218 | Triticonazole |
| 28 | EST268 | Saflufenacil | 175 | GOICDS_C DS0218 | Prochloraz |
| 28 | EST268 | Topramezone; | 175 | GOICDS_C DS0218 | Carbendazim |
| 30 | EST293 | acephate | 175 | GOICDS_C DS0218 | Cyprodinil |
| 30 | EST293 | chlorpyrifos | 175 | GOICDS_C DS0218 | Pyrimethanil |
| 30 | EST293 | dimethoate | 175 | GOICDS_C DS0218 | Fenpropimorph |
| 30 | EST293 | methamidophos | 175 | GOICDS_C DS0218 | Tridemorph |
| 30 | EST293 | terbufos | 175 | GOICDS_C DS0218 | Iprodione |
| 30 | EST293 | aldicarb | 175 | GOICDS_C DS0218 | 5-ethyl-6-octyl-[1,2,4] triazolo [1,5-a]pyrimidine-7-ylamine |
| 30 | EST293 | carbofuran | 175 | GOICDS_C DS0218 | Mancozeb |

(continued)

| Line in Table B | Gene Name/ LocusID | Active ingredientB | Line in Table B | Gene Name/ LocusID | Active ingredientB |
|---|---|---|---|---|---|
| 30 | EST293 | bifenthrin | 175 | GOICDS_C DS0218 | Maneb |
| 30 | EST293 | cypermethrin | 175 | GOICDS_C DS0218 | Metiram |
| 30 | EST293 | alpha-cypermethrin | 175 | GOICDS_C DS0218 | Dithianon |
| 30 | EST293 | deltamethrin | 175 | GOICDS_C DS0218 | Chlorothalonil |
| 30 | EST293 | lambda-cyhalothrin | 175 | GOICDS_C DS0218 | ThiophanateMethyl |
| 30 | EST293 | tefluthrin | 175 | GOICDS_C DS0218 | Cymoxanil |
| 30 | EST293 | flufenoxuron | 175 | GOICDS_C DS0218 | Metrafenone |
| 30 | EST293 | teflubenzuron | 175 | GOICDS_C DS0218 | Acetochlor |
| 30 | EST293 | spirotetramat; | 175 | GOICDS_C DS0218 | Dimethenamid |
| 30 | EST293 | clothianidin | 175 | GOICDS_C DS0218 | Metolachlor |
| 30 | EST293 | imidacloprid | 175 | GOICDS_C DS0218 | Metazachlor |
| 30 | EST293 | thiamethoxam | 175 | GOICDS_C DS0218 | Glyphosate |
| 30 | EST293 | endosulfan | 175 | GOICDS_C DS0218 | Glufosinate |
| 30 | EST293 | fipronil | 175 | GOICDS_C DS0218 | Sulfosate |
| 30 | EST293 | abamectin | 175 | GOICDS_C DS0218 | Fenoxaprop |
| 30 | EST293 | spinosad | 175 | GOICDS_C DS0218 | Paraquat |
| 30 | EST293 | spinetoram | 175 | GOICDS_C DS0218 | Cycloxydim |
| 30 | EST293 | hydramethylnon; | 175 | GOICDS_C DS0218 | Profoxydim |
| 30 | EST293 | chlorfenapyr; | 175 | GOICDS_C DS0218 | Sethoxydim |
| 30 | EST293 | indoxacarb | 175 | GOICDS_C DS0218 | Tepraloxydim |
| 30 | EST293 | metaflumizone | 175 | GOICDS8_C DS0218 | Pendimethalin |
| 30 | EST293 | flubendiamide | 175 | GOICDS C DS0218 | Acifluorfen |

(continued)

| Line in Table B | Gene Name/ LocusID | Active ingredientB | Line in Table B | Gene Name/ LocusID | Active ingredientB |
|---|---|---|---|---|---|
| 30 | EST293 | chlorantraniliprole | 175 | GOICDS_C DS0218 | Imazamethabenz |
| 30 | EST293 | cyazypyr(HGW86) | 175 | GOICDS_C DS0218 | Imazamox |
| 30 | EST293 | Azoxystrobin | 175 | GOICDS_C DS0218 | Imazapic |
| 30 | EST293 | Dimoxystrobin | 175 | GOICDS_C DS0218 | Imazapyr |
| 30 | EST293 | Kresoxim-methyl | 175 | GOICDS_C DS0218 | Imazaquin |
| 30 | EST293 | Orysastrobin | 175 | GOICDS_C DS0218 | Imazethapyr |
| 30 | EST293 | Pyraclostrobin | 175 | GOICDS_C DS0218 | 2,4-D |
| 30 | EST293 | Trifloxystrobin | 175 | GOICDS_C DS0218 | Chloridazon |
| 30 | EST293 | Bixafen | 175 | GOICDS_C DS0218 | Picloram |
| 30 | EST293 | Boscalid | 175 | GOICDS_C DS0218 | Picolinafen |
| 30 | EST293 | Isopyrazam | 175 | GOICDS_C DS0218 | Cyclosulfamuron |
| 30 | EST293 | Metalaxyl | 175 | GOICDS_C DS0218 | Triflusulfuron |
| 30 | EST293 | Penthiopyrad | 175 | GOICDS_C DS0218 | Atrazine |
| 30 | EST293 | 3-Difluoromethyl-1-methyl-1 H-pyrazole-4-carboxylic acid(2',4',5'-trifluorobiphenyl-2-yl)-amide | 175 | GOICDS_C DS0218 | Pyroxasulfone |
| 30 | EST293 | Dimethomorph | 175 | GOICDS_C DS0218 | Bentazone |
| 30 | EST293 | Difenoconazole | 175 | GOICDS_C DS0218 | Cinidon-ethly |
| 30 | EST293 | Epoxiconazole | 175 | GOICDS_C DS0218 | Cinmethylin |
| 30 | EST293 | Fluquinconazole | 175 | GOICDS_C DS0218 | Dicamba |
| 30 | EST293 | Metconazol | 175 | GOICDS_C DS0218 | Diflufenzopyr |
| 30 | EST293 | Propiconazole | 175 | GOICDS_C DS0218 | Quinclorac |

(continued)

| Line in Table B | Gene Name/ LocusID | Active ingredientB | Line in Table B | Gene Name/ LocusID | Active ingredientB |
|---|---|---|---|---|---|
| 30 | EST293 | Prothioconazole | 175 | GOICDS_C DS0218 | Quinmerac |
| 30 | EST293 | Tebuconazole | 175 | GOICDS_C DS0218 | Mesotrione |
| 30 | EST293 | Triticonazole | 175 | GOICDS_C DS0218 | Saflufenacil |
| 30 | EST293 | Prochloraz | 175 | GOICDS_C DS0218 | Topramezone; |
| 30 | EST293 | Carbendazim | 176 | GOICDS_C DS1536 | acephate |
| 30 | EST293 | Cyprodinil | 176 | GOICDS_C DS1536 | chlorpyrifos |
| 30 | EST293 | Pyrimethanil | 176 | GOICDS_C DS1536 | dimethoate |
| 30 | EST293 | Fenpropimorph | 176 | GOICDS_C DS1536 | methamidophos |
| 30 | EST293 | Tridemorph | 176 | GOICDS_C DS1536 | terbufos |
| 30 | EST293 | Iprodione | 176 | GOICDS_C DS1536 | aldicarb |
| 30 | EST293 | 5-ethyl-6-octyl-[1,2,4] triazolo [1,5-a]pyrimidine-7-ylamine | 176 | GOICDS_C DS1536 | carbofuran |
| 30 | EST293 | Mancozeb | 176 | GOICDS_C DS1536 | bifenthrin |
| 30 | EST293 | Maneb | 176 | GOICDS_C DS1536 | cypermethrin |
| 30 | EST293 | Metiram | 176 | GOICDS_C DS1536 | alpha-cypermethrin |
| 30 | EST293 | Dithianon | 176 | GOICDS_C DS1536 | deltamethrin |
| 30 | EST293 | Chlorothalonil | 176 | GOICDS_C DS1536 | lambda-cyhalothrin |
| 30 | EST293 | ThiophanateMethyl | 176 | GOICDS_C DS1536 | tefluthrin |
| 30 | EST293 | Cymoxanil | 176 | GOICDS_C DS1536 | flufenoxuron |
| 30 | EST293 | Metrafenone | 176 | GOICDS_C DS1536 | teflubenzuron |
| 30 | EST293 | Acetochlor | 176 | GOICDS_C DS1536 | spirotetramat; |
| 30 | EST293 | Dimethenamid | 176 | GOICDS_C DS1536 | clothianidin |

(continued)

| Line in Table B | Gene Name/ LocusID | Active ingredientB | Line in Table B | Gene Name/ LocusID | Active ingredientB |
|---|---|---|---|---|---|
| 30 | EST293 | Metolachlor | 176 | GOICDS_C DS1536 | imidacloprid |
| 30 | EST293 | Metazachlor | 176 | GOICDS_C DS1536 | thiamethoxam |
| 30 | EST293 | Glyphosate | 176 | GOICDS_C DS1536 | endosulfan |
| 30 | EST293 | Glufosinate | 176 | GOICDS_C DS1536 | fipronil |
| 30 | EST293 | Sulfosate | 176 | GOICDS_C DS1536 | abamectin |
| 30 | EST293 | Fenoxaprop | 176 | GOICDS_C DS1536 | spinosad |
| 30 | EST293 | Paraquat | 176 | GOICDS_C DS1536 | spinetoram |
| 30 | EST293 | Cycloxydim | 176 | GOICDS_C DS1536 | hydramethylnon; |
| 30 | EST293 | Profoxydim | 176 | GOICDS_C DS1536 | chlorfenapyr; |
| 30 | EST293 | Sethoxydim | 176 | GOICDS_C DS1536 | indoxacarb |
| 30 | EST293 | Tepraloxydim | 176 | GOICDS_C DS1536 | metaflumizone |
| 30 | EST293 | Pendimethalin | 176 | GOICDS_C DS1536 | flubendiamide |
| 30 | EST293 | Acifluorfen | 176 | GOICDS_C DS1536 | chlorantraniliprole |
| 30 | EST293 | Imazamethabenz | 176 | GOICDS_C DS1536 | cyazypyr(HGW86) |
| 30 | EST293 | Imazamox | 176 | GOICDS_C DS1536 | Azoxystrobin |
| 30 | EST293 | Imazapic | 176 | GOICDS_C DS1536 | Dimoxystrobin |
| 30 | EST293 | Imazapyr | 176 | GOICDS_C DS1536 | Kresoxim-methyl |
| 30 | EST293 | Imazaquin | 176 | GOICDS_C DS1536 | Orysastrobin |
| 30 | EST293 | Imazethapyr | 176 | GOICDS_C DS1536 | Pyraclostrobin |
| 30 | EST293 | 2,4-D | 176 | GOICDS_C DS1536 | Trifloxystrobin |
| 30 | EST293 | Chloridazon | 176 | GOICDS_C DS1536 | Bixafen |
| 30 | EST293 | Picloram | 176 | GOICDS_C DS1536 | Boscalid |

(continued)

| Line in Table B | Gene Name/ LocusID | Active ingredientB | Line in Table B | Gene Name/ LocusID | Active ingredientB |
|---|---|---|---|---|---|
| 30 | EST293 | Picolinafen | 176 | GOICDS_C DS1536 | Isopyrazam |
| 30 | EST293 | Cyclosulfamuron | 176 | GOICDS_C DS1536 | Metalaxyl |
| 30 | EST293 | Triflusulfuron | 176 | GOICDS_C DS1536 | Penthiopyrad |
| 30 | EST293 | Atrazine | 176 | GOICDS_C DS1536 | 3-Difluoromethyl-1-methyl-1H-pyrazole-4-carboxylic acid(2',4',5'-trifluorobiphenyl-2-yl)-amide |
| 30 | EST293 | Pyroxasulfone | 176 | GOICDS_C DS1536 | Dimethomorph |
| 30 | EST293 | Bentazone | 176 | GOICDS_C DS1536 | Difenoconazole |
| 30 | EST293 | Cinidon-ethly | 176 | GOICDS_C DS1536 | Epoxiconazole |
| 30 | EST293 | Cinmethylin | 176 | GOICDS_C DS1536 | Fluquinconazole |
| 30 | EST293 | Dicamba | 176 | GOICDS_C DS1536 | Metconazol |
| 30 | EST293 | Diflufenzopyr | 176 | GOICDS_C DS1536 | Propiconazole |
| 30 | EST293 | Quinclorac | 176 | GOICDS_C DS1536 | Prothioconazole |
| 30 | EST293 | Quinmerac | 176 | GOICDS_C DS1536 | Tebuconazole |
| 30 | EST293 | Mesotrione | 176 | GOICDS_C DS1536 | Triticonazole |
| 30 | EST293 | Saflufenacil | 176 | GOICDS_C DS1536 | Prochloraz |
| 30 | EST293 | Topramezone; | 176 | GOICDS_C DS1536 | Carbendazim |
| 31 | EST295 | acephate | 176 | GOICDS_C DS1536 | Cyprodinil |
| 31 | EST295 | chlorpyrifos | 176 | GOICDS_C DS1536 | Pyrimethanil |
| 31 | EST295 | dimethoate | 176 | GOICDS_C DS1536 | Fenpropimorph |
| 31 | EST295 | methamidophos | 176 | GOICDS_C DS1536 | Tridemorph |
| 31 | EST295 | terbufos | 176 | GOICDS_C DS1536 | Iprodione |

(continued)

| Line in Table B | Gene Name/ LocusID | Active ingredientB | Line in Table B | Gene Name/ LocusID | Active ingredientB |
|---|---|---|---|---|---|
| 31 | EST295 | aldicarb | 176 | GOICDS_C : DS1536 | 5-ethyl-6-octyl-[1,2,4] triazolo [1,5-a]pyrimidine-7-ylamine |
| 31 | EST295 | carbofuran | 176 | GOICDS_C DS1536 | Mancozeb |
| 31 | EST295 | bifenthrin | 176 | GOICDS_C DS1536 | Maneb |
| 31 | EST295 | cypermethrin | 176 | GOICDS_C DS1536 | Metiram |
| 31 | EST295 | alpha-cypermethrin | 176 | GOICDS_C DS1536 | Dithianon |
| 31 | EST295 | deltamethrin | 176 | GOICDS_C DS1536 | Chlorothalonil |
| 31 | EST295 | lambda-cyhalothrin | 176 | GOICDS_C DS1536 | ThiophanateMethyl |
| 31 | EST295 | tefluthrin | 176 | GOICDS_C DS1536 | Cymoxanil |
| 31 | EST295 | flufenoxuron | 176 | GOICDS_C DS1536 | Metrafenone |
| 31 | EST295 | teflubenzuron | 176 | GOICDS_C DS1536 | Acetochlor |
| 31 | EST295 | spirotetramat; | 176 | GOICDS_C DS1536 | Dimethenamid |
| 31 | EST295 | clothianidin | 176 | GOICDS_C DS1536 | Metolachlor |
| 31 | EST295 | imidacloprid | 176 | GOICDS_C DS1536 | Metazachlor |
| 31 | EST295 | thiamethoxam | 176 | GOICDS_C DS1536 | Glyphosate |
| 31 | EST295 | endosulfan | 176 | GOICDS_C DS1536 | Glufosinate |
| 31 | EST295 | fipronil | 176 | GOICDS_C DS1536 | Sulfosate |
| 31 | EST295 | abamectin | 176 | GOICDS_C DS1536 | Fenoxaprop |
| 31 | EST295 | spinosad | 176 | GOICDS_C DS1536 | Paraquat |
| 31 | EST295 | spinetoram | 176 | GOICDS_C DS1536 | Cycloxydim |
| 31 | EST295 | hydramethylnon; | 176 | GOICDS_C DS1536 | Profoxydim |
| 31 | EST295 | chlorfenapyr; | 176 | GOICDS_C DS1536 | Sethoxydim |

(continued)

| Line in Table B | Gene Name/ LocusID | Active ingredientB | Line in Table B | Gene Name/ LocusID | Active ingredientB |
|---|---|---|---|---|---|
| 31 | EST295 | indoxacarb | 176 | GOICDS_C DS1536 | Tepraloxydim |
| 31 | EST295 | metaflumizone | 176 | GOICDS_C DS1536 | Pendimethalin |
| 31 | EST295 | flubendiamide | 176 | GOICDS C DS1536 | Acifluorfen |
| 31 | EST295 | chlorantraniliprole | 176 | GOICDS_C DS1536 | Imazamethabenz |
| 31 | EST295 | cyazypyr(HGW86) | 176 | GOICDS_C DS1536 | Imazamox |
| 31 | EST295 | Azoxystrobin | 176 | GOICDS_C DS1536 | Imazapic |
| 31 | EST295 | Dimoxystrobin | 176 | GOICDS_C DS1536 | Imazapyr |
| 31 | EST295 | Kresoxim-methyl | 176 | GOICDS_C DS1536 | Imazaquin |
| 31 | EST295 | Orysastrobin | 176 | GOICDS_C DS1536 | Imazethapyr |
| 31 | EST295 | Pyraclostrobin | 176 | GOICDS_C DS1536 | 2,4-D |
| 31 | EST295 | Trifloxystrobin | 176 | GOICDS_C DS1536 | Chloridazon |
| 31 | EST295 | Bixafen | 176 | GOICDS_C DS1536 | Picloram |
| 31 | EST295 | Boscalid | 176 | GOICDS_C DS1536 | Picolinafen |
| 31 | EST295 | Isopyrazam | 176 | GOICDS_C DS1536 | Cyclosulfamuron |
| 31 | EST295 | Metalaxyl | 176 | GOICDS_C DS1536 | Triflusulfuron |
| 31 | EST295 | Penthiopyrad | 176 | GOICDS_C DS1536 | Atrazine |
| 31 | EST295 | 3-Difluoromethyl-1-methyl-1 H-pyrazole-4-carboxylic acid(2',4',5'-trifluorobiphenyl-2-yl)-amide | 176 | GOICDS_C DS1536 | Pyroxasulfone |
| 31 | EST295 | Dimethomorph | 176 | GOICDS_C DS1536 | Bentazone |
| 31 | EST295 | Difenoconazole | 176 | GOICDS_C DS1536 | Cinidon-ethly |
| 31 | EST295 | Epoxiconazole | 176 | GOICDS_C DS1536 | Cinmethylin |

(continued)

| Line in Table B | Gene Name/ LocusID | Active ingredientB | Line in Table B | Gene Name/ LocusID | Active ingredientB |
|---|---|---|---|---|---|
| 31 | EST295 | Fluquinconazole | 176 | GOICDS_C DS1536 | Dicamba |
| 31 | EST295 | Metconazol | 176 | GOICDS_C DS1536 | Diflufenzopyr |
| 31 | EST295 | Propiconazole | 176 | GOICDS_C DS1536 | Quinclorac |
| 31 | EST295 | Prothioconazole | 176 | GOICDS_C DS1536 | Quinmerac |
| 31 | EST295 | Tebuconazole | 176 | GOICDS_C DS1536 | Mesotrione |
| 31 | EST295 | Triticonazole | 176 | GOICDS_C DS1536 | Saflufenacil |
| 31 | EST295 | Prochloraz | 176 | GOICDS_C DS1536 | Topramezone; |
| 31 | EST295 | Carbendazim | 177 | | acephate |
| 31 | EST295 | Cyprodinil | 177 | | chlorpyrifos |
| 31 | EST295 | Pyrimethanil | 177 | | dimethoate |
| 31 | EST295 | Fenpropimorph | 177 | | methamidophos |
| 31 | EST295 | Tridemorph | 177 | | terbufos |
| 31 | EST295 | Iprodione | 177 | | aldicarb |
| 31 | EST295 | 5-ethyl-6-octyl-[1,2,4] triazolo [1,5-a]pyrimidine-7-ylamine | 177 | | carbofuran |
| 31 | EST295 | Mancozeb | 177 | | bifenthrin |
| 31 | EST295 | Maneb | 177 | | cypermethrin |
| 31 | EST295 | Metiram | 177 | | alpha-cypermethrin |
| 31 | EST295 | Dithianon | 177 | | deltamethrin |
| 31 | EST295 | Chlorothalonil | 177 | | lambda-cyhalothrin |
| 31 | EST295 | ThiophanateMethyl | 177 | | tefluthrin |
| 31 | EST295 | Cymoxanil | 177 | | flufenoxuron |
| 31 | EST295 | Metrafenone | 177 | | teflubenzuron |
| 31 | EST295 | Acetochlor | 177 | | spirotetramat; |
| 31 | EST295 | Dimethenamid | 177 | | clothianidin |
| 31 | EST295 | Metolachlor | 177 | | imidacloprid |
| 31 | EST295 | Metazachlor | 177 | | thiamethoxam |
| 31 | EST295 | Glyphosate | 177 | | endosulfan |
| 31 | EST295 | Glufosinate | 177 | | fipronil |
| 31 | EST295 | Sulfosate | 177 | | abamectin |
| 31 | EST295 | Fenoxaprop | 177 | | spinosad |

(continued)

| Line in Table B | Gene Name/ LocusID | Active ingredientB | Line in Table B | Gene Name/ LocusID | Active ingredientB |
|---|---|---|---|---|---|
| 31 | EST295 | Paraquat | 177 | | spinetoram |
| 31 | EST295 | Cycloxydim | 177 | | hydramethylnon; |
| 31 | EST295 | Profoxydim | 177 | | chlorfenapyr; |
| 31 | EST295 | Sethoxydim | 177 | | indoxacarb |
| 31 | EST295 | Tepraloxydim | 177 | | metaflumizone |
| 31 | EST295 | Pendimethalin | 177 | | flubendiamide |
| 31 | EST295 | Acifluorfen | 177 | | chlorantraniliprole |
| 31 | EST295 | Imazamethabenz | 177 | | cyazypyr(HGW86) |
| 31 | EST295 | Imazamox | 177 | | Azoxystrobin |
| 31 | EST295 | Imazapic | 177 | | Dimoxystrobin |
| 31 | EST295 | Imazapyr | 177 | | Kresoxim-methyl |
| 31 | EST295 | Imazaquin | 177 | | Orysastrobin |
| 31 | EST295 | Imazethapyr | 177 | | Pyraclostrobin |
| 31 | EST295 | 2,4-D | 177 | | Trifloxystrobin |
| 31 | EST295 | Chloridazon | 177 | | Bixafen |
| 31 | EST295 | Picloram | 177 | | Boscalid |
| 31 | EST295 | Picolinafen | 177 | | Isopyrazam |
| 31 | EST295 | Cyclosulfamuron | 177 | | Metalaxyl |
| 31 | EST295 | Triflusulfuron | 177 | | Penthiopyrad |
| 31 | EST295 | Atrazine | 177 | | 3-Difluoromethyl-1-methyl-1H-pyrazole-4-carboxylic acid(2', 4',5'-trifluorobiphenyl-2-yl)-amide |
| 31 | EST295 | Pyroxasulfone | 177 | | Dimethomorph |
| 31 | EST295 | Bentazone | 177 | | Difenoconazole |
| 31 | EST295 | Cinidon-ethly | 177 | | Epoxiconazole |
| 31 | EST295 | Cinmethylin | 177 | | Fluquinconazole |
| 31 | EST295 | Dicamba | 177 | | Metconazol |
| 31 | EST295 | Diflufenzopyr | 177 | | Propiconazole |
| 31 | EST295 | Quinclorac | 177 | | Prothioconazole |
| 31 | EST295 | Quinmerac | 177 | | Tebuconazole |
| 31 | EST295 | Mesotrione | 177 | | Triticonazole |
| 31 | EST295 | Saflufenacil | 177 | | Prochloraz |
| 31 | EST295 | Topramezone; | 177 | | Carbendazim |
| 33 | EST335 | acephate | 177 | | Cyprodinil |
| 33 | EST335 | chlorpyrifos | 177 | | Pyrimethanil |

(continued)

| Line in Table B | Gene Name/ LocusID | Active ingredientB | Line in Table B | Gene Name/ LocusID | Active ingredientB |
|---|---|---|---|---|---|
| 33 | EST335 | dimethoate | 177 | | Fenpropimorph |
| 33 | EST335 | methamidophos | 177 | | Tridemorph |
| 33 | EST335 | terbufos | 177 | | Iprodione |
| 33 | EST335 | aldicarb | 177 | | 5-ethyl-6-octyl-[1,2,4] triazolo [1,5-a]pyrimidine-7-ylamine |
| 33 | EST335 | carbofuran | 177 | | Mancozeb |
| 33 | EST335 | bifenthrin | 177 | | Maneb |
| 33 | EST335 | cypermethrin | 177 | | Metiram |
| 33 | EST335 | alpha-cypermethrin | 177 | | Dithianon |
| 33 | EST335 | deltamethrin | 177 | | Chlorothalonil |
| 33 | EST335 | lambda-cyhalothrin | 177 | | ThiophanateMethyl |
| 33 | EST335 | tefluthrin | 177 | | Cymoxanil |
| 33 | EST335 | flufenoxuron | 177 | | Metrafenone |
| 33 | EST335 | teflubenzuron | 177 | | Acetochlor |
| 33 | EST335 | spirotetramat; | 177 | | Dimethenamid |
| 33 | EST335 | clothianidin | 177 | | Metolachlor |
| 33 | EST335 | imidacloprid | 177 | | Metazachlor |
| 33 | EST335 | thiamethoxam | 177 | | Glyphosate |
| 33 | EST335 | endosulfan | 177 | | Glufosinate |
| 33 | EST335 | fipronil | 177 | | Sulfosate |
| 33 | EST335 | abamectin | 177 | | Fenoxaprop |
| 33 | EST335 | spinosad | 177 | | Paraquat |
| 33 | EST335 | spinetoram | 177 | | Cycloxydim |
| 33 | EST335 | hydramethylnon; | 177 | | Profoxydim |
| 33 | EST335 | chlorfenapyr; | 177 | | Sethoxydim |
| 33 | EST335 | indoxacarb | 177 | | Tepraloxydim |
| 33 | EST335 | metaflumizone | 177 | | Pendimethalin |
| 33 | EST335 | flubendiamide | 177 | | Acifluorfen |
| 33 | EST335 | chlorantraniliprole | 177 | | Imazamethabenz |
| 33 | EST335 | cyazypyr(HGW86) | 177 | | Imazamox |
| 33 | EST335 | Azoxystrobin | 177 | | Imazapic |
| 33 | EST335 | Dimoxystrobin | 177 | | Imazapyr |
| 33 | EST335 | Kresoxim-methyl | 177 | | Imazaquin |
| 33 | EST335 | Orysastrobin | 177 | | Imazethapyr |
| 33 | EST335 | Pyraclostrobin | 177 | | 2,4-D |
| 33 | EST335 | Trifloxystrobin | 177 | | Chloridazon |

(continued)

| Line in Table B | Gene Name/ LocusID | Active ingredientB | Line in Table B | Gene Name/ LocusID | Active ingredientB |
|---|---|---|---|---|---|
| 33 | EST335 | Bixafen | 177 | | Picloram |
| 33 | EST335 | Boscalid | 177 | | Picolinafen |
| 33 | EST335 | Isopyrazam | 177 | | Cyclosulfamuron |
| 33 | EST335 | Metalaxyl | 177 | | Triflusulfuron |
| 33 | EST335 | Penthiopyrad | 177 | | Atrazine |
| 33 | EST335 | 3-Difluoromethyl-1-methyl-1 H-pyrazole-4-carboxylic acid(2',4',5'-trifluorobiphenyl-2-yl)-amide | 177 | | Pyroxasulfone |
| 33 | EST335 | Dimethomorph | 177 | | Bentazone |
| 33 | EST335 | Difenoconazole | 177 | | Cinidon-ethly |
| 33 | EST335 | Epoxiconazole | 177 | | Cinmethylin |
| 33 | EST335 | Fluquinconazole | 177 | | Dicamba |
| 33 | EST335 | Metconazol | 177 | | Diflufenzopyr |
| 33 | EST335 | Propiconazole | 177 | | Quinclorac |
| 33 | EST335 | Prothioconazole | 177 | | Quinmerac |
| 33 | EST335 | Tebuconazole | 177 | | Mesotrione |
| 33 | EST335 | Triticonazole | 177 | | Saflufenacil |
| 33 | EST335 | Prochloraz | 177 | | Topramezone; |
| 33 | EST335 | Carbendazim | 178 | GOICDS_CDS3074 | acephate |
| 33 | EST335 | Cyprodinil | 178 | GOICDS_CDS3074 | chlorpyrifos |
| 33 | EST335 | Pyrimethanil | 178 | GOICDS_CDS3074 | dimethoate |
| 33 | EST335 | Fenpropimorph | 178 | GOICDS_CDS3074 | methamidophos |
| 33 | EST335 | Tridemorph | 178 | GOICDS_CDS3074 | terbufos |
| 33 | EST335 | Iprodione | 178 | GOICDS_CDS3074 | aldicarb |
| 33 | EST335 | 5-ethyl-6-octyl-[1,2,4] triazolo [1,5-a]pyrimidine-7-ylamine | 178 | GOICDS_CDS3074 | carbofuran |
| 33 | EST335 | Mancozeb | 178 | GOICDS_CDS3074 | bifenthrin |
| 33 | EST335 | Maneb | 178 | GOICDS_CDS3074 | cypermethrin |

(continued)

| Line in Table B | Gene Name/ LocusID | Active ingredientB | Line in Table B | Gene Name/ LocusID | Active ingredientB |
|---|---|---|---|---|---|
| 33 | EST335 | Metiram | 178 | GOICDS_C DS3074 | alpha-cypermethrin |
| 33 | EST335 | Dithianon | 178 | GOICDS_C DS3074 | deltamethrin |
| 33 | EST335 | Chlorothalonil | 178 | GOICDS_C DS3074 | lambda-cyhalothrin |
| 33 | EST335 | ThiophanateMethyl | 178 | GOICDS_C DS3074 | tefluthrin |
| 33 | EST335 | Cymoxanil | 178 | GOICDS_C DS3074 | flufenoxuron |
| 33 | EST335 | Metrafenone | 178 | GOICDS_C DS3074 | teflubenzuron |
| 33 | EST335 | Acetochlor | 178 | GOICDS_C DS3074 | spirotetramat; |
| 33 | EST335 | Dimethenamid | 178 | GOICDS_C DS3074 | clothianidin |
| 33 | EST335 | Metolachlor | 178 | GOICDS_C DS3074 | imidacloprid |
| 33 | EST335 | Metazachlor | 178 | GOICDS_C DS3074 | thiamethoxam |
| 33 | EST335 | Glyphosate | 178 | GOICDS_C DS3074 | endosulfan |
| 33 | EST335 | Glufosinate | 178 | GOICDS_C DS3074 | fipronil |
| 33 | EST335 | Sulfosate | 178 | GOICDS_C DS3074 | abamectin |
| 33 | EST335 | Fenoxaprop | 178 | GOICDS_C DS3074 | spinosad |
| 33 | EST335 | Paraquat | 178 | GOICDS_C DS3074 | spinetoram |
| 33 | EST335 | Cycloxydim | 178 | GOICDS_C DS3074 | hydramethylnon; |
| 33 | EST335 | Profoxydim | 178 | GOICDS_C DS3074 | chlorfenapyr; |
| 33 | EST335 | Sethoxydim | 178 | GOICDS_C DS3074 | indoxacarb |
| 33 | EST335 | Tepraloxydim | 178 | GOICDS_C DS3074 | metaflumizone |
| 33 | EST335 | Pendimethalin | 178 | GOICDS_C DS3074 | flubendiamide |
| 33 | EST335 | Acifluorfen | 178 | GOICDS_C DS3074 | chlorantraniliprole |
| 33 | EST335 | Imazamethabenz | 178 | GOICDS_C DS3074 | cyazypyr(HGW86) |

(continued)

| Line in Table B | Gene Name/ LocusID | Active ingredientB | Line in Table B | Gene Name/ LocusID | Active ingredientB |
|---|---|---|---|---|---|
| 33 | EST335 | Imazamox | 178 | GOICDS_C DS3074 | Azoxystrobin |
| 33 | EST335 | Imazapic | 178 | GOICDS_C DS3074 | Dimoxystrobin |
| 33 | EST335 | Imazapyr | 178 | GOICDS_C DS3074 | Kresoxim-methyl |
| 33 | EST335 | Imazaquin | 178 | GOICDS_C DS3074 | Orysastrobin |
| 33 | EST335 | Imazethapyr | 178 | GOICDS_C DS3074 | Pyraclostrobin |
| 33 | EST335 | 2,4-D | 178 | GOICDS_C DS3074 | Trifloxystrobin |
| 33 | EST335 | Chloridazon | 178 | GOICDS_C DS3074 | Bixafen |
| 33 | EST335 | Picloram | 178 | GOICDS_C DS3074 | Boscalid |
| 33 | EST335 | Picolinafen | 178 | GOICDS_C DS3074 | Isopyrazam |
| 33 | EST335 | Cyclosulfamuron | 178 | GOICDS_C DS3074 | Metalaxyl |
| 33 | EST335 | Triflusulfuron | 178 | GOICDS_C DS3074 | Penthiopyrad |
| 33 | EST335 | Atrazine | 178 | GOICDS_C DS3074 | 3-Difluoromethyl-1-methyl-1H-pyrazole-4-carboxylic acid(2’, 4’,5’-trifluorobiphenyl-2-yl)-amide |
| 33 | EST335 | Pyroxasulfone | 178 | GOICDS_C DS3074 | Dimethomorph |
| 33 | EST335 | Bentazone | 178 | GOICDS_C DS3074 | Difenoconazole |
| 33 | EST335 | Cinidon-ethly | 178 | GOICDS_C DS3074 | Epoxiconazole |
| 33 | EST335 | Cinmethylin | 178 | GOICDS_C DS3074 | Fluquinconazole |
| 33 | EST335 | Dicamba | 178 | GOICDS_C DS3074 | Metconazol |
| 33 | EST335 | Diflufenzopyr | 178 | GOICDS_C DS3074 | Propiconazole |
| 33 | EST335 | Quinclorac | 178 | GOICDS_C DS3074 | Prothioconazole |
| 33 | EST335 | Quinmerac | 178 | GOICDS_C DS3074 | Tebuconazole |

(continued)

| Line in Table B | Gene Name/ LocusID | Active ingredientB | Line in Table B | Gene Name/ LocusID | Active ingredientB |
|---|---|---|---|---|---|
| 33 | EST335 | Mesotrione | 178 | GOICDS_CDS3074 | Triticonazole |
| 33 | EST335 | Saflufenacil | 178 | GOICDS_CDS3074 | Prochloraz |
| 33 | EST335 | Topramezone; | 178 | GOICDS_CDS3074 | Carbendazim |
| 37 | EST4 | acephate | 178 | GOICDS_CDS3074 | Cyprodinil |
| 37 | EST4 | chlorpyrifos | 178 | GOICDS_CDS3074 | Pyrimethanil |
| 37 | EST4 | dimethoate | 178 | GOICDS_CDS3074 | Fenpropimorph |
| 37 | EST4 | methamidophos | 178 | GOICDS_CDS3074 | Tridemorph |
| 37 | EST4 | terbufos | 178 | GOICDS_CDS3074 | Iprodione |
| 37 | EST4 | aldicarb | 178 | GOICDS_CDS3074 | 5-ethyl-6-octyl-[1,2,4] triazolo [1,5-a]pyrimidine-7-ylamine |
| 37 | EST4 | carbofuran | 178 | GOICDS_CDS3074 | Mancozeb |
| 37 | EST4 | bifenthrin | 178 | GOICDS_CDS3074 | Maneb |
| 37 | EST4 | cypermethrin | 178 | GOICDS_CDS3074 | Metiram |
| 37 | EST4 | alpha-cypermethrin | 178 | GOICDS_CDS3074 | Dithianon |
| 37 | EST4 | deltamethrin | 178 | GOICDS_CDS3074 | Chlorothalonil |
| 37 | EST4 | lambda-cyhalothrin | 178 | GOICDS_CDS3074 | ThiophanateMethyl |
| 37 | EST4 | tefluthrin | 178 | GOICDS_CDS3074 | Cymoxanil |
| 37 | EST4 | flufenoxuron | 178 | GOICDS_CDS3074 | Metrafenone |
| 37 | EST4 | teflubenzuron | 178 | GOICDS_CDS3074 | Acetochlor |
| 37 | EST4 | spirotetramat; | 178 | GOICDS_CDS3074 | Dimethenamid |
| 37 | EST4 | clothianidin | 178 | GOICDS_CDS3074 | Metolachlor |
| 37 | EST4 | imidacloprid | 178 | GOICDS_CDS3074 | Metazachlor |

(continued)

| Line in Table B | Gene Name/ LocusID | Active ingredientB | Line in Table B | Gene Name/ LocusID | Active ingredientB |
|---|---|---|---|---|---|
| 37 | EST4 | thiamethoxam | 178 | GOICDS_C DS3074 | Glyphosate |
| 37 | EST4 | endosulfan | 178 | GOICDS_C DS3074 | Glufosinate |
| 37 | EST4 | fipronil | 178 | GOICDS_C DS3074 | Sulfosate |
| 37 | EST4 | abamectin | 178 | GOICDS_C DS3074 | Fenoxaprop |
| 37 | EST4 | spinosad | 178 | GOICDS_C DS3074 | Paraquat |
| 37 | EST4 | spinetoram | 178 | GOICDS_C DS3074 | Cycloxydim |
| 37 | EST4 | hydramethylnon; | 178 | GOICDS_C DS3074 | Profoxydim |
| 37 | EST4 | chlorfenapyr; | 178 | GOICDS_C DS3074 | Sethoxydim |
| 37 | EST4 | indoxacarb | 178 | GOICDS_C DS3074 | Tepraloxydim |
| 37 | EST4 | metaflumizone | 178 | GOICDS_C DS3074 | Pendimethalin |
| 37 | EST4 | flubendiamide | 178 | GOICDS_C DS3074 | Acifluorfen |
| 37 | EST4 | chlorantraniliprole | 178 | GOICDS_C DS3074 | Imazamethabenz |
| 37 | EST4 | cyazypyr(HGW86) | 178 | GOICDS_C DS3074 | Imazamox |
| 37 | EST4 | Azoxystrobin | 178 | GOICDS_C DS3074 | Imazapic |
| 37 | EST4 | Dimoxystrobin | 178 | GOICDS_C DS3074 | Imazapyr |
| 37 | EST4 | Kresoxim-methyl | 178 | GOICDS_C DS3074 | Imazaquin |
| 37 | EST4 | Orysastrobin | 178 | GOICDS_C DS3074 | Imazethapyr |
| 37 | EST4 | Pyraclostrobin | 178 | GOICDS_C DS3074 | 2,4-D |
| 37 | EST4 | Trifloxystrobin | 178 | GOICDS_C DS3074 | Chloridazon |
| 37 | EST4 | Bixafen | 178 | GOICDS_C DS3074 | Picloram |
| 37 | EST4 | Boscalid | 178 | GOICDS_C DS3074 | Picolinafen |
| 37 | EST4 | Isopyrazam | 178 | GOICDS_C DS3074 | Cyclosulfamuron |

(continued)

| Line in Table B | Gene Name/ LocusID | Active ingredientB | Line in Table B | Gene Name/ LocusID | Active ingredientB |
|---|---|---|---|---|---|
| 37 | EST4 | Metalaxyl | 178 | GOICDS_C DS3074 | Triflusulfuron |
| 37 | EST4 | Penthiopyrad | 178 | GOICDS_C DS3074 | Atrazine |
| 37 | EST4 | 3-Difluoromethyl-1-methyl-1 H-pyrazole-4-carboxylic acid(2',4',5'-trifluorobiphenyl-2-yl)-amide | 178 | GOICDS_C DS3074 | Pyroxasulfone |
| 37 | EST4 | Dimethomorph | 178 | GOICDS_C DS3074 | Bentazone |
| 37 | EST4 | Difenoconazole | 178 | GOICDS_C DS3074 | Cinidon-ethly |
| 37 | EST4 | Epoxiconazole | 178 | GOICDS_C DS3074 | Cinmethylin |
| 37 | EST4 | Fluquinconazole | 178 | GOICDS_C DS3074 | Dicamba |
| 37 | EST4 | Metconazol | 178 | GOICDS_C DS3074 | Diflufenzopyr |
| 37 | EST4 | Propiconazole | 178 | GOICDS_C DS3074 | Quinclorac |
| 37 | EST4 | Prothioconazole | 178 | GOICDS_C DS3074 | Quinmerac |
| 37 | EST4 | Tebuconazole | 178 | GOICDS_C DS3074 | Mesotrione |
| 37 | EST4 | Triticonazole | 178 | GOICDS_C DS3074 | Saflufenacil |
| 37 | EST4 | Prochloraz | 178 | GOICDS_C DS3074 | Topramezone; |
| 37 | EST4 | Carbendazim | 182 | | acephate |
| 37 | EST4 | Cyprodinil | 182 | | chlorpyrifos |
| 37 | EST4 | Pyrimethanil | 182 | | dimethoate |
| 37 | EST4 | Fenpropimorph | 182 | | methamidophos |
| 37 | EST4 | Tridemorph | 182 | | terbufos |
| 37 | EST4 | Iprodione | 182 | | aldicarb |
| 37 | EST4 | 5-ethyl-6-octyl-[1,2,4] triazolo [1,5-a]pyrimidine-7-ylamine | 182 | | carbofuran |
| 37 | EST4 | Mancozeb | 182 | | bifenthrin |
| 37 | EST4 | Maneb | 182 | | cypermethrin |
| 37 | EST4 | Metiram | 182 | | alpha-cypermethrin |

(continued)

| Line in Table B | Gene Name/ LocusID | Active ingredientB | Line in Table B | Gene Name/ LocusID | Active ingredientB |
|---|---|---|---|---|---|
| 37 | EST4 | Dithianon | 182 | | deltamethrin |
| 37 | EST4 | Chlorothalonil | 182 | | lambda-cyhalothrin |
| 37 | EST4 | ThiophanateMethyl | 182 | | tefluthrin |
| 37 | EST4 | Cymoxanil | 182 | | flufenoxuron |
| 37 | EST4 | Metrafenone | 182 | | teflubenzuron |
| 37 | EST4 | Acetochlor | 182 | | spirotetramat; |
| 37 | EST4 | Dimethenamid | 182 | | clothianidin |
| 37 | EST4 | Metolachlor | 182 | | imidacloprid |
| 37 | EST4 | Metazachlor | 182 | | thiamethoxam |
| 37 | EST4 | Glyphosate | 182 | | endosulfan |
| 37 | EST4 | Glufosinate | 182 | | fipronil |
| 37 | EST4 | Sulfosate | 182 | | abamectin |
| 37 | EST4 | Fenoxaprop | 182 | | spinosad |
| 37 | EST4 | Paraquat | 182 | | spinetoram |
| 37 | EST4 | Cycloxydim | 182 | | hydramethylnon; |
| 37 | EST4 | Profoxydim | 182 | | chlorfenapyr; |
| 37 | EST4 | Sethoxydim | 182 | | indoxacarb |
| 37 | EST4 | Tepraloxydim | 182 | | metaflumizone |
| 37 | EST4 | Pendimethalin | 182 | | flubendiamide |
| 37 | EST4 | Acifluorfen | 182 | | chlorantraniliprole |
| 37 | EST4 | Imazamethabenz | 182 | | cyazypyr(HGW86) |
| 37 | EST4 | Imazamox | 182 | | Azoxystrobin |
| 37 | EST4 | Imazapic | 182 | | Dimoxystrobin |
| 37 | EST4 | Imazapyr | 182 | | Kresoxim-methyl |
| 37 | EST4 | Imazaquin | 182 | | Orysastrobin |
| 37 | EST4 | Imazethapyr | 182 | | Pyraclostrobin |
| 37 | EST4 | 2,4-D | 182 | | Trifloxystrobin |
| 37 | EST4 | Chloridazon | 182 | | Bixafen |
| 37 | EST4 | Picloram | 182 | | Boscalid |
| 37 | EST4 | Picolinafen | 182 | | Isopyrazam |
| 37 | EST4 | Cyclosulfamuron | 182 | | Metalaxyl |
| 37 | EST4 | Triflusulfuron | 182 | | Penthiopyrad |
| 37 | EST4 | Atrazine | 182 | | 3-Difluoromethyl-1-methyl-1H-pyrazole-4-carboxylic acid(2',4',5'-trifluorobiphenyl-2-yl)-amide |

(continued)

| Line in Table B | Gene Name/ LocusID | Active ingredientB | Line in Table B | Gene Name/ LocusID | Active ingredientB |
|---|---|---|---|---|---|
| 37 | EST4 | Pyroxasulfone | 182 | | Dimethomorph |
| 37 | EST4 | Bentazone | 182 | | Difenoconazole |
| 37 | EST4 | Cinidon-ethly | 182 | | Epoxiconazole |
| 37 | EST4 | Cinmethylin | 182 | | Fluquinconazole |
| 37 | EST4 | Dicamba | 182 | | Metconazol |
| 37 | EST4 | Diflufenzopyr | 182 | | Propiconazole |
| 37 | EST4 | Quinclorac | 182 | | Prothioconazole |
| 37 | EST4 | Quinmerac | 182 | | Tebuconazole |
| 37 | EST4 | Mesotrione | 182 | | Triticonazole |
| 37 | EST4 | Saflufenacil | 182 | | Prochloraz |
| 37 | EST4 | Topramezone; | 182 | | Carbendazim |
| 39 | EST443 | acephate | 182 | | Cyprodinil |
| 39 | EST443 | chlorpyrifos | 182 | | Pyrimethanil |
| 39 | EST443 | dimethoate | 182 | | Fenpropimorph |
| 39 | EST443 | methamidophos | 182 | | Tridemorph |
| 39 | EST443 | terbufos | 182 | | Iprodione |
| 39 | EST443 | aldicarb | 182 | | 5-ethyl-6-octyl-[1,2,4] triazolo [1,5-a]pyrimidine-7-ylamine |
| 39 | EST443 | carbofuran | 182 | | Mancozeb |
| 39 | EST443 | bifenthrin | 182 | | Maneb |
| 39 | EST443 | cypermethrin | 182 | | Metiram |
| 39 | EST443 | alpha-cypermethrin | 182 | | Dithianon |
| 39 | EST443 | deltamethrin | 182 | | Chlorothalonil |
| 39 | EST443 | lambda-cyhalothrin | 182 | | ThiophanateMethyl |
| 39 | EST443 | tefluthrin | 182 | | Cymoxanil |
| 39 | EST443 | flufenoxuron | 182 | | Metrafenone |
| 39 | EST443 | teflubenzuron | 182 | | Acetochlor |
| 39 | EST443 | spirotetramat; | 182 | | Dimethenamid |
| 39 | EST443 | clothianidin | 182 | | Metolachlor |
| 39 | EST443 | imidacloprid | 182 | | Metazachlor |
| 39 | EST443 | thiamethoxam | 182 | | Glyphosate |
| 39 | EST443 | endosulfan | 182 | | Glufosinate |
| 39 | EST443 | fipronil | 182 | | Sulfosate |
| 39 | EST443 | abamectin | 182 | | Fenoxaprop |
| 39 | EST443 | spinosad | 182 | | Paraquat |
| 39 | EST443 | spinetoram | 182 | | Cycloxydim |

(continued)

| Line in Table B | Gene Name/ LocusID | Active ingredientB | Line in Table B | Gene Name/ LocusID | Active ingredientB |
|---|---|---|---|---|---|
| 39 | EST443 | hydramethylnon; | 182 | | Profoxydim |
| 39 | EST443 | chlorfenapyr; | 182 | | Sethoxydim |
| 39 | EST443 | indoxacarb | 182 | | Tepraloxydim |
| 39 | EST443 | metaflumizone | 182 | | Pendimethalin |
| 39 | EST443 | flubendiamide | 182 | | Acifluorfen |
| 39 | EST443 | chlorantraniliprole | 182 | | Imazamethabenz |
| 39 | EST443 | cyazypyr(HGW86) | 182 | | Imazamox |
| 39 | EST443 | Azoxystrobin | 182 | | Imazapic |
| 39 | EST443 | Dimoxystrobin | 182 | | Imazapyr |
| 39 | EST443 | Kresoxim-methyl | 182 | | Imazaquin |
| 39 | EST443 | Orysastrobin | 182 | | Imazethapyr |
| 39 | EST443 | Pyraclostrobin | 182 | | 2,4-D |
| 39 | EST443 | Trifloxystrobin | 182 | | Chloridazon |
| 39 | EST443 | Bixafen | 182 | | Picloram |
| 39 | EST443 | Boscalid | 182 | | Picolinafen |
| 39 | EST443 | Isopyrazam | 182 | | Cyclosulfamuron |
| 39 | EST443 | Metalaxyl | 182 | | Triflusulfuron |
| 39 | EST443 | Penthiopyrad | 182 | | Atrazine |
| 39 | EST443 | 3-Difluoromethyl-1-methyl-1 H-pyrazole-4-carboxylic acid(2', 4',5'-trifluorobiphenyl-2-yl)-amide | 182 | | Pyroxasulfone |
| 39 | EST443 | Dimethomorph | 182 | | Bentazone |
| 39 | EST443 | Difenoconazole | 182 | | Cinidon-ethly |
| 39 | EST443 | Epoxiconazole | 182 | | Cinmethylin |
| 39 | EST443 | Fluquinconazole | 182 | | Dicamba |
| 39 | EST443 | Metconazol | 182 | | Diflufenzopyr |
| 39 | EST443 | Propiconazole | 182 | | Quinclorac |
| 39 | EST443 | Prothioconazole | 182 | | Quinmerac |
| 39 | EST443 | Tebuconazole | 182 | | Mesotrione |
| 39 | EST443 | Triticonazole | 182 | | Saflufenacil |
| 39 | EST443 | Prochloraz | 182 | | Topramezone; |
| 39 | EST443 | Carbendazim | 186 | | acephate |
| 39 | EST443 | Cyprodinil | 186 | | chlorpyrifos |
| 39 | EST443 | Pyrimethanil | 186 | | dimethoate |
| 39 | EST443 | Fenpropimorph | 186 | | methamidophos |

(continued)

| Line in Table B | Gene Name/ LocusID | Active ingredientB | Line in Table B | Gene Name/ LocusID | Active ingredientB |
|---|---|---|---|---|---|
| 39 | EST443 | Tridemorph | 186 | | terbufos |
| 39 | EST443 | Iprodione | 186 | | aldicarb |
| 39 | EST443 | 5-ethyl-6-octyl-[1,2,4] triazolo [1,5-a]pyrimidine-7-ylamine | 186 | | carbofuran |
| 39 | EST443 | Mancozeb | 186 | | bifenthrin |
| 39 | EST443 | Maneb | 186 | | cypermethrin |
| 39 | EST443 | Metiram | 186 | | alpha-cypermethrin |
| 39 | EST443 | Dithianon | 186 | | deltamethrin |
| 39 | EST443 | Chlorothalonil | 186 | | lambda-cyhalothrin |
| 39 | EST443 | ThiophanateMethyl | 186 | | tefluthrin |
| 39 | EST443 | Cymoxanil | 186 | | flufenoxuron |
| 39 | EST443 | Metrafenone | 186 | | teflubenzuron |
| 39 | EST443 | Acetochlor | 186 | | spirotetramat; |
| 39 | EST443 | Dimethenamid | 186 | | clothianidin |
| 39 | EST443 | Metolachlor | 186 | | imidacloprid |
| 39 | EST443 | Metazachlor | 186 | | thiamethoxam |
| 39 | EST443 | Glyphosate | 186 | | endosulfan |
| 39 | EST443 | Glufosinate | 186 | | fipronil |
| 39 | EST443 | Sulfosate | 186 | | abamectin |
| 39 | EST443 | Fenoxaprop | 186 | | spinosad |
| 39 | EST443 | Paraquat | 186 | | spinetoram |
| 39 | EST443 | Cycloxydim | 186 | | hydramethylnon; |
| 39 | EST443 | Profoxydim | 186 | | chlorfenapyr; |
| 39 | EST443 | Sethoxydim | 186 | | indoxacarb |
| 39 | EST443 | Tepraloxydim | 186 | | metaflumizone |
| 39 | EST443 | Pendimethalin | 186 | | flubendiamide |
| 39 | EST443 | Acifluorfen | 186 | | chlorantraniliprole |
| 39 | EST443 | Imazamethabenz | 186 | | cyazypyr(HGW86) |
| 39 | EST443 | Imazamox | 186 | | Azoxystrobin |
| 39 | EST443 | Imazapic | 186 | | Dimoxystrobin |
| 39 | EST443 | Imazapyr | 186 | | Kresoxim-methyl |
| 39 | EST443 | Imazaquin | 186 | | Orysastrobin |
| 39 | EST443 | Imazethapyr | 186 | | Pyraclostrobin |
| 39 | EST443 | 2,4-D | 186 | | Trifloxystrobin |
| 39 | EST443 | Chloridazon | 186 | | Bixafen |
| 39 | EST443 | Picloram | 186 | | Boscalid |

(continued)

| Line in Table B | Gene Name/ LocusID | Active ingredientB | Line in Table B | Gene Name/ LocusID | Active ingredientB |
|---|---|---|---|---|---|
| 39 | EST443 | Picolinafen | 186 | | Isopyrazam |
| 39 | EST443 | Cyclosulfamuron | 186 | | Metalaxyl |
| 39 | EST443 | Triflusulfuron | 186 | | Penthiopyrad |
| 39 | EST443 | Atrazine | 186 | | 3-Difluoromethyl-1-methyl-1H-pyrazole-4-carboxylicacid(2',4',5'-trifluorobiphenyl-2-yl)-amide |
| 39 | EST443 | Pyroxasulfone | 186 | | Dimethomorph |
| 39 | EST443 | Bentazone | 186 | | Difenoconazole |
| 39 | EST443 | Cinidon-ethly | 186 | | Epoxiconazole |
| 39 | EST443 | Cinmethylin | 186 | | Fluquinconazole |
| 39 | EST443 | Dicamba | 186 | | Metconazol |
| 39 | EST443 | Diflufenzopyr | 186 | | Propiconazole |
| 39 | EST443 | Quinclorac | 186 | | Prothioconazole |
| 39 | EST443 | Quinmerac | 186 | | Tebuconazole |
| 39 | EST443 | Mesotrione | 186 | | Triticonazole |
| 39 | EST443 | Saflufenacil | 186 | | Prochloraz |
| 39 | EST443 | Topramezone; | 186 | | Carbendazim |
| 40 | EST46 | acephate | 186 | | Cyprodinil |
| 40 | EST46 | chlorpyrifos | 186 | | Pyrimethanil |
| 40 | EST46 | dimethoate | 186 | | Fenpropimorph |
| 40 | EST46 | methamidophos | 186 | | Tridemorph |
| 40 | EST46 | terbufos | 186 | | Iprodione |
| 40 | EST46 | aldicarb | 186 | | 5-ethyl-6-octyl-[1,2,4]triazolo [1,5-a]pyrimidine-7-ylamine |
| 40 | EST46 | carbofuran | 186 | | Mancozeb |
| 40 | EST46 | bifenthrin | 186 | | Maneb |
| 40 | EST46 | cypermethrin | 186 | | Metiram |
| 40 | EST46 | alpha-cypermethrin | 186 | | Dithianon |
| 40 | EST46 | deltamethrin | 186 | | Chlorothalonil |
| 40 | EST46 | lambda-cyhalothrin | 186 | | ThiophanateMethyl |
| 40 | EST46 | tefluthrin | 186 | | Cymoxanil |
| 40 | EST46 | flufenoxuron | 186 | | Metrafenone |
| 40 | EST46 | teflubenzuron | 186 | | Acetochlor |
| 40 | EST46 | spirotetramat; | 186 | | Dimethenamid |

(continued)

| Line in Table B | Gene Name/ LocusID | Active ingredientB | Line in Table B | Gene Name/ LocusID | Active ingredientB |
|---|---|---|---|---|---|
| 40 | EST46 | clothianidin | 186 | | Metolachlor |
| 40 | EST46 | imidacloprid | 186 | | Metazachlor |
| 40 | EST46 | thiamethoxam | 186 | | Glyphosate |
| 40 | EST46 | endosulfan | 186 | | Glufosinate |
| 40 | EST46 | fipronil | 186 | | Sulfosate |
| 40 | EST46 | abamectin | 186 | | Fenoxaprop |
| 40 | EST46 | spinosad | 186 | | Paraquat |
| 40 | EST46 | spinetoram | 186 | | Cycloxydim |
| 40 | EST46 | hydramethylnon; | 186 | | Profoxydim |
| 40 | EST46 | chlorfenapyr; | 186 | | Sethoxydim |
| 40 | EST46 | indoxacarb | 186 | | Tepraloxydim |
| 40 | EST46 | metaflumizone | 186 | | Pendimethalin |
| 40 | EST46 | flubendiamide | 186 | | Acifluorfen |
| 40 | EST46 | chlorantraniliprole | 186 | | Imazamethabenz |
| 40 | EST46 | cyazypyr(HGW86) | 186 | | Imazamox |
| 40 | EST46 | Azoxystrobin | 186 | | Imazapic |
| 40 | EST46 | Dimoxystrobin | 186 | | Imazapyr |
| 40 | EST46 | Kresoxim-methyl | 186 | | Imazaquin |
| 40 | EST46 | Orysastrobin | 186 | | Imazethapyr |
| 40 | EST46 | Pyraclostrobin | 186 | | 2,4-D |
| 40 | EST46 | Trifloxystrobin | 186 | | Chloridazon |
| 40 | EST46 | Bixafen | 186 | | Picloram |
| 40 | EST46 | Boscalid | 186 | | Picolinafen |
| 40 | EST46 | Isopyrazam | 186 | | Cyclosulfamuron |
| 40 | EST46 | Metalaxyl | 186 | | Triflusulfuron |
| 40 | EST46 | Penthiopyrad | 186 | | Atrazine |
| 40 | EST46 | 3-Difluoromethyl-1-methyl-1 H-pyrazole-4-carboxylic acid(2',4',5'-trifluorobiphenyl-2-yl)-amide | 186 | | Pyroxasulfone |
| 40 | EST46 | Dimethomorph | 186 | | Bentazone |
| 40 | EST46 | Difenoconazole | 186 | | Cinidon-ethly |
| 40 | EST46 | Epoxiconazole | 186 | | Cinmethylin |
| 40 | EST46 | Fluquinconazole | 186 | | Dicamba |
| 40 | EST46 | Metconazol | 186 | | Diflufenzopyr |
| 40 | EST46 | Propiconazole | 186 | | Quinclorac |

(continued)

| Line in Table B | Gene Name/ LocusID | Active ingredientB | Line in Table B | Gene Name/ LocusID | Active ingredientB |
|---|---|---|---|---|---|
| 40 | EST46 | Prothioconazole | 186 | | Quinmerac |
| 40 | EST46 | Tebuconazole | 186 | | Mesotrione |
| 40 | EST46 | Triticonazole | 186 | | Saflufenacil |
| 40 | EST46 | Prochloraz | 186 | | Topramezone; |
| 40 | EST46 | Carbendazim | 187 | | acephate |
| 40 | EST46 | Cyprodinil | 187 | | chlorpyrifos |
| 40 | EST46 | Pyrimethanil | 187 | | dimethoate |
| 40 | EST46 | Fenpropimorph | 187 | | methamidophos |
| 40 | EST46 | Tridemorph | 187 | | terbufos |
| 40 | EST46 | Iprodione | 187 | | aldicarb |
| 40 | EST46 | 5-ethyl-6-octyl-[1,2,4] triazolo [1,5-a]pyrimidine-7-ylamine | 187 | | carbofuran |
| 40 | EST46 | Mancozeb | 187 | | bifenthrin |
| 40 | EST46 | Maneb | 187 | | cypermethrin |
| 40 | EST46 | Metiram | 187 | | alpha-cypermethrin |
| 40 | EST46 | Dithianon | 187 | | deltamethrin |
| 40 | EST46 | Chlorothalonil | 187 | | lambda-cyhalothrin |
| 40 | EST46 | ThiophanateMethyl | 187 | | tefluthrin |
| 40 | EST46 | Cymoxanil | 187 | | flufenoxuron |
| 40 | EST46 | Metrafenone | 187 | | teflubenzuron |
| 40 | EST46 | Acetochlor | 187 | | spirotetramat; |
| 40 | EST46 | Dimethenamid | 187 | | clothianidin |
| 40 | EST46 | Metolachlor | 187 | | imidacloprid |
| 40 | EST46 | Metazachlor | 187 | | thiamethoxam |
| 40 | EST46 | Glyphosate | 187 | | endosulfan |
| 40 | EST46 | Glufosinate | 187 | | fipronil |
| 40 | EST46 | Sulfosate | 187 | | abamectin |
| 40 | EST46 | Fenoxaprop | 187 | | spinosad |
| 40 | EST46 | Paraquat | 187 | | spinetoram |
| 40 | EST46 | Cycloxydim | 187 | | hydramethylnon; |
| 40 | EST46 | Profoxydim | 187 | | chlorfenapyr; |
| 40 | EST46 | Sethoxydim | 187 | | indoxacarb |
| 40 | EST46 | Tepraloxydim | 187 | | metaflumizone |
| 40 | EST46 | Pendimethalin | 187 | | flubendiamide |
| 40 | EST46 | Acifluorfen | 187 | | chlorantraniliprole |
| 40 | EST46 | Imazamethabenz | 187 | | cyazypyr(HGW86) |

(continued)

| Line in Table B | Gene Name/ LocusID | Active ingredientB | Line in Table B | Gene Name/ LocusID | Active ingredientB |
|---|---|---|---|---|---|
| 40 | EST46 | Imazamox | 187 | | Azoxystrobin |
| 40 | EST46 | Imazapic | 187 | | Dimoxystrobin |
| 40 | EST46 | Imazapyr | 187 | | Kresoxim-methyl |
| 40 | EST46 | Imazaquin | 187 | | Orysastrobin |
| 40 | EST46 | Imazethapyr | 187 | | Pyraclostrobin |
| 40 | EST46 | 2,4-D | 187 | | Trifloxystrobin |
| 40 | EST46 | Chloridazon | 187 | | Bixafen |
| 40 | EST46 | Picloram | 187 | | Boscalid |
| 40 | EST46 | Picolinafen | 187 | | Isopyrazam |
| 40 | EST46 | Cyclosulfamuron | 187 | | Metalaxyl |
| 40 | EST46 | Triflusulfuron | 187 | | Penthiopyrad |
| 40 | EST46 | Atrazine | 187 | | 3-Difluoromethyl-1-methyl-1H-pyrazole-4-carboxylic acid(2',4',5'-trifluorobiphenyl-2-yl)-amide |
| 40 | EST46 | Pyroxasulfone | 187 | | Dimethomorph |
| 40 | EST46 | Bentazone | 187 | | Difenoconazole |
| 40 | EST46 | Cinidon-ethly | 187 | | Epoxiconazole |
| 40 | EST46 | Cinmethylin | 187 | | Fluquinconazole |
| 40 | EST46 | Dicamba | 187 | | Metconazol |
| 40 | EST46 | Diflufenzopyr | 187 | | Propiconazole |
| 40 | EST46 | Quinclorac | 187 | | Prothioconazole |
| 40 | EST46 | Quinmerac | 187 | | Tebuconazole |
| 40 | EST46 | Mesotrione | 187 | | Triticonazole |
| 40 | EST46 | Saflufenacil | 187 | | Prochloraz |
| 40 | EST46 | Topramezone; | 187 | | Carbendazim |
| 42 | EST512 | acephate | 187 | | Cyprodinil |
| 42 | EST512 | chlorpyrifos | 187 | | Pyrimethanil |
| 42 | EST512 | dimethoate | 187 | | Fenpropimorph |
| 42 | EST512 | methamidophos | 187 | | Tridemorph |
| 42 | EST512 | terbufos | 187 | | Iprodione |
| 42 | EST512 | aldicarb | 187 | | 5-ethyl-6-octyl-[1,2,4] triazolo [1,5-a]pyrimidine-7-ylamine |
| 42 | EST512 | carbofuran | 187 | | Mancozeb |
| 42 | EST512 | bifenthrin | 187 | | Maneb |

(continued)

| Line in Table B | Gene Name/ LocusID | Active ingredientB | Line in Table B | Gene Name/ LocusID | Active ingredientB |
|---|---|---|---|---|---|
| 42 | EST512 | cypermethrin | 187 | | Metiram |
| 42 | EST512 | alpha-cypermethrin | 187 | | Dithianon |
| 42 | EST512 | deltamethrin | 187 | | Chlorothalonil |
| 42 | EST512 | lambda-cyhalothrin | 187 | | ThiophanateMethyl |
| 42 | EST512 | tefluthrin | 187 | | Cymoxanil |
| 42 | EST512 | flufenoxuron | 187 | | Metrafenone |
| 42 | EST512 | teflubenzuron | 187 | | Acetochlor |
| 42 | EST512 | spirotetramat; | 187 | | Dimethenamid |
| 42 | EST512 | clothianidin | 187 | | Metolachlor |
| 42 | EST512 | imidacloprid | 187 | | Metazachlor |
| 42 | EST512 | thiamethoxam | 187 | | Glyphosate |
| 42 | EST512 | endosulfan | 187 | | Glufosinate |
| 42 | EST512 | fipronil | 187 | | Sulfosate |
| 42 | EST512 | abamectin | 187 | | Fenoxaprop |
| 42 | EST512 | spinosad | 187 | | Paraquat |
| 42 | EST512 | spinetoram | 187 | | Cycloxydim |
| 42 | EST512 | hydramethylnon; | 187 | | Profoxydim |
| 42 | EST512 | chlorfenapyr; | 187 | | Sethoxydim |
| 42 | EST512 | indoxacarb | 187 | | Tepraloxydim |
| 42 | EST512 | metaflumizone | 187 | | Pendimethalin |
| 42 | EST512 | flubendiamide | 187 | | Acifluorfen |
| 42 | EST512 | chlorantraniliprole | 187 | | Imazamethabenz |
| 42 | EST512 | cyazypyr(HGW86) | 187 | | Imazamox |
| 42 | EST512 | Azoxystrobin | 187 | | Imazapic |
| 42 | EST512 | Dimoxystrobin | 187 | | Imazapyr |
| 42 | EST512 | Kresoxim-methyl | 187 | | Imazaquin |
| 42 | EST512 | Orysastrobin | 187 | | Imazethapyr |
| 42 | EST512 | Pyraclostrobin | 187 | | 2,4-D |
| 42 | EST512 | Trifloxystrobin | 187 | | Chloridazon |
| 42 | EST512 | Bixafen | 187 | | Picloram |
| 42 | EST512 | Boscalid | 187 | | Picolinafen |
| 42 | EST512 | Isopyrazam | 187 | | Cyclosulfamuron |
| 42 | EST512 | Metalaxyl | 187 | | Triflusulfuron |
| 42 | EST512 | Penthiopyrad | 187 | | Atrazine |

(continued)

| Line in Table B | Gene Name/ LocusID | Active ingredientB | Line in Table B | Gene Name/ LocusID | Active ingredientB |
|---|---|---|---|---|---|
| 42 | EST512 | 3-Difluoromethyl-1-methyl-1 H-pyrazole-4-carboxylic acid(2',4',5'-trifluorobiphenyl-2-yl)-amide | 187 | | Pyroxasulfone |
| 42 | EST512 | Dimethomorph | 187 | | Bentazone |
| 42 | EST512 | Difenoconazole | 187 | | Cinidon-ethly |
| 42 | EST512 | Epoxiconazole | 187 | | Cinmethylin |
| 42 | EST512 | Fluquinconazole | 187 | | Dicamba |
| 42 | EST512 | Metconazol | 187 | | Diflufenzopyr |
| 42 | EST512 | Propiconazole | 187 | | Quinclorac |
| 42 | EST512 | Prothioconazole | 187 | | Quinmerac |
| 42 | EST512 | Tebuconazole | 187 | | Mesotrione |
| 42 | EST512 | Triticonazole | 187 | | Saflufenacil |
| 42 | EST512 | Prochloraz | 187 | | Topramezone; |
| 42 | EST512 | Carbendazim | 188 | | acephate |
| 42 | EST512 | Cyprodinil | 188 | | chlorpyrifos |
| 42 | EST512 | Pyrimethanil | 188 | | dimethoate |
| 42 | EST512 | Fenpropimorph | 188 | | methamidophos |
| 42 | EST512 | Tridemorph | 188 | | terbufos |
| 42 | EST512 | Iprodione | 188 | | aldicarb |
| 42 | EST512 | 5-ethyl-6-octyl-[1,2,4] triazolo [1,5-a]pyrimidine-7-ylamine | 188 | | carbofuran |
| 42 | EST512 | Mancozeb | 188 | | bifenthrin |
| 42 | EST512 | Maneb | 188 | | cypermethrin |
| 42 | EST512 | Metiram | 188 | | alpha-cypermethrin |
| 42 | EST512 | Dithianon | 188 | | deltamethrin |
| 42 | EST512 | Chlorothalonil | 188 | | lambda-cyhalothrin |
| 42 | EST512 | ThiophanateMethyl | 188 | | tefluthrin |
| 42 | EST512 | Cymoxanil | 188 | | flufenoxuron |
| 42 | EST512 | Metrafenone | 188 | | teflubenzuron |
| 42 | EST512 | Acetochlor | 188 | | spirotetramat; |
| 42 | EST512 | Dimethenamid | 188 | | clothianidin |
| 42 | EST512 | Metolachlor | 188 | | imidacloprid |
| 42 | EST512 | Metazachlor | 188 | | thiamethoxam |
| 42 | EST512 | Glyphosate | 188 | | endosulfan |

(continued)

| Line in Table B | Gene Name/ LocusID | Active ingredientB | Line in Table B | Gene Name/ LocusID | Active ingredientB |
|---|---|---|---|---|---|
| 42 | EST512 | Glufosinate | 188 | | fipronil |
| 42 | EST512 | Sulfosate | 188 | | abamectin |
| 42 | EST512 | Fenoxaprop | 188 | | spinosad |
| 42 | EST512 | Paraquat | 188 | | spinetoram |
| 42 | EST512 | Cycloxydim | 188 | | hydramethylnon; |
| 42 | EST512 | Profoxydim | 188 | | chlorfenapyr; |
| 42 | EST512 | Sethoxydim | 188 | | indoxacarb |
| 42 | EST512 | Tepraloxydim | 188 | | metaflumizone |
| 42 | EST512 | Pendimethalin | 188 | | flubendiamide |
| 42 | EST512 | Acifluorfen | 188 | | chlorantraniliprole |
| 42 | EST512 | Imazamethabenz | 188 | | cyazypyr(HGW86) |
| 42 | EST512 | Imazamox | 188 | | Azoxystrobin |
| 42 | EST512 | Imazapic | 188 | | Dimoxystrobin |
| 42 | EST512 | Imazapyr | 188 | | Kresoxim-methyl |
| 42 | EST512 | Imazaquin | 188 | | Orysastrobin |
| 42 | EST512 | Imazethapyr | 188 | | Pyraclostrobin |
| 42 | EST512 | 2,4-D | 188 | | Trifloxystrobin |
| 42 | EST512 | Chloridazon | 188 | | Bixafen |
| 42 | EST512 | Picloram | 188 | | Boscalid |
| 42 | EST512 | Picolinafen | 188 | | Isopyrazam |
| 42 | EST512 | Cyclosulfamuron | 188 | | Metalaxyl |
| 42 | EST512 | Triflusulfuron | 188 | | Penthiopyrad |
| 42 | EST512 | Atrazine | 188 | | 3-Difluoromethyl-1-methyl-1H-pyrazole-4-carboxylic acid(2',4',5'-trifluorobiphenyl-2-yl)-amide |
| 42 | EST512 | Pyroxasulfone | 188 | | Dimethomorph |
| 42 | EST512 | Bentazone | 188 | | Difenoconazole |
| 42 | EST512 | Cinidon-ethly | 188 | | Epoxiconazole |
| 42 | EST512 | Cinmethylin | 188 | | Fluquinconazole |
| 42 | EST512 | Dicamba | 188 | | Metconazol |
| 42 | EST512 | Diflufenzopyr | 188 | | Propiconazole |
| 42 | EST512 | Quinclorac | 188 | | Prothioconazole |
| 42 | EST512 | Quinmerac | 188 | | Tebuconazole |
| 42 | EST512 | Mesotrione | 188 | | Triticonazole |
| 42 | EST512 | Saflufenacil | 188 | | Prochloraz |

(continued)

| Line in Table B | Gene Name/ LocusID | Active ingredientB | Line in Table B | Gene Name/ LocusID | Active ingredientB |
|---|---|---|---|---|---|
| 42 | EST512 | Topramezone; | 188 | | Carbendazim |
| 43 | PpVTP_EST 513 | acephate | 188 | | Cyprodinil |
| 43 | PpVTP_EST 513 | chlorpyrifos | 188 | | Pyrimethanil |
| 43 | PpVTP_EST 513 | dimethoate | 188 | | Fenpropimorph |
| 43 | PpVTP_EST 513 | methamidophos | 188 | | Tridemorph |
| 43 | PpVTP_EST 513 | terbufos | 188 | | Iprodione |
| 43 | PpVTP_EST 513 | aldicarb | 188 | | 5-ethyl-6-octyl-[1,2,4] triazolo [1,5-a]pyrimidine-7-ylamine |
| 43 | PpVTP_EST 513 | carbofuran | 188 | | Mancozeb |
| 43 | PpVTP_EST 513 | bifenthrin | 188 | | Maneb |
| 43 | PpVTP_EST 513 | cypermethrin | 188 | | Metiram |
| 43 | PpVTP_EST 513 | alpha-cypermethrin | 188 | | Dithianon |
| 43 | PpVTP_EST 513 | deltamethrin | 188 | | Chlorothalonil |
| 43 | PpVTP_EST 513 | lambda-cyhalothrin | 188 | | ThiophanateMethyl |
| 43 | PpVTP_EST 513 | tefluthrin | 188 | | Cymoxanil |
| 43 | PpVTP_EST 513 | flufenoxuron | 188 | | Metrafenone |
| 43 | PpVTP_EST 513 | teflubenzuron | 188 | | Acetochlor |
| 43 | PpVTP_EST 513 | spirotetramat; | 188 | | Dimethenamid |
| 43 | PpVTP_EST 513 | clothianidin | 188 | | Metolachlor |
| 43 | PpVTP_EST 513 | imidacloprid | 188 | | Metazachlor |
| 43 | PpVTP_EST 513 | thiamethoxam | 188 | | Glyphosate |
| 43 | PpVTP_EST 513 | endosulfan | 188 | | Glufosinate |

(continued)

| Line in Table B | Gene Name/ LocusID | Active ingredientB | Line in Table B | Gene Name/ LocusID | Active ingredientB |
|---|---|---|---|---|---|
| 43 | PpVTP_EST 513 | fipronil | 188 | | Sulfosate |
| 43 | PpVTP_EST 513 | abamectin | 188 | | Fenoxaprop |
| 43 | PpVTP_EST 513 | spinosad | 188 | | Paraquat |
| 43 | PpVTP_EST 513 | spinetoram | 188 | | Cycloxydim |
| 43 | PpVTP_EST 513 | hydramethylnon; | 188 | | Profoxydim |
| 43 | PpVTP_EST 513 | chlorfenapyr; | 188 | | Sethoxydim |
| 43 | PpVTP_EST 513 | indoxacarb | 188 | | Tepraloxydim |
| 43 | PpVTP_EST 513 | metaflumizone | 188 | | Pendimethalin |
| 43 | PpVTP_EST 513 | flubendiamide | 188 | | Acifluorfen |
| 43 | PpVTP_EST 513 | chlorantraniliprole | 188 | | Imazamethabenz |
| 43 | PpVTP_EST 513 | cyazypyr(HGW86) | 188 | | Imazamox |
| 43 | PpVTP_EST 513 | Azoxystrobin | 188 | | Imazapic |
| 43 | PpVTP_EST 513 | Dimoxystrobin | 188 | | Imazapyr |
| 43 | PpVTP_EST 513 | Kresoxim-methyl | 188 | | Imazaquin |
| 43 | PpVTP_EST 513 | Orysastrobin | 188 | | Imazethapyr |
| 43 | PpVTP_EST 513 | Pyraclostrobin | 188 | | 2,4-D |
| 43 | PpVTP_EST 513 | Trifloxystrobin | 188 | | Chloridazon |
| 43 | PpVTP_EST 513 | Bixafen | 188 | | Picloram |
| 43 | PpVTP_EST 513 | Boscalid | 188 | | Picolinafen |
| 43 | PpVTP_EST 513 | Isopyrazam | 188 | | Cyclosulfamuron |
| 43 | PpVTP_EST 513 | Metalaxyl | 188 | | Triflusulfuron |
| 43 | PpVTP_EST 513 | Penthiopyrad | 188 | | Atrazine |

(continued)

| Line in Table B | Gene Name/ LocusID | Active ingredientB | Line in Table B | Gene Name/ LocusID | Active ingredientB |
|---|---|---|---|---|---|
| 43 | PpVTP_EST 513 | 3-Difluoromethyl-1-methyl-1 H-pyrazole-4-carboxylic acid(2',4',5'-trifluorobiphenyl-2-yl)-amide | 188 | | Pyroxasulfone |
| 43 | PpVTP_EST 513 | Dimethomorph | 188 | | Bentazone |
| 43 | PpVTP_EST 513 | Difenoconazole | 188 | | Cinidon-ethly |
| 43 | PpVTP_EST 513 | Epoxiconazole | 188 | | Cinmethylin |
| 43 | PpVTP_EST 513 | Fluquinconazole | 188 | | Dicamba |
| 43 | PpVTP_EST 513 | Metconazol | 188 | | Diflufenzopyr |
| 43 | PpVTP_EST 513 | Propiconazole | 188 | | Quinclorac |
| 43 | PpVTP_EST 513 | Prothioconazole | 188 | | Quinmerac |
| 43 | PpVTP_EST 513 | Tebuconazole | 188 | | Mesotrione |
| 43 | PpVTP_EST 513 | Triticonazole | 188 | | Saflufenacil |
| 43 | PpVTP_EST 513 | Prochloraz | 188 | | Topramezone; |
| 43 | PpVTP _EST 513 | Carbendazim | 189 | _CDS3298 | acephate |
| 43 | PpVTP _EST 513 | Cyprodinil | 189 | _CDS3298 | chlorpyrifos |
| 43 | PpVTP_EST 513 | Pyrimethanil | 189 | _CDS3298 | dimethoate |
| 43 | PpVTP_EST 513 | Fenpropimorph | 189 | _CDS3298 | methamidophos |
| 43 | PpVTP_EST 513 | Tridemorph | 189 | _CDS3298 | terbufos |
| 43 | PpVTP_EST 513 | Iprodione | 189 | _CDS3298 | aldicarb |
| 43 | PpVTP_EST 513 | 5-ethyl-6-octyl-[1,2,4] triazolo [1,5-a]pyrimidine-7-ylamine | 189 | _CDS3298 | carbofuran |
| 43 | PpVTP_EST 513 | Mancozeb | 189 | _CDS3298 | bifenthrin |

(continued)

| Line in Table B | Gene Name/ LocusID | Active ingredientB | Line in Table B | Gene Name/ LocusID | Active ingredientB |
|---|---|---|---|---|---|
| 43 | PpVTP_EST 513 | Maneb | 189 | _CDS3298 | cypermethrin |
| 43 | PpVTP_EST 513 | Metiram | 189 | _CDS3298 | alpha-cypermethrin |
| 43 | PpVTP_EST 513 | Dithianon | 189 | _CDS3298 | deltamethrin |
| 43 | PpVTP _EST 513 | Chlorothalonil | 189 | _CDS3298 | lambda-cyhalothrin |
| 43 | PpVTP_EST' 513 | ThiophanateMethyl | 189 | _CDS3298 | tefluthrin |
| 43 | PpVTP _EST 513 | Cymoxanil | 189 | _CDS3298 | flufenoxuron |
| 43 | PpVTP_EST 513 | Metrafenone | 189 | _CDS3298 | teflubenzuron |
| 43 | PpVTP_EST, 513 | Acetochlor | 189 | _CDS3298 | spirotetramat; |
| 43 | PpVTP_EST 513 | Dimethenamid | 189 | _CDS3298 | clothianidin |
| 43 | PpVTP_EST 513 | Metolachlor | 189 | _CDS3298 | imidacloprid |
| 43 | PpVTP_EST 513 | Metazachlor | 189 | _CDS3298 | thiamethoxam |
| 43 | PpVTP _EST 513 | Glyphosate | 189 | _CDS3298 | endosulfan |
| 43 | PpVTP_EST 513 | Glufosinate | 189 | _CDS3298 | fipronil |
| 43 | PpVTP_EST: 513 | Sulfosate | 189 | _CDS3298 | abamectin |
| 43 | PpVTP_EST 513 | Fenoxaprop | 189 | _CDS3298 | spinosad |
| 43 | PpVTP_EST 513 | Paraquat | 189 | _CDS3298 | spinetoram |
| 43 | PpVTP _EST 513 | Cycloxydim | 189 | _CDS3298 | hydramethylnon; |
| 43 | PpVTP_EST 513 | Profoxydim | 189 | _CDS3298 | chlorfenapyr; |
| 43 | PpVTP_EST: 513 | Sethoxydim | 189 | _CDS3298 | indoxacarb |
| 43 | PpVTP_EST' 513 | Tepraloxydim | 189 | _CDS3298 | metaflumizone |
| 43 | PpVTP_EST 513 | Pendimethalin | 189 | _CDS3298 | flubendiamide |
| 43 | PpVTP_EST 513 | Acifluorfen | 189 | _CDS3298 | chlorantraniliprole |

(continued)

| Line in Table B | Gene Name/ LocusID | Active ingredientB | Line in Table B | Gene Name/ LocusID | Active ingredientB |
|---|---|---|---|---|---|
| 43 | PpVTP_EST 513 | Imazamethabenz | 189 | _CDS3298 | cyazypyr(HGW86) |
| 43 | PpVTP_EST 513 | Imazamox | 189 | _CDS3298 | Azoxystrobin |
| 43 | PpVTP_EST 513 | Imazapic | 189 | _CDS3298 | Dimoxystrobin |
| 43 | PpVTP_EST 513 | Imazapyr | 189 | _CDS3298 | Kresoxim-methyl |
| 43 | PpVTP_EST 513 | Imazaquin | 189 | _CDS3298 | Orysastrobin |
| 43 | PpVTP_EST 513 | Imazethapyr | 189 | _CDS3298 | Pyraclostrobin |
| 43 | PpVTP_EST: 513 | 2,4-D | 189 | _CDS3298 | Trifloxystrobin |
| 43 | PpVTP_EST 513 | Chloridazon | 189 | _CDS3298 | Bixafen |
| 43 | PpVTP_EST 513 | Picloram | 189 | _CDS3298 | Boscalid |
| 43 | PpVTP_EST 513 | Picolinafen | 189 | _CDS3298 | Isopyrazam |
| 43 | PpVTP _EST 513 | Cyclosulfamuron | 189 | _CDS3298 | Metalaxyl |
| 43 | PpVTP_EST' 513 | Triflusulfuron | 189 | _CDS3298 | Penthiopyrad |
| 43 | PpVTP_EST, 513 | Atrazine | 189 | _CDS3298 | 3-Difluoromethyl-1-methyl-1H-pyrazole-4-carboxylic acid(2',4',5'-trifluorobiphenyl-2-yl)-amide |
| 43 | PpVTP_EST 513 | Pyroxasulfone | 189 | _CDS3298 | Dimethomorph |
| 43 | PpVTP_EST 513 | Bentazone | 189 | _CDS3298 | Difenoconazole |
| 43 | PpVTP _EST 513 | Cinidon-ethly | 189 | _CDS3298 | Epoxiconazole |
| 43 | PpVTP _EST 513 | Cinmethylin | 189 | _CDS3298 | Fluquinconazole |
| 43 | PpVTP_EST 513 | Dicamba | 189 | _CDS3298 | Metconazol |
| 43 | PpVTP_EST 513 | Diflufenzopyr | 189 | _CDS3298 | Propiconazole |
| 43 | PpVTP_EST 513 | Quinclorac | 189 | _CDS3298 | Prothioconazole |

(continued)

| Line in Table B | Gene Name/ LocusID | Active ingredientB | Line in Table B | Gene Name/ LocusID | Active ingredientB |
|---|---|---|---|---|---|
| 43 | PpVTP_EST 513 | Quinmerac | 189 | _CDS3298 | Tebuconazole |
| 43 | PpVTP_EST 513 | Mesotrione | 189 | _CDS3298 | Triticonazole |
| 43 | PpVTP_EST 513 | Saflufenacil | 189 | _CDS3298 | Prochloraz |
| 43 | PpVTP_EST' 513 | Topramezone; | 189 | _CDS3298 | Carbendazim |
| 91 | YKL150w | acephate | 189 | _CDS3298 | Cyprodinil |
| 91 | YKL150w | chlorpyrifos | 189 | _CDS3298 | Pyrimethanil |
| 91 | YKL150w | dimethoate | 189 | _CDS3298 | Fenpropimorph |
| 91 | YKL150w | methamidophos | 189 | _CDS3298 | Tridemorph |
| 91 | YKL150w | terbufos | 189 | _CDS3298 | Iprodione |
| 91 | YKL150w | aldicarb | 189 | _CDS3298 | 5-ethyl-6-octyl-[1,2,4] triazolo [1,5-a]pyrimidine-7-ylamine |
| 91 | YKL150w | carbofuran | 189 | _CDS3298 | Mancozeb |
| 91 | YKL150w | bifenthrin | 189 | _CDS3298 | Maneb |
| 91 | YKL150w | cypermethrin | 189 | _CDS3298 | Metiram |
| 91 | YKL150w | alpha-cypermethrin | 189 | _CDS3298 | Dithianon |
| 91 | YKL150w | deltamethrin | 189 | _CDS3298 | Chlorothalonil |
| 91 | YKL150w | lambda-cyhalothrin | 189 | _CDS3298 | ThiophanateMethyl |
| 91 | YKL150w | tefluthrin | 189 | _CDS3298 | Cymoxanil |
| 91 | YKL150w | flufenoxuron | 189 | _CDS3298 | Metrafenone |
| 91 | YKL150w | teflubenzuron | 189 | _CDS3298 | Acetochlor |
| 91 | YKL150w | spirotetramat; | 189 | _CDS3298 | Dimethenamid |
| 91 | YKL150w | clothianidin | 189 | _CDS3298 | Metolachlor |
| 91 | YKL150w | imidacloprid | 189 | _CDS3298 | Metazachlor |
| 91 | YKL150w | thiamethoxam | 189 | _CDS3298 | Glyphosate |
| 91 | YKL150w | endosulfan | 189 | _CDS3298 | Glufosinate |
| 91 | YKL150w | fipronil | 189 | _CDS3298 | Sulfosate |
| 91 | YKL150w | abamectin | 189 | _CDS3298 | Fenoxaprop |
| 91 | YKL150w | spinosad | 189 | _CDS3298 | Paraquat |
| 91 | YKL150w | spinetoram | 189 | _CDS3298 | Cycloxydim |
| 91 | YKL150w | hydramethylnon; | 189 | _CDS3298 | Profoxydim |
| 91 | YKL150w | chlorfenapyr; | 189 | _CDS3298 | Sethoxydim |
| 91 | YKL150w | indoxacarb | 189 | _CDS3298 | Tepraloxydim |
| 91 | YKL150w | metaflumizone | 189 | _CDS3298 | Pendimethalin |

(continued)

| Line in Table B | Gene Name/ LocusID | Active ingredientB | Line in Table B | Gene Name/ LocusID | Active ingredientB |
|---|---|---|---|---|---|
| 91 | YKL150w | flubendiamide | 189 | _CDS3298 | Acifluorfen |
| 91 | YKL150w | chlorantraniliprole | 189 | _CDS3298 | Imazamethabenz |
| 91 | YKL150w | cyazypyr(HGW86) | 189 | _CDS3298 | Imazamox |
| 91 | YKL150w | Azoxystrobin | 189 | _CDS3298 | Imazapic |
| 91 | YKL150w | Dimoxystrobin | 189 | _CDS3298 | Imazapyr |
| 91 | YKL150w | Kresoxim-methyl | 189 | _CDS3298 | Imazaquin |
| 91 | YKL150w | Orysastrobin | 189 | _CDS3298 | Imazethapyr |
| 91 | YKL150w | Pyraclostrobin | 189 | _CDS3298 | 2,4-D |
| 91 | YKL150w | Trifloxystrobin | 189 | _CDS3298 | Chloridazon |
| 91 | YKL150w | Bixafen | 189 | _CDS3298 | Picloram |
| 91 | YKL150w | Boscalid | 189 | _CDS3298 | Picolinafen |
| 91 | YKL150w | Isopyrazam | 189 | _CDS3298 | Cyclosulfamuron |
| 91 | YKL150w | Metalaxyl | 189 | _CDS3298 | Triflusulfuron |
| 91 | YKL150w | Penthiopyrad | 189 | _CDS3298 | Atrazine |
| 91 | YKL150w | 3-Difluoromethyl-1-methyl-1 H-pyrazole-4-carboxylic acid (2',4',5'-trifluorobiphenyl-2-yl)-amide | 189 | _CDS3298 | Pyroxasulfone |
| 91 | YKL150w | Dimethomorph | 189 | _CDS3298 | Bentazone |
| 91 | YKL150w | Difenoconazole | 189 | _CDS3298 | Cinidon-ethly |
| 91 | YKL150w | Epoxiconazole | 189 | _CDS3298 | Cinmethylin |
| 91 | YKL150w | Fluquinconazole | 189 | _CDS3298 | Dicamba |
| 91 | YKL150w | Metconazol | 189 | _CDS3298 | Diflufenzopyr |
| 91 | YKL150w | Propiconazole | 189 | _CDS3298 | Quinclorac |
| 91 | YKL150w | Prothioconazole | 189 | _CDS3298 | Quinmerac |
| 91 | YKL150w | Tebuconazole | 189 | _CDS3298 | Mesotrione |
| 91 | YKL150w | Triticonazole | 189 | _CDS3298 | Saflufenacil |
| 91 | YKL150w | Prochloraz | 189 | _CDS3298 | Topramezone; |
| 91 | YKL150w | Carbendazim | 190 | | acephate |
| 91 | YKL150w | Cyprodinil | 190 | | chlorpyrifos |
| 91 | YKL150w | Pyrimethanil | 190 | | dimethoate |
| 91 | YKL150w | Fenpropimorph | 190 | | methamidophos |
| 91 | YKL150w | Tridemorph | 190 | | terbufos |
| 91 | YKL150w | Iprodione | 190 | | aldicarb |

(continued)

| Line in Table B | Gene Name/ LocusID | Active ingredientB | Line in Table B | Gene Name/ LocusID | Active ingredientB |
|---|---|---|---|---|---|
| 91 | YKL150w | 5-ethyl-6-octyl-[1,2,4] triazolo [1,5-a]pyrimidine-7-ylamine | 190 | | carbofuran |
| 91 | YKL150w | Mancozeb | 190 | | bifenthrin |
| 91 | YKL150w | Maneb | 190 | | cypermethrin |
| 91 | YKL150w | Metiram | 190 | | alpha-cypermethrin |
| 91 | YKL150w | Dithianon | 190 | | deltamethrin |
| 91 | YKL150w | Chlorothalonil | 190 | | lambda-cyhalothrin |
| 91 | YKL150w | ThiophanateMethyl | 190 | | tefluthrin |
| 91 | YKL150w | Cymoxanil | 190 | | flufenoxuron |
| 91 | YKL150w | Metrafenone | 190 | | teflubenzuron |
| 91 | YKL150w | Acetochlor | 190 | | spirotetramat; |
| 91 | YKL150w | Dimethenamid | 190 | | clothianidin |
| 91 | YKL150w | Metolachlor | 190 | | imidacloprid |
| 91 | YKL150w | Metazachlor | 190 | | thiamethoxam |
| 91 | YKL150w | Glyphosate | 190 | | endosulfan |
| 91 | YKL150w | Glufosinate | 190 | | fipronil |
| 91 | YKL150w | Sulfosate | 190 | | abamectin |
| 91 | YKL150w | Fenoxaprop | 190 | | spinosad |
| 91 | YKL150w | Paraquat | 190 | | spinetoram |
| 91 | YKL150w | Cycloxydim | 190 | | hydramethylnon; |
| 91 | YKL150w | Profoxydim | 190 | | chlorfenapyr; |
| 91 | YKL150w | Sethoxydim | 190 | | indoxacarb |
| 91 | YKL150w | Tepraloxydim | 190 | | metaflumizone |
| 91 | YKL150w | Pendimethalin | 190 | | flubendiamide |
| 91 | YKL150w | Acifluorfen | 190 | | chlorantraniliprole |
| 91 | YKL150w | Imazamethabenz | 190 | | cyazypyr(HGW86) |
| 91 | YKL150w | Imazamox | 190 | | Azoxystrobin |
| 91 | YKL150w | Imazapic | 190 | | Dimoxystrobin |
| 91 | YKL150w | Imazapyr | 190 | | Kresoxim-methyl |
| 91 | YKL150w | Imazaquin | 190 | | Orysastrobin |
| 91 | YKL150w | Imazethapyr | 190 | | Pyraclostrobin |
| 91 | YKL150w | 2,4-D | 190 | | Trifloxystrobin |
| 91 | YKL150w | Chloridazon | 190 | | Bixafen |
| 91 | YKL150w | Picloram | 190 | | Boscalid |
| 91 | YKL150w | Picolinafen | 190 | | Isopyrazam |
| 91 | YKL150w | Cyclosulfamuron | 190 | | Metalaxyl |

(continued)

| Line in Table B | Gene Name/ LocusID | Active ingredientB | Line in Table B | Gene Name/ LocusID | Active ingredientB |
|---|---|---|---|---|---|
| 91 | YKL150w | Triflusulfuron | 190 | | Penthiopyrad |
| 91 | YKL150w | Atrazine | 190 | | 3-Difluoromethyl-1-methyl-1H-pyrazole-4-carboxylic acid(2',4',5'-trifluorobiphenyl-2-yl)-amide |
| 91 | YKL150w | Pyroxasulfone | 190 | | Dimethomorph |
| 91 | YKL150w | Bentazone | 190 | | Difenoconazole |
| 91 | YKL150w | Cinidon-ethly | 190 | | Epoxiconazole |
| 91 | YKL150w | Cinmethylin | 190 | | Fluquinconazole |
| 91 | YKL150w | Dicamba | 190 | | Metconazol |
| 91 | YKL150w | Diflufenzopyr | 190 | | Propiconazole |
| 91 | YKL150w | Quinclorac | 190 | | Prothioconazole |
| 91 | YKL150w | Quinmerac | 190 | | Tebuconazole |
| 91 | YKL150w | Mesotrione | 190 | | Triticonazole |
| 91 | YKL150w | Saflufenacil | 190 | | Prochloraz |
| 91 | YKL150w | Topramezone; | 190 | | Carbendazim |
| 105 | YNL079C | acephate | 190 | | Cyprodinil |
| 105 | YNL079C | chlorpyrifos | 190 | | Pyrimethanil |
| 105 | YNL079C | dimethoate | 190 | | Fenpropimorph |
| 105 | YNL079C | methamidophos | 190 | | Tridemorph |
| 105 | YNL079C | terbufos | 190 | | Iprodione |
| 105 | YNL079C | aldicarb | 190 | | 5-ethyl-6-octyl-[1,2,4] triazolo [1,5-a]pyrimidine-7-ylamine |
| 105 | YNL079C | carbofuran | 190 | | Mancozeb |
| 105 | YNL079C | bifenthrin | 190 | | Maneb |
| 105 | YNL079C | cypermethrin | 190 | | Metiram |
| 105 | YNL079C | alpha-cypermethrin | 190 | | Dithianon |
| 105 | YNL079C | deltamethrin | 190 | | Chlorothalonil |
| 105 | YNL079C | lambda-cyhalothrin | 190 | | ThiophanateMethyl |
| 105 | YNL079C | tefluthrin | 190 | | Cymoxanil |
| 105 | YNL079C | flufenoxuron | 190 | | Metrafenone |
| 105 | YNL079C | teflubenzuron | 190 | | Acetochlor |
| 105 | YNL079C | spirotetramat; | 190 | | Dimethenamid |
| 105 | YNL079C | clothianidin | 190 | | Metolachlor |
| 105 | YNL079C | imidacloprid | 190 | | Metazachlor |

(continued)

| Line in Table B | Gene Name/ LocusID | Active ingredientB | Line in Table B | Gene Name/ LocusID | Active ingredientB |
|---|---|---|---|---|---|
| 105 | YNL079C | thiamethoxam | 190 | | Glyphosate |
| 105 | YNL079C | endosulfan | 190 | | Glufosinate |
| 105 | YNL079C | fipronil | 190 | | Sulfosate |
| 105<br>105 | YNL079C<br>YNL079C | abamectin<br>spinosad | 190<br>190 | | Fenoxaprop<br>Paraquat |
| 105 | YNL079C | spinetoram | 190 | | Cycloxydim |
| 105 | YNL079C | hydramethylnon; | 190 | | Profoxydim |
| 105 | YNL079C | chlorfenapyr; | 190 | | Sethoxydim |
| 105 | YNL079C | indoxacarb | 190 | | Tepraloxydim |
| 105 | YNL079C | metaflumizone | 190 | | Pendimethalin |
| 105 | YNL079C | flubendiamide | 190 | | Acifluorfen |
| 105 | YNL079C | chlorantraniliprole | 190 | | Imazamethabenz |
| 105 | YNL079C | cyazypyr(HGW86) | 190 | | Imazamox |
| 105 | YNL079C | Azoxystrobin | 190 | | Imazapic |
| 105 | YNL079C | Dimoxystrobin | 190 | | Imazapyr |
| 105 | YNL079C | Kresoxim-methyl | 190 | | Imazaquin |
| 105 | YNL079C | Orysastrobin | 190 | | Imazethapyr |
| 105 | YNL079C | Pyraclostrobin | 190 | | 2,4-D |
| 105 | YNL079C | Trifloxystrobin | 190 | | Chloridazon |
| 105 | YNL079C | Bixafen | 190 | | Picloram |
| 105 | YNL079C | Boscalid | 190 | | Picolinafen |
| 105 | YNL079C | Isopyrazam | 190 | | Cyclosulfamuron |
| 105 | YNL079C | Metalaxyl | 190 | | Triflusulfuron |
| 105 | YNL079C | Penthiopyrad | 190 | | Atrazine |
| 105 | YNL079C | 3-Difluoromethyl-1-methyl-1H-pyrazole-4-carboxylic acid(2',4',5'-trifluorobiphenyl-2-yl)-amide | 190 | | Pyroxasulfone |
| 105 | YNL079C | Dimethomorph | 190 | | Bentazone |
| 105 | YNL079C | Difenoconazole | 190 | | Cinidon-ethly |
| 105 | YNL079C | Epoxiconazole | 190 | | Cinmethylin |
| 105 | YNL079C | Fluquinconazole | 190 | | Dicamba |
| 105 | YNL079C | Metconazol | 190 | | Diflufenzopyr |
| 105 | YNL079C | Propiconazole | 190 | | Quinclorac |
| 105 | YNL079C | Prothioconazole | 190 | | Quinmerac |
| 105 | YNL079C | Tebuconazole | 190 | | Mesotrione |

(continued)

| Line in Table B | Gene Name/ LocusID | Active ingredientB | Line in Table B | Gene Name/ LocusID | Active ingredientB |
|---|---|---|---|---|---|
| 105 | YNL079C | Triticonazole | 190 | | Saflufenacil |
| 105 | YNL079C | Prochloraz | 190 | | Topramezone; |
| 105 | YNL079C | Carbendazim | 194 | YIL172C | acephate |
| 105 | YNL079C | Cyprodinil | 194 | YIL172C | chlorpyrifos |
| 105 | YNL079C | Pyrimethanil | 194 | YIL172C | dimethoate |
| 105 | YNL079C | Fenpropimorph | 194 | YIL172C | methamidophos |
| 105 | YNL079C | Tridemorph | 194 | YIL172C | terbufos |
| 105 | YNL079C | Iprodione | 194 | YIL172C | aldicarb |
| 105 | YNL079C | 5-ethyl-6-octyl-[1,2,4] triazolo [1,5-a]pyrimidine-7-ylamine | 194 | YIL172C | carbofuran |
| 105 | YNL079C | Mancozeb | 194 | YIL172C | bifenthrin |
| 105 | YNL079C | Maneb | 194 | YIL172C | cypermethrin |
| 105 | YNL079C | Metiram | 194 | YIL172C | alpha-cypermethrin |
| 105 | YNL079C | Dithianon | 194 | YIL172C | deltamethrin |
| 105 | YNL079C | Chlorothalonil | 194 | YIL172C | lambda-cyhalothrin |
| 105 | YNL079C | ThiophanateMethyl | 194 | YIL172C | tefluthrin |
| 105 | YNL079C | Cymoxanil | 194 | YIL172C | flufenoxuron |
| 105 | YNL079C | Metrafenone | 194 | YIL172C | teflubenzuron |
| 105 | YNL079C | Acetochlor | 194 | YIL172C | spirotetramat; |
| 105 | YNL079C | Dimethenamid | 194 | YIL172C | clothianidin |
| 105 | YNL079C | Metolachlor | 194 | YIL172C | imidacloprid |
| 105 | YNL079C | Metazachlor | 194 | YIL172C | thiamethoxam |
| 105 | YNL079C | Glyphosate | 194 | YIL172C | endosulfan |
| 105 | YNL079C | Glufosinate | 194 | YIL172C | fipronil |
| 105 | YNL079C | Sulfosate | 194 | YIL172C | abamectin |
| 105 | YNL079C | Fenoxaprop | 194 | YIL172C | spinosad |
| 105 | YNL079C | Paraquat | 194 | YIL172C | spinetoram |
| 105 | YNL079C | Cycloxydim | 194 | YIL172C | hydramethylnon; |
| 105 | YNL079C | Profoxydim | 194 | YIL172C | chlorfenapyr; |
| 105 | YNL079C | Sethoxydim | 194 | YIL172C | indoxacarb |
| 105 | YNL079C | Tepraloxydim | 194 | YIL172C | metaflumizone |
| 105 | YNL079C | Pendimethalin | 194 | YIL172C | flubendiamide |
| 105 | YNL079C | Acifluorfen | 194 | YIL172C | chlorantraniliprole |
| 105 | YNL079C | Imazamethabenz | 194 | YIL172C | cyazypyr(HGW86) |
| 105 | YNL079C | Imazamox | 194 | YIL172C | Azoxystrobin |
| 105 | YNL079C | Imazapic | 194 | YIL172C | Dimoxystrobin |

(continued)

| Line in Table B | Gene Name/ LocusID | Active ingredientB | Line in Table B | Gene Name/ LocusID | Active ingredientB |
|---|---|---|---|---|---|
| 105 | YNL079C | Imazapyr | 194 | YIL172C | Kresoxim-methyl |
| 105 | YNL079C | Imazaquin | 194 | YIL172C | Orysastrobin |
| 105 | YNL079C | Imazethapyr | 194 | YIL172C | Pyraclostrobin |
| 105 | YNL079C | 2,4-D | 194 | YIL172C | Trifloxystrobin |
| 105 | YNL079C | Chloridazon | 194 | YIL172C | Bixafen |
| 105 | YNL079C | Picloram | 194 | YIL172C | Boscalid |
| 105 | YNL079C | Picolinafen | 194 | YIL172C | Isopyrazam |
| 105 | YNL079C | Cyclosulfamuron | 194 | YIL172C | Metalaxyl |
| 105 | YNL079C | Triflusulfuron | 194 | YIL172C | Penthiopyrad |
| 105 | YNL079C | Atrazine | 194 | YIL172C | 3-Difluoromethyl-1-methyl-1H-pyrazole-4-carboxylic acid(2',4',5'-trifluorobiphenyl-2-yl)-amide |
| 105 | YNL079C | Pyroxasulfone | 194 | YIL172C | Dimethomorph |
| 105 | YNL079C | Bentazone | 194 | YIL172C | Difenoconazole |
| 105 | YNL079C | Cinidon-ethly | 194 | YIL172C | Epoxiconazole |
| 105 | YNL079C | Cinmethylin | 194 | YIL172C | Fluquinconazole |
| 105 | YNL079C | Dicamba | 194 | YIL172C | Metconazol |
| 105 | YNL079C | Diflufenzopyr | 194 | YIL172C | Propiconazole |
| 105 | YNL079C | Quinclorac | 194 | YIL172C | Prothioconazole |
| 105 | YNL079C | Quinmerac | 194 | YIL172C | Tebuconazole |
| 105 | YNL079C | Mesotrione | 194 | YIL172C | Triticonazole |
| 105 | YNL079C | Saflufenacil | 194 | YIL172C | Prochloraz |
| 105 | YNL079C | Topramezone; | 194 | YIL172C | Carbendazim |
| 107 | YMR100W | acephate | 194 | YIL172C | Cyprodinil |
| 107 | YMR100W | chlorpyrifos | 194 | YIL172C | Pyrimethanil |
| 107 | YMR100W | dimethoate | 194 | YIL172C | Fenpropimorph |
| 107 | YMR100W | methamidophos | 194 | YIL172C | Tridemorph |
| 107 | YMR100W | terbufos | 194 | YIL172C | Iprodione |
| 107 | YMR100W | aldicarb | 194 | YIL172C | 5-ethyl-6-octyl-[1,2,4] triazolo [1,5-a]pyrimidine-7-ylamine |
| 107 | YMR100W | carbofuran | 194 | YIL172C | Mancozeb |
| 107 | YMR100W | bifenthrin | 194 | YIL172C | Maneb |
| 107 | YMR100W | cypermethrin | 194 | YIL172C | Metiram |
| 107 | YMR100W | alpha-cypermethrin | 194 | YIL172C | Dithianon |

(continued)

| Line in Table B | Gene Name/ LocusID | Active ingredientB | Line in Table B | Gene Name/ LocusID | Active ingredientB |
|---|---|---|---|---|---|
| 107 | YMR100W | deltamethrin | 194 | YIL172C | Chlorothalonil |
| 107 | YMR100W | lambda-cyhalothrin | 194 | YIL172C | ThiophanateMethyl |
| 107 | YMR100W | tefluthrin | 194 | YIL172C | Cymoxanil |
| 107 | YMR100W | flufenoxuron | 194 | YIL172C | Metrafenone |
| 107 | YMR100W | teflubenzuron | 194 | YIL172C | Acetochlor |
| 107 | YMR100W | spirotetramat; | 194 | YIL172C | Dimethenamid |
| 107 | YMR100W | clothianidin | 194 | YIL172C | Metolachlor |
| 107 | YMR100W | imidacloprid | 194 | YIL172C | Metazachlor |
| 107 | YMR100W | thiamethoxam | 194 | YIL172C | Glyphosate |
| 107 | YMR100W | endosulfan | 194 | YIL172C | Glufosinate |
| 107 | YMR100W | fipronil | 194 | YIL172C | Sulfosate |
| 107 | YMR100W | abamectin | 194 | YIL172C | Fenoxaprop |
| 107 | YMR100W | spinosad | 194 | YIL172C | Paraquat |
| 107 | YMR100W | spinetoram | 194 | YIL172C | Cycloxydim |
| 107 | YMR100W | hydramethylnon; | 194 | YIL172C | Profoxydim |
| 107 | YMR100W | chlorfenapyr; | 194 | YIL172C | Sethoxydim |
| 107 | YMR100W | indoxacarb | 194 | YIL172C | Tepraloxydim |
| 107 | YMR100W | metaflumizone | 194 | YIL172C | Pendimethalin |
| 107 | YMR100W | flubendiamide | 194 | YIL172C | Acifluorfen |
| 107 | YMR100W | chlorantraniliprole | 194 | YIL172C | Imazamethabenz |
| 107 | YMR100W | cyazypyr(HGW86) | 194 | YIL172C | Imazamox |
| 107 | YMR100W | Azoxystrobin | 194 | YIL172C | Imazapic |
| 107 | YMR100W | Dimoxystrobin | 194 | YIL172C | Imazapyr |
| 107 | YMR100W | Kresoxim-methyl | 194 | YIL172C | Imazaquin |
| 107 | YMR100W | Orysastrobin | 194 | YIL172C | Imazethapyr |
| 107 | YMR100W | Pyraclostrobin | 194 | YIL172C | 2,4-D |
| 107 | YMR100W | Trifloxystrobin | 194 | YIL172C | Chloridazon |
| 107 | YMR100W | Bixafen | 194 | YIL172C | Picloram |
| 107 | YMR100W | Boscalid | 194 | YIL172C | Picolinafen |
| 107 | YMR100W | Isopyrazam | 194 | YIL172C | Cyclosulfamuron |
| 107 | YMR100W | Metalaxyl | 194 | YIL172C | Triflusulfuron |
| 107 | YMR100W | Penthiopyrad | 194 | YIL172C | Atrazine |
| 107 | YMR100W | 3-Difluoromethyl-1-methyl-1 H-pyrazole-4-carboxylic acid(2',4',5'-trifluorobiphenyl-2-yl)-amide | 194 | YIL172C | Pyroxasulfone |

(continued)

| Line in Table B | Gene Name/ LocusID | Active ingredientB | Line in Table B | Gene Name/ LocusID | Active ingredientB |
|---|---|---|---|---|---|
| 107 | YMR100W | Dimethomorph | 194 | YIL172C | Bentazone |
| 107 | YMR100W | Difenoconazole | 194 | YIL172C | Cinidon-ethly |
| 107 | YMR100W | Epoxiconazole | 194 | YIL172C | Cinmethylin |
| 107 | YMR100W | Fluquinconazole | 194 | YIL172C | Dicamba |
| 107 | YMR100W | Metconazol | 194 | YIL172C | Diflufenzopyr |
| 107 | YMR100W | Propiconazole | 194 | YIL172C | Quinclorac |
| 107 | YMR100W | Prothioconazole | 194 | YIL172C | Quinmerac |
| 107 | YMR100W | Tebuconazole | 194 | YIL172C | Mesotrione |
| 107 | YMR100W | Triticonazole | 194 | YIL172C | Saflufenacil |
| 107 | YMR100W | Prochloraz | 194 | YIL172C | Topramezone; |
| 107 | YMR100W | Carbendazim | 196 | YMR217W | acephate |
| 107 | YMR100W | Cyprodinil | 196 | YMR217W | chlorpyrifos |
| 107 | YMR100W | Pyrimethanil | 196 | YMR217W | dimethoate |
| 107 | YMR100W | Fenpropimorph | 196 | YMR217W | methamidophos |
| 107 | YMR100W | Tridemorph | 196 | YMR217W | terbufos |
| 107 | YMR100W | Iprodione | 196 | YMR217W | aldicarb |
| 107 | YMR100W | 5-ethyl-6-octyl-[1,2,4]triazolo [1,5-a]pyrimidine-7-ylamine | 196 | YMR217W | carbofuran |
| 107 | YMR100W | Mancozeb | 196 | YMR217W | bifenthrin |
| 107 | YMR100W | Maneb | 196 | YMR217W | cypermethrin |
| 107 | YMR100W | Metiram | 196 | YMR217W | alpha-cypermethrin |
| 107 | YMR100W | Dithianon | 196 | YMR217W | deltamethrin |
| 107 | YMR100W | Chlorothalonil | 196 | YMR217W | lambda-cyhalothrin |
| 107 | YMR100W | ThiophanateMethyl | 196 | YMR217W | tefluthrin |
| 107 | YMR100W | Cymoxanil | 196 | YMR217W | flufenoxuron |
| 107 | YMR100W | Metrafenone | 196 | YMR217W | teflubenzuron |
| 107 | YMR100W | Acetochlor | 196 | YMR217W | spirotetramat; |
| 107 | YMR100W | Dimethenamid | 196 | YMR217W | clothianidin |
| 107 | YMR100W | Metolachlor | 196 | YMR217W | imidacloprid |
| 107 | YMR100W | Metazachlor | 196 | YMR217W | thiamethoxam |
| 107 | YMR100W | Glyphosate | 196 | YMR217W | endosulfan |
| 107 | YMR100W | Glufosinate | 196 | YMR217W | fipronil |
| 107 | YMR100W | Sulfosate | 196 | YMR217W | abamectin |
| 107 | YMR100W | Fenoxaprop | 196 | YMR217W | spinosad |
| 107 | YMR100W | Paraquat | 196 | YMR217W | spinetoram |
| 107 | YMR100W | Cycloxydim | 196 | YMR217W | hydramethylnon; |

(continued)

| Line in Table B | Gene Name/ LocusID | Active ingredientB | Line in Table B | Gene Name/ LocusID | Active ingredientB |
|---|---|---|---|---|---|
| 107 | YMR100W | Profoxydim | 196 | YMR217W | chlorfenapyr; |
| 107 | YMR100W | Sethoxydim | 196 | YMR217W | indoxacarb |
| 107 | YMR100W | Tepraloxydim | 196 | YMR217W | metaflumizone |
| 107 | YMR100W | Pendimethalin | 196 | YMR217W | flubendiamide |
| 107 | YMR100W | Acifluorfen | 196 | YMR217W | chlorantraniliprole |
| 107 | YMR100W | Imazamethabenz | 196 | YMR217W | cyazypyr(HGW86) |
| 107 | YMR100W | Imazamox | 196 | YMR217W | Azoxystrobin |
| 107 | YMR100W | Imazapic | 196 | YMR217W | Dimoxystrobin |
| 107 | YMR100W | Imazapyr | 196 | YMR217W | Kresoxim-methyl |
| 107 | YMR100W | Imazaquin | 196 | YMR217W | Orysastrobin |
| 107 | YMR100W | Imazethapyr | 196 | YMR217W | Pyraclostrobin |
| 107 | YMR100W | 2,4-D | 196 | YMR217W | Trifloxystrobin |
| 107 | YMR100W | Chloridazon | 196 | YMR217W | Bixafen |
| 107 | YMR100W | Picloram | 196 | YMR217W | Boscalid |
| 107 | YMR100W | Picolinafen | 196 | YMR217W | Isopyrazam |
| 107 | YMR100W | Cyclosulfamuron | 196 | YMR217W | Metalaxyl |
| 107 | YMR100W | Triflusulfuron | 196 | YMR217W | Penthiopyrad |
| 107 | YMR100W | Atrazine | 196 | YMR217W | 3-Difluoromethyl-1-methyl-1H-pyrazole-4-carboxylic acid(2',4',5'-trifluorobiphenyl-2-yl)-amide |
| 107 | YMR100W | Pyroxasulfone | 196 | YMR217W | Dimethomorph |
| 107 | YMR100W | Bentazone | 196 | YMR217W | Difenoconazole |
| 107 | YMR100W | Cinidon-ethly | 196 | YMR217W | Epoxiconazole |
| 107 | YMR100W | Cinmethylin | 196 | YMR217W | Fluquinconazole |
| 107 | YMR100W | Dicamba | 196 | YMR217W | Metconazol |
| 107 | YMR100W | Diflufenzopyr | 196 | YMR217W | Propiconazole |
| 107 | YMR100W | Quinclorac | 196 | YMR217W | Prothioconazole |
| 107 | YMR100W | Quinmerac | 196 | YMR217W | Tebuconazole |
| 107 | YMR100W | Mesotrione | 196 | YMR217W | Triticonazole |
| 107 | YMR100W | Saflufenacil | 196 | YMR217W | Prochloraz |
| 107 | YMR100W | Topramezone; | 196 | YMR217W | Carbendazim |
| 112 | YNL024c | acephate | 196 | YMR217W | Cyprodinil |
| 112 | YNL024c | chlorpyrifos | 196 | YMR217W | Pyrimethanil |
| 112 | YNL024c | dimethoate | 196 | YMR217W | Fenpropimorph |
| 112 | YNL024c | methamidophos | 196 | YMR217W | Tridemorph |

(continued)

| Line in Table B | Gene Name/ LocusID | Active ingredientB | Line in Table B | Gene Name/ LocusID | Active ingredientB |
|---|---|---|---|---|---|
| 112 | YNL024c | terbufos | 196 | YMR217W | Iprodione |
| 112 | YNL024c | aldicarb | 196 | YMR217W | 5-ethyl-6-octyl-[1,2,4] triazolo [1,5-a]pyrimidine-7-ylamine |
| 112 | YNL024c | carbofuran | 196 | YMR217W | Mancozeb |
| 112 | YNL024c | bifenthrin | 196 | YMR217W | Maneb |
| 112 | YNL024c | cypermethrin | 196 | YMR217W | Metiram |
| 112 | YNL024c | alpha-cypermethrin | 196 | YMR217W | Dithianon |
| 112 | YNL024c | deltamethrin | 196 | YMR217W | Chlorothalonil |
| 112 | YNL024c | lambda-cyhalothrin | 196 | YMR217W | ThiophanateMethyl |
| 112 | YNL024c | tefluthrin | 196 | YMR217W | Cymoxanil |
| 112 | YN L024c | flufenoxuron | 196 | YMR217W | Metrafenone |
| 112 | YNL024c | teflubenzuron | 196 | YMR217W | Acetochlor |
| 112 | YNL024c | spirotetramat; | 196 | YMR217W | Dimethenamid |
| 112 | YNL024c | clothianidin | 196 | YMR217W | Metolachlor |
| 112 | YNL024c | imidacloprid | 196 | YMR217W | Metazachlor |
| 112 | YNL024c | thiamethoxam | 196 | YMR217W | Glyphosate |
| 112 | YNL024c | endosulfan | 196 | YMR217W | Glufosinate |
| 112 | YNL024c | fipronil | 196 | YMR217W | Sulfosate |
| 112 | YNL024c | abamectin | 196 | YMR217W | Fenoxaprop |
| 112 | YNL024c | spinosad | 196 | YMR217W | Paraquat |
| 112 | YNL024c | spinetoram | 196 | YMR217W | Cycloxydim |
| 112 | YNL024c | hydramethylnon; | 196 | YMR217W | Profoxydim |
| 112 | YNL024c | chlorfenapyr; | 196 | YMR217W | Sethoxydim |
| 112 | YNL024c | indoxacarb | 196 | YMR217W | Tepraloxydim |
| 112 | YNL024c | metaflumizone | 196 | YMR217W | Pendimethalin |
| 112 | YNL024c | flubendiamide | 196 | YMR217W | Acifluorfen |
| 112 | YNL024c | chlorantraniliprole | 196 | YMR217W | Imazamethabenz |
| 112 | YNL024c | cyazypyr(HGW86) | 196 | YMR217W | Imazamox |
| 112 | YNL024c | Azoxystrobin | 196 | YMR217W | Imazapic |
| 112 | YNL024c | Dimoxystrobin | 196 | YMR217W | Imazapyr |
| 112 | YNL024c | Kresoxim-methyl | 196 | YMR217W | Imazaquin |
| 112 | YNL024c | Orysastrobin | 196 | YMR217W | Imazethapyr |
| 112 | YNL024c | Pyraclostrobin | 196 | YMR217W | 2,4-D |
| 112 | YNL024c | Trifloxystrobin | 196 | YMR217W | Chloridazon |
| 112 | YNL024c | Bixafen | 196 | YMR217W | Picloram |
| 112 | YNL024c | Boscalid | 196 | YMR217W | Picolinafen |

(continued)

| Line in Table B | Gene Name/ LocusID | Active ingredientB | Line in Table B | Gene Name/ LocusID | Active ingredientB |
|---|---|---|---|---|---|
| 112 | YNL024c | Isopyrazam | 196 | YMR217W | Cyclosulfamuron |
| 112 | YNL024c | Metalaxyl | 196 | YMR217W | Triflusulfuron |
| 112 | YNL024c | Penthiopyrad | 196 | YMR217W | Atrazine |
| 112 | YNL024c | 3-Difluoromethyl-1-methyl-1 H-pyrazole-4-carboxylic acid(2',4',5'-trifluorobiphenyl-2-yl)-amide | 196 | YMR217W | Pyroxasulfone |
| 112 | YNL024c | Dimethomorph | 196 | YMR217W | Bentazone |
| 112 | YNL024c | Difenoconazole | 196 | YMR217W | Cinidon-ethly |
| 112 | YNL024c | Epoxiconazole | 196 | YMR217W | Cinmethylin |
| 112 | YNL024c | Fluquinconazole | 196 | YMR217W | Dicamba |
| 112 | YNL024c | Metconazol | 196 | YMR217W | Diflufenzopyr |
| 112 | YNL024c | Propiconazole | 196 | YMR217W | Quinclorac |
| 112 | YNL024c | Prothioconazole | 196 | YMR217W | Quinmerac |
| 112 | YNL024c | Tebuconazole | 196 | YMR217W | Mesotrione |
| 112 | YNL024c | Triticonazole | 196 | YMR217W | Saflufenacil |
| 112 | YNL024c | Prochloraz | 196 | YMR217W | Topramezone; |
| 112 | YNL024c | Carbendazim | | | |
| 203 | GOICDS_C DS1608 | acephate | 204 | | acephate |
| 203 | GOICDS_C DS1608 | chlorpyrifos | 204 | | chlorpyrifos |
| 203 | GOICDS_C DS1608 | dimethoate | 204 | | dimethoate |
| 203 | GOICDS_C DS1608 | methamidophos | 204 | | methamidophos |
| 203 | GOICDS_C DS1608 | terbufos | 204 | | terbufos |
| 203 | GOICDS_C DS1608 | aldicarb | 204 | | aldicarb |
| 203 | GOICDS_C DS1608 | carbofuran | 204 | | carbofuran |
| 203 | GOICDS_C DS1608 | bifenthrin | 204 | | bifenthrin |
| 203 | GOICDS_C DS1608 | cypermethrin | 204 | | cypermethrin |
| 203 | GOICDS_C DS1608 | alpha-cypermethrin | 204 | | alpha-cypermethrin |

(continued)

| Line in Table B | Gene Name/ LocusID | Active ingredientB | Line in Table B | Gene Name/ LocusID | Active ingredientB |
|---|---|---|---|---|---|
| 203 | GOICDS_C DS1608 | deltamethrin | 204 | | deltamethrin |
| 203 | GOICDS_C DS1608 | lambda-cyhalothrin | 204 | | lambda-cyhalothrin |
| 203 | GOICDS_C DS1608 | tefluthrin | 204 | | tefluthrin |
| 203 | GOICDS_C DS1608 | flufenoxuron | 204 | | flufenoxuron |
| 203 | GOICDS_C DS1608 | teflubenzuron | 204 | | teflubenzuron |
| 203 | GOICDS_C DS1608 | spirotetramat; | 204 | | spirotetramat; |
| 203 | GOICDS_C DS1608 | clothianidin | 204 | | clothianidin |
| 203 | GOICDS_C DS1608 | imidacloprid | 204 | | imidacloprid |
| 203 | GOICDS_C DS1608 | thiamethoxam | 204 | | thiamethoxam |
| 203 | GOICDS_C DS1608 | endosulfan | 204 | | endosulfan |
| 203 | GOICDS_C DS1608 | fipronil | 204 | | fipronil |
| 203 | GOICDS_C DS1608 | abamectin | 204 | | abamectin |
| 203 | GOICDS_C DS1608 | spinosad | 204 | | spinosad |
| 203 | GOICDS_C DS1608 | spinetoram | 204 | | spinetoram |
| 203 | GOICDS_C DS1608 | hydramethylnon; | 204 | | hydramethylnon; |
| 203 | GOICDS_C DS1608 | chlorfenapyr; | 204 | | chlorfenapyr; |
| 203 | GOICDS_C DS1608 | indoxacarb | 204 | | indoxacarb |
| 203 | GOICDS_C DS1608 | metaflumizone | 204 | | metaflumizone |
| 203 | GOICDS_C DS1608 | flubendiamide | 204 | | flubendiamide |
| 203 | GOICDS_C DS1608 | chlorantraniliprole | 204 | | chlorantraniliprole |
| 203 | GOICDS_C DS1608 | cyazypyr(HGW86) | 204 | | cyazypyr(HGW86) |
| 203 | GOICDS_C DS1608 | Azoxystrobin | 204 | | Azoxystrobin |

(continued)

| Line in Table B | Gene Name/ LocusID | Active ingredientB | Line in Table B | Gene Name/ LocusID | Active ingredientB |
|---|---|---|---|---|---|
| 203 | GOICDS_CDS1608 | Dimoxystrobin | 204 | | Dimoxystrobin |
| 203 | GOICDS_CDS1608 | Kresoxim-methyl | 204 | | Kresoxim-methyl |
| 203 | GOICDS_CDS1608 | Orysastrobin | 204 | | Orysastrobin |
| 203 | GOICDS_CDS1608 | Pyraclostrobin | 204 | | Pyraclostrobin |
| 203 | GOICDS_CDS1608 | Trifloxystrobin | 204 | | Trifloxystrobin |
| 203 | GOICDS_CDS1608 | Bixafen | 204 | | Bixafen |
| 203 | GOICDS_CDS1608 | Boscalid | 204 | | Boscalid |
| 203 | GOICDS_CDS1608 | Isopyrazam | 204 | | Isopyrazam |
| 203 | GOICDS_CDS1608 | Metalaxyl | 204 | | Metalaxyl |
| 203 | GOICDS_CDS1608 | Penthiopyrad | 204 | | Penthiopyrad |
| 203 | GOICDS_CDS1608 | 3-Difluoromethyl-1-methyl-1 H-pyrazole-4-carboxylic acid(2',4',5'-trifluorobiphenyl-2-yl)-amide | 204 | | 3-Difluoromethyl-1-methyl-1H-pyrazole-4-carboxylic acid(2',4',5'-trifluorobiphenyl-2-yl)-amide |
| 203 | GOICDS_CDS1608 | Dimethomorph | 204 | | Dimethomorph |
| 203 | GOICDS_CDS1608 | Difenoconazole | 204 | | Difenoconazole |
| 203 | GOICDS_CDS1608 | Epoxiconazole | 204 | | Epoxiconazole |
| 203 | GOICDS_CDS1608 | Fluquinconazole | 204 | | Fluquinconazole |
| 203 | GOICDS_CDS1608 | Metconazol | 204 | | Metconazol |
| 203 | GOICDS_CDS1608 | Propiconazole | 204 | | Propiconazole |
| 203 | GOICDS_CDS1608 | Prothioconazole | 204 | | Prothioconazole |
| 203 | GOICDS_CDS1608 | Tebuconazole | 204 | | Tebuconazole |
| 203 | GOICDS_CDS1608 | Triticonazole | 204 | | Triticonazole |

(continued)

| Line in Table B | Gene Name/ LocusID | Active ingredientB | Line in Table B | Gene Name/ LocusID | Active ingredientB |
|---|---|---|---|---|---|
| 203 | GOICDS_CDS1608 | Prochloraz | 204 | | Prochloraz |
| 203 | GOICDS_CDS1608 | Carbendazim | 204 | | Carbendazim |
| 203 | GOICDS_CDS1608 | Cyprodinil | 204 | | Cyprodinil |
| 203 | GOICDS_CDS1608 | Pyrimethanil | 204 | | Pyrimethanil |
| 203 | GOICDS_CDS1608 | Fenpropimorph | 204 | | Fenpropimorph |
| 203 | GOICDS_CDS1608 | Tridemorph | 204 | | Tridemorph |
| 203 | GOICDS_CDS1608 | Iprodione | 204 | | Iprodione |
| 203 | GOICDS_CDS1608 | 5-ethyl-6-octyl-[1,2,4] triazolo [1,5-a]pyrimidine-7-ylamine | 204 | | 5-ethyl-6-octyl-[1,2,4] triazolo [1,5-a]pyrimidine-7-ylamine |
| 203 | GOICDS_CDS1608 | Mancozeb | 204 | | Mancozeb |
| 203 | GOICDS_CDS1608 | Maneb | 204 | | Maneb |
| 203 | GOICDS_CDS1608 | Metiram | 204 | | Metiram |
| 203 | GOICDS_CDS1608 | Dithianon | 204 | | Dithianon |
| 203 | GOICDS_CDS1608 | Chlorothalonil | 204 | | Chlorothalonil |
| 203 | GOICDS_CDS1608 | ThiophanateMethyl | 204 | | ThiophanateMethyl |
| 203 | GOICDS_CDS1608 | Cymoxanil | 204 | | Cymoxanil |
| 203 | GOICDS_CDS1608 | Metrafenone | 204 | | Metrafenone |
| 203 | GOICDS_CDS1608 | Acetochlor | 204 | | Acetochlor |
| 203 | GOICDS_CDS1608 | Dimethenamid | 204 | | Dimethenamid |
| 203 | GOICDS_CDS1608 | Metolachlor | 204 | | Metolachlor |
| 203 | GOICDS_CDS1608 | Metazachlor | 204 | | Metazachlor |
| 203 | GOICDS_CDS1608 | Glyphosate | 204 | | Glyphosate |

(continued)

| Line in Table B | Gene Name/ LocusID | Active ingredientB | Line in Table B | Gene Name/ LocusID | Active ingredientB |
|---|---|---|---|---|---|
| 203 | GOICDS_CDS1608 | Glufosinate | 204 | | Glufosinate |
| 203 | GOICDS_CDS1608 | Sulfosate | 204 | | Sulfosate |
| 203 | GOICDS_CDS1608 | Fenoxaprop | 204 | | Fenoxaprop |
| 203 | GOICDS_CDS1608 | Paraquat | 204 | | Paraquat |
| 203 | GOICDS_CDS1608 | Cycloxydim | 204 | | Cycloxydim |
| 203 | GOICDS_CDS1608 | Profoxydim | 204 | | Profoxydim |
| 203 | GOICDS_CDS1608 | Sethoxydim | 204 | | Sethoxydim |
| 203 | GOICDS_CDS1608 | Tepraloxydim | 204 | | Tepraloxydim |
| 203 | GOICDS_CDS1608 | Pendimethalin | 204 | | Pendimethalin |
| 203 | GOICDS_CDS1608 | Acifluorfen | 204 | | Acifluorfen |
| 203 | GOICDS_CDS1608 | Imazamethabenz | 204 | | Imazamethabenz |
| 203 | GOICDS_CDS1608 | Imazamox | 204 | | Imazamox |
| 203 | GOICDS_CDS1608 | Imazapic | 204 | | Imazapic |
| 203 | GOICDS_CDS1608 | Imazapyr | 204 | | Imazapyr |
| 203 | GOICDS_CDS1608 | Imazaquin | 204 | | Imazaquin |
| 203 | GOICDS_CDS1608 | Imazethapyr | 204 | | Imazethapyr |
| 203 | GOICDS_CDS1608 | 2,4-D | 204 | | 2,4-D |
| 203 | GOICDS_CDS1608 | Chloridazon | 204 | | Chloridazon |
| 203 | GOICDS_CDS1608 | Picloram | 204 | | Picloram |
| 203 | GOICDS_CDS1608 | Picolinafen | 204 | | Picolinafen |
| 203 | GOICDS_CDS1608 | Cyclosulfamuron | 204 | | Cyclosulfamuron |
| 203 | GOICDS_CDS1608 | Triflusulfuron | 204 | | Triflusulfuron |

(continued)

| Line in Table B | Gene Name/ LocusID | Active ingredientB | Line in Table B | Gene Name/ LocusID | Active ingredientB |
|---|---|---|---|---|---|
| 203 | GOICDS_C DS1608 | Atrazine | 204 | | Atrazine |
| 203 | GOICDS_C DS1608 | Pyroxasulfone | 204 | | Pyroxasulfone |
| 203 | GOICDS_C DS1608 | Bentazone | 204 | | Bentazone |
| 203 | GOICDS_C DS1608 | Cinidon-ethly | 204 | | Cinidon-ethly |
| 203 | GOICDS_C DS1608 | Cinmethylin | 204 | | Cinmethylin |
| 203 | GOICDS_C DS1608 | Dicamba | 204 | | Dicamba |
| 203 | GOICDS_C DS1608 | Diflufenzopyr | 204 | | Diflufenzopyr |
| 203 | GOICDS_C DS1608 | Quinclorac | 204 | | Quinclorac |
| 203 | GOICDS_C DS1608 | Quinmerac | 204 | | Quinmerac |
| 203 | GOICDS_C DS1608 | Mesotrione | 204 | | Mesotrione |
| 203 | GOICDS_C DS1608 | Saflufenacil | 204 | | Saflufenacil |
| 203 | GOICDS_C DS1608 | Topramezone; | 204 | | Topramezone; |
| 205 | GOICDS_C DS0644_7 | acephate | 206 | | acephate |
| 205 | GOICDS_C DS0644_7 | chlorpyrifos | 206 | | chlorpyrifos |
| 205 | GOICDS_C DS0644_7 | dimethoate | 206 | | dimethoate |
| 205 | GOICDS_C DS0644_7 | methamidophos | 206 | | methamidophos |
| 205 | GOICDS_C DS0644_7 | terbufos | 206 | | terbufos |
| 205 | GOICDS_C DS0644_7 | aldicarb | 206 | | aldicarb |
| 205 | GOICDS_C DS0644_7 | carbofuran | 206 | | carbofuran |
| 205 | GOICDS_C DS0644_7 | bifenthrin | 206 | | bifenthrin |
| 205 | GOICDS_C DS0644_7 | cypermethrin | 206 | | cypermethrin |
| 205 | GOICDS_C DS0644_7 | alpha-cypermethrin | 206 | | alpha-cypermethrin |

(continued)

| Line in Table B | Gene Name/ LocusID | Active ingredientB | Line in Table B | Gene Name/ LocusID | Active ingredientB |
|---|---|---|---|---|---|
| 205 | GOICDS_CDS0644_7 | deltamethrin | 206 | | deltamethrin |
| 205 | GOICDS_CDS0644_7 | lambda-cyhalothrin | 206 | | lambda-cyhalothrin |
| 205 | GOICDS_CDS0644_7 | tefluthrin | 206 | | tefluthrin |
| 205 | GOICDS_CDS0644_7 | flufenoxuron | 206 | | flufenoxuron |
| 205 | GOICDS_CDS0644_7 | teflubenzuron | 206 | | teflubenzuron |
| 205 | GOICDS_CDS0644_7 | spirotetramat; | 206 | | spirotetramat; |
| 205 | GOICDS_CDS0644_7 | clothianidin | 206 | | clothianidin |
| 205 | GOICDS_CDS0644_7 | imidacloprid | 206 | | imidacloprid |
| 205 | GOICDS_CDS0644_7 | thiamethoxam | 206 | | thiamethoxam |
| 205 | GOICDS_CDS0644_7 | endosulfan | 206 | | endosulfan |
| 205 | GOICDS_CDS0644_7 | fipronil | 206 | | fipronil |
| 205 | GOICDS_CDS0644_7 | abamectin | 206 | | abamectin |
| 205 | GOICDS_CDS0644_7 | spinosad | 206 | | spinosad |
| 205 | GOICDS_CDS0644_7 | spinetoram | 206 | | spinetoram |
| 205 | GOICDS_CDS0644_7 | hydramethylnon; | 206 | | hydramethylnon; |
| 205 | GOICDS_CDS0644_7 | chlorfenapyr; | 206 | | chlorfenapyr; |
| 205 | GOICDS_CDS0644_7 | indoxacarb | 206 | | indoxacarb |
| 205 | GOICDS_CDS0644_7 | metaflumizone | 206 | | metaflumizone |
| 205 | GOICDS_CDS0644_7 | flubendiamide | 206 | | flubendiamide |
| 205 | GOICDS_CDS0644_7 | chlorantraniliprole | 206 | | chlorantraniliprole |
| 205 | GOICDS_CDS0644_7 | cyazypyr(HGW86) | 206 | | cyazypyr(HGW86) |
| 205 | GOICDS_CDS0644_7 | Azoxystrobin | 206 | | Azoxystrobin |

(continued)

| Line in Table B | Gene Name/ LocusID | Active ingredientB | Line in Table B | Gene Name/ LocusID | Active ingredientB |
|---|---|---|---|---|---|
| 205 | GOICDS_CDS0644_7 | Dimoxystrobin | 206 | | Dimoxystrobin |
| 205 | GOICDS_CDS0644_7 | Kresoxim-methyl | 206 | | Kresoxim-methyl |
| 205 | GOICDS_CDS0644_7 | Orysastrobin | 206 | | Orysastrobin |
| 205 | GOICDS_CDS0644_7 | Pyraclostrobin | 206 | | Pyraclostrobin |
| 205 | GOICDS_CDS0644_7 | Trifloxystrobin | 206 | | Trifloxystrobin |
| 205 | GOICDS_CDS0644_7 | Bixafen | 206 | | Bixafen |
| 205 | GOICDS_CDS0644_7 | Boscalid | 206 | | Boscalid |
| 205 | GOICDS_CDS0644_7 | Isopyrazam | 206 | | Isopyrazam |
| 205 | GOICDS_CDS0644_7 | Metalaxyl | 206 | | Metalaxyl |
| 205 | GOICDS_CDS0644_7 | Penthiopyrad | 206 | | Penthiopyrad |
| 205 | GOICDS_CDS0644_7 | 3-Difluoromethyl-1-methyl-1 H-pyrazole-4-carboxylic acid(2',4',5'-trifluorobiphenyl-2-yl)-amide | 206 | | 3-Difluoromethyl-1-methyl-1H-pyrazole-4-carboxylic acid(2',4',5'-trifluorobiphenyl-2-yl)-amide |
| 205 | GOICDS_CDS0644_7 | Dimethomorph | 206 | | Dimethomorph |
| 205 | GOICDS_CDS0644_7 | Difenoconazole | 206 | | Difenoconazole |
| 205 | GOICDS_CDS0644_7 | Epoxiconazole | 206 | | Epoxiconazole |
| 205 | GOICDS_CDS0644_7 | Fluquinconazole | 206 | | Fluquinconazole |
| 205 | GOICDS_CDS0644_7 | Metconazol | 206 | | Metconazol |
| 205 | GOICDS_CDS0644_7 | Propiconazole | 206 | | Propiconazole |
| 205 | GOICDS_CDS0644_7 | Prothioconazole | 206 | | Prothioconazole |
| 205 | GOICDS_CDS0644_7 | Tebuconazole | 206 | | Tebuconazole |
| 205 | GOICDS_CDS0644_7 | Triticonazole | 206 | | Triticonazole |

(continued)

| Line in Table B | Gene Name/ LocusID | Active ingredientB | Line in Table B | Gene Name/ LocusID | Active ingredientB |
|---|---|---|---|---|---|
| 205 | GOICDS_C DS0644_7 | Prochloraz | 206 | | Prochloraz |
| 205 | GOICDS_C DS0644_7 | Carbendazim | 206 | | Carbendazim |
| 205 | GOICDS_C DS0644_7 | Cyprodinil | 206 | | Cyprodinil |
| 205 | GOICDS_C DS0644_7 | Pyrimethanil | 206 | | Pyrimethanil |
| 205 | GOICDS_C DS0644_7 | Fenpropimorph | 206 | | Fenpropimorph |
| 205 | GOICDS_C DS0644_7 | Tridemorph | 206 | | Tridemorph |
| 205 | GOICDS_C DS0644_7 | Iprodione | 206 | | Iprodione |
| 205 | GOICDS_C DS0644_7 | 5-ethyl-6-octyl-[1,2,4] triazolo [1,5-a]pyrimidine-7-ylamine | 206 | | 5-ethyl-6-octyl-[1,2,4] triazolo [1,5-a]pyrimidine-7-ylamine |
| 205 | GOICDS_C DS0644_7 | Mancozeb | 206 | | Mancozeb |
| 205 | GOICDS_C DS0644_7 | Maneb | 206 | | Maneb |
| 205 | GOICDS_C DS0644_7 | Metiram | 206 | | Metiram |
| 205 | GOICDS_C DS0644_7 | Dithianon | 206 | | Dithianon |
| 205 | GOICDS_C DS0644_7 | Chlorothalonil | 206 | | Chlorothalonil |
| 205 | GOICDS_C DS0644_7 | ThiophanateMethyl | 206 | | ThiophanateMethyl |
| 205 | GOICDS_C DS0644_7 | Cymoxanil | 206 | | Cymoxanil |
| 205 | GOICDS_C DS0644_7 | Metrafenone | 206 | | Metrafenone |
| 205 | GOICDS_C DS0644_7 | Acetochlor | 206 | | Acetochlor |
| 205 | GOICDS_C DS0644_7 | Dimethenamid | 206 | | Dimethenamid |
| 205 | GOICDS_C DS0644_7 | Metolachlor | 206 | | Metolachlor |
| 205 | GOICDS_C DS0644_7 | Metazachlor | 206 | | Metazachlor |
| 205 | GOICDS_C DS0644_7 | Glyphosate | 206 | | Glyphosate |

(continued)

| Line in Table B | Gene Name/ LocusID | Active ingredientB | Line in Table B | Gene Name/ LocusID | Active ingredientB |
|---|---|---|---|---|---|
| 205 | GOICDS_CDS0644_7 | Glufosinate | 206 | | Glufosinate |
| 205 | GOICDS_CDS0644_7 | Sulfosate | 206 | | Sulfosate |
| 205 | GOICDS_CDS0644_7 | Fenoxaprop | 206 | | Fenoxaprop |
| 205 | GOICDS_CDS0644_7 | Paraquat | 206 | | Paraquat |
| 205 | GOICDS_CDS0644_7 | Cycloxydim | 206 | | Cycloxydim |
| 205 | GOICDS_CDS0644_7 | Profoxydim | 206 | | Profoxydim |
| 205 | GOICDS_CDS0644_7 | Sethoxydim | 206 | | Sethoxydim |
| 205 | GOICDS_CDS0644_7 | Tepraloxydim | 206 | | Tepraloxydim |
| 205 | GOICDS_CDS0644_7 | Pendimethalin | 206 | | Pendimethalin |
| 205 | GOICDS_CDS0644_7 | Acifluorfen | 206 | | Acifluorfen |
| 205 | GOICDS_CDS0644_7 | Imazamethabenz | 206 | | Imazamethabenz |
| 205 | GOICDS_CDS0644_7 | Imazamox | 206 | | Imazamox |
| 205 | GOICDS_CDS0644_7 | Imazapic | 206 | | Imazapic |
| 205 | GOICDS_CDS0644_7 | Imazapyr | 206 | | Imazapyr |
| 205 | GOICDS_CDS0644_7 | Imazaquin | 206 | | Imazaquin |
| 205 | GOICDS_CDS0644_7 | Imazethapyr | 206 | | Imazethapyr |
| 205 | GOICDS_CDS0644_7 | 2,4-D | 206 | | 2,4-D |
| 205 | GOICDS_CDS0644_7 | Chloridazon | 206 | | Chloridazon |
| 205 | GOICDS_CDS0644_7 | Picloram | 206 | | Picloram |
| 205 | GOICDS_CDS0644_7 | Picolinafen | 206 | | Picolinafen |
| 205 | GOICDS_CDS0644_7 | Cyclosulfamuron | 206 | | Cyclosulfamuron |
| 205 | GOICDS_CDS0644_7 | Triflusulfuron | 206 | | Triflusulfuron |

(continued)

| Line in Table B | Gene Name/ LocusID | Active ingredientB | Line in Table B | Gene Name/ LocusID | Active ingredientB |
|---|---|---|---|---|---|
| 205 | GOICDS_CDS0644_7 | Atrazine | 206 | | Atrazine |
| 205 | GOICDS_CDS0644_7 | Pyroxasulfone | 206 | | Pyroxasulfone |
| 205 | GOICDS_CDS0644_7 | Bentazone | 206 | | Bentazone |
| 205 | GOICDS_CDS0644_7 | Cinidon-ethly | 206 | | Cinidon-ethly |
| 205 | GOICDS_CDS0644_7 | Cinmethylin | 206 | | Cinmethylin |
| 205 | GOICDS_CDS0644_7 | Dicamba | 206 | | Dicamba |
| 205 | GOICDS_CDS0644_7 | Diflufenzopyr | 206 | | Diflufenzopyr |
| 205 | GOICDS_CDS0644_7 | Quinclorac | 206 | | Quinclorac |
| 205 | GOICDS_CDS0644_7 | Quinmerac | 206 | | Quinmerac |
| 205 | GOICDS_CDS0644_7 | Mesotrione | 206 | | Mesotrione |
| 205 | GOICDS_CDS0644_7 | Saflufenacil | 206 | | Saflufenacil |
| 205 | GOICDS_CDS0644_7 | Topramezone; | 206 | | Topramezone; |
| 207 | GOICDS_CDS2447 | acephate | 209 | GOISCDS_CDS3159 | acephate |
| 207 | GOICDS_CDS2447 | chlorpyrifos | 209 | GOISCDS_CDS3159 | chlorpyrifos |
| 207 | GOICDS_CDS2447 | dimethoate | 209 | GOISCDS_CDS3159 | dimethoate |
| 207 | GOICDS_CDS2447 | methamidophos | 209 | GOISCDS_CDS3159 | methamidophos |
| 207 | GOICDS_CDS2447 | terbufos | 209 | GOISCDS_CDS3159 | terbufos |
| 207 | GOICDS_CDS2447 | aldicarb | 209 | GOISCDS_CDS3159 | aldicarb |
| 207 | GOICDS_CDS2447 | carbofuran | 209 | GOISCDS_CDS3159 | carbofuran |
| 207 | GOICDS_CDS2447 | bifenthrin | 209 | GOISCDS_CDS3159 | bifenthrin |
| 207 | GOICDS_CDS2447 | cypermethrin | 209 | GOISCDS_CDS3159 | cypermethrin |
| 207 | GOICDS_CDS2447 | alpha-cypermethrin | 209 | GOISCDS_CDS3159 | alpha-cypermethrin |

(continued)

| Line in Table B | Gene Name/ LocusID | Active ingredientB | Line in Table B | Gene Name/ LocusID | Active ingredientB |
|---|---|---|---|---|---|
| 207 | GOICDS_C DS2447 | deltamethrin | 209 | GOISCDS_ CDS3159 | deltamethrin |
| 207 | GOICDS_C DS2447 | lambda-cyhalothrin | 209 | GOISCDS_ CDS3159 | lambda-cyhalothrin |
| 207 | GOICDS_C DS2447 | tefluthrin | 209 | GOISCDS_ CDS3159 | tefluthrin |
| 207 | GOICDS_C DS2447 | flufenoxuron | 209 | GOISCDS_ CDS3159 | flufenoxuron |
| 207 | GOICDS_C DS2447 | teflubenzuron | 209 | GOISCDS_ CDS3159 | teflubenzuron |
| 207 | GOICDS_C DS2447 | spirotetramat; | 209 | GOISCDS_ CDS3159 | spirotetramat; |
| 207 | GOICDS_C DS2447 | clothianidin | 209 | GOISCDS_ CDS3159 | clothianidin |
| 207 | GOICDS_C DS2447 | imidacloprid | 209 | GOISCDS_ CDS3159 | imidacloprid |
| 207 | GOICDS_C DS2447 | thiamethoxam | 209 | GOISCDS_ CDS3159 | thiamethoxam |
| 207 | GOICDS_C DS2447 | endosulfan | 209 | GOISCDS_ CDS3159 | endosulfan |
| 207 | GOICDS_C DS2447 | fipronil | 209 | GOISCDS_ CDS3159 | fipronil |
| 207 | GOICDS_C DS2447 | abamectin | 209 | GOISCDS_ CDS3159 | abamectin |
| 207 | GOICDS_C DS2447 | spinosad | 209 | GOISCDS_ CDS3159 | spinosad |
| 207 | GOICDS_C DS2447 | spinetoram | 209 | GOISCDS_ CDS3159 | spinetoram |
| 207 | GOICDS_C DS2447 | hydramethylnon; | 209 | GOISCDS_ CDS3159 | hydramethylnon; |
| 207 | GOICDS_C DS2447 | chlorfenapyr; | 209 | GOISCDS_ CDS3159 | chlorfenapyr; |
| 207 | GOICDS_C DS2447 | indoxacarb | 209 | GOISCDS_ CDS3159 | indoxacarb |
| 207 | GOICDS_C DS2447 | metaflumizone | 209 | GOISCDS_ CDS3159 | metaflumizone |
| 207 | GOICDS_C DS2447 | flubendiamide | 209 | GOISCDS_ CDS3159 | flubendiamide |
| 207 | GOICDS_C DS2447 | chlorantraniliprole | 209 | GOISCDS_ CDS3159 | chlorantraniliprole |
| 207 | GOICDS_C DS2447 | cyazypyr(HGW86) | 209 | GOISCDS_ CDS3159 | cyazypyr(HGW86) |
| 207 | GOICDS_C DS2447 | Azoxystrobin | 209 | GOISCDS_ CDS3159 | Azoxystrobin |

(continued)

| Line in Table B | Gene Name/ LocusID | Active ingredientB | Line in Table B | Gene Name/ LocusID | Active ingredientB |
|---|---|---|---|---|---|
| 207 | GOICDS_CDS2447 | Dimoxystrobin | 209 | GOISCDS_CDS3159 | Dimoxystrobin |
| 207 | GOICDS_CDS2447 | Kresoxim-methyl | 209 | GOISCDS_CDS3159 | Kresoxim-methyl |
| 207 | GOICDS_CDS2447 | Orysastrobin | 209 | GOISCDS_CDS3159 | Orysastrobin |
| 207 | GOICDS_CDS2447 | Pyraclostrobin | 209 | GOISCDS_CDS3159 | Pyraclostrobin |
| 207 | GOICDS_CDS2447 | Trifloxystrobin | 209 | GOISCDS_CDS3159 | Trifloxystrobin |
| 207 | GOICDS_CDS2447 | Bixafen | 209 | GOISCDS_CDS3159 | Bixafen |
| 207 | GOICDS_CDS2447 | Boscalid | 209 | GOISCDS_CDS3159 | Boscalid |
| 207 | GOICDS_CDS2447 | Isopyrazam | 209 | GOISCDS_CDS3159 | Isopyrazam |
| 207 | GOICDS_CDS2447 | Metalaxyl | 209 | GOISCDS_CDS3159 | Metalaxyl |
| 207 | GOICDS_CDS2447 | Penthiopyrad | 209 | GOISCDS_CDS3159 | Penthiopyrad |
| 207 | GOICDS_CDS2447 | 3-Difluoromethyl-1-methyl-1H-pyrazole-4-carboxylic acid(2',4',5'-trifluorobiphenyl-2-yl)-amide | 209 | GOISCDS_CDS3159 | 3-Difluoromethyl-1-methyl-1H-pyrazole-4-carboxylic acid(2',4',5'-trifluorobiphenyl-2-yl)-amide |
| 207 | GOICDS_CDS2447 | Dimethomorph | 209 | GOISCDS_CDS3159 | Dimethomorph |
| 207 | GOICDS_CDS2447 | Difenoconazole | 209 | GOISCDS_CDS3159 | Difenoconazole |
| 207 | GOICDS_CDS2447 | Epoxiconazole | 209 | GOISCDS_CDS3159 | Epoxiconazole |
| 207 | GOICDS_CDS2447 | Fluquinconazole | 209 | GOISCDS_CDS3159 | Fluquinconazole |
| 207 | GOICDS_CDS2447 | Metconazol | 209 | GOISCDS_ | Metconazol |
| 207 | GOICDS_CDS2447 | Propiconazole | 209 | GOISCDS_CDS3159 | Propiconazole |
| 207 | GOICDS_CDS2447 | Prothioconazole | 209 | GOISCDS_CDS3159 | Prothioconazole |
| 207 | GOICDS_CDS2447 | Tebuconazole | 209 | GOISCDS_CDS3159 | Tebuconazole |
| 207 | GOICDS_CDS2447 | Triticonazole | 209 | GOISCDS_CDS3159 | Triticonazole |

(continued)

| Line in Table B | Gene Name/ LocusID | Active ingredientB | Line in Table B | Gene Name/ LocusID | Active ingredientB |
|---|---|---|---|---|---|
| 207 | GOICDS_CDS2447 | Prochloraz | 209 | GOISCDS_CDS3159 | Prochloraz |
| 207 | GOICDS_CDS2447 | Carbendazim | 209 | GOISCDS_CDS3159 | Carbendazim |
| 207 | GOICDS_CDS2447 | Cyprodinil | 209 | GOISCDS_CDS3159 | Cyprodinil |
| 207 | GOICDS_CDS2447 | Pyrimethanil | 209 | GOISCDS_CDS3159 | Pyrimethanil |
| 207 | GOICDS_CDS2447 | Fenpropimorph | 209 | GOISCDS_CDS3159 | Fenpropimorph |
| 207 | GOICDS_CDS2447 | Tridemorph | 209 | GOISCDS_CDS3159 | Tridemorph |
| 207 | GOICDS_CDS2447 | Iprodione | 209 | GOISCDS_CDS3159 | Iprodione |
| 207 | GOICDS_CDS2447 | 5-ethyl-6-octyl-[1,2,4] triazolo [1,5-a]pyrimidine-7-ylamine | 209 | GOISCDS_CDS3159 | 5-ethyl-6-octyl-[1,2,4] triazolo [1,5-a]pyrimidine-7-ylamine |
| 207 | GOICDS_CDS2447 | Mancozeb | 209 | GOISCDS_CDS3159 | Mancozeb |
| 207 | GOICDS_CDS2447 | Maneb | 209 | GOISCDS_CDS3159 | Maneb |
| 207 | GOICDS_CDS2447 | Metiram | 209 | GOISCDS_CDS3159 | Metiram |
| 207 | GOICDS_CDS2447 | Dithianon | 209 | GOISCDS_CDS3159 | Dithianon |
| 207 | GOICDS_CDS2447 | Chlorothalonil | 209 | GOISCDS_CDS3159 | Chlorothalonil |
| 207 | GOICDS_CDS2447 | ThiophanateMethyl | 209 | GOISCDS_CDS3159 | ThiophanateMethyl |
| 207 | GOICDS_CDS2447 | Cymoxanil | 209 | GOISCDS_CDS3159 | Cymoxanil |
| 207 | GOICDS_CDS2447 | Metrafenone | 209 | GOISCDS_CDS3159 | Metrafenone |
| 207 | GOICDS_CDS2447 | Acetochlor | 209 | GOISCDS_CDS3159 | Acetochlor |
| 207 | GOICDS_CDS2447 | Dimethenamid | 209 | GOISCDS_CDS3159 | Dimethenamid |
| 207 | GOICDS_CDS2447 | Metolachlor | 209 | GOISCDS_CDS3159 | Metolachlor |
| 207 | GOICDS_CDS2447 | Metazachlor | 209 | GOISCDS_CDS3159 | Metazachlor |
| 207 | GOICDS_CDS2447 | Glyphosate | 209 | GOISCDS_CDS3159 | Glyphosate |

(continued)

| Line in Table B | Gene Name/ LocusID | Active ingredientB | Line in Table B | Gene Name/ LocusID | Active ingredientB |
|---|---|---|---|---|---|
| 207 | GOICDS_C DS2447 | Glufosinate | 209 | GOISCDS_ CDS3159 | Glufosinate |
| 207 | GOICDS_C DS2447 | Sulfosate | 209 | GOISCDS_ CDS3159 | Sulfosate |
| 207 | GOICDS_C DS2447 | Fenoxaprop | 209 | GOISCDS_ CDS3159 | Fenoxaprop |
| 207 | GOICDS_C DS2447 | Paraquat | 209 | GOISCDS_ CDS3159 | Paraquat |
| 207 | GOICDS_C DS2447 | Cycloxydim | 209 | GOISCDS_ CDS3159 | Cycloxydim |
| 207 | GOICDS_C DS2447 | Profoxydim | 209 | GOISCDS_ CDS3159 | Profoxydim |
| 207 | GOICDS_C DS2447 | Sethoxydim | 209 | GOISCDS_ CDS3159 | Sethoxydim |
| 207 | GOICDS_C DS2447 | Tepraloxydim | 209 | GOISCDS_ CDS3159 | Tepraloxydim |
| 207 | GOICDS_C DS2447 | Pendimethalin | 209 | GOISCDS_ CDS3159 | Pendimethalin |
| 207 | GOICDS_C DS2447 | Acifluorfen | 209 | GOISCDS_ CDS3159 | Acifluorfen |
| 207 | GOICDS_C DS2447 | Imazamethabenz | 209 | GOISCDS_ CDS3159 | Imazamethabenz |
| 207 | GOICDS_C DS2447 | Imazamox | 209 | GOISCDS_ CDS3159 | Imazamox |
| 207 | GOICDS_C DS2447 | Imazapic | 209 | GOISCDS_ CDS3159 | Imazapic |
| 207 | GOICDS_C DS2447 | Imazapyr | 209 | GOISCDS_ CDS3159 | Imazapyr |
| 207 | GOICDS_C DS2447 | Imazaquin | 209 | GOISCDS_ CDS3159 | Imazaquin |
| 207 | GOICDS_C DS2447 | Imazethapyr | 209 | GOISCDS_ CDS3159 | Imazethapyr |
| 207 | GOICDS_C DS2447 | 2,4-D | 209 | GOISCDS_ CDS3159 | 2,4-D |
| 207 | GOICDS_C DS2447 | Chloridazon | 209 | GOISCDS_ CDS3159 | Chloridazon |
| 207 | GOICDS_C DS2447 | Picloram | 209 | GOISCDS_ CDS3159 | Picloram |
| 207 | GOICDS_C DS2447 | Picolinafen | 209 | GOISCDS_ CDS3159 | Picolinafen |
| 207 | GOICDS_C DS2447 | Cyclosulfamuron | 209 | GOISCDS_ CDS3159 | Cyclosulfamuron |
| 207 | GOICDS_C DS2447 | Triflusulfuron | 209 | GOISCDS_ CDS3159 | Triflusulfuron |

(continued)

| Line in Table B | Gene Name/ LocusID | Active ingredientB | Line in Table B | Gene Name/ LocusID | Active ingredientB |
|---|---|---|---|---|---|
| 207 | GOICDS_C DS2447 | Atrazine | 209 | GOISCDS_ CDS3159 | Atrazine |
| 207 | GOICDS_C DS2447 | Pyroxasulfone | 209 | GOISCDS_ CDS3159 | Pyroxasulfone |
| 207 | GOICDS_C DS2447 | Bentazone | 209 | GOISCDS_ CDS3159 | Bentazone |
| 207 | GOICDS_C DS2447 | Cinidon-ethly | 209 | GOISCDS_ CDS3159 | Cinidon-ethly |
| 207 | GOICDS_C DS2447 | Cinmethylin | 209 | GOISCDS_ CDS3159 | Cinmethylin |
| 207 | GOICDS_C DS2447 | Dicamba | 209 | GOISCDS_ CDS3159 | Dicamba |
| 207 | GOICDS_C DS2447 | Diflufenzopyr | 209 | GOISCDS_ CDS3159 | Diflufenzopyr |
| 207 | GOICDS_C DS2447 | Quinclorac | 209 | GOISCDS_ CDS3159 | Quinclorac |
| 207 | GOICDS_C DS2447 | Quinmerac | 209 | GOISCDS_ CDS3159 | Quinmerac |
| 207 | GOICDS_C DS2447 | Mesotrione | 209 | GOISCDS_ CDS3159 | Mesotrione |
| 207 | GOICDS_C DS2447 | Saflufenacil | 209 | GOISCDS_ CDS3159 | Saflufenacil |
| 207 | GOICDS_C DS2447 | Topramezone; | 209 | GOISCDS_ CDS3159 | Topramezone; |
| 210 | GOISCDS_ | acephate | 211 | GOICDS_C DS0644_7 | acephate |
| 210 | GOISCDS_ | chlorpyrifos | 211 | GOICDS_C DS0644_7 | chlorpyrifos |
| 210 | GOISCDS_ | dimethoate | 211 | GOICDS_C DS0644_7 | dimethoate |
| 210 | GOISCDS_ | methamidophos | 211 | GOICDS_C DS0644_7 | methamidophos |
| 210 | GOISCDS_ | terbufos | 211 | GOICDS_C DS0644_7 | terbufos |
| 210 | GOISCDS_ | aldicarb | 211 | GOICDS_C DS0644_7 | aldicarb |
| 210 | GOISCDS_ | carbofuran | 211 | GOICDS_C DS0644_7 | carbofuran |
| 210 | GOISCDS_ | bifenthrin | 211 | GOICDS_C DS0644_7 | bifenthrin |
| 210 | GOISCDS_ | cypermethrin | 211 | GOICDS_C DS0644_7 | cypermethrin |
| 210 | GOISCDS_ | alpha-cypermethrin | 211 | GOICDS_C DS0644_7 | alpha-cypermethrin |

(continued)

| Line in Table B | Gene Name/ LocusID | Active ingredientB | Line in Table B | Gene Name/ LocusID | Active ingredientB |
|---|---|---|---|---|---|
| 210 | GOISCDS_ | deltamethrin | 211 | GOICDS_CDS0644_7 | deltamethrin |
| 210 | GOISCDS_ | lambda-cyhalothrin | 211 | GOICDS_CDS0644_7 | lambda-cyhalothrin |
| 210 | GOISCDS_ | tefluthrin | 211 | GOICDS_CDS0644_7 | tefluthrin |
| 210 | GOISCDS_ | flufenoxuron | 211 | GOICDS_CDS0644_7 | flufenoxuron |
| 210 | GOISCDS_ | teflubenzuron | 211 | GOICDS_CDS0644_7 | teflubenzuron |
| 210 | GOISCDS_ | spirotetramat; | 211 | GOICDS_CDS0644_7 | spirotetramat; |
| 210 | GOISCDS_ | clothianidin | 211 | GOICDS_CDS0644_7 | clothianidin |
| 210 | GOISCDS_ | imidacloprid | 211 | GOICDS_CDS0644_7 | imidacloprid |
| 210 | GOISCDS_ | thiamethoxam | 211 | GOICDS_CDS0644_7 | thiamethoxam |
| 210 | GOISCDS_ | endosulfan | 211 | GOICDS_CDS0644_7 | endosulfan |
| 210 | GOISCDS_ | fipronil | 211 | GOICDS_CDS0644_7 | fipronil |
| 210 | GOISCDS_ | abamectin | 211 | GOICDS_CDS0644_7 | abamectin |
| 210 | GOISCDS_ | spinosad | 211 | GOICDS_CDS0644_7 | spinosad |
| 210 | GOISCDS_ | spinetoram | 211 | GOICDS_CDS0644_7 | spinetoram |
| 210 | GOISCDS_ | hydramethylnon; | 211 | GOICDS_CDS0644_7 | hydramethylnon; |
| 210 | GOISCDS_ | chlorfenapyr; | 211 | GOICDS_CDS0644_7 | chlorfenapyr; |
| 210 | GOISCDS_ | indoxacarb | 211 | GOICDS_CDS0644_7 | indoxacarb |
| 210 | GOISCDS_ | metaflumizone | 211 | GOICDS_CDS0644_7 | metaflumizone |
| 210 | GOISCDS_ | flubendiamide | 211 | GOICDS_CDS0644_7 | flubendiamide |
| 210 | GOISCDS_ | chlorantraniliprole | 211 | GOICDS_CDS0644_7 | chlorantraniliprole |
| 210 | GOISCDS_ | cyazypyr(HGW86) | 211 | GOICDS_CDS0644_7 | cyazypyr(HGW86) |
| 210 | GOISCDS_ | Azoxystrobin | 211 | GOICDS_CDS0644_7 | Azoxystrobin |

(continued)

| Line in Table B | Gene Name/ LocusID | Active ingredientB | Line in Table B | Gene Name/ LocusID | Active ingredientB |
|---|---|---|---|---|---|
| 210 | GOISCDS_ | Dimoxystrobin | 211 | GOICDS_CDS0644_7 | Dimoxystrobin |
| 210 | GOISCDS_ | Kresoxim-methyl | 211 | GOICDS_CDS0644_7 | Kresoxim-methyl |
| 210 | GOISCDS_ | Orysastrobin | 211 | GOICDS_CDS0644_7 | Orysastrobin |
| 210 | GOISCDS_ | Pyraclostrobin | 211 | GOICDS_CDS0644_7 | Pyraclostrobin |
| 210 | GOISCDS_ | Trifloxystrobin | 211 | GOICDS_CDS0644_7 | Trifloxystrobin |
| 210 | GOISCDS_ | Bixafen | 211 | GOICDS_CDS0644_7 | Bixafen |
| 210 | GOISCDS_ | Boscalid | 211 | GOICDS_CDS0644_7 | Boscalid |
| 210 | GOISCDS_ | Isopyrazam | 211 | GOICDS_CDS0644_7 | Isopyrazam |
| 210 | GOISCDS_ | Metalaxyl | 211 | GOICDS_CDS0644_7 | Metalaxyl |
| 210 | GOISCDS_ | Penthiopyrad | 211 | GOICDS_CDS0644_7 | Penthiopyrad |
| 210 | GOISCDS_ | 3-Difluoromethyl-1-methyl-1H-pyrazole-4-carboxylic acid(2',4',5'-trifluorobiphenyl-2-yl)-amide | 211 | GOICDS_CDS0644_7 | *3-Difluoromethyl-1-methyl-1H-pyrazole-4-carboxylic acid(2',4',5'-trifluorobiphenyl-2-yl)-amide |
| 210 | GOISCDS_ | Dimethomorph | 211 | GOICDS_CDS0644_7 | Dimethomorph |
| 210 | GOISCDS_ | Difenoconazole | 211 | GOICDS_CDS0644_7 | Difenoconazole |
| 210 | GOISCDS_ | Epoxiconazole | 211 | GOICDS_CDS0644_7 | Epoxiconazole |
| 210 | GOISCDS_ | Fluquinconazole | 211 | GOICDS_CDS0644_7 | Fluquinconazole |
| 210 | GOISCDS_ | Metconazol | 211 | GOICDS_CDS0644_7 | Metconazol |
| 210 | GOISCDS_ | Propiconazole | 211 | GOICDS_CDS0644_7 | Propiconazole |
| 210 | GOISCDS_ | Prothioconazole | 211 | GOICDS_CDS0644_7 | Prothioconazole |
| 210 | GOISCDS_ | Tebuconazole | 211 | GOICDS_CDS0644_7 | Tebuconazole |
| 210 | GOISCDS_ | Triticonazole | 211 | GOICDS_CDS0644_7 | Triticonazole |

(continued)

| Line in Table B | Gene Name/ LocusID | Active ingredientB | Line in Table B | Gene Name/ LocusID | Active ingredientB |
|---|---|---|---|---|---|
| 210 | GOISCDS_ | Prochloraz | 211 | GOICDS_CDS0644_7 | Prochloraz |
| 210 | GOISCDS_ | Carbendazim | 211 | GOICDS_CDS0644_7 | Carbendazim |
| 210 | GOISCDS_ | Cyprodinil | 211 | GOICDS_CDS0644_7 | Cyprodinil |
| 210 | GOISCDS_ | Pyrimethanil | 211 | GOICDS_CDS0644_7 | Pyrimethanil |
| 210 | GOISCDS_ | Fenpropimorph | 211 | GOICDS_CDS0644_7 | Fenpropimorph |
| 210 | GOISCDS_ | Tridemorph | 211 | GOICDS_CDS0644_7 | Tridemorph |
| 210 | GOISCDS_ | Iprodione | 211 | GOICDS_CDS0644_7 | Iprodione |
| 210 | GOISCDS_ | 5-ethyl-6-octyl-[1,2,4] triazolo [1,5-a]pyrimidine-7-ylamine | 211 | GOICDS_CDS0644_7 | 5-ethyl-6-octyl-[1,2,4] triazolo [1,5-a]pyrimidine-7-ylamine |
| 210 | GOISCDS_ | Mancozeb | 211 | GOICDS_CDS0644_7 | Mancozeb |
| 210 | GOISCDS_ | Maneb | 211 | GOICDS_CDS0644_7 | Maneb |
| 210 | GOISCDS_ | Metiram | 211 | GOICDS_CDS0644_7 | Metiram |
| 210 | GOISCDS_ | Dithianon | 211 | GOICDS_CDS0644_7 | Dithianon |
| 210 | GOISCDS_ | Chlorothalonil | 211 | GOICDS_CDS0644_7 | Chlorothalonil |
| 210 | GOISCDS_ | ThiophanateMethyl | 211 | GOICDS_CDS0644_7 | ThiophanateMethyl |
| 210 | GOISCDS_ | Cymoxanil | 211 | GOICDS_CDS0644_7 | Cymoxanil |
| 210 | GOISCDS_ | Metrafenone | 211 | GOICDS_CDS0644_7 | Metrafenone |
| 210 | GOISCDS_ | Acetochlor | 211 | GOICDS_CDS0644_7 | Acetochlor |
| 210 | GOISCDS_ | Dimethenamid | 211 | GOICDS_CDS0644_7 | Dimethenamid |
| 210 | GOISCDS_ | Metolachlor | 211 | GOICDS_CDS0644_7 | Metolachlor |
| 210 | GOISCDS_ | Metazachlor | 211 | GOICDS_CDS0644_7 | Metazachlor |
| 210 | GOISCDS_ | Glyphosate | 211 | GOICDS_CDS0644_7 | Glyphosate |

(continued)

| Line in Table B | Gene Name/ LocusID | Active ingredientB | Line in Table B | Gene Name/ LocusID | Active ingredientB |
|---|---|---|---|---|---|
| 210 | GOISCDS_ | Glufosinate | 211 | GOICDS_C DS0644_7 | Glufosinate |
| 210 | GOISCDS_ | Sulfosate | 211 | GOICDS_C DS0644_7 | Sulfosate |
| 210 | GOISCDS_ | Fenoxaprop | 211 | GOICDS_C DS0644_7 | Fenoxaprop |
| 210 | GOISCDS_ | Paraquat | 211 | GOICDS_C DS0644_7 | Paraquat |
| 210 | GOISCDS_ | Cycloxydim | 211 | GOICDS_C DS0644_7 | Cycloxydim |
| 210 | GOISCDS_ | Profoxydim | 211 | GOICDS_C DS0644_7 | Profoxydim |
| 210 | GOISCDS_ | Sethoxydim | 211 | GOICDS_C DS0644_7 | Sethoxydim |
| 210 | GOISCDS_ | Tepraloxydim | 211 | GOICDS_C DS0644_7 | Tepraloxydim |
| 210 | GOISCDS_ | Pendimethalin | 211 | GOICDS_C DS0644_7 | Pendimethalin |
| 210 | GOISCDS_ | Acifluorfen | 211 | GOICDS_C DS0644_7 | Acifluorfen |
| 210 | GOISCDS_ | Imazamethabenz | 211 | GOICDS_C DS0644_7 | Imazamethabenz |
| 210 | GOISCDS_ | Imazamox | 211 | GOICDS_C DS0644_7 | Imazamox |
| 210 | GOISCDS_ | Imazapic | 211 | GOICDS_C DS0644_7 | Imazapic |
| 210 | GOISCDS_ | Imazapyr | 211 | GOICDS_C DS0644_7 | Imazapyr |
| 210 | GOISCDS_ | Imazaquin | 211 | GOICDS_C DS0644_7 | Imazaquin |
| 210 | GOISCDS_ | Imazethapyr | 211 | GOICDS_C DS0644_7 | Imazethapyr |
| 210 | GOISCDS_ | 2,4-D | 211 | GOICDS_C DS0644_7 | 2,4-D |
| 210 | GOISCDS_ | Chloridazon | 211 | GOICDS_C DS0644_7 | Chloridazon |
| 210 | GOISCDS_ | Picloram | 211 | GOICDS_C DS0644_7 | Picloram |
| 210 | GOISCDS_ | Picolinafen | 211 | GOICDS_C DS0644_7 | Picolinafen |
| 210 | GOISCDS_ | Cyclosulfamuron | 211 | GOICDS_C DS0644_7 | Cyclosulfamuron |
| 210 | GOISCDS_ | Triflusulfuron | 211 | GOICDS_C DS0644_7 | Triflusulfuron |

(continued)

| Line in Table B | Gene Name/ LocusID | Active ingredientB | Line in Table B | Gene Name/ LocusID | Active ingredientB |
|---|---|---|---|---|---|
| 210 | GOISCDS_ | Atrazine | 211 | GOICDS_C DS0644_7 | Atrazine |
| 210 | GOISCDS_ | Pyroxasulfone | 211 | GOICDS_C DS0644_7 | Pyroxasulfone |
| 210 | GOISCDS_ | Bentazone | 211 | GOICDS_C DS0644_7 | Bentazone |
| 210 | GOISCDS_ | Cinidon-ethly | 211 | GOICDS_C DS0644_7 | Cinidon-ethly |
| 210 | GOISCDS_ | Cinmethylin | 211 | GOICDS_C DS0644_7 | Cinmethylin |
| 210 | GOISCDS_ | Dicamba | 211 | GOICDS_C DS0644_7 | Dicamba |
| 210 | GOISCDS_ | Diflufenzopyr | 211 | GOICDS_C DS0644_7 | Diflufenzopyr |
| 210 | GOISCDS_ | Quinclorac | 211 | GOICDS_C DS0644_7 | Quinclorac |
| 210 | GOISCDS_ | Quinmerac | 211 | GOICDS_C DS0644_7 | Quinmerac |
| 210 | GOISCDS_ | Mesotrione | 211 | GOICDS_C DS0644_7 | Mesotrione |
| 210 | GOISCDS_ | Saflufenacil | 211 | GOICDS_C DS0644_7 | Saflufenacil |
| 210 | GOISCDS_ | Topramezone; | 211 | GOICDS_C DS0644_7 | Topramezone; |
| 213 | | acephate | 213 | | methamidophos |
| 213 | | chlorpyrifos | 213 | | terbufos |
| 213 | | dimethoate | 213 | | aldicarb |
| 213 | | carbofuran | 213 | | alpha-cypermethrin |
| 213 | | bifenthrin | 213 | | deltamethrin |
| 213 | | cypermethrin | 213 | | lambda-cyhalothrin |
| 213 | | tefluthrin | 213 | | spirotetramat; |
| 213 | | flufenoxuron | 213 | | clothianidin |
| 213 | | teflubenzuron | 213 | | imidacloprid |
| 213 | | thiamethoxam | 213 | | abamectin |
| 213 | | endosulfan | 213 | | spinosad |
| 213 | | fipronil | 213 | | spinetoram |
| 213 | | hydramethylnon; | 213 | | metaflumizone |
| 213 | | chlorfenapyr; | 213 | | flubendiamide |
| 213 | | indoxacarb | 213 | | chlorantraniliprole |
| 213 | | cyazypyr(HGW86) | 213 | | Kresoxim-methyl |
| 213 | | Azoxystrobin | 213 | | Orysastrobin |

(continued)

| Line in Table B | Gene Name/ LocusID | Active ingredientB | Line in Table B | Gene Name/ LocusID | Active ingredientB |
|---|---|---|---|---|---|
| 213 | | Dimoxystrobin | 213 | | Pyraclostrobin |
| 213 | | Trifloxystrobin | 213 | | Isopyrazam |
| 213 | | Bixafen | 213 | | Metalaxyl |
| 213 | | Boscalid | 213 | | Penthiopyrad |
| 213 | | 3-Difluoromethyl-1-methyl-1 H-pyrazole-4-carboxylic acid(2',4',5'-trifluorobiphenyl-2-yl)-amide | 213 | | Epoxiconazole |
| 213 | | Dimethomorph | 213 | | Fluquinconazole |
| 213 | | Difenoconazole | 213 | | Metconazol |
| 213 | | Propiconazole | 213 | | Triticonazole |
| 213 | | Prothioconazole | 213 | | Prochloraz |
| 213 | | Tebuconazole | 213 | | Carbendazim |
| 213 | | Cyprodinil | 213 | | Tridemorph |
| 213 | | Pyrimethanil | 213 | | Iprodione |
| 213 | | Fenpropimorph | 213 | | 5-ethyl-6-octyl-[1,2,4] triazolo [1,5-a]pyrimidine-7-ylamine |
| 213 | | Mancozeb | 213 | | Dithianon |
| 213 | | Maneb | 213 | | Chlorothalonil |
| 213 | | Metiram | 213 | | ThiophanateMethyl |
| 213 | | Cymoxanil | 213 | | Dimethenamid |
| 213 | | Metrafenone | 213 | | Metolachlor |
| 213 | | Acetochlor | 213 | | Metazachlor |
| 213 | | Glyphosate | 213 | | Paraquat |
| 213 | | Glufosinate | 213 | | Cycloxydim |
| 213 | | Sulfosate | 213 | | Profoxydim |
| 213 | | Fenoxaprop | 213 | | Sethoxydim |
| 213 | | Tepraloxydim | 213 | | Imazamethabenz |
| 213 | | Pendimethalin | 213 | | Imazamox |
| 213 | | Acifluorfen | 213 | | Imazapic |
| 213 | | Imazapyr | 213 | | 2,4-D |
| 213 | | Imazaquin | 213 | | Chloridazon |
| 213 | | Imazethapyr | 213 | | Picloram |
| 213 | | Picolinafen | 213 | | Atrazine |
| 213 | | Cyclosulfamuron | 213 | | Pyroxasulfone |

(continued)

| Line in Table B | Gene Name/ LocusID | Active ingredientB | Line in Table B | Gene Name/ LocusID | Active ingredientB |
|---|---|---|---|---|---|
| 213 | | Triflusulfuron | 213 | | Bentazone |
| 213 | | Cinidon-ethly | 213 | | Diflufenzopyr |
| 213 | | Cinmethylin | 213 | | Quinclorac |
| 213 | | Dicamba | 213 | | Quinmerac |
| 213 | | Mesotrione | 213 | | Topramezone; |
| 213 | | Saflufenacil | | | |

## Claims

**1.** A method for increasing plant health and/or controlling pests in plants with at least one transgenic modification related to yield increase as compared to a corresponding wild-type plant
comprising treating the location where the plant with at least one transgenic modification is growing or is expected to grow and/or the transgenic plant with at least one transgenic modification or propagation material of the plant with at least one transgenic modification with an effective amount of a chemical composition comprising at least one active ingredient,
wherein at least one transgenic modification related to yield increase as compared to a corresponding wild-type plant is a transgenic modification conferring one or more increased or generated activities selected from the group consisting of the polypeptides encoded by:

a) a nucleic acid molecule encoding the polypeptide as depicted in any of the SEQ ID NO: 5, 17, 22, 28, 44, 45, 48, 67, 76, 81, 82, 85, 124, 155 or 192;
b) a nucleic acid molecule as depicted in table B, column 1 or 3 and encoding a polypeptide as depicted in a);
c) a nucleic acid molecule, which, as a result of the degeneracy of the genetic code, can be derived from a polypeptide sequence as depicted in table B, column 1 or 3 and encoding a polypeptide as depicted in a) and/or in SEQ ID NO: 5, 17, 22, 28, 44, 45, 48, 67, 76, 81, 82, 85, 124, 155 or 192;
d) a nucleic acid molecule having at least 30 % identity with the nucleic acid molecule sequence of a polynucleotide comprising the nucleic acid molecule according to a) to c);
e) a nucleic acid molecule encoding a polypeptide having at least 30 % identity with the amino acid sequence of the polypeptide encoded by the nucleic acid molecule of (a) to (c) and having the activity represented by a nucleic acid molecule of (a) to (c) ;
f) nucleic acid molecule which hybridizes with a nucleic acid molecule of (a) to (c) under stringent hybridization conditions;
g) a nucleic acid molecule encoding a polypeptide which can be isolated with the aid of monoclonal or polyclonal antibodies made against a polypeptide encoded by one of the nucleic acid molecules of (a) to (e) and having the activity represented by the nucleic acid molecule comprising a polynucleotide of (a) to (c);
h) a nucleic acid molecule encoding a polypeptide comprising the consensus sequence or one or more polypeptide motifs resulting from 2 ore more of the polypeptides as depicted in table B, column 1 or 3 and/or in SEQ ID NO: 1 to 270;
h) a nucleic acid molecule encoding a polypeptide having the activity represented by a protein as depicted in table B, column 1 or 3 or 5 and encoding a polypeptide as depicted in a) and/or in SEQ ID NO: 5, 17, 22, 28, 44, 45, 48, 67, 76, 81, 82, 85, 124, 155 or 192;
i) nucleic acid molecule which comprises a polynucleotide, which is obtained by amplifying a cDNA library or a genomic library using the primers for the polynucleotides as depicted in table B, column 1 or 3 and encoding a polypeptide as depicted in a) and/or in SEQ ID NO: 5, 17, 22, 28, 44, 45, 48, 67, 76, 81, 82, 85, 124, 155 or 192 which do not start at their 5'-end with the nucleotides ATA;
and
j) a nucleic acid molecule which is obtainable by screening a suitable nucleic acid library under stringent hybridization conditions with a probe comprising a complementary sequence of a nucleic acid molecule of (a) or (b) or with a fragment thereof, having at least 15 nt, preferably 20 nt, 30 nt, 50 nt, 100 nt, 200 nt or 500 nt of a nucleic acid molecule complementary to a nucleic acid molecule sequence **characterized in** (a) to (e) and

encoding a polypeptide having the activity represented by a protein comprising a polypeptide as depicted in table B, column 1 or 3 and encoding a polypeptide as depicted in a) and/or in SEQ ID NO: 5, 17, 22, 28, 44, 45, 48, 67, 76, 81, 82, 85, 124, 155 or 192.

**2.** A method for increasing plant productivity in plants with at least one transgenic modification related to yield increase as compared to a corresponding wild-type plant comprising treating the location where the plant with at least one transgenic modification is growing or is expected to grow and/or the transgenic plant with at least one transgenic modification or propagation material of the plant with at least one transgenic modification with an effective amount of a chemical composition comprising at least one active ingredient,
wherein at least one transgenic modification related to yield increase as compared to a corresponding wild-type plant is a transgenic modification conferring one or more increased or generated activities selected from the group consisting of the polypeptides encoded by:

a) a nucleic acid molecule encoding the polypeptide as depicted in any of the SEQ ID NO: 5, 17, 22, 28, 44, 45, 48, 67, 76, 81, 82, 85, 124, 155 or 192;
b) a nucleic acid molecule as depicted in table B, column 1 or 3 and encoding a polypeptide as depicted in a);
c) a nucleic acid molecule, which, as a result of the degeneracy of the genetic code, can be derived from a polypeptide sequence as depicted in table B, column 1 or 3 and encoding a polypeptide as depicted in a) and/or in SEQ ID NO: 5, 17, 22, 28, 44, 45, 48, 67, 76, 81, 82, 85, 124, 155 or 192;
d) a nucleic acid molecule having at least 30 % identity with the nucleic acid molecule sequence of a polynucleotide comprising the nucleic acid molecule according to a) to c);
e) a nucleic acid molecule encoding a polypeptide having at least 30 % identity with the amino acid sequence of the polypeptide encoded by the nucleic acid molecule of (a) to (c) and having the activity represented by a nucleic acid molecule of (a) to (c) ;
f) nucleic acid molecule which hybridizes with a nucleic acid molecule of (a) to (c) under stringent hybridization conditions;
g) a nucleic acid molecule encoding a polypeptide which can be isolated with the aid of monoclonal or polyclonal antibodies made against a polypeptide encoded by one of the nucleic acid molecules of (a) to (e) and having the activity represented by the nucleic acid molecule comprising a polynucleotide of (a) to (c);
h) a nucleic acid molecule encoding a polypeptide comprising the consensus sequence or one or more polypeptide motifs resulting from 2 ore more of the polypeptides as depicted in table B, column 1 or 3 and/or in SEQ ID NO: 1 to 270;
h) a nucleic acid molecule encoding a polypeptide having the activity represented by a protein as depicted in table B, column 1 or 3 or 5 and encoding a polypeptide as depicted in a) and/or in SEQ ID NO: 5, 17, 22, 28, 44, 45, 48, 67, 76, 81, 82, 85, 124, 155 or 192;
i) nucleic acid molecule which comprises a polynucleotide, which is obtained by amplifying a cDNA library or a genomic library using the primers for the polynucleotides as depicted in table B, column 1 or 3 and encoding a polypeptide as depicted in a) and/or in SEQ ID NO: 5, 17, 22, 28, 44, 45, 48, 67, 76, 81, 82, 85, 124, 155 or 192 which do not start at their 5'-end with the nucleotides ATA;
and
j) a nucleic acid molecule which is obtainable by screening a suitable nucleic acid library under stringent hybridization conditions with a probe comprising a complementary sequence of a nucleic acid molecule of (a) or (b) or with a fragment thereof, having at least 15 nt, preferably 20 nt, 30 nt, 50 nt, 100 nt, 200 nt or 500 nt of a nucleic acid molecule complementary to a nucleic acid molecule sequence **characterized in** (a) to (e) and encoding a polypeptide having the activity represented by a protein comprising a polypeptide as depicted in table B, column 1 or 3 and encoding a polypeptide as depicted in a) and/or in SEQ ID NO: 5, 17, 22, 28, 44, 45, 48, 67, 76, 81, 82, 85, 124, 155 or 192.

**3.** The method of claim 1 or 2 whereby at least one transgenic modification of said plant does not confer resistance to the active ingredient which is employed.

**4.** The method of claim 1 whereby the are plants with one or more increased or generated activities selected from the group consisting of the stress related proteins which confers an increase in tolerance and/or resistance to environmental stress as compared to a corresponding non-transformed wild-type plant..

**5.** The method of any one of the preceding claims wherein the active ingredient is selected from the group consisting of insecticides, preferably an insecticide selected from the group consisting of acephate, chlorpyrifos, diazinon, dichlorvos, dimethoate, fenitrothion, methamidophos, methidathion, methyl-parathion, monocrotophos, phorate, profenofos, terbufos, aldicarb, carbaryl, carbofuran, carbosulfan, methomyl, thiodicarb, bifenthrin, cyfluthrin, cypermethrin, alpha-cypermethrin, zeta-cypermethrin, deltamethrin, esfenvalerate, lambda-cyhalothrin, permethrin, tefluthrin, diflubenzuron, flufenoxuron, lufenuron, teflubenzuron, spirotetramat; clothianidin, dinotefuran, imidacloprid, thiamethoxam, acetamiprid, thiacloprid; endosulfan, fipronil, abamectin, emamectin, spinosad, spinetoram, hydramethylnon; chlorfenapyr; fenbutatin oxide, indoxacarb, metaflumizone, flonicamid, flubendiamide, chlorantraniliprole, cyazypyr (HGW86), cyflumetofen ;
fungicides, preferably a fungicide selected from the group consisting of azoxystrobin, dimoxystrobin, kresoxim-methyl, orysastrobin, pyraclostrobin, trifloxystrobin, bixafen, boscalid, isopyrazam, metalaxyl, penthiopyrad, 3-difluoromethyl-1-methyl-1H-pyrazole-4-carboxylic acid (2',4',5'-trifluorobiphenyl-2-yl)-amide, N-(2-bicyclopropyl-2-yl-phenyl)-3-difluoromethyl-1-methyl-1 H-pyrazole-4-carboxylic acid amide, dimethomorph, fluopicolide, difenoconazole, epoxiconazole, fluquinconazole, flusilazole, flutriafol, metconazol, myclobutanil, propiconazole, prothioconazole, tebuconazole, tetraconazole, triticonazole, prochloraz, carbendazim, fluazinam, cyprodinil, pyrimethanil, fludioxonil, dodemorph, fenpropimorph, tridemorph, fenpropidin, iprodione, vinclozolin, famoxadone, probenazole, captan, folpet, 5-ethyl-6-octyl-[1,2,4]triazolo[1,5-a]pyrimidine-7-ylamine, mancozeb, maneb, metiram, thiram, dithianon, fosetyl, fosetyl-aluminium, chlorothalonil, thiophanate methyl, cymoxanil, metrafenone, spiroxamine, bixafen, N-(3',4',5'-trifluorobiphenyl-2-yl)- 3-difluoromethyl-1-methyl-1H-pyrazole-4-carboxamide, N-[2-(4'-trifluoromethylthio)-biphenyl]-3-difluoromethyl-1-methyl-1H-pyrazole-4-carboxamide, N-[2-(1,3-dimethylbutyl)-phenyl]-1,3-dimethyl-5-fluoro-1H-pyrazole-4-carboxamide, N-(2-bicyclopropyl-2-yl-phenyl)-3-difluoromethyl-1-methyl-1H-pyrazole-4- carboxamide, N-(cis- 2- bicyclopropyl- 2- yl- phenyl)- 3- difluoromethyl- 1- methyl- 1H- pyrazole- 4- carboxamide, N-(trans-2-bicyclopropyl-2-yl-phenyl)-3-difluoromethyl-1-methyl-1H-pyrazole-4-carboxamide, N-[1,2,3,4-tetrahydro- 9-(1- methylethyl)- 1,4- methanonaphthalen- 5- yl]- 3-(difluoromethyl)- 1- methyl- 1H- pyrazole- 4- carboxamide and/or
herbicides, preferably a herbicide selected from the group consisting of acetochlor, dimethenamid, metolachlor, metazachlor, glyphosate, glufosinate, sulfosate, clodinafop, fenoxaprop, fluazifop, haloxyfop, paraquat, phenmedipham, clethodim, cycloxydim, profoxydim, sethoxydim, tepraloxydim, pendimethalin, trifluralin, acifluorfen, bromoxynil, imazamethabenz, imazamox, imazapic, imazapyr, imazaquin, imazethapyr, 2,4-D, chloridazon, clopyralid, fluroxypyr, picloram, picolinafen, bensulfuron, chlorimuron ethyl, cyclosulfamuron, iodosulfuron, mesosulfuron, metsulfuron-methyl, nicosulfuron, rimsulfuron, triflusulfuron, atrazine, hexazinone, diuron, florasulam, pyroxasulfone, bentazone, cinidon-ethly, cinmethylin, dicamba, diflufenzopyr, quinclorac, quinmerac, mesotrione, saflufenacil, topramezone.

6. The method of any one of the preceding claims, wherein the active ingredient (B) is a strobilurin selected from pyraclostrobin, kresoxim-methyl, dimoxystrobin, 2-(ortho-((2,5-dimethylphenyl-oxymethylene)phenyl)-3-methoxy-acrylic acid methyl ester, picoxystrobin, trifloxystrobin, enestroburin, orysastrobin, metominostrobin, azoxystrobin and fluoxastrobin and/or the active ingredient (B) is selected from thiamethoxam, fipronil and imidacloprid and clothianidin.

7. The method of any one of the preceding claims, wherein the chemical composition comprises at least one active ingredient (B) and a component (A) which is a glucan or a glucan derivative.

8. The method of any one of the preceding claims wherein the propagation material is seed.

9. The method of any one of the preceding claims wherein the transgenic seed or transgenic plant contains one or more genes which lead to an increase in plant health, stress resistance pest control and/or increase in yield.

10. The method of any one of the preceding claims, wherein the treatment(s) are carried out as foliar application and/or in furrow or wherein the active compound is applied to seed.

11. The method of any one of the preceding claims, wherein the transgenic plant is a monocotyledonous plant, a dicotyledonous plant and/or gymnosperm plant , preferably the plant with the transgenic modification is selected from the group comprising maize, wheat, rye, oat, triticale, rice, barley, soybean, peanut, cotton, oilseed rape, canola/OSR, manihot, pepper, sunflower, flax, borage, sugar cane, safflower, linseed, primrose, rapeseed, turnip rape, tagetes, solanaceous plants, potato, tobacco, eggplant, tomato, lettuce, Vicia species, pea, alfalfa, coffee, cacao, tea, Salix species, oil palm, coconut, perennial grass, snip grass and forage crops, especially the plant with the transgenic modification is selected from the group comprising maize, soybean, cotton, canola/OSR and snip grass.

12. A method for the detection of increased plant health and/or increased plant productivity comprising at least one of

the following steps:

i) growing transgenic seedlings and/or plants in a greenhouse under optimal, well water conditions, preferably administrating supplemental nutrients and light,
ii) collecting phenotyic data in an imaging procedure,
iii) harvesting the plants for determining fresh weight, dry weight and/or plant volume and standardize and correlate the data from the imaging procedure with the physiological features,
iv) measuring with the imaging system, preferably scanalyzer, the plant volume, internode length, greenness, yellowness, leaf angle, area of the leaves, number of leaves and/or stem length of the plants,
v) comparing the data of chemically treated plants with non treated plants.

**13.** Use of a composition comprising a pesticide for increasing the health, the productivity, the yield and/or improving the vigor of a transgenic plant of the invention, preferably an agricultural transgenic plant, whereby at least one transgenic modification of said plant does not confer resistance to the active ingredient which is employed.

**14.** Use of a composition comprising a pesticide for stimulating the natural defensive reactions of a plant against a pathogen and/or a pest.

**15.** A method for obtaining synergistic effects conferring plant productivity enhancement and/or plant health enhancement and/or pest control comprising treating the location where the plant with at least one transgenic modification is growing or is expected to grow and/or the transgenic plant with at least one transgenic modification or propagation material of the plant with at least one transgenic modification with an effective amount of a chemical composition comprising at least one active ingredient.

Fig. 1

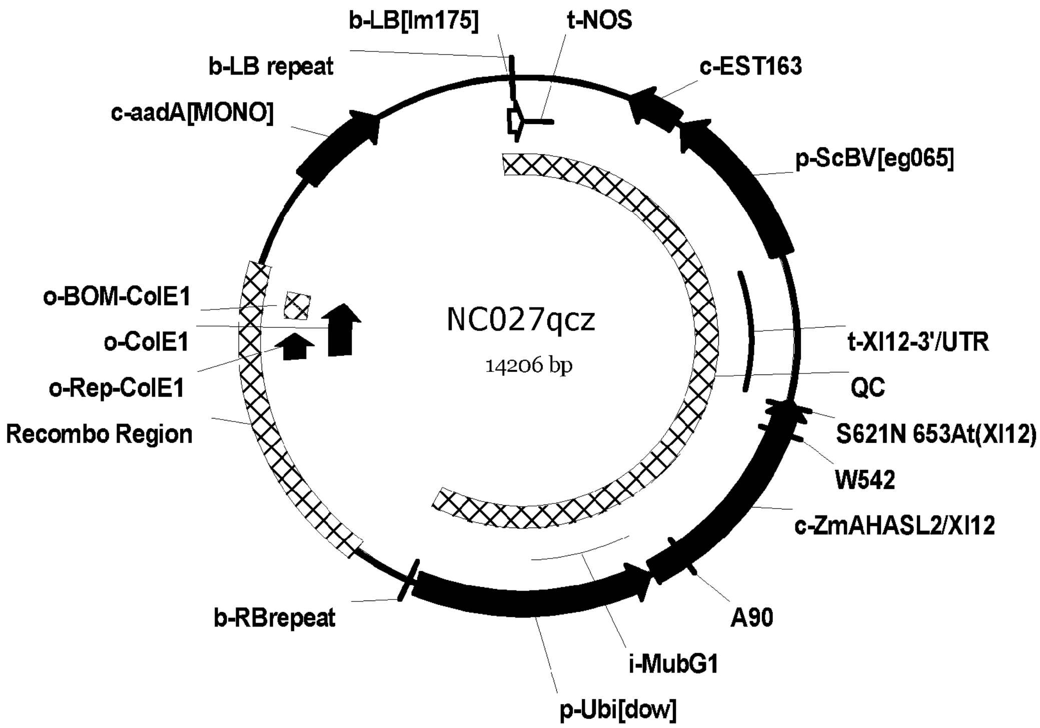
b-LB[lm175]
t-NOS
b-LB repeat
c-EST163
c-aadA[MONO]
p-ScBV[eg065]
o-BOM-ColE1
NC027qcz
o-ColE1
14206 bp
t-Xl12-3'/UTR
o-Rep-ColE1
QC
Recombo Region
S621N 653At(Xl12)
W542
c-ZmAHASL2/Xl12
b-RBrepeat
A90
i-MubG1
p-Ubi[dow]

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6593273 B
- WO 1999035913 A
- US 20030060371 A
- US 20040023081 A
- US 20030114308 A
- WO 2004018687 A
- WO 2004092398 A
- WO 2006032708 A
- WO 2004092349 A
- WO 2006032707 A
- US 6979665 B
- US 6387847 B
- US 6303587 B
- US 5750472 A
- FR 9208387
- DE 2903612
- EP 545099 A
- US 3249499 A
- US 3937840 A
- JP 1104514 A
- GB 1500581 A
- GB 2058059 A
- US 3399214 A
- JP 10130268 B
- EP 120321 A
- EP 860438 A
- WO 9942447 A
- DE 2324020
- US 4664696 A
- GB 2098607 A
- EP 196038 A
- EP 15756 A
- EP 267778 A
- GB 857383 A
- BE 835579
- WO 9616048 A
- EP 40345 A
- EP 234242 A
- FR 2641277
- US 3991071 A
- JP 54119462 A
- US 3631176 A
- US 3657443 A
- DE 1209799
- US 3017415 A
- EP 639574 A
- JP 518249 A
- JP 532202 A
- EP 49854 A
- EP 310550 A
- GB 1218623 A
- EP 224339 A
- DD 151404 A
- DE 1901421
- DE 1198125
- DE 2752096
- DE 1164152
- GB 1312536 A
- US 3903090 A
- DE OS2207576
- US 2720480 A
- US 2553770 A
- US 4052395 A
- EP 262393 A
- WO 9748684 A
- GB 13943373 A
- US 5240940 A
- GB 1419121 A
- US 1972961 A
- US 3379610 A
- US 2504404 A
- US 3248400 A
- US 2791605 A
- BE 611960
- DE 642532
- US 2457674 A
- EP 78663 A
- JP 9323984 A
- EP 472996 A
- DE 1643040
- US 2867562 A
- GB 1114155 A
- US 3499086 A
- DE 1493736 A
- FR 2254276
- DE 1545790
- GB 1467561 A
- DE 1930540
- US 3290353 A
- DE 1193498
- EP 199433 A
- DE 1643347
- DE 2732257
- DE 682048
- US 2526660 A
- EP 281842 A
- WO 9619442 A
- US 3957847 A

- US 5945567 A
- WO 03066609 A
- WO 9924413 A
- WO 0449804 A
- EP 1035122 A
- WO 0314103 A
- EP 1028125 A
- WO 199828279 A
- EP 454621 A1
- WO 199828277 A
- US 4822779 A
- JP 2002193709 B
- WO 200100614 A
- WO 199845274 A
- US 6335357 B
- US 6221890 B
- JP 21010907 B
- WO 2003007717 A
- WO 2003007718 A
- WO 2004080180 A
- WO 200170671 A
- WO 200248137 A
- WO 200324222 A
- WO 200315518 A
- WO 200467528 A
- WO 200433468 A
- WO 2005118552 A
- EP 178826 A
- EP 253213 A
- WO 9315046 A
- WO 9518789 A
- WO 9521153 A
- WO 9521154 A
- WO 9524396 A
- WO 9601256 A
- WO 9715552 A
- WO 9727189 A
- EP 278595 A
- EP 398692 A
- EP 628540 A
- EP 280185 A
- EP 460575 A
- EP 463488 A
- EP 382375 A
- WO 9705103 A
- WO 9706133 A
- WO 9306730 A
- WO 9828279 A
- WO 9828277 A
- EP 462456 A1
- WO 0100614 A
- WO 9845274 A
- WO 03007717 A
- WO 03007718 A
- US 6300348 B
- WO 0170671 A
- WO 03015519 A
- WO 05118552 A
- WO 04067528 A
- WO 04080180 A
- EP 1097932 A
- JP 2006131529 B
- WO 2007060839 A
- WO 02089579 A
- WO 02090320 A
- WO 02090321 A
- WO 04006677 A
- WO 05068423 A
- WO 05068432 A
- WO 05063694 A
- WO 2007011625 A
- WO 2007011771 A
- WO 2007011681 A
- US 2006027384 A
- WO 200216625 A
- WO 2007020198 A
- WO 2006069610 A
- US 20070028333 A
- US 20070157343 A
- US 20070261131 A
- US 7176026 B
- US 20070192908 A
- US 20040194163 A
- US 20070157344 A
- US 6677504 A
- US 5041018 A
- US 7166767 A
- US 767611 A
- WO 2006133983 A
- WO 2006020717 A
- US 20070157334 A
- WO 2003040171 A
- US 20040107463 A
- US 20040128721 A
- WO 2006044912 A
- US 7164057 A
- US 0571017 A
- US 20070079400 A
- US 7091402 B
- WO 200246442 A
- US 20070157345 A
- US 7125719 B
- US 20040148658 A
- WO 2006050038 A
- WO 2006134162 A
- US 20030172408 A
- WO 2003020914 A
- WO 2004074440 A
- US 2004011888 A
- WO 2006013010 A
- US 20060122375 A
- WO 9209685 A
- WO 2005024029 A
- WO 2007064724 A
- WO 2005094562 A
- WO 2004038027 A

- WO 2005083094 A
- WO 2006008271 A
- WO 0196580 A
- WO 2006045829 A
- WO 2007113237 A
- WO 2004058980 A
- WO 2006079655 A
- WO 2005117568 A
- WO 2006018432 A
- WO 2006005771 A
- WO 2004090142 A
- WO 2006005751 A
- WO 2004087755 A
- WO 2006131547 A
- WO 2007138070 A
- WO 2004090141 A
- WO 2005049646 A
- WO 2006067232 A
- WO 2005059147 A
- WO 2004087927 A
- WO 20070647249 A
- WO 2004106528 A
- WO 2006056590 A
- WO 2006058897 A
- WO 2007099096 A
- WO 2007051866 A
- WO 2006067236 A
- WO 2007003409 A


**Non-patent literature cited in the description**


- **W. R. PEARSON ; D. J. LIPMAN.** Improved Tools for Biological Sequence Comparison. *PNAS,* 1988, vol. 85, 2444-2448
- **W. R. PEARSON.** Rapid and Sensitive Sequence Comparison with FASTP and FASTA. *Methods in Enzymology,* 1990, vol. 183, 63-98
- **W. R. PEARSON.** Rapid and Sensitive Sequence Comparison with FASTP and FASTAMethods. *Enzymology,* 1990, vol. 183, 63-98
- *J. Mol. Evolution,* 1987, vol. 25, 351-360
- **HIGGINS et al.** *CABIOS,* 1989, vol. 5, 151-153
- **NEEDLEMAN ; WUNSCH.** *J. Mol. Biol.,* 1970, vol. 48, 443-453
- **SMITH ; WATERMAN.** *Adv. Appl. Math.,* 1981, vol. 2, 482-489
- **ALTSCHUL et al.** *Nucleic Acids Res.,* 1997, vol. 25, 3389
- **ALBAN S. et al.** Synthesis of laminarin sulfates with anticoagulant activity. *Arzneim. Forsch.,* 1992, vol. 42, 1005-1008
- **BLACK et al.** *Appl. Chem.,* 1951, vol. 1, 505-517
- The Pesticide Manual. British Crop Protection Council, 2003
- *Proc. Br. Crop Prot. Conf. - Pests Dis.,* 1998, vol. 2, 327
- *AGROW,* 1995, 22
- *Proc. 1990 Br. Crop. Prot. Conf. - Pests Dis.,* vol. 1, 459
- *Noyaku Kagaku,* 1983, vol. 8, 575
- *Fruits,* 1973, vol. 28, 545
- *Proc. 1988 Br. Crop Prot. Conf. - Pests Dis.,* 1988, vol. 1, 33
- *Proc. Br. Crop Prot. Conf.-Pests Dis,* 1984, vol. 1, 413
- *Proc. Br. Crop Prot. Conf.-Pests Dis.,* 1992, vol. 5-3, 411
- *Proc. 1988 Br. Crop Prot. Conf. - Pests Dis.,* 1988, vol. 2, 519
- Pesticide Manual. 2000, 712
- The Pesticide Manual. The British Crop Protection Council, 1995, 474
- The Pesticide Manual. The British Crop Protection Council, 1995, 482
- Proc. 1988 Br. Crop Prot. Conf.-Pests Dis. 1988, vol. 1, 65
- *Phytopathology,* 1962, vol. 52, 754
- Bull. Soc. Chim. Fr. 1900, vol. 15, 891
- *J. Am. Chem. Soc.,* 1947, vol. 69, 1234
- *Proc. Insectic. Fungic. Conf. 8. Bd.,* 1975, vol. 2, 715
- *Jpn. Pesticide Inf.,* 1970, 11
- *C. R. Seances Acad. Agric. Fr.,* 1945, vol. 31, 24
- Farm Chemicals Handbook. Meister Publishing Company, 2001, vol. 88
- *Pesticide Science,* 1988, vol. 54, 237-243
- Dechema-Monographien. VCH Verlagsgemeinschaft, 1993, vol. 129, 27-38
- *Natural Product Reports,* 1993, 565-574
- *Biochem. Soc. Trans.,* 1993, vol. 22, 63S